# EUROPEAN PATENT APPLICATION

(11) **EP 4 495 240 A1**
(43) Date of publication of application: **22.01.2025**
(21) Application number: 23770815.1
(22) Date of filing: 15.03.2023
(51) Int. Cl.: C12N 15/113, A61K 31/712, A61K 31/7125, A61K 31/713, A61K 47/26, A61K 47/54, A61K 48/00, A61P 7/02, A61P 43/00, C07H 21/04

(54) **SIRNA FOR SUPPRESSING EXPRESSION OF TRANSFERRIN RECEPTOR-2**

(30) Priority: 16.03.2022 JP 2022041864
(71) Applicant: Daiichi Sankyo Company, Limited, Tokyo 103-8426 (JP)
(72) Inventor: KOIZUMI, Makoto, Tokyo 103-8426 (JP); SHOJI, Takao, Tokyo 103-8426 (JP); FUKUNAGA, Taichi, Tokyo 103-8426 (JP)
(74) Representative: Marks & Clerk LLP
(86) International application number: PCT/JP2023/009973
(87) International publication number: WO 2023/176862

(57) **Abstract**

Provided is novel siRNA having an RNA interfering action and/or a gene expression suppressing action on mRNA encoding TfR2, or the like. An oligonucleotide or a pharmaceutically acceptable salt thereof, or the like, has a knockdown action on mRNA of transferrin receptor 2, and consists of an antisense strand region containing a target-matching sequence substantially complementary to a target sequence in mRNA encoding TfR2, and a sense strand region containing a nucleotide sequence substantially complementary to the target-matching sequence of the antisense strand region.

## Description

### Technical Field

The present invention relates to siRNA having an RNA interfering action and/or a gene expression suppressing action on mRNA encoding transferrin receptor 2 (TfR2), use of the siRNA, a method for suppressing TfR2 gene expression using the siRNA, a medical drug comprising the siRNA, and the like.

### Background Art

siRNA against mRNA encoding a TfR2 protein can suppress mRNA expression of TfR2, and suppress production of hepcidin that plays a key role in iron metabolism. As a result, iron metabolism in a living organism may be activated to promote bioavailability of iron in erythroid precursor cells, thus enabling treatment of anemia. The use of siRNA for suppressing mRNA expression of TfR2, with the aim of suppressing hepcidin production, is being studied.

Hepcidin is a peptide hormone which consists of 25 amino acids, and is produced mainly from the liver and secreted into the blood. Hepcidin plays a key role in iron metabolism in living organisms. It is known that hepcidin suppresses absorption of iron from the intestinal tract and release of iron from macrophages by binding to the iron transporter Ferroportin (SLC40A1) and promoting proteolysis of Ferroportin, and thus negatively controls iron metabolism. It has been reported that an increase in hepcidin concentration in the blood limits bioavailability of iron in erythroid precursor cells by suppressing iron metabolism, and thus contributes to various anemic diseases (Non-Patent Literature 1).

Examples of anemic diseases in which there is an increase in hepcidin in blood include anemia associated with chronic kidney disease, anemia associated with cancer, anemia induced by chemotherapy, iron refractory iron-deficiency anemia (IRIDA), anemia associated with chronic inflammation (anemia of chronic disease (ACD)) such as articular rheumatism or Castleman disease, Diamond-Blackfan anemia, aplastic anemia, β thalassemia, sickle cell disease, paroxysmal nocturnal hemoglobinuria, autoimmune hemolytic anemia, anemia associated with myelodysplastic syndrome, chronic myelomonocytic leukemia, and anemia associated with myelofibrosis. In myelofibrosis patients, the hepcidin concentration in the blood can increase in correlation to iron loading associated with erythrocyte transfusion and a chronic increase of inflammatory cytokines (Non-Patent Literature 2). However, in the case of anemia that is caused mainly by an insufficient ability to produce erythrocytes itself due to hematopoietic dysfunction in the bone marrow, such as myelofibrosis, the contribution of defective iron utilization associated with an increase in hepcidin in the blood to a pathological condition may be low, and a therapeutic effect of the suppression of hepcidin production on anemia has not heretofore been reported. At present, drugs such as ESA (erythropoiesis stimulating agent) may be used for treating anemia associated with bone-marrow dysfunction such as myelofibrosis, but have a limited effect.

Patent Literature 1 discloses that administration of AD-52590 as TfR2 siRNA to a monkey resulted in a decrease in mRNA expression of TfR2 in the liver, a decrease in mRNA expression of hepcidin, a decrease in hepcidin concentration in the blood, and an increase in iron concentration in the blood. Patent Literature 1 also discloses that administration of AD-47882 to a rat having a pathological condition of anemia associated with inflammation resulted in a decrease in TfR2 mRNA expression in the liver, a decrease in hepcidin concentration in the blood, an increase in iron concentration in the blood, and an increase in hemoglobin levels. However, Patent Literature 1 shows the results of administering siRNA encapsulated in lipid nanoparticles (LNPs). The route of administration is limited to an intravenous route. The therapeutic effect of TfR2 siRNA disclosed in Patent Literature 1 is limited to a therapeutic effect on anemia associated with inflammation. Therapeutic effects on other anemias, such as anemia associated with loss of bone-marrow function, are not disclosed.

Methods for inhibiting expression a target gene in cells, tissues or individuals include methods in which double-stranded RNA is introduced into the cells, tissues or individuals. The introduction of double-stranded RNA degrades mRNA homologous to the sequence of the double-stranded RNA, and inhibits expression of a target gene. This effect is called "RNA interference" or "RNAi". Double-stranded RNA having an overhang with two nucleotides at the 3'-terminus, and a sense strand and an antisense strand each having 21 nucleotides (small interfering RNA: siRNA) has been reported to have an RNA interfering action in cultured cells of a vertebrate (see, for example, Non-Patent Literature 3).

siRNA is useful for identification of a gene function, screening of a cell strain suitable for production of a useful substance, control of a gene involved in disease, and the like, but is easily degraded by a ribonuclease, and therefore has the disadvantages that it is difficult to maintain activity in the body and the cost for synthesis is high. For this reason, various chemical modifications have been attempted for the development of an oligonucleotide which has high stability against a ribonuclease, can be produced at low cost, and retains RNAi activity.

A phosphorothioate (PS) bond obtained by replacing a non-bridging oxygen atom of a phosphate group of a phosphodiester bond in an oligonucleotide by a sulfur atom is known to have resistance to a nuclease. However, it has been reported that an increased number of PS bonds in an oligonucleotide is not preferred because double-stranded RNA is thermodynamically destabilized and nonspecifically binds to protein (see, for example, Non-Patent Literature 4).

In addition, siRNA having, as nucleosides, sugar-modified nucleosides such as DNA, 2'-O-methylnucleosides, 2'-deoxy-2'fluoronucleosides or 2'-O,4'-C-bridged nucleosides is disclosed in, for example, Patent Literatures 2, 3 and 4.

Attempts have also been made to obtain stable siRNA by replacing natural RNA with modified RNA. For example, many derivatives have been reported in which the 2'-OH group of RNA is alkylated to obtain a 2'-O-alkylnucleoside which does not form a substrate for RNase. 2'-O-methylnucleotide, which is a naturally occurring modified nucleotide that is also found in tRNA, has been studied since the early days of antisense studies (see, for example, Non-Patent Literature 5).

Replacement of all RNAs of one or both of the sense strand and the antisense strand of siRNA by a 2'-O-methylnucleotide has been reported to reduce or completely eliminate RNAi activity (see, for example, Non-Patent Literatures 6, 7, 8 and 9). It has been reported that introduction of three 2'-O-methylnucleotides in a row does not decrease the activity in the case of the introduction into the sense strand, but decreases the activity in the case of the introduction into the antisense strand, and particularly significantly decreases the activity in the case of the introduction at the 5'-terminus of the sense strand (see, for example, Non-Patent Literature 10).

An oligonucleotide with 2'-deoxy-2'-fluoronucleotide (2'-F or 2'-F RNA), which is an artificially synthesized modified RNA, preferentially forms the same N-conformation as a ribonucleotide, and has increased affinity to RNA, but when the oligonucleotide has a phosphodiester bond, it does not have nuclease resistance, and therefore replacement with a phosphorothioate bond for imparting nuclease resistance is required (see, for example, Non-Patent Literature 11).

ENA (2'-O,4'-C-ethylene-bridged nucleic acid) is a modified nucleic acid having stability against a nuclease, but it has been reported that introduction of ENA into the two nucleotides of an overhang site at the 3'-terminus of one or both of the sense strand and the antisense strand of siRNA decreases the RNAi activity (see, for example, Non-Patent Literature 12).

There have been many reports on siRNA in which a plurality of modified nucleic acids are combined. For example, it has been reported that a 2'-O-methylnucleotide and 2'-F are introduced into every other sense strand and antisense strand of siRNA, and RNAi activity equivalent to or higher than that of unmodified siRNA can be obtained, and relatively stably maintained in the serum (see, for example, Non-Patent Literature 13).

Double-stranded oligonucleotides in which DNA, 2'-O-methyl RNA and 2'-F are combined, in particular, siRNA in which the 14th residue from the 5'-terminus in the antisense strand is 2'-F, have been reported (see, for example, Patent Literatures 5 and 6). Double-stranded oligonucleotides in which DNA, 2'-O-methyl RNA, 2'-F RNA and LNA (2'-O,4'-C-methylene-bridged nucleic acid) are combined, in particular, the activity of siRNA in which the 2nd or 14th residue from the 5'-terminus in the antisense strand is 2'-F, DNA or LNA, have been reported (see, for example, Patent Literatures 5 and 7).

### Citation List

### Patent Literatures

Patent Literature 1: International Patent Publication No. WO 2012/177921
Patent Literature 2: U.S. Patent No. 9,399,775
Patent Literature 3: U.S. Patent No. 9,796,974
Patent Literature 4: U.S. Patent No. 10,612,024
Patent Literature 5: International Patent Publication No. WO 2012/058210
Patent Literature 6: International Patent Publication No. WO 2013/074974, U.S. Patent No. 9,399,775, and U.S. Patent No. 9,796,974
Patent Literature 7: International Patent Publication No. WO 2016/028649, and U.S. Patent No. 10,612,024

### Non-Patent Literatures

Non-Patent Literature 1: Pharmaceuticals 2019, 12, 170
Non-Patent Literature 2: Am. J. Hematol. 88:312-316, 2013.
Non-Patent Literature 3: Nature, 2001, Vol. 411, p. 494-498
Non-Patent Literature 4: Antisense Nucleic Acid Drug Development, 2000, Vol. 10, p. 117-121
Non-Patent Literature 5: Nucleic Acids Research, 1987, Vol. 15, p.6131-6148
Non-Patent Literature 6: EMBO Journal, 2001, Vol. 20, p. 6877-6888
Non-Patent Literature 7: RNA, Vol. 9, 2003, p. 1034-1048
Non-Patent Literature 8: Biochemistry, 2003, Vol. 42, p. 7967-7975
Non-Patent Literature 9: Nucleic Acids Research, 2003, Vol. 31, p. 2705-2716
Non-Patent Literature 10: Journal of Medical Chemistry, 2005, Vol. 48, p. 4247-4253
Non-Patent Literature 11: Journal of Medical Chemistry, 1993, Vol. 36, p. 831-841
Non-Patent Literature 12: Antisense and Nucleic Acid Drug Development, 2002, Vol. 12, p.301-309
Non-Patent Literature 13: Journal of Medicinal Chemistry., 2005, Vol. 48, p. 901-904

### Summary of Invention

### Technical Problem

TfR2 siRNA with high pharmacological activity, which is capable of preventing, ameliorating and/or treating anemia, is desired. TfR2 siRNA having low toxicity to cells and excellent stability and drug delivery properties in the blood is desired.

When there is an increase in hepcidin in anemia associated with chronic inflammation, a therapeutic effect can be obtained by suppressing production of hepcidin in the blood, and the anemia is caused mainly by defective iron utilization due to an increase in hepcidin rather than abnormal production of erythrocytes. However, for anemia associated with bone-marrow dysfunction such as myelofibrosis, the cause is often a decrease in production of erythrocytes, and therefore, even when there is an increase in hepcidin, it cannot be expected that a therapeutic effect can be obtained by suppressing production of hepcidin, and there is no report to indicate so. Thus, in particular, a novel effective drug for treating anemia associated with bone-marrow dysfunction such as myelofibrosis is desired. Studies by the present inventors have shown that the target sequence of TfR2 siRNA in Patent Literature 1 has sequence-specific cytotoxicity, and therefore, there is a safety concern in the conventional art siRNA.

An object of the present invention is to provide novel siRNA having an RNA interfering action and/or a gene expression suppressing action on mRNA encoding TfR2 (these actions are sometimes referred to as a knockdown action). Another object of the present invention is to provide novel TfR2 siRNA having improved cytotoxicity, stability and/or drug delivery properties. Still another object of the present invention is to provide a novel pharmaceutical composition for treating or preventing a disease that can be treated or prevented by suppressing expression of TfR2 mRNA.

### Solution to Problem

The present inventors have conducted diligent studies, and found that TfR2 siRNA whose target sequence is a nucleotide sequence at a specific position in TfR2 mRNA has an RNA interfering action and/or a gene expression suppressing action, and the target sequence in the present invention does not have cytotoxicity to cells which is observed in the target sequence in Patent Literature 1. In addition, the present inventors have found that siRNA having an oligonucleotide in which DNA, RNA, 2'-O-methyl RNA, 2'-F RNA, LNA, ENA and 3'-O-methyl RNA are appropriately combined has an RNA interfering action and/or a gene expression suppressing action, and is stable in a living organism. In addition, the present inventors have found that drug delivery properties are improved by adding a unit for delivery such as GalNAc to siRNA. Further, the present inventors have found that by suppressing expression of TfR2 mRNA using TfR2 siRNA, anemic symptoms are improved not only in a mouse model of anemia associated with inflammation but also in a mouse model of anemia associated with loss of bone-marrow function. In this way, the present invention has been completed.

That is, the present invention provides the following:
[1] An oligonucleotide or a pharmaceutically acceptable salt thereof comprising the following antisense strand region (A) and sense strand region (B), and having a knockdown action on mRNA of transferrin receptor 2:
   (A) an antisense strand region which is 18 to 31 bases in length and consists of a target-matching sequence substantially complementary to a target sequence consisting of 18 to 21 consecutive bases in a region between nucleotide positions 2845 and 2867 or a region between nucleotide positions 1534 and 1556 in SEQ ID NO: 1, in which a nucleoside at the 5'-terminus of the target-matching sequence is a nucleoside substantially complementary to a nucleoside at the 3'-terminus of the target sequence, a nucleoside having adenine, or a nucleoside having uracil, and an overhang structure of 5 or less bases may be present at the 5'-terminus and/or the 3'-terminus of the target-matching sequence; and
   (B) a sense strand region which is 18 to 31 bases in length and contains a nucleotide sequence substantially complementary to the target-matching sequence of the antisense strand region, in which an overhang structure of 5 or less bases is optionally present at the 5'-terminus and/or the 3'-terminus of the nucleotide sequence.
[2] The oligonucleotide or a pharmaceutically acceptable salt thereof according to [1], wherein the target sequence is a nucleotide sequence consisting of 19 bases from nucleotide positions 2847 to 2865 or from nucleotide positions 1536 to 1554 in SEQ ID NO: 1.
[3] The oligonucleotide or a pharmaceutically acceptable salt thereof according to [1] or [2], wherein the nucleoside at the 5'-terminus of the target-matching sequence of the antisense strand region is a nucleoside having adenine or uracil.
[4] The oligonucleotide or a pharmaceutically acceptable salt thereof according to any one of [1] to [3], wherein the target-matching sequence of the antisense strand region is a nucleotide sequence fully complementary to the target sequence consisting of 19 bases from nucleotide positions 2847 to 2865 or from nucleotide positions 1536 to 1554 in SEQ ID NO: 1.
[5] The oligonucleotide or a pharmaceutically acceptable salt thereof according to any one of [1] to [4], wherein at least one of sugars and/or phosphodiester bonds forming the oligonucleotide is modified.
[6] The oligonucleotide or a pharmaceutically acceptable salt thereof according to [5], wherein the sugar forming the oligonucleotide is D-ribofuranose, and the modification of the sugar is modification of a hydroxyl group at the 2'-position of D-ribofuranose.
[7] The oligonucleotide or a pharmaceutically acceptable salt thereof according to [6], wherein the modification of the sugar is 2'-deoxidation, 2'-O-alkylation, 2'-O-alkoxyalkylation, 2'-halogenation and/or 2'-O,4'-C-alkylenation of D-ribofuranose.
[8] The oligonucleotide or a pharmaceutically acceptable salt thereof according to [7], wherein the 2'-O-alkylation is 2'-O-methylation, the 2'-O-alkoxyalkylation is 2'-O-methoxyethylation, the 2'-halogenation is 2'-fluorination, and the 2'-O,4'-C-alkylenation is 2'-O,4'-C-methylenation and/or 2'-O,4'-C-ethylenation.
[9] The oligonucleotide or a pharmaceutically acceptable salt thereof according to any one of [5] to [8], wherein the phosphodiester bond is modified into phosphorothioate.
[10] The oligonucleotide or a pharmaceutically acceptable salt thereof according to any one of [5] to [9], wherein the D-ribofuranose in the nucleoside at the 3'-terminus of the sense strand region and/or the antisense strand region is 3'-O-alkylated.
[11] The oligonucleotide or a pharmaceutically acceptable salt thereof according to [10], wherein the 3'-O-alkylation is 3'-O-methylation.
[12] The oligonucleotide or a pharmaceutically acceptable salt thereof according to any one of [1] to [11], wherein an overhang structure of 3 or less bases is present at the 5'-terminus and/or the 3'-terminus of the sense strand region and/or the antisense strand region.
[13] The oligonucleotide or a pharmaceutically acceptable salt thereof according to [12], wherein the overhang structure has 2 bases.
[14] The oligonucleotide or a pharmaceutically acceptable salt thereof according to [13], wherein the overhang structure is nucleosides containing two consecutive thymines, or nucleosides containing two consecutive uracils.
[15] The oligonucleotide or a pharmaceutically acceptable salt thereof according to [14], wherein the overhang structure is two consecutive U(M) present at the 3'-terminus of the antisense strand region.
[16] The oligonucleotide or a pharmaceutically acceptable salt thereof according to [1], wherein the sense strand region consists of an oligonucleotide represented by the following formula (I), the antisense strand region consists of an oligonucleotide represented by the following formula (II), and having the following characteristics (a) to (g),:
   sense strand region: 5' S_{O5}-Sₐ-S₁₉-S₁₈-S₁₇-S₁₆-S₁₅-S₁₄-S₁₃-S₁₂-S₁₁-S₁₀-S₉-S₈-S₇-S₆-S₅-S₄-S₃-S₂-S₁-S_{O3} 3' (I)
   antisense strand region: 5' A₀₅-A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-Aₐ-A₀₃ 3' (II)
      (a) S₁ is a 3'-modified nucleoside, S₂ to S₁₉ each represent a nucleoside, and are each independently a 2'-OMe RNA, a 2'-F RNA or a DNA, Sₐ represents 0 to 3 nucleosides, where the nucleosides each independently represent 2'-OMe RNA, 2'-F RNA or DNA, S_{O3} and S_{O5} each independently represent 0 to 3 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
      (b) A₁₁, A₁₂, A₁₃, A₁₄ and A₁₅ each represent a nucleoside, one of A₁₁, A₁₂, A₁₃, A₁₄ and A₁₅ is DNA, RNA, a 2'-O,4'-C-bridging-modified nucleoside or 2'-MOE RNA, the others are 2'-OMe RNA or 2'-F RNA, A₁ to A₁₀ and A₁₆ to A₁₉ each represent a nucleoside, and each independently represent 2'-OMe RNA, 2'-F RNA or DNA, Aₐ represents 0 to 3 nucleosides, where the nucleosides each independently represent 2'-OMe RNA, 2'-F RNA or DNA, A_{O3} and A_{O5} each independently represent 0 to 3 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
      (c) the nucleotide sequence between A₂ and Aₐ consists of a nucleotide sequence substantially complementary to a target sequence, A₁ is a nucleoside having a base complementary to a corresponding nucleoside of the target sequence, a nucleoside having adenine, a nucleoside having thymine, or a nucleoside having uracil, and when A₀₃ and A₀₅ are present, the nucleotide sequences thereof are each independently a nucleotide sequence selected independently of a corresponding nucleoside of the target sequence;
      (d) the nucleotide sequence between S₁ and Sₐ and the nucleotide sequence between A₁ and Aₐ are nucleotide sequences substantially complementary to each other, and form a double-stranded structure;
      (e) when both S_{O5} and A_{O3} are present, S_{O5} and A_{O3} are nucleotide sequences not complementary to each other;
      (f) when both S_{O3} and A_{O5} are present, S_{O3} and A_{O5} are nucleotide sequences not complementary to each other; and
      (g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.
[17] The oligonucleotide or a pharmaceutically acceptable salt thereof according to [16], wherein in formula (II), S₁ is 3'-OMe RNA.
[18] The oligonucleotide or a pharmaceutically acceptable salt thereof according to [16] or [17], wherein the 2'-O,4'-C-bridging-modified nucleoside is LNA or ENA.
[19] The oligonucleotide or a pharmaceutically acceptable salt thereof according to any one of [16] to [18], wherein in formula (II), two or more of A₁₁, A₁₂, A₁₃, A₁₄ and A₁₅ are the same or different, and are DNA, RNA, a 2'-O,4'-C-bridging-modified nucleoside or 2'-MOE RNA.
[20] The oligonucleotide or a pharmaceutically acceptable salt thereof according to [19], wherein in formula (II), A₁₄ is RNA or a 2'-O,4'-C-bridging-modified nucleoside.
[21] The oligonucleotide or a pharmaceutically acceptable salt thereof according to [20], wherein in formula (II), A₁₃ is a 2'-O,4'-C-bridging-modified nucleoside or 2'-OMe RNA, and A₁₄ is RNA.
[22] The oligonucleotide or a pharmaceutically acceptable salt thereof according to [21], wherein in formula (II), A₁₁-A₁₂-A₁₃-A₁₄-A₁₅ is one of:
   A₁₁(2'-OMe RNA)-A₁₂(2'-OMe RNA)-A₁₃(ENA)-A₁₄(RNA)-A₁₅(2'-OMe RNA),
   A₁₁(2'-FRNA)-A₁₂(2'-OMeRNA)-A₁₃(ENA)-A₁₄(RNA)-A₁₅(2'-OMe RNA),
   A₁₁(2'-OMe RNA)-A₁₂(2'-F RNA)-A₁₃(ENA)-A₁₄(RNA)-A₁₅(2'-OMe RNA),
   A₁₁(2'-OMe RNA) -A₁₂(2' -OMe RNA) -A₁₃(LNA)-A₁₄(RNA)-A₁₅(2'-OMe RNA),
   A₁₁(2' -F RNA)-A₁₂(2'-OMe RNA)-A₁₃(LNA)-A₁₄(RNA)-A₁₅(2'-OMe RNA),
   A₁₁(2'-OMe RNA)-A₁₂(2'-F RNA)-A₁₃(LNA)-A₁₄(RNA)-A₁₅(2'-OMe RNA),
   A₁₁(2'-F RNA)-A₁₂(2'-OMe RNA)-A₁₃(2' -OMe RNA)-A₁₄(RNA)-A₁₅(2'-OMe RNA), and,
   A₁₁(2'-OMe RNA)-A₁₂(2'-F RNA)-A₁₃(2' -OMe RNA)-A₁₄(RNA)-A₁₅(2'-OMe RNA).
[23] The oligonucleotide or a pharmaceutically acceptable salt thereof according to [20], wherein in formula (II), A₁₂ is DNA, and A₁₄ is a 2'-O,4'-C-bridging-modified nucleoside.
[24] The oligonucleotide or a pharmaceutically acceptable salt thereof according to [23], wherein in formula (II), A₁₁-A₁₂-A₁₃-A₁₄-A₁₅ is one of:
   A₁₁(2'-F RNA)-A₁₂(DNA)-A₁₃(2'-OMe RNA)-A₁₄(ENA)-A₁₅(2'-OMe RNA),
   A₁₁(2'-OMe RNA)-A₁₂(DNA)-A₁₃(2'-OMe RNA)-A₁₄(ENA)-A₁₅(2'-OMe RNA),
   A₁₁(2'-F RNA)-A₁₂(DNA)-A₁₃(2'-OMe RNA)-A₁₄(LNA)-A₁₅(2'-OMe RNA), and,
   A₁₁(2'-OMe RNA)-A₁₂(DNA)-A₁₃(2'-OMe RNA)-A₁₄(LNA)-A₁₅(2'-OMe RNA) .
[25] The oligonucleotide or a pharmaceutically acceptable salt thereof according to any one of [16] to [24], wherein in formula (II), A₂, A₆ and A₁₆ are 2'-F RNA.
[26] The oligonucleotide or a pharmaceutically acceptable salt thereof according to [25], wherein in formula (II), one or two nucleosides selected from A₈, A₉ and A₁₀ are 2'-F RNA, and the nucleosides A₁, A₃ to A₅, A₇, A₁₇ to A₁₉ and Aₐ are 2'-OMe RNA.
[27] The oligonucleotide or a pharmaceutically acceptable salt thereof according to [26], wherein in formula (II), A₂, A₆, A₈, A₁₀ and A₁₆ are 2'-F RNA, and the nucleosides A₁, A₃ to A₅, A₇, A₉, A₁₇ to A₁₉ and Aₐ are 2'-OMe RNA.
[28] The oligonucleotide or a pharmaceutically acceptable salt thereof according to any one of [16] to [27], wherein in formula (I), S₁₁, S₁₂, S₁₃ and S₁₅ are 2'-F RNA.
[29] The oligonucleotide or a pharmaceutically acceptable salt thereof according to [28], wherein in formula (I), S₂ to S₁₀, S₁₄, S₁₆ to S₁₉ and Sₐ are 2'-OMe RNA.
[30] The oligonucleotide or a pharmaceutically acceptable salt thereof according to any one of [16] to [29], wherein when RNA is used in formula (II), a bond between the RNA and a 3' adjacent nucleoside is a phosphorothioate bond.
[31] The oligonucleotide or a pharmaceutically acceptable salt thereof according to any one of [16] to [30], wherein in 2 to 5 nucleotides from each of the 5'-terminus and the 3'-terminus of the oligonucleotide, each inter-nucleoside bond is a phosphorothioate bond.
[32] The oligonucleotide or a pharmaceutically acceptable salt thereof according to any one of [16] to [31], wherein the number of nucleosides in each of S_{O3}, S_{O5}, A_{O5} and A_{O3} is 0 to 2.
[33] The oligonucleotide or a pharmaceutically acceptable salt thereof according to [32], wherein the number of nucleosides in S_{O3} is 0.
[34] The oligonucleotide or a pharmaceutically acceptable salt thereof according to [33], wherein the number of nucleosides in each of S_{O3}, S_{O5} and A_{O5} is 0, and the number of nucleosides in A_{O3} is 2.
[35] The oligonucleotide or a pharmaceutically acceptable salt thereof according to any one of [1] to [34], wherein the antisense strand region and the sense strand region form a double-stranded oligonucleotide as independent oligonucleotides.
[36] The oligonucleotide or a pharmaceutically acceptable salt thereof according to any one of [1] to [34], wherein the nucleoside at the 5'-terminus of the antisense strand region and the nucleoside at the 3'-terminus of the sense strand region are connected by a linker structure.
[37] The oligonucleotide or a pharmaceutically acceptable salt thereof according to [36], wherein the linker structure is represented by the following formula: 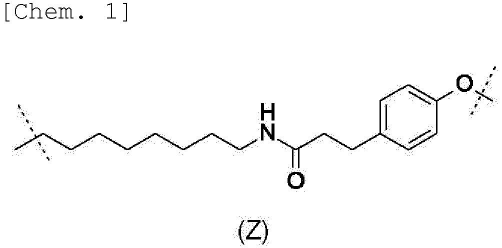 wherein the broken line represents a point of attachment, and means that the oxygen atom bonded to the phenyl group forms a phosphodiester bond or a phosphorothioate bond with a 5'-phosphate group of a nucleotide at the 5'-terminus of an adjacent antisense strand region, and the methylene group at the other end forms a phosphodiester bond or a phosphorothioate bond with a 3'-phosphate group (phosphate group at the 2'-position in the case of a nucleotide modified at the 3'-position) of a nucleotide at the 3'-terminus of an adjacent sense strand region.
[38] The oligonucleotide or a pharmaceutically acceptable salt thereof according to any one of [1] to [37], wherein there is a chemical modification with a GalNAc unit at the 5'-position of a nucleoside at the 5'-terminus of the oligonucleotide and/or the 3'-position of a nucleoside at the 3'-terminus of the oligonucleotide, or the 2'-position of the nucleoside at the 3'-terminus of the oligonucleotide in the case where the nucleoside at the 3'-terminus is a 3'-modified nucleoside.
[39] The oligonucleotide or a pharmaceutically acceptable salt thereof according to [38], wherein the GalNAc unit is represented by: 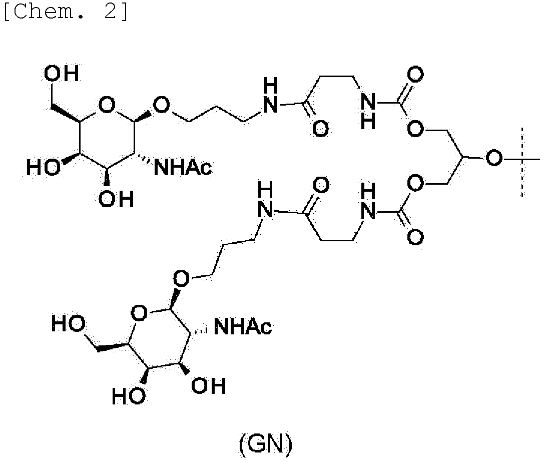 wherein the broken line represents a phosphodiester bond formed with a phosphate group at the 5'-terminus and/or a phosphate group (phosphate group at the 2'-position in the case of a nucleotide modified at the 3'-position) at the 3'-terminus of an adjacent nucleotide.
[40] The oligonucleotide or a pharmaceutically acceptable salt thereof according to [1], wherein the sense strand region excluding the overhang structure has the following formula (I-1), the antisense strand region excluding the overhang structure has any of the following formulae (II-1) to (II-10), the sense strand region and the antisense strand region form a double-stranded oligonucleotide as independent oligonucleotides, and the sense strand region and the antisense strand region may each independently contain an overhang structure:
   (formulae)

   5' C(M)G(M)U(M)G(M)G(F)A(M)G(F)U(F)U(F)U(M)C(M)A(M)A(M)U(M)A (M)U(M)C(M)A(M)A(3M) 3' (I-1)

   5' U(M)U(F)G(M)A(M)U(M)A(F)U(M)U(F)G(F)A(M)A(M)A(D)C(M)T(E)C (M)C(F)A(M)C(M)G(M) 3' (II-1)

   5' U(M)U(F)G(M)A(F)U(M)A(F)U(M)U(M)G(F)A(M)A(M)A(D)C(M)T(E)C (M)C(F)A(M)C(M)G(M) 3' (11-2)

   5' U(M)U(F)G(M)A(M)U(M)A(F)U(M)U(M)G(F)A(F)A(M)A(D)C(M)T(E)C (M)C(F)A(M)C(M)G(M)3' (11-3)

   5' U(M)U(F)G(M)A(M)U(M)A(F)U(M)U(M)G(F)A(M)A(M)A(D)C(M)T(E)C (M)C(F)A(M)C(F)G(M) 3' (11-4)

   5' U(M)U(F)G(M)A(M)U(M)A(F)U(M)U(F)G(F)A(M)A(M)A(M)C(E)U(R)^ C(M)C(F)A(M)C(M)G(M) 3' (II-5)

   5' U(M)U(F)G(M)A(M)U(M)A(F)U(M)U(M)G(F)A(F)A(M)A(M)C(E)U(R)^ C(M)C(F)A(M)C(M)G(M) 3' (11-6)

   5' U(M)U(F)G(M)A(M)U(M)A(F)U(M)U(M)G(F)A(M)A(F)A(M)C(E)U(R)^ C(M)C(F)A(M)C(M)G(M) 3' (II-7)

   5' U(M)U(F)G(M)A(M)U(M)A(F)U(M)U(M)G(F)A(M)A(M)A(F)C(E)U(R)^ C(M)C(F)A(M)C(M)G(M) 3' (II-8)

   5' U(M)U(F)G(M)A(M)U(M)A(F)U(M)U(M)G(F)A(M)A(F)A(M)C(M)U(R)^ C(M)C(F)A(M)C(M)G(M) 3' (II-9)

   5' U(M)U(F)G(M)A(M)U(M)A(F)U(M)U(M)G(F)A(M)A(M)A(F)C(M)U(R)^ C(M)C(F)A(M)C(M)G(M) 3' (II-10)

   wherein for nucleic acid bases, A represents adenine, U represents uracil, T represents thymine, G represents guanine, and C represents cytosine (provided that C represents 2'-O,4'-C-ethylene-bridged-5-methylcytidine when C is ENA), and for nucleic acids, (R) represents RNA, (D) represents DNA, (M) represents 2'-OMe RNA, (3M) represents 3'-OMe RNA, (F) represents 2'-F RNA, and (E) represents ENA; and "^" means that the bond between nucleosides is a phosphorothioate bond (-P(=S) (OH)-), and the bond between nucleosides is a phosphodiester bond (-P(=O) (OH)-) if no specific indication is given, but two inter-nucleoside bonds in three nucleosides from each of the 5'-terminus and the 3'-terminus of the sense strand region, and two inter-nucleoside bonds in three nucleosides from the 5'-terminus and three inter-nucleoside bonds in four nucleosides from the 3'-terminus of the antisense strand region are phosphorothioate bonds, even if no specific indication is given.
[41] The oligonucleotide or a pharmaceutically acceptable salt thereof according to [40], wherein an overhang structure of 3 or less bases is present at the 5'-terminus and/or the 3'-terminus of the sense strand region and/or the antisense strand region.
[42] The oligonucleotide or a pharmaceutically acceptable salt thereof according to [41], wherein the overhang structure has 2 bases.
[43] The oligonucleotide or a pharmaceutically acceptable salt thereof according to [42], wherein the overhang structure is nucleosides containing two consecutive thymines, or nucleosides containing two consecutive uracils.
[44] The oligonucleotide or a pharmaceutically acceptable salt thereof according to [43], wherein the overhang structure is two consecutive U(M) added at the 3'-terminus of the antisense strand region.
[45] The oligonucleotide or a pharmaceutically acceptable salt thereof according to any one of [40] to [44], wherein the 5'-terminus of the sense strand region is chemically modified with a GalNAc unit represented by the following formula: 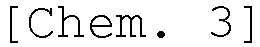 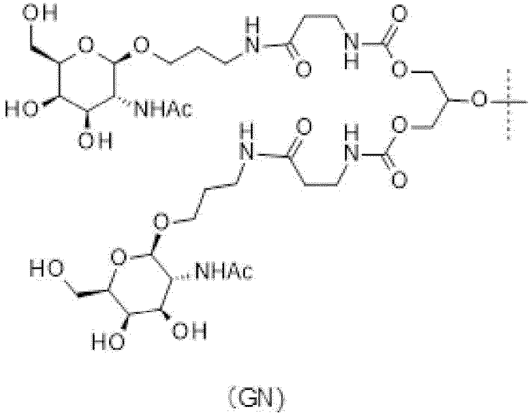 wherein the broken line represents a phosphodiester bond formed with a 5'-phosphate group of a nucleotide at the 5'-terminus of the adjacent sense strand region.
[46] The oligonucleotide or a pharmaceutically acceptable salt thereof according to [1], wherein the sense strand region has the following formula (I-2), the antisense strand region has any of the following formulae (II-11) to (II-20), and the sense strand region and the antisense strand region form a double-stranded oligonucleotide as independent oligonucleotides:
   (formulae)

   5' GNC(M)^G(M)^U(M)G(M)G(F)A(M)G(F)U(F)U(F)U(M)C(M)A(M)A(M)U (M)A(M)U(M)C(M)^A(M)^A(3M) 3' (I-2)

   5' U(M)^U(F)^G(M)A(M)U(M)A(F)U(M)U(F)G(F)A(M)A(M)A(D)C(M)T(E )C(M)C(F)A(M)C(M)^G(M)^U(M)^U(M) 3' (II-11)

   5' U(M)^U(F)^G(M)A(F)U(M)A(F)U(M)U(M)G(F)A(M)A(M)A(D)C(M)T(E )C(M)C(F)A(M)C(M)^G(M)^U(M)^U(M) 3' (II-12)

   5' U(M)^U(F)^G(M)A(M)U(M)A(F)U(M)U(M)G(F)A(F)A(M)A(D)C(M)T(E )C(M)C(F)A(M)C(M)^G(M)^U(M)^U(M) 3' (II-13)

   5' U(M)^U(F)^G(M)A(M)U(M)A(F)U(M)U(M)G(F)A(M)A(M)A(D)C(M)T(E )C(M)C(F)A(M)C(F)^G(M)^U(M)^U(M) 3' (II-14)

   5' U(M)^U(F)^G(M)A(M)U(M)A(F)U(M)U(F)G(F)A(M)A(M)A(M)C(E)U(R )^C(M)C(F)A(M)C(M)^G(M)^U(M)^U(M) 3' (II-15)

   5' U(M)^U(F)^G(M)A(M)U(M)A(F)U(M)U(M)G(F)A(F)A(M)A(M)C(E)U(R )^C(M)C(F)A(M)C(M)^G(M)^U(M)^U(M) 3' (II-16)

   5' U(M)^U(F)^G(M)A(M)U(M)A(F)U(M)U(M)G(F)A(M)A(F)A(M)C(E)U(R )^C(M)C(F)A(M)C(M)^G(M)^U(M)^U(M) 3' (II-17)

   5' U(M)^U(F)^G(M)A(M)U(M)A(F)U(M)U(M)G(F)A(M)A(M)A(F)C(E)U(R )^C(M)C(F)A(M)C(M)^G(M)^U(M)^U(M) 3' (II-18)

   5' U(M)^U(F)^G(M)A(M)U(M)A(F)U(M)U(M)G(F)A(M)A(F)A(M)C(M)U(R )^C(M)C(F)A(M)C(M)^G(M)^U(M)^U(M) 3' (II-19)

   5' U(M)^U(F)^G(M)A(M)U(M)A(F)U(M)U(M)G(F)A(M)A(M)A(F)C(M)U(R )^C(M)C(F)A(M)C(M)^G(M)^U(M)^U(M) 3' (II-20)

   wherein "GN" represents a GalNAc unit represented by the following formula: 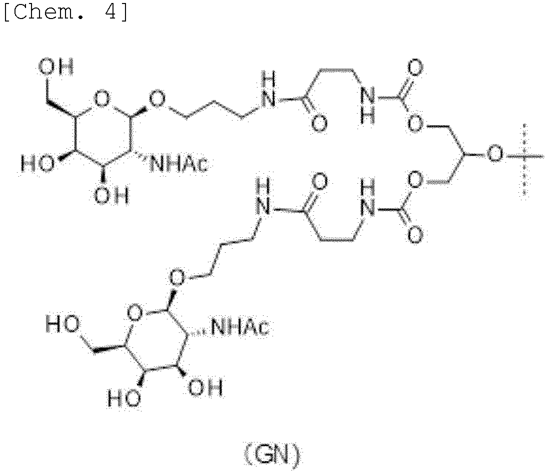 wherein the broken line represents a phosphodiester bond formed with a 5'-phosphate group of a nucleotide at the 5'-terminus of the adjacent sense strand region; for nucleobases, A represents adenine, U represents uracil, T represents thymine, G represents guanine, and C represents cytosine (provided that C represents 2'-O,4'-C-ethylene-bridged-5-methylcytidine when C is ENA), and for nucleic acids, (R) represents RNA, (D) represents DNA, (M) represents 2'-OMe RNA, (3M) represents 3'-OMe RNA, (F) represents 2'-F RNA, and (E) represents ENA; "^" means that the bond between nucleosides is a phosphorothioate bond (-P(=S) (OH)-), and the bond between nucleosides is a phosphodiester bond (-P(=O) (OH)-) if no specific indication is given; and each of residues at the 3'-position of the nucleotide at the 3'-terminus of the sense strand region, at the 5'-position of the nucleotide at the 5'-terminus of the antisense strand region, and the 3'-position of the nucleotide at the 3'-terminus of the antisense strand region is a hydroxyl group.
[47] A pharmaceutical composition comprising the oligonucleotide or a pharmaceutically acceptable salt thereof according to any one of [1] to [46] as an active ingredient.
[48] The pharmaceutical composition according to [47], for treating or preventing a disease that can be treated or prevented by suppressing expression of a transferrin receptor 2.
[49] The pharmaceutical composition according to [48], for preventing or treating anemia.
[50] The pharmaceutical composition according to [49], wherein the anemia is anemia associated with chronic inflammation or anemia associated with bone-marrow dysfunction.
[51] The pharmaceutical composition according to [50], wherein the anemia associated with chronic inflammation is anemia associated with an autoimmune disease, anemia associated with an infectious disease, anemia associated with inflammatory enteric disease, anemia associated with heart failure, anemia associated with chronic kidney disease, cancerous anemia, or anemia associated with Castleman disease.
[52] The pharmaceutical composition according to [50], wherein the anemia associated with bone-marrow dysfunction is anemia associated with myelofibrosis, anemia associated with myelodysplastic syndrome, anemia associated with chronic myelomonocytic leukemia, or anemia associated with chemotherapeutic myelosuppression.
[53] The pharmaceutical composition according to any one of [47] to [52], which is intended for a patient undergoing treatment with another drug agent.
[54] The pharmaceutical composition according to [53], wherein the other drug agent is a drug for treating anemia, a drug for treating a disease that causes anemia, or a drug for treating iron overload disorder.
[55] The pharmaceutical composition according to [54], wherein the drug for treating anemia is an erythropoietic stimulation factor preparation, a HIFPHD (hypoxia inducible factor prolyl hydroxylase) inhibitor, an oral iron supplement, an intravenous iron supplement, luspatercept, or danazol.
[56] The pharmaceutical composition according to [55], wherein the erythropoietic stimulation factor preparation is epoetin alfa, epoetin beta, epoetin beta pegol, epoetin kappa, or darbepoetin alfa.
[57] The pharmaceutical composition according to [55], wherein the HIFPHD (hypoxia inducible factor prolyl hydroxylase) inhibitor is roxadustat, vadadustat, daprodustat, enarodustat, or molidustat.
[58] The pharmaceutical composition according to [55], wherein the oral iron supplement is ferrous citrate, ferric citrate, ferrous fumarate, soluble ferric pyrophosphate, or dry iron sulfide.
[59] The pharmaceutical composition according to [55], wherein the intravenous iron supplement is ferric carboxymaltose, or sugar-containing iron oxide.
[60] The pharmaceutical composition according to [54], wherein the drug for treating a disease that causes anemia is a drug for treating myelofibrosis, a drug for treating myelodysplastic syndrome, a drug for treating chronic kidney disease, or an anticancer agent.
[61] The pharmaceutical composition according to [60], wherein the drug for treating myelofibrosis is a JAK2 inhibitor.
[62] The pharmaceutical composition according to [61], wherein the JAK2 inhibitor is ruxolitinib, fedratinib, pacritinib, or momelotinib.
[63] The pharmaceutical composition according to [60], wherein the drug for treating myelodysplastic syndrome is azacytidine, or lenalidomide.
[64] The pharmaceutical composition according to [60], wherein the drug for treating chronic kidney disease is a SGLT2 (sodium glucose co-transporter 2) inhibitor, a renin-angiotensin system inhibitor, a calcium antagonist, a diuretic drug, a drug for treating diabetes, a drug for treating hyperlipidemia, a steroid, or an immunosuppressive drug.
[65] The pharmaceutical composition according to [64], wherein the renin-angiotensin system inhibitor is an angiotensin II receptor antagonist or an angiotensin-converting enzyme inhibitor.
[66] The pharmaceutical composition according to [60], wherein the anticancer agent is a chemotherapeutic drug.
[67] The pharmaceutical composition according to [66], wherein the chemotherapeutic drug is cisplatin, carboplatin, doxorubicin, paclitaxel, or amrubicin.
[68] The pharmaceutical composition according to [54], wherein the drug for treating iron overload disorder is an iron chelating agent.
[69] The pharmaceutical composition according to [68], wherein the iron chelating agent is deferasirox, deferoxamine, or deferiprone.
[70] A method for treating or preventing anemia, comprising administering an effective amount of the oligonucleotide or a pharmaceutically acceptable salt thereof according to any one of [1] to [46] to a subject.
[71] A method for treating or preventing anemia, comprising administering the pharmaceutical composition according to any one of [47] to [69] to a subject.
[72] The oligonucleotide or a pharmaceutically acceptable salt thereof according to any one of [1] to [46], for use in the treatment or prevention of anemia.
[73] The oligonucleotide or a pharmaceutically acceptable salt thereof according to any one of [1] to [46], for use as an active ingredient of the pharmaceutical composition according to any one of [47] to [69].
[74] Use of the oligonucleotide or a pharmaceutically acceptable salt thereof according to any one of [1] to [46] in the production of the pharmaceutical composition according to any one of [47] to [69].

Further, in one aspect, the present invention provides the following:
[75] A pharmaceutical composition for treatment or prevention of anemia associated with bone-marrow dysfunction, comprising, as an active ingredient, a compound having an effect of knocking down the transferrin receptor 2 gene.
[76] The pharmaceutical composition according to [75], wherein the compound is an RNAi-oligonucleotide.

### Advantageous Effects of Invention

The siRNA of the present invention can suppress expression of TfR2 mRNA. The siRNA of the present invention can suppress expression of TfR2 mRNA in the liver in a living organism, and is capable of preventing, ameliorating, and/or treating anemia.

### Brief Description of Drawings

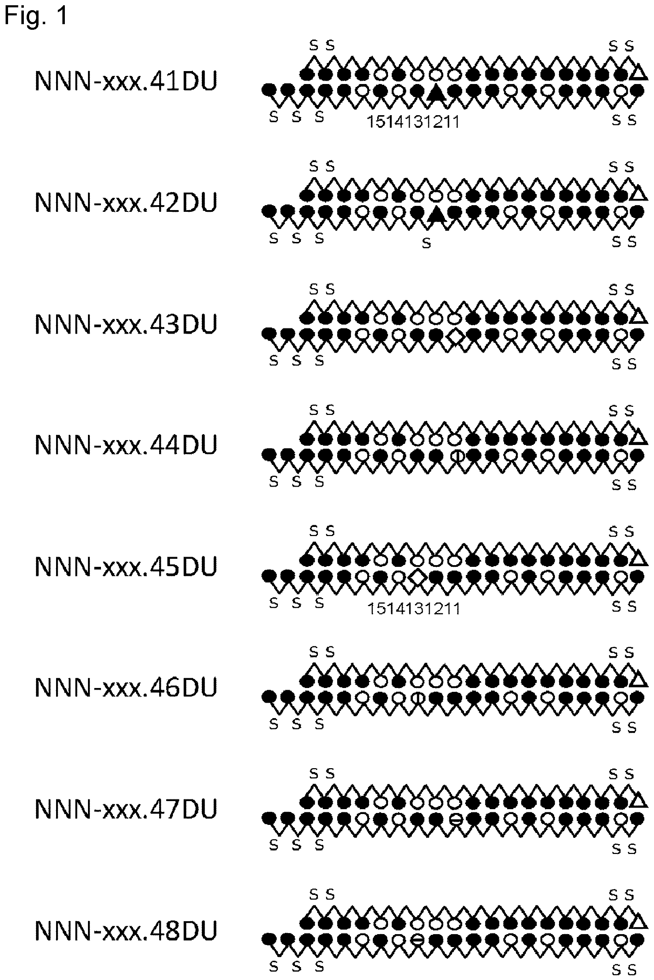

[Figure 1] Figure 1 shows modification patterns of double-stranded siRNA. In the figure, the black circle represents 2'-O-methyl RNA, the white circle represents 2'-deoxy-2'-fluoro RNA, the black triangle represents DNA, the white triangle represents 3'-O-methyl RNA, the white diamond represents ENA, the black diamond represents RNA, the white circle with a vertical bar represents LNA, the white circle with a horizontal bar represents 2'-O-methoxyethyl RNA, and the line connecting marks represents a phosphodiester bond, where the line connecting marks represents a phosphorothioate bond when labelled "S". In the figure, the upper strand represents a sense strand (or a passenger strand), the left terminus of the sense strand represents the 5'-terminus, and the right terminus of the sense strand represents the 3'-terminus. The lower strand represents an antisense strand (or a guide strand), the right terminus of the antisense strand represents the 5'-terminus, and the left terminus of the antisense strand represents the 3'-terminus. Each siRNA is represented by NNN-xxx, and the numbers and letters written after NNN-xxx indicate an abbreviation of each of the modification patterns shown in Figures 1 to 6.

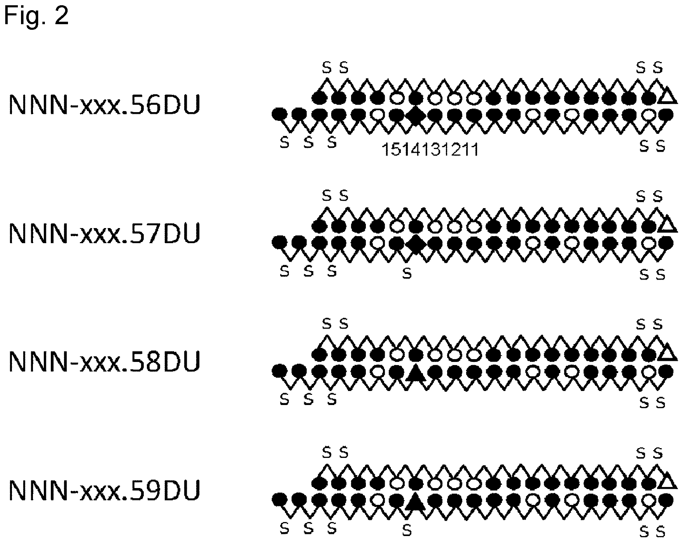

[Figure 2] Figure 2 shows modification patterns of double-stranded siRNA in which A₁₄ of the antisense strand is DNA or RNA. The symbols, arrangements, modifications and the like are shown in the same form as in Figure 1.

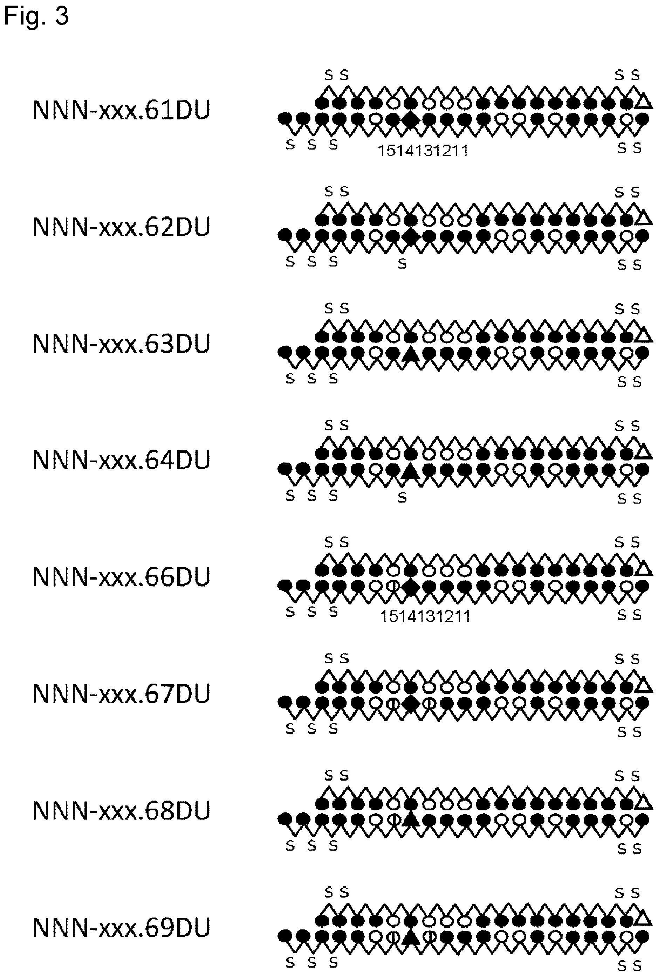

[Figure 3] Figure 3 shows modification patterns of double-stranded siRNA in which A₈ and A₉ of the antisense strand are 2'-deoxy-2'-fluoro RNA. The symbols, arrangements, modifications and the like are shown in the same form as in Figure 1.

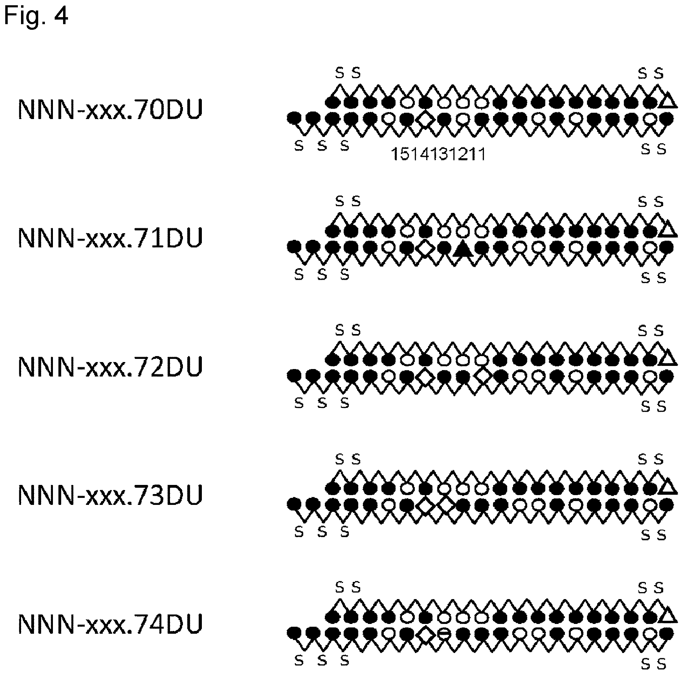

[Figure 4] Figure 4 shows modification patterns of double-stranded siRNA in which A₁₄ of the antisense strand is ENA. The symbols, arrangements, modifications and the like are shown in the same form as in Figure 1.

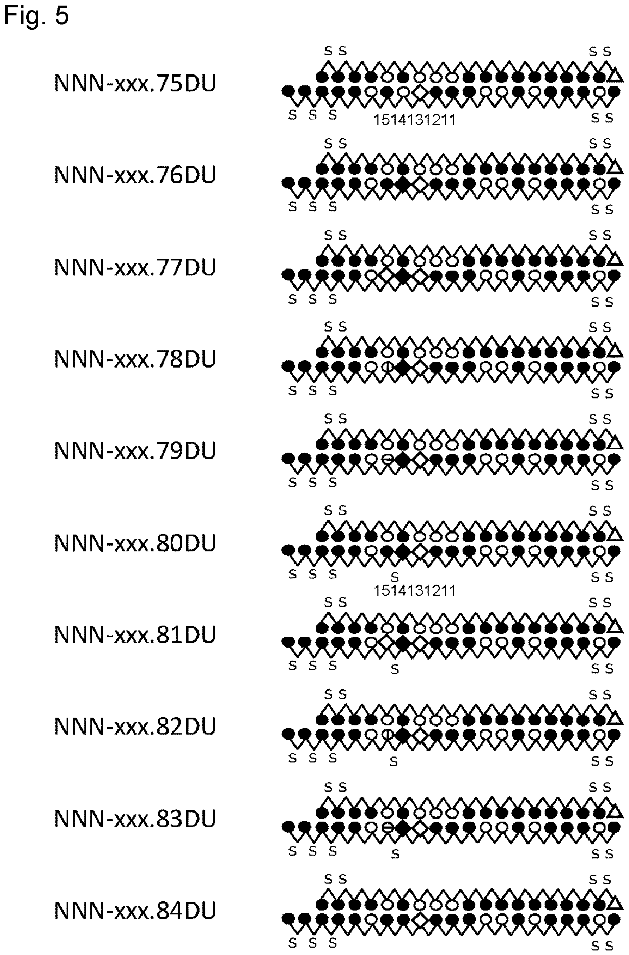

[Figure 5] Figure 5 shows modification patterns of double-stranded siRNA in which A₈ and A₉ of the antisense strand are 2'-deoxy-2'-fluoro RNA and A₁₃ of the antisense strand is ENA. The symbols, arrangements, modifications and the like are shown in the same form as in Figure 1.

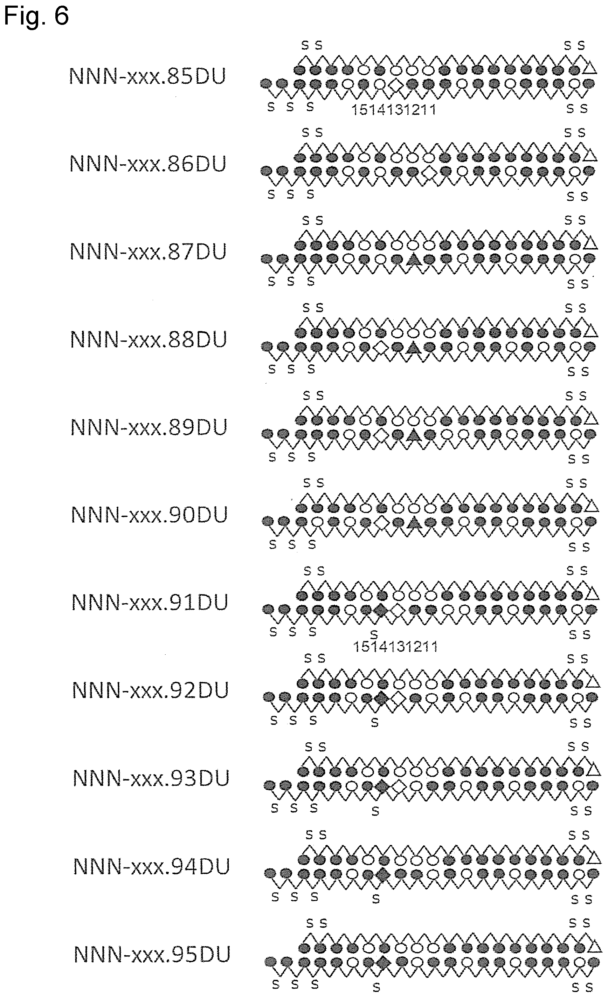

[Figure 6] Figure 6 shows modification patterns of double-stranded siRNA in which A₈ of the antisense strand is 2'-O-methyl RNA and A₉ of the antisense strand is 2'-deoxy-2'-fluoro RNA. The symbols, arrangements, modifications and the like are shown in the same form as in Figure 1.

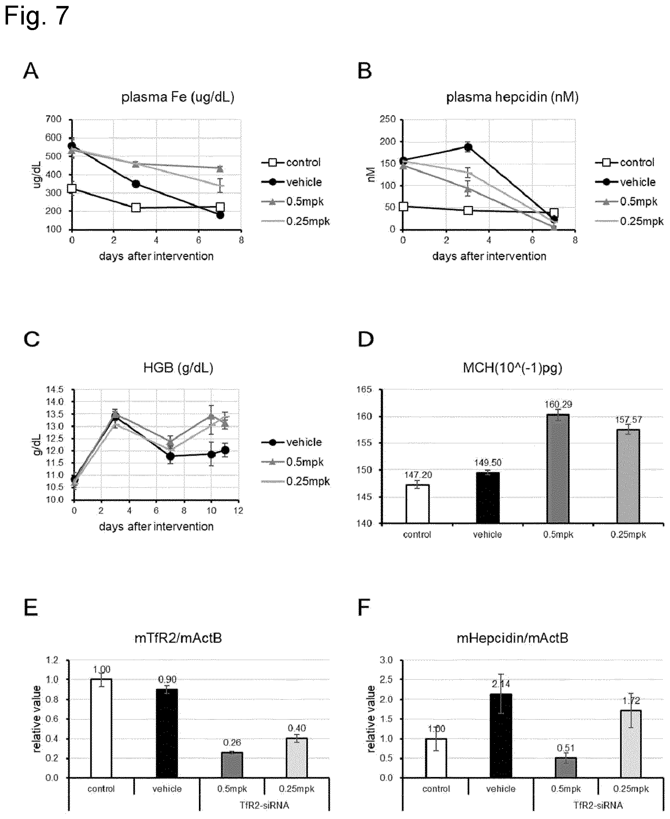

[Figure 7] Figure 7A shows the change in plasma iron concentration after treatment with nucleic acid lipid particles encapsulating TfR2 siRNA in a mouse model of anemia associated with loss of bone-marrow function.
Figure 7B shows the change in plasma hepcidin concentration after treatment with LNP-encapsulated TfR2 siRNA in a mouse model of anemia associated with loss of bone-marrow function. Figure 7C shows the change in hemoglobin (HGB) concentration after treatment with LNP-encapsulated TfR2 siRNA in a mouse model of anemia associated with loss of bone-marrow function. Figure 7D shows the mean corpuscular hemoglobin (MCH) amount after treatment with LNP-encapsulated TfR2 siRNA in a mouse model of anemia associated with loss of bone-marrow function. Figure 7E shows the liver Tfr2 (mTfr2) mRNA expression level after treatment with LNP-encapsulated TfR2 siRNA in a mouse model of anemia associated with loss of bone-marrow function. Figure 7F shows the liver hepcidin (mHepcidin) mRNA expression level after treatment with LNP-encapsulated TfR2 siRNA in a mouse model of anemia associated with loss of bone-marrow function.

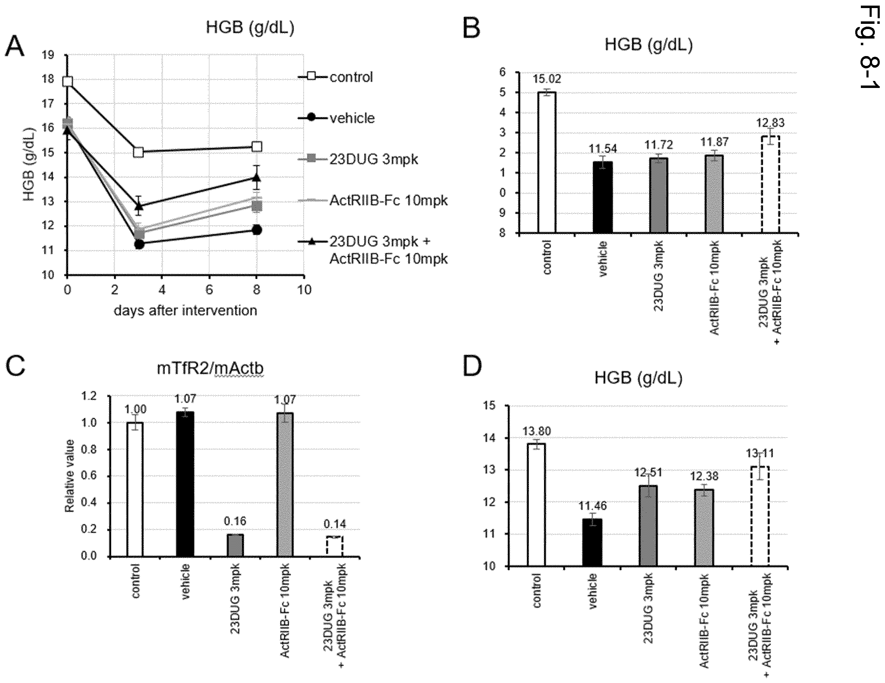

[Figure 8-1] Figure 8A shows the change of the HGB concentration after treatment with GalNAc-conjugated TfR2 siRNA in a mouse model of anemia associated with loss of bone-marrow function. Figure 8B shows the HGB concentration 3 days after drug agent treatment in a mouse model of anemia associated with loss of bone-marrow function. Figure 8C shows the liver mTfR2 mRNA expression level after treatment with GalNAc-conjugated TfR2 siRNA in a mouse model of anemia associated with loss of bone-marrow function. Figure 8D shows the HGB concentration after treatment with GalNAc-conjugated TfR2 siRNA in a mouse model of anemia associated with loss of bone-marrow function.

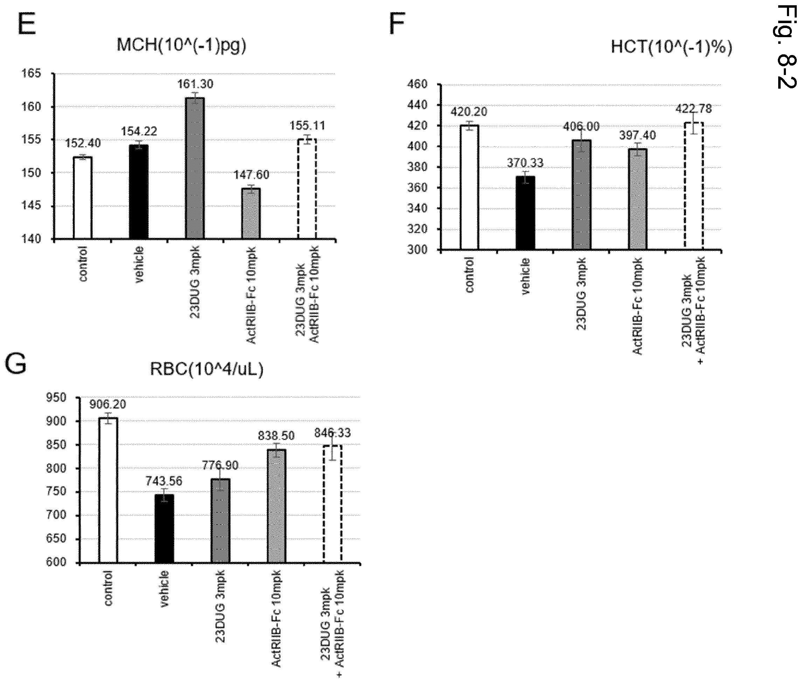

[Figure 8-2] Figure 8E shows the MCH amount after treatment with GalNAc-conjugated TfR2 siRNA in a mouse model of anemia associated with loss of bone-marrow function. Figure 8F shows the hematocrit (HCT) level after treatment with GalNAc-conjugated TfR2 siRNA in a mouse model of anemia associated with loss of bone-marrow function. Figure 8G shows the number of red blood cells (RBCs) after treatment with GalNAc-conjugated TfR2 siRNA in a mouse model of anemia associated with loss of bone-marrow function.

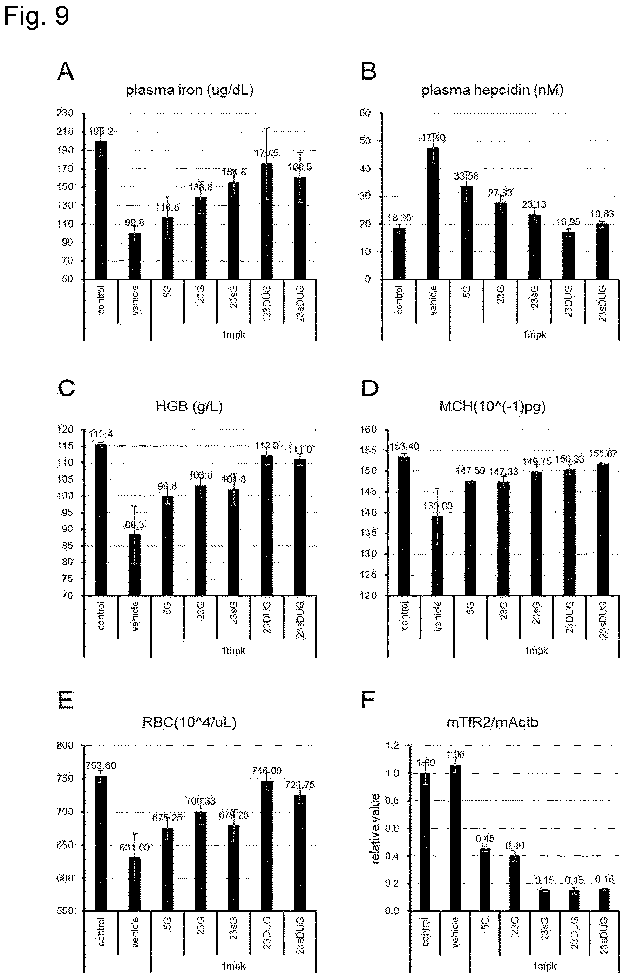

[Figure 9] Figure 9A shows the plasma iron concentration after treatment with GalNAc-conjugated TfR2 siRNA in a mouse model of anemia associated with inflammation. Figure 9B shows the plasma hepcidin concentration after treatment with GalNAc-conjugated TfR2 siRNA in a mouse model of anemia associated with inflammation. Figure 9C shows the HGB concentration after treatment with GalNAc-conjugated TfR2 siRNA in a mouse model of anemia associated with inflammation. Figure 9D shows the MCH amount after treatment with GalNAc-conjugated TfR2 siRNA in a mouse model of anemia associated with inflammation. Figure 9E shows the number of RBCs after treatment with GalNAc-conjugated TfR2 siRNA in a mouse model of anemia associated with inflammation. Figure 9F shows the liver mTfR2 mRNA expression level after treatment with GalNAc-conjugated TfR2 siRNA in a mouse model of anemia associated with inflammation.

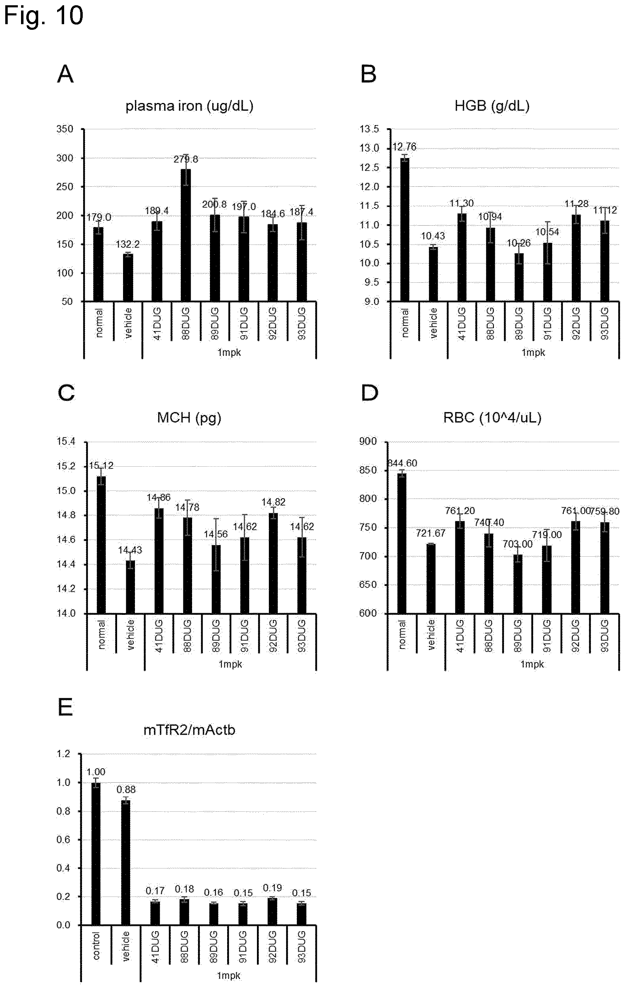

[Figure 10] Figure 10A shows the plasma iron concentration after treatment with GalNAc-conjugated TfR2 siRNA in a mouse model of anemia associated with inflammation. Figure 10B shows the HGB concentration after treatment with GalNAc-conjugated TfR2 siRNA in a mouse model of anemia associated with inflammation.
Figure 10C shows the MCH amount after treatment with GalNAc-conjugated TfR2 siRNA in a mouse model of anemia associated with inflammation. Figure 10D shows the number of RBCs after treatment with GalNAc-conjugated TfR2 siRNA in a mouse model of anemia associated with inflammation. Figure 10E shows the liver mTfR2 mRNA expression level after treatment with GalNAc-conjugated TfR2 siRNA in a mouse model of anemia associated with inflammation.

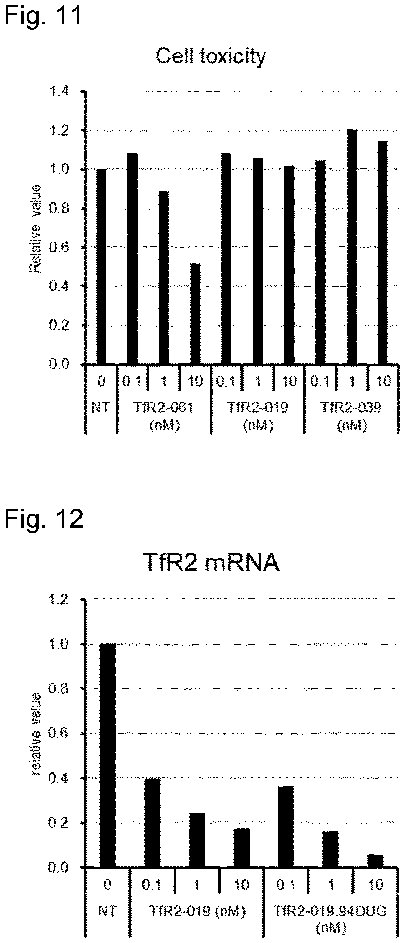

[Figure 11] Figure 11 shows the results of evaluation of cytotoxicity of siRNA in HepG2 cells.
[Figure 12] Figure 12 shows the hTfR2 knockdown activity of siRNA in HepG2 cells.

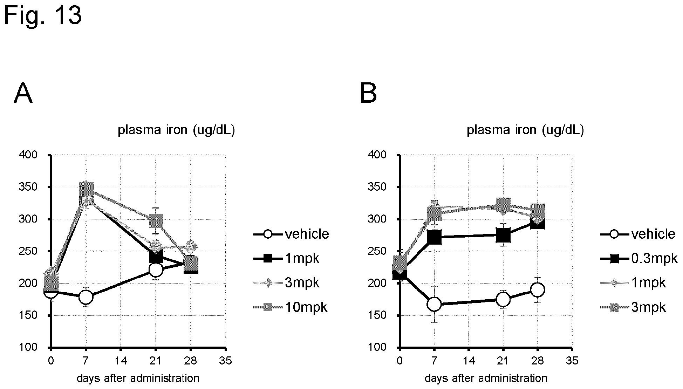

[Figure 13] Figure 13 shows the change in plasma iron concentration after treatment with GalNAc-conjugated TfR2 siRNA in normal mice.

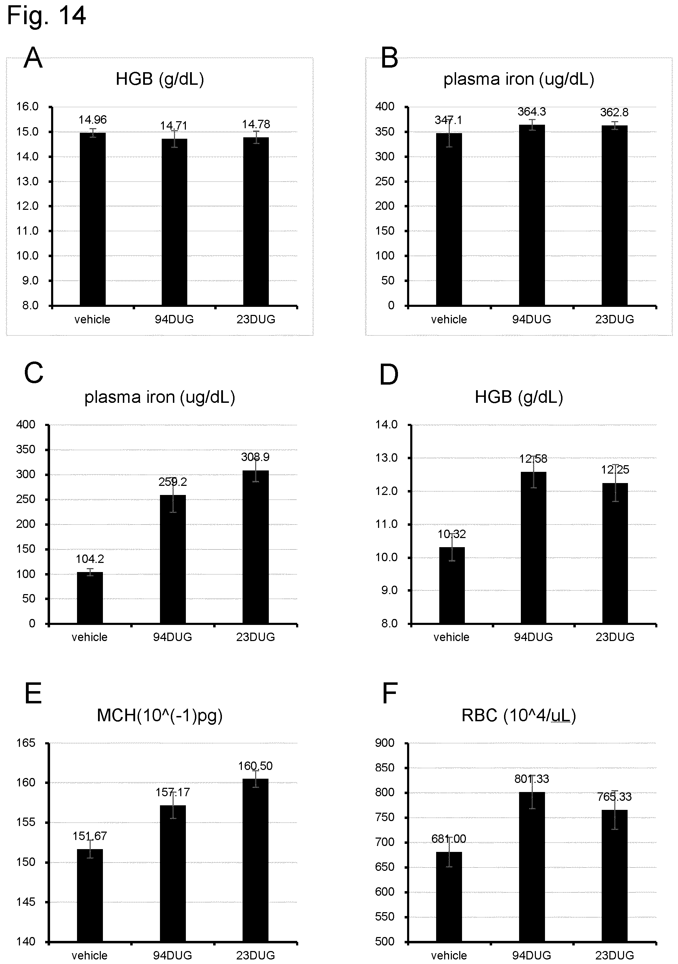

[Figure 14] Figure 14A shows the HGB concentration before treatment with GalNAc-conjugated TfR2 siRNA in a mouse model of anemia associated with loss of bone-marrow function. Figure 14B shows the plasma iron concentration before treatment with GalNAc-conjugated TfR2 siRNA in a mouse model of anemia associated with loss of bone-marrow function. Figure 14C shows the plasma iron concentration 8 days after treatment with GalNAc-conjugated TfR2 siRNA in a mouse model of anemia associated with loss of bone-marrow function. Figure 14D shows the HGB concentration 9 days after treatment with GalNAc-conjugated TfR2 siRNA in a mouse model of anemia associated with loss of bone-marrow function. Figure 14E shows the MCH amount 9 days after treatment with GalNAc-conjugated TfR2 siRNA in a mouse model of anemia associated with loss of bone-marrow function. Figure 14F shows the number of RBCs 9 days after treatment with GalNAc-conjugated TfR2 siRNA in a mouse model of anemia associated with loss of bone-marrow function.

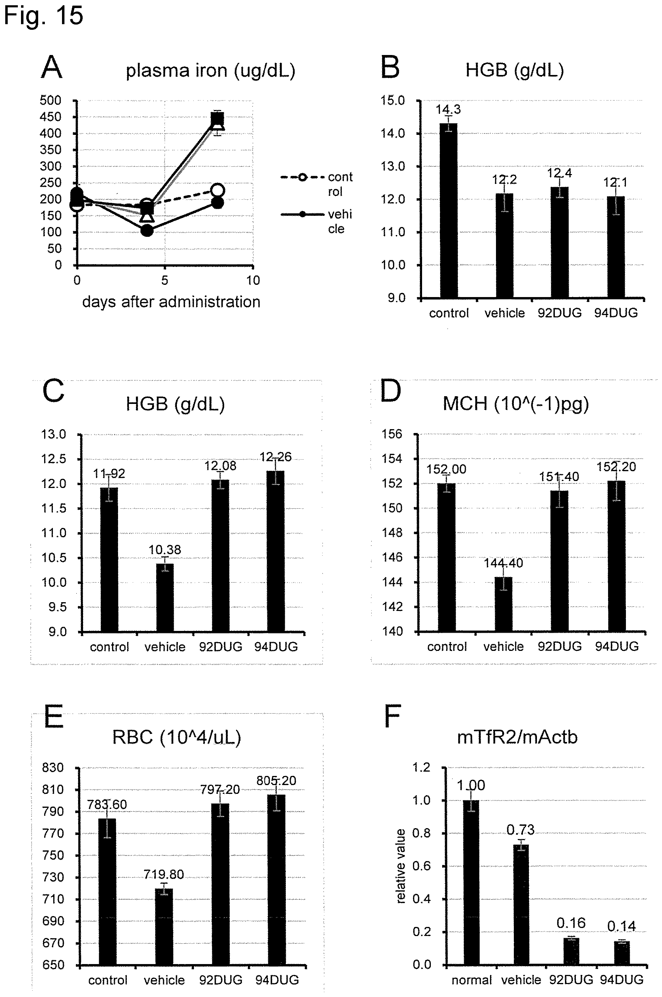

[Figure 15] Figure 15A shows the change in the plasma iron concentration after treatment with GalNAc-conjugated TfR2 siRNA in a mouse model of anemia associated with inflammation. Figure 15B shows the HGB concentration before treatment with GalNAc-conjugated TfR2 siRNA in a mouse model of anemia associated with inflammation. Figure 15C shows the HGB concentration 8 days after treatment with GalNAc-conjugated TfR2 siRNA in a mouse model of anemia associated with inflammation. Figure 15D shows the MCH amount 8 days after treatment with GalNAc-conjugated TfR2 siRNA in a mouse model of anemia associated with inflammation. Figure 15E shows the number of RBCs 8 days after treatment with GalNAc-conjugated TfR2 siRNA in a mouse model of anemia associated with inflammation. Figure 15F shows the liver mTfR2 mRNA expression level 8 days after treatment with GalNAc-conjugated TfR2 siRNA in a mouse model of anemia associated with inflammation.

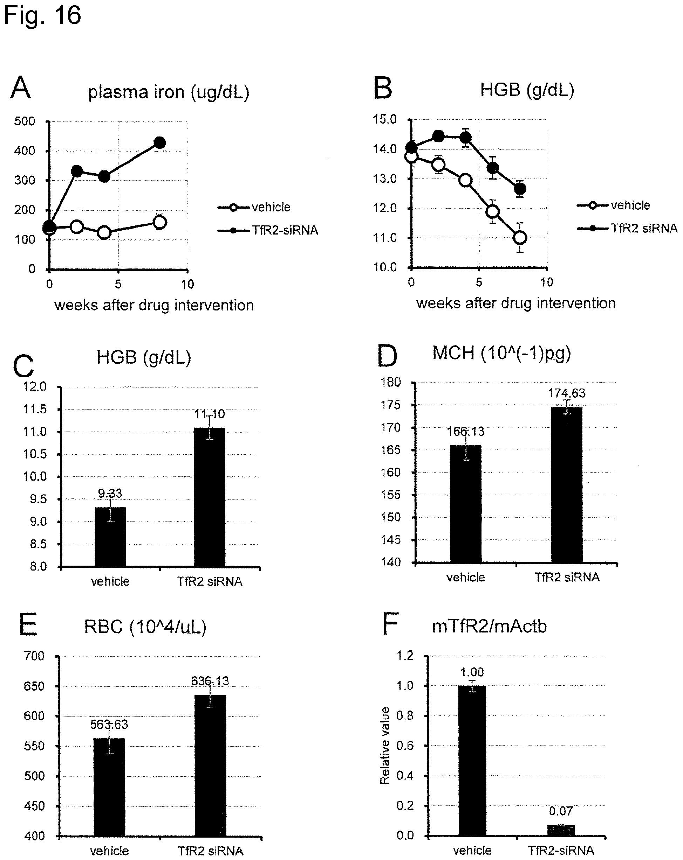

[Figure 16] Figure 16A shows the change in plasma iron concentration after treatment with GalNAc-conjugated TfR2 siRNA in a mouse model of MDS. Figure 16B shows the change in HGB concentration after treatment with GalNAc-conjugated TfR2 siRNA in a mouse model of MDS. Figure 16C shows the HGB concentration 8 weeks after the start of treatment with GalNAc-conjugated TfR2 siRNA in a mouse model of MDS. Figure 16D shows the MCH amount 8 weeks after the start of treatment with GalNAc-conjugated TfR2 siRNA in a mouse model of MDS. Figure 16E shows the number of RBCs 8 weeks after the start of treatment with GalNAc-conjugated TfR2 siRNA in a mouse model of MDS. Figure 16F shows the liver mTfR2 mRNA expression level 8 weeks after the start of treatment with GalNAc-conjugated TfR2 siRNA in a mouse model of MDS.

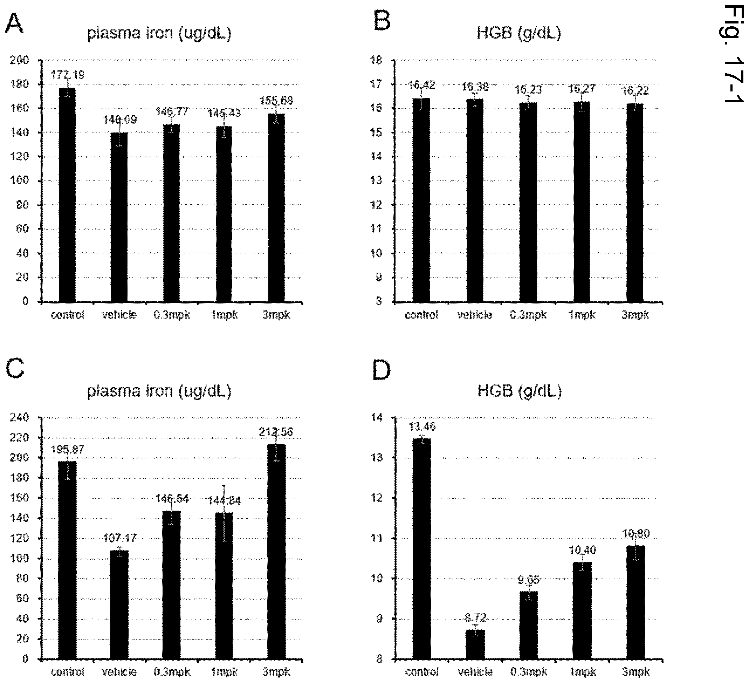

[Figure 17-1] Figure 17A shows the plasma iron concentration before treatment with GalNAc-conjugated TfR2 siRNA in a mouse model of anemia associated with chronic kidney disease. Figure 17B shows the HGB concentration before treatment with GalNAc-conjugated TfR2 siRNA in a mouse model of anemia associated with chronic kidney disease. Figure 17C shows the plasma iron concentration 6 weeks after the start of treatment with GalNAc-conjugated TfR2 siRNA in a mouse model of anemia associated with chronic kidney disease. Figure 17D shows the HGB concentration 6 weeks after the start of treatment with GalNAc-conjugated TfR2 siRNA in a mouse model of anemia associated with chronic kidney disease.

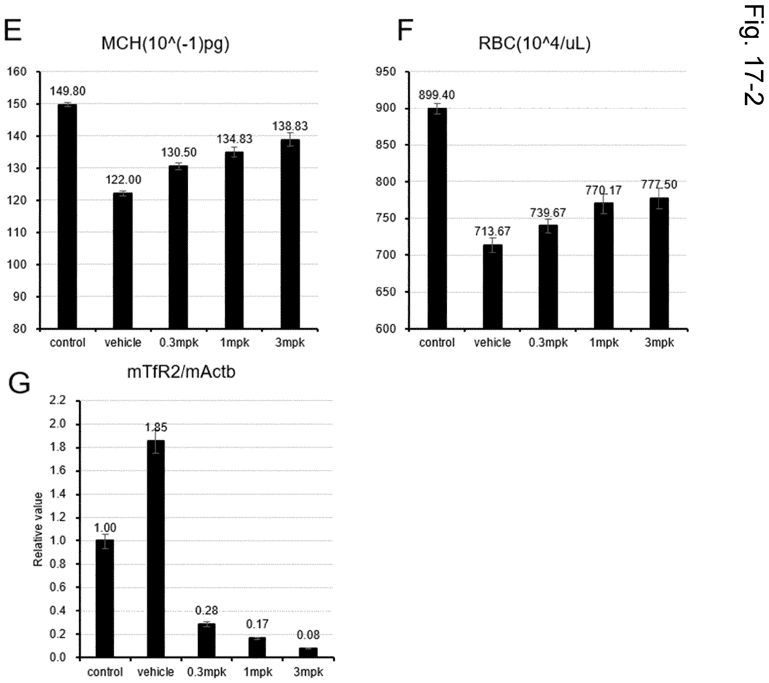

[Figure 17-2] Figure 17E shows the MCH amount 6 weeks after the start of treatment with GalNAc-conjugated TfR2 siRNA in a mouse model of anemia associated with chronic kidney disease. Figure 17F shows the number of RBCs 6 weeks after the start of treatment with GalNAc-conjugated TfR2 siRNA in a mouse model of anemia associated with chronic kidney disease. Figure 17G shows the liver mTfR2 mRNA expression level 6 weeks after the start of treatment with GalNAc-conjugated TfR2 siRNA in a mouse model of anemia associated with chronic kidney disease.

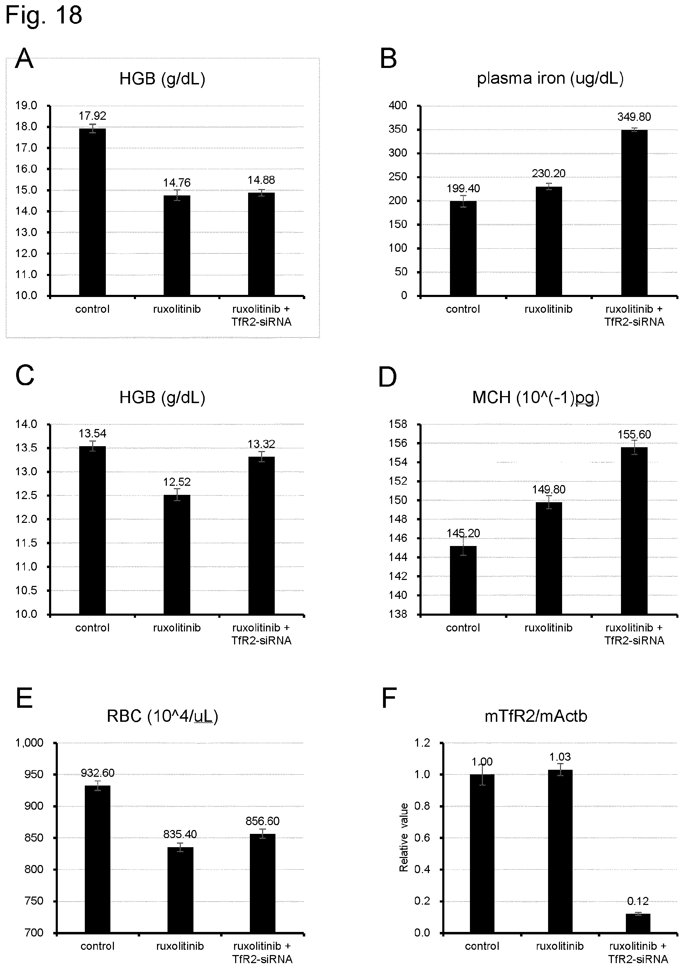

[Figure 18] Figure 18A shows the HGB concentration before treatment with GalNAc-conjugated TfR2 siRNA in a mouse model of anemia caused by ruxolitinib. Figure 18B shows the plasma iron concentration 3 weeks after the start of treatment with GalNAc-conjugated TfR2 siRNA in a mouse model of anemia caused by ruxolitinib. Figure 18C shows the HGB concentration 6 weeks after the start of treatment with GalNAc-conjugated TfR2 siRNA in a mouse model of anemia caused by ruxolitinib. Figure 18D shows the MCH amount 6 weeks after the start of treatment with GalNAc-conjugated TfR2 siRNA in a mouse model of anemia caused by ruxolitinib. Figure 18E shows the number of RBCs 6 weeks after the start of treatment with GalNAc-conjugated TfR2 siRNA in a mouse model of anemia caused by ruxolitinib. Figure 18F shows the liver mTfR2 mRNA expression level 6 weeks after the start of treatment with GalNAc-conjugated TfR2 siRNA in a mouse model of anemia caused by ruxolitinib.

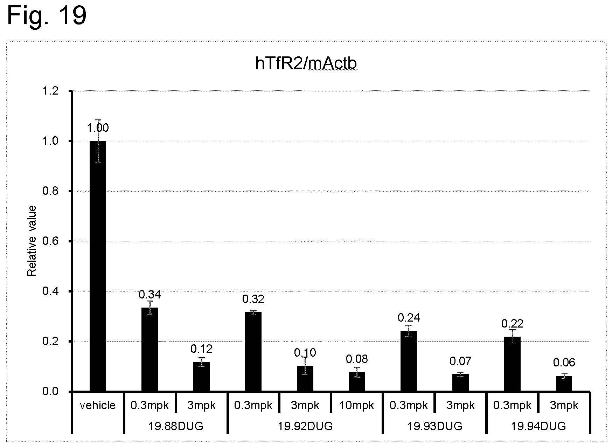

[Figure 19] Figure 19 shows the liver hTfR2 mRNA expression level 7 days after treatment with GalNAc-conjugated TfR2 siRNA in hTfR2 Tg mice.

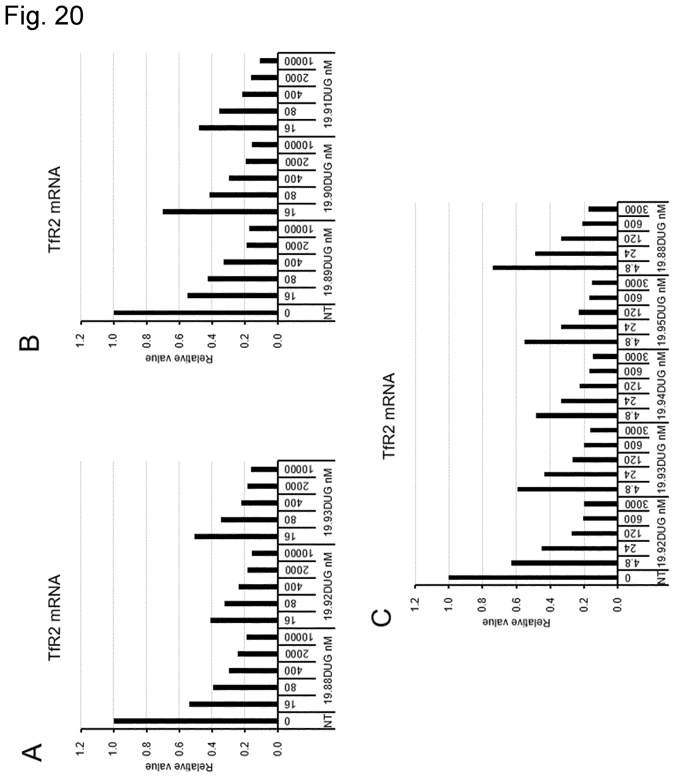

[Figure 20] Figure 20 shows the hTfR2 mRNA expression level after treatment with GalNAc-conjugated TfR2 siRNA in human primary liver cells.

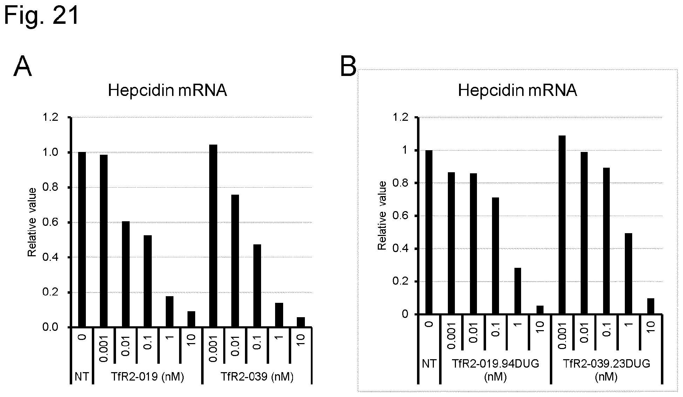

[Figure 21] Figure 21A shows the hepcidin mRNA expression suppressing activity of TfR2-019 and TfR2-039 in HepG2 cells. Figure 21B shows the hepcidin mRNA expression suppressing activity of TfR2-019.94DUG and TfR2-039.23DUG in HepG2 cells.

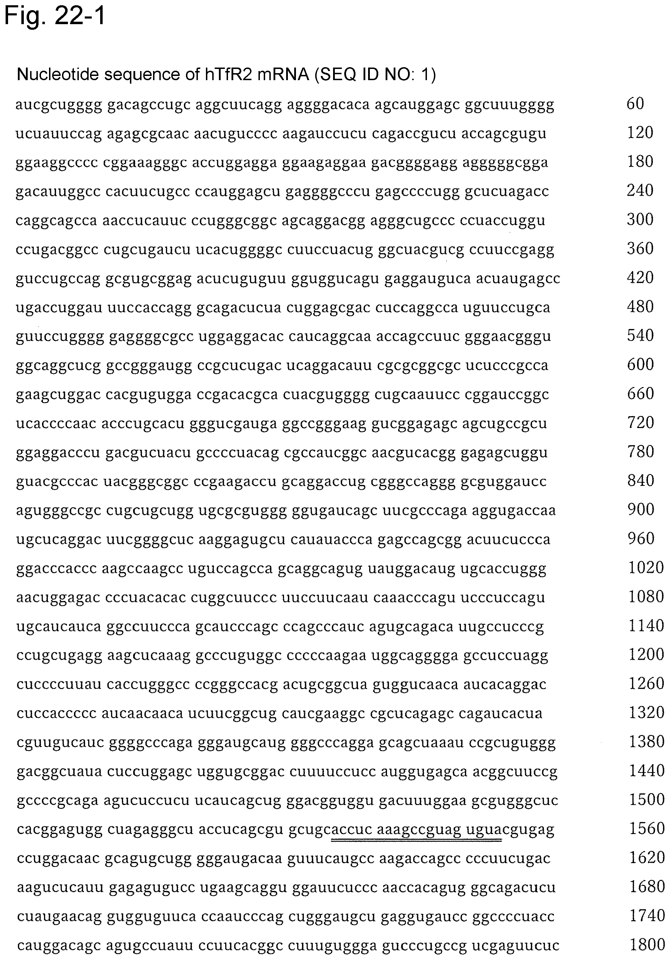

[Figure 22-1] Figure 22-1 shows the nucleotide sequence of mRNA of the gene encoding human transferrin receptor 2 (hTfR2) (SEQ ID NO: 1). The underlined sequence (the 2847th-to-2865th sequence) indicates the sequence targeted by TfR2-019, and the double-underlined sequence (the 1536th-to-1554th sequence) indicates the sequence targeted by TfR2-039.

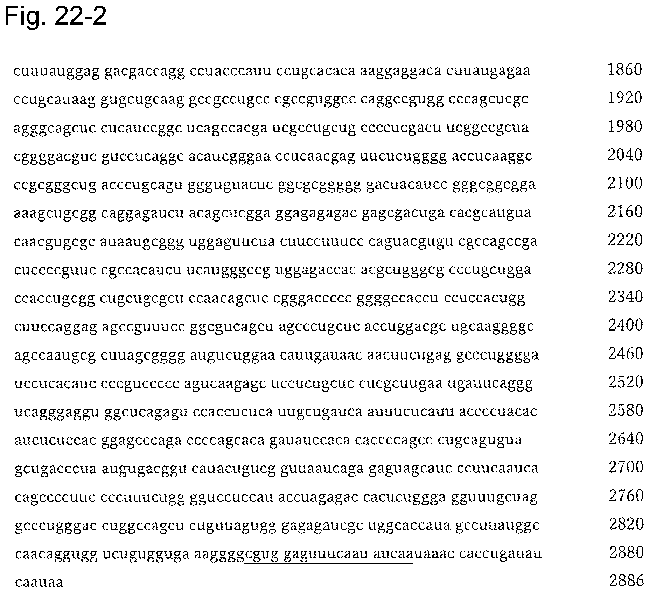

[Figure 22-2] Figure 22-2 shows a continuation of Figure 22-1.

### Description of Embodiments

### <1. Description of terms>

In the present specification, the term "target gene" refers to mRNA corresponding to a gene encoding transferrin receptor 2 (TfR2), and may be immature mRNA before undergoing RNA processing (also referred to as a mRNA precursor, or pre-mRNA), or mRNA matured by undergoing RNA processing. The RNA processing includes, for example, RNA splicing, formation of a cap structure at the 5'-terminus, and formation of a poly-A tail moiety at the 3'-terminus.

TfR2 is a type 2 membrane protein expressed mainly in the liver, and is known to consist of an intracellular region, a transmembrane region, and an extracellular region (The International Journal of Biochemistry & Cell Biology 35 (2003) 292-296). TfR2 is known to play a key role in production of hepcidin, which has an important role in iron metabolism, and control of iron metabolism (Front. Pharmacol., 06 March 2014, Haematologica 2011; 96(4): 500-506., Blood. 2011; 117(10): 2960-2966).

The term "anemia" refers to a pathological condition in which the concentration of hemoglobin existing in erythrocytes in the blood and having the ability to transport oxygen decreases. Anemia is involved in a decline in physical activity, and an increase in mortality (Ann N Y Acad Sci. 2019 August; 1450(1): 15-31). Anemia is classified into various types according to the cause thereof, clinical characteristics, the erythrocyte index such as a mean corpuscular volume (MCV) (The Journal of the Japanese Society of Internal Medicine, Vol. 104, No. 7: 1375-1382, The Guideline for Laboratory Medicine JSLM 2015: 175-180), and the like. Anemia is considered to be caused mainly by a deficiency in production of erythrocytes, excessive destruction of erythrocytes, iron deficiency and the like, which are known to be intricately linked to a variety of pathological conditions such as defective iron utilization associated with chronic inflammation and the like, and bone-marrow dysfunction (Ann N Y Acad Sci. 2019 August; 1450(1): 15-31.).

For anemia associated with chronic inflammation, it is known that a permanent increase in inflammatory cytokines such as interleukin 6 (IL6) leads to an increase in blood concentration of hepcidin, which plays a major role in iron metabolism, which causes defective iron utilization, resulting in a decline in hematopoietic function (Med Clin North Am. 2017 March; 101(2): 285-296.). Examples of the cause of such chronic inflammation include chronic diseases such as autoimmune diseases, infectious diseases, inflammatory enteric diseases, heart failure, chronic kidney disease, and cancer. Examples of anemia associated with chronic inflammation include anemia associated with an autoimmune disease such as rheumatoid arthritis, systemic erythematosus or autoimmune hematolytic anemia, anemia associated with an infectious disease, anemia associated with macrophage activation syndrome, anemia associated with Castleman disease, anemia associated with an inflammatory enteric disease such as inflammatory colitis, anemia associated with heart failure, anemia associated with chronic kidney disease, and cancerous anemia. The cancerous anemia includes, for example, anemia associated with cancer such as multiple myeloma, acute leukemia, chronic leukemia, lung cancer or breast cancer.

For anemia associated with bone-marrow dysfunction, the ability to produce erythrocytes is significantly impaired by some factors, leading to anemia. Examples of anemia associated with bone-marrow dysfunction include anemia associated with myelodysplastic syndrome, anemia associated with myelofibrosis, anemia associated with chronic myelomonocytic leukemia (CMML), anemia associated with chemotherapeutic myelosuppression, aplastic anemia, Diamond-Blackfan anemia, and Fanconi anemia. Myelofibrosis is a disease in which a gene variant such as JAK2V617F is generated in hematopoietic stem cells a posteriori, and thus abnormal hematopoietic stem cell-derived clones proliferate, so that there is a permanent increase in inflammation and fibrosis of the bone marrow, resulting in loss of bone-marrow function. Myelofibrosis is known to produce severe anemia, enlargement of the spleen, and the like (Am J Hematol. 2021: 96: 145-162.).

In the present specification, nucleic acid or nucleic acid molecule is a general term for nucleosides, nucleotides, oligonucleotides and polynucleotides.

The term "nucleoside" means a chemical structure moiety in which a base moiety important for genetic information and a sugar moiety important for physicochemical properties of the molecule are bound.

The term "nucleotide" refers to a compound in which the hydroxyl group at the 3'-position (2'-position in the case of modification at the 3'-position) of a sugar moiety of a nucleoside forms an ester with a phosphate group.

The term "oligonucleotide" refers to an oligomer having a structure in which there are two or more nucleotides, and the phosphate group moiety of one of the nucleotides and the hydroxyl group at the 5'-position of the sugar moiety of another nucleotide are bound (phosphodiester bond). However, the residues at the 3'-position (2'-position in the case of a nucleotide modified at the 3'-position) of the nucleotide at the 3'-terminus and/or the 5'-position of the nucleotide at the 5'-terminus may be hydroxyl groups or phosphate groups, and these groups may be chemically modified. The phosphodiester bond between the nucleosides may be chemically modified. In the present specification, the term "oligonucleotide" and the term "polynucleotide" are used interchangeably with each other.

The base moiety of a nucleic acid is adenine (A), guanine (G), cytosine (C), uracil (U) or thymine (T), A and U or T, G and C, respectively, forms Watson-Crick base pairs through a hydrogen bond, and the relationship between bases which form a base pair is referred to as "complementary". Each base moiety may be chemically modified, and the modified base may have the same ability to form a base pair as the original base. As typical modified bases, 5-methylcytosine (5C), 5-methyluracil (5U) and the like are known. 5U is identical in structure to T. In the sequence listing attached to the present specification, 5C is represented as C, and 5U is represented as T or U.

In the present specification, "symbols for bases (symbols for sugar moieties)" may be used in the notation for nucleic acids. The symbols for sugar moieties will be described in the following sections where various nucleosides are described.

In the present specification, as long as no specific indication is given, the term "modification pattern" of the oligonucleotide means an arrangement of modifications of the sugar moieties of the nucleosides forming an oligonucleotide, independently of the base sequence. If a specific indication is given, this term means a modification pattern further combined with an inter-nucleoside bond form. Since the structure of the sugar moiety and the inter-nucleoside bond are elements that do not depend on a target sequence, and have influences on the stability of molecules, and bond performance associated with the stability, a modification pattern confirmed to have an action and effect on a target sequence is likely to exhibit the same action and effect on another target sequence (see, for example, Mol Ther. (2018) 26: 708-717).

In the present specification, the term "natural nucleoside" refers to a 2'-deoxynucleoside or a ribonucleoside. The 2'-deoxynucleoside (also referred to as "DNA"; the symbol representing a sugar moiety is (D), and may be expressed by a lowercase mark for a base moiety) corresponding to each base has a structure represented by one of the following formulae. 2'-deoxyadenosine may be represented as A(D) or a, 2'-deoxyguanosine may be represented as G(D) or g, 2'-deoxycytidine may be represented as C(D) or c, thymidine may be represented as T(D) or t, 2'-deoxy-5-methylcytidine may be represented as 5C(D) or 5c, and 2'-deoxyuridine may be represented as U(D) or u. A 2-deoxynucleoside whose base is not specified may be represented as N(D). 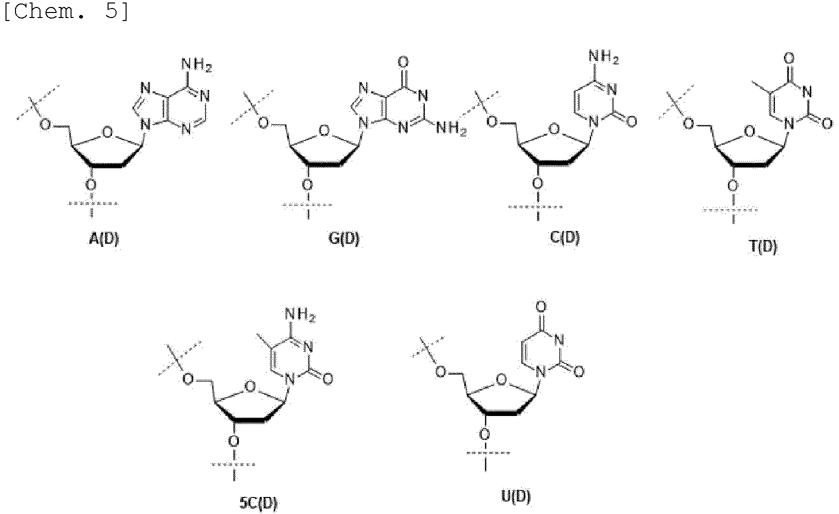 wherein the broken line indicates a point of attachment.

The ribonucleoside (also referred to as "RNA"; the symbol representing a sugar moiety is (R), and may be represented as a capital indicating a base moiety) corresponding to each base has a structure represented by one of the following formulae. Adenosine may be represented as A(R) or A, guanosine may be represented as G(R) or G, cytidine may be represented as C(R) or C, and uridine may be represented as U(R) or U. A ribonucleoside whose base is not specified may be represented as N(R). 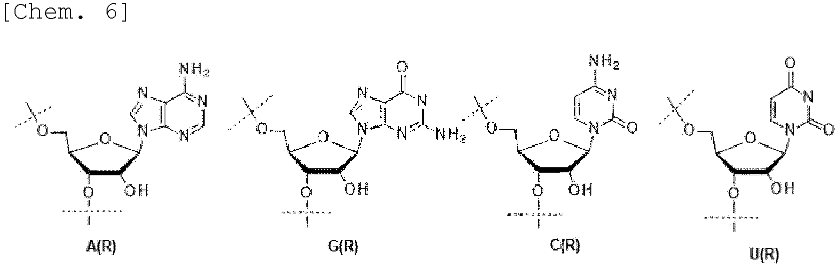 wherein the broken line indicates a point of attachment.

In the present specification, the terms "modified nucleoside" and "modified nucleotide" mean a nucleoside or a nucleotide containing at least one chemically modified structure out of the base moiety, the sugar moiety or the phosphodiester moiety of a natural nucleoside.

In the present specification, the term "phosphodiester bond that may be chemically modified" means a phosphodiester bond adopted as a natural inter-nucleoside bond, or a chemically modified phosphodiester bond. The natural inter-nucleoside bond is a phosphodiester bond represented by the following formula (P), and is shown with no character and symbol between nucleosides, or between a nucleoside and an adjacent structural unit (for example, a GalNAc unit), a chemical modification structure (for example, a later-described structure indicated by "2"), or a chemical linker or an oligonucleotide linker (for example, a later-described structure indicated by "Z") in the display of nucleotide sequences in the present specification. In the present specification, the term "chemically modified phosphodiester bond" means a bond form in which the phosphorus atom contained in the phosphodiester bond is chemically modified. Examples of the chemically modified phosphodiester bond include a phosphorothioate bond represented by the following formula (PS), a phosphoramidate bond that is a modified bond in which a nitrogen atom is added to a phosphorus atom, an alkyl phosphonate bond that is a modified bond in which a replaceable alkyl group is added to a phosphorus atom (for example, a methyl phosphonate bond when the alkyl group is a methyl group), and a phosphotriester bond that is a modified bond in which a replaceable alkyl group is added to an oxygen atom of a noncovalent bond of a phosphate group. When a phosphorothioate bond is adopted, "^" is shown between nucleosides, or between a nucleoside and its adjacent structural unit (for example, a GalNAc unit), a chemical modification structure (for example, a later-described structure indicated by "2"), or a chemical linker or an oligonucleotide linker (for example, a later-described structure indicated by "Z") in the display of nucleotide sequences in the present specification. 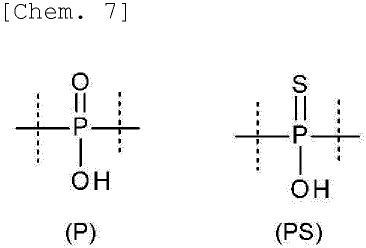 wherein the broken line represents a point of attachment, the point of attachment on one side represents an ester bond formed with the oxygen atom of the 3'-hydroxyl group (oxygen atom at the 2'-position in the case of a nucleoside modified at the 3'-position) of a nucleoside adjacent on the 5' side, an adjacent structural unit, or a chemical linker or an oligonucleotide linker, and the point of attachment on the other side represents an ester bond formed with the oxygen atom of the 5'-hydroxyl group of a nucleoside adjacent on the 3' side, a structural unit, or a chemical linker or an oligonucleotide linker.

In the present specification, the term "sugar-modified nucleoside" refers to a nucleoside in which the sugar moiety of the nucleoside is modified. The sugar-modified nucleoside includes all forms of sugar modification that are known in the technical field to which the present invention belongs. Examples of the sugar-modified nucleoside include 2'-modified nucleosides, 3'-modified nucleosides, 4'-thio-modified nucleosides, 4'-thio-2'-modified nucleosides, and 2'-O,4'-C-bridged modified nucleosides. Examples of preferred sugar modifications are 2'-O-alkylation (methylation, ethylation, propylation, isopropylation, butylation and the like), 2'-O-alkoxyalkylation (methoxyethylation, methoxypropylation, methoxybutylation and the like), 2'-halogenation (chlorination, fluorination and the like), 3'-O-alkylation (methylation, ethylation, propylation, isopropylation, butylation and the like), 3'-O-alkoxyalkylation (methoxyethylation, methoxypropylation, methoxybutylation and the like), 3'-halogenation (chlorination, fluorination and the like), 2'-O,4'-C-bridging (alkylene-bridging and the like), and the like.

In the present specification, the term "2'-modified nucleoside" refers to a nucleoside in which ribofuranose contained in the nucleoside is chemically modified at the 2'-position. Examples of relevant modifications are 2'-O-alkylated (for example, methylated, ethylated, propylated, isopropylated and butylated) nucleosides, 2'-O-alkoxyalkylated (for example, methoxyethylated, methoxypropylated and methoxybutyl) nucleosides, 2'-halogenated (for example, chlorinated and fluorinated) nucleosides, 2'-allylated nucleosides, 2'-aminated nucleosides, 2'-azidated nucleosides, 2'-O-allylated nucleosides, 2'-OCF₃ nucleosides, 2'-O(CH₂)₂SCH₃ nucleosides, 2'-O-(CH₂)₂-O-N-(Rₘ)(Rₙ) nucleosides, or 2'-O-CH₂-C(=O)-N(Rₘ)(Rₙ) nucleosides (each Rₘ and Rₙ are independently H, an amino protective group, or a substituted or unsubstituted C₁-C₁₀ alkyl). The preferred 2'-modified nucleoside is a 2'-O-alkylated nucleoside, a 2'-O-alkoxyalkylated nucleoside, or a 2'-halogenated nucleoside.

Examples of a 2'-O-alkylation modification of the sugar-modified nucleoside include 2'-O-methylnucleoside (sometimes referred to as "2'-OMe RNA" or "2'-O-methyl RNA"; the symbol representing a sugar moiety is (M)). The 2'-O-methylnucleoside corresponding to each base has a structure represented by one of the following formulae: 2'-O-methyladenosine may be represented as A(M), 2'-O-methylguanosine may be represented as G(M), 2'-O-methylcytidine may be represented as C(M), 2'-O-methyl-5-methylcytidine may be represented as 5C(M), 2'-O-methyluridine may be represented as U(M), and 2'-O-methyl-5-methyluridine may be represented as T(M). A 2'-O-methylnucleoside whose base is not specified may be represented as N(M). 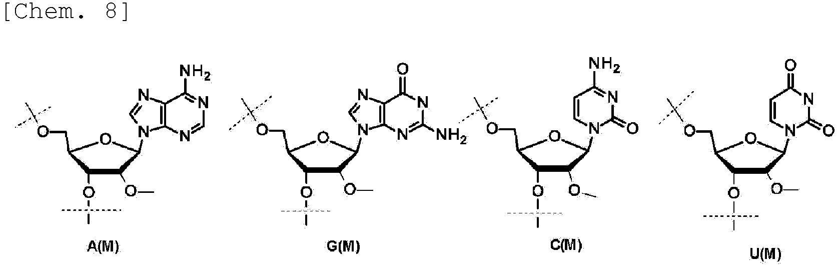 wherein the broken line indicates a point of attachment.

Examples of a 2'-O-alkoxyalkylation modification include 2'-O-methoxyethylnucleosides (sometimes referred to as "2'-MOE RNA", "2'-O-methoxyethyl RNA" or "2'-methoxyethoxy RNA"; the symbol representing a sugar moiety is (m)). The 2'-O-methoxyethylnucleoside corresponding to each base has a structure represented by one of the following formulae: 2'-O-methoxyethyladenosine may be represented as A(m), 2'-O-methoxyethylguanosine may be represented as G(m), 2'-O-methoxyethyl-5-methylcytidine may be represented as C(m) (written as C(m) for convenience although the structure of the base is 5C; 2'-O-methoxyethylcytidine can also be used in replacement of 2'-O-methoxyethyl-5-methylcytidine), 2'-O-methoxyethyluridine may be represented as U(m), and 2'-O-methoxyethyl-5-methyluridine may be represented as T(m). A 2'-O-methoxyethylnucleoside whose base is not specified may be represented as N(m). 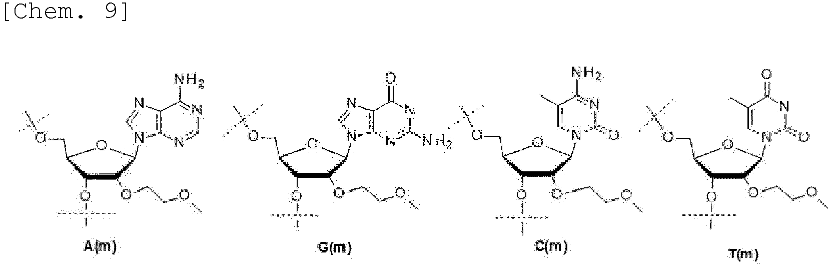 wherein the broken line indicates a point of attachment.

Examples of a 2'-halogenation modification include 2'-deoxy-2'-fluoronucleosides ("sometimes referred to as "2'-F RNA" or "2'-deoxy-2'-fluoro RNA"; the symbol representing a sugar moiety is (F)). The 2'-deoxy-2'-fluoronucleoside corresponding to each base has a structure represented by one of the following formulae: 2'-deoxy-2'-fluoroadenosine may be represented as A(F), 2'-deoxy-2'-fluoroguanosine may be represented as G(F), 2'-deoxy-2'-fluorocytidine may be represented as C(F), 2'-deoxy-2'-fluoro-5-methylcytidine may be represented as 5mC(F), 2'-deoxy-2'-fluorouridine may be represented as U(F), and 2'-deoxy-2'-fluoro-5-methyluridine may be represented as T(F). A 2'-deoxy-2'-fluoronucleoside whose base is not specified may be represented as N(F). 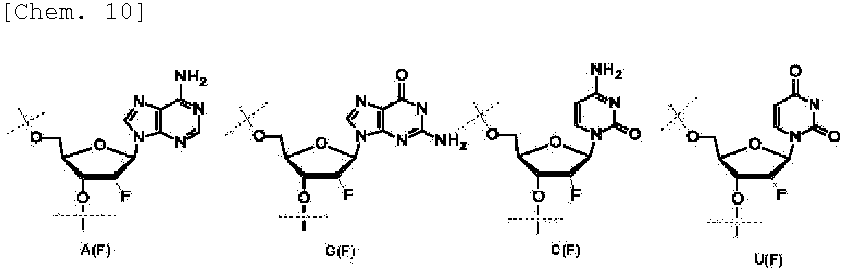 wherein the broken line indicates a point of attachment.

In the present specification, the term "3'-modified nucleoside" refers to a nucleoside in which ribofuranose contained in the nucleoside is chemically modified at the 3'-position. The hydroxyl group at the 2'-position in the 3'-modified nucleoside may be modified. Examples of a relevant modification are 3'-O-alkylated (for example, methylated, ethylated, propylated, isopropylated and butylated) nucleosides, 3'-O-alkoxyalkylated (for example, methoxyethylated, methoxypropylated and methoxybutylated) nucleosides, 3'-halogenated (for example, chlorinated and fluorinated) nucleosides, 3'-allylated nucleosides, 3'-aminated nucleosides, 3'-azidated nucleosides, 3'-O-allylated nucleosides, 3'-OCF₃ nucleosides, 3'-O(CH₂)₂SCH₃ nucleosides, 3'-O-(CH₂)₂-O-N-(Rₘ)(Rₙ) nucleosides, or 3' -O-CH₂-C(=O)-N(Rₘ)(Rₙ) nucleosides (each Rₘ and Rₙ are independently H, an amino protective group, or a substituted or unsubstituted C₁-C₁₀ alkyl). The preferred 3'-modified nucleoside is a 3'-O-alkylated nucleoside, a 3'-O-alkoxyalkylated nucleoside, or a 3'-halogenated nucleoside. The above-described nucleosides and the like can be exemplified.

Examples of a 3'-O-alkylation modification of a 3'-modified nucleoside include 3'-O-methylnucleoside (sometimes referred to as "3'-OMe RNA"; the symbol representing a sugar moiety is (3M)). The 3'-O-methylnucleoside corresponding to each base has a structure represented by one of the following formulae: 3'-O-methyladenosine may be represented as A(3M), 3'-O-methylguanosine may be represented as G(3M), 3'-O-methylcytidine may be represented as C(3M), 3'-O-methyl-5-methylcytidine may be represented as 5mC(3M), 3'-O-methyluridine may be represented as U(3M), and 3'-O-methyl-5- methyluridine may be represented as T(3M). A 3'-O-methylnucleoside whose base is not specified may be represented as N(3M). 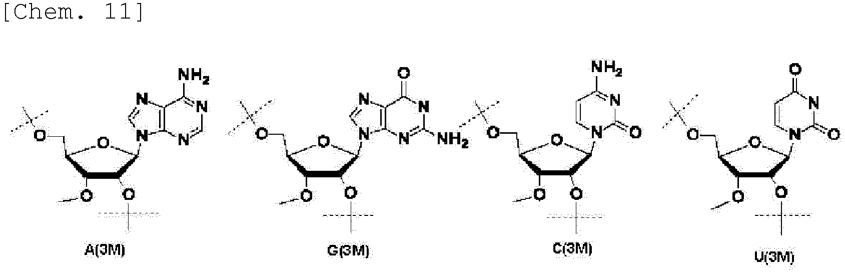 wherein the broken line indicates a point of attachment.

Examples of a 3'-O-alkoxyalkylation modification include 3'-O-methoxyethylnucleosides (sometimes referred to as "3'-MOE RNA", "3'-O-methoxyethyl RNA" or "3'-methoxyethoxy RNA"; the symbol representing a sugar moiety is (3m)). The 3'-O-methoxyethylnucleoside corresponding to each base has a structure in which a methoxyethyl group is bonded in place of the methyl group bonded to the oxygen atom at the 3'-position in the structural formula of the 3'-O-methylnucleoside corresponding to each base. For the 3'-O-methoxyethylnucleoside corresponding to each base, 3'-O-methoxyethyladenosine may be represented as A(3m), 3'-O-methoxyethylguanosine may be represented as G(3m), 3'-O-methoxyethyl-5-methylcytidine may represented as C(3m) (written as C(3m) for convenience although the structure of the base is 5C; 3'-O-methoxyethylcytidine can also be used in replacement of 3'-O-methoxyethyl-5-methylcytidine), 3'-O-methoxyethyluridine may be represented as U(3m), and 3'-O-methoxyethyl-5-methyluridine may be represented as T(3m). A 3'-O-methoxyethylnucleoside whose base is not specified may be represented as N(3m).

Examples of a 3'-halogenation modification include 3'-deoxy-3'-fluoronucleosides (sometimes referred to as "3'-F RNA" or "3'-deoxy-3'-fluoro RNA"; the symbol representing a sugar moiety is (3F)). The 3'-deoxy-3'-fluoronucleoside corresponding to each base has a structure in which a fluorine atom is bonded in place of the methoxy group bonded to the carbon atom at the 3'-position in the structural formula of the 3'-O-methylnucleoside corresponding to each base. For the 3'-deoxy-3'-fluoronucleoside corresponding to each base, 3'-deoxy-3'-fluoroadenosine may be represented as A(3F), 3'-deoxy-3'-fluoroguanosine may be represented as G(3F), 3'-deoxy-3'-fluorocytidine may be represented as C(3F), 3'-deoxy-3'-fluoro-5-methylcytidine may be represented as 5mC(3F), 3'-deoxy-3'-fluorouridine may be represented as U(3F), the 3'-deoxy-3'-fluoro-5-methyluridine may be represented as T(3F). A 3'-deoxy-3'-fluoronucleoside whose base is not specified may be represented as N(3F).

In the present specification, the term "4'-thio-modified nucleoside" refers to a nucleoside in which the oxygen atom at the 4'-position of a ribofuranose contained in the nucleoside is replaced by a sulfur atom. Examples of a 4'-thio-modified nucleoside can include β-D-ribonucleosides in which the 4'-oxygen atom is replaced by a sulfur atom (Hoshika, S. et al. FEBS Lett. 579, p.3115-3118, (2005); Dande, P. et al. J. Med. Chem. 49, p.1624-1634 (2006); Hoshika, S. et al. ChemBioChem. 8, p.2133-2138, (2007)).

In the present specification, the term "4'-thio-2'-modified nucleoside" refers to a nucleoside in which a ribofuranose contained in the nucleoside is chemically modified at the 2'-position, and the oxygen atom at the 4'-position of the ribofuranose is replaced by a sulfur atom. Examples of the 4'-thio-2'-modified nucleoside can include 4'-thio-2'-modified nucleosides holding 2'-H or 2'-O-methyl (Matsugami, et al. Nucleic Acids Res. 36, 1805 (2008)).

Examples of a 2'-O,4'-C-bridging modification for the sugar-modified nucleoside include 2'-O,4'-C-ethylene-bridged nucleosides (sometimes referred to as "ENA" or an "ENA unit"; the symbol representing a sugar moiety is (E)) (Morita, K. et al. Bioorg. Med. Chem. Lett., 12, p.73 (2002).; Morita, K. et al. Bioorg. Med. Chem., 11, p.2211 (2003).). The 2'-O,4'-C-ethylene-bridged nucleoside corresponding to each base has a structure represented by one of the following formulae: 2'-O,4'-C-ethylene-bridged adenosine may be represented as A(E), 2'-O,4'-C-ethylene-bridged guanosine may be represented as G(E), 2'-O,4'-C-ethylene-bridged-5-methylcytidine may be represented as C(E) (written as C(E) for convenience although the structure of the base is 5C; 2'-O,4'-C-ethylene-bridged cytidine can also be used in replacement of 2'-O,4'-C-ethylene-bridged-5-methylcytidine), 2'-O,4'-C-ethylene-bridged uridine may be represented as U(E), and 2'-O,4'-C-ethylene-bridged-5-methyluridine may be represented as T(E). A 2'-O,4'-C-ethylene-bridged nucleoside whose base is not specified may be represented as N(E). 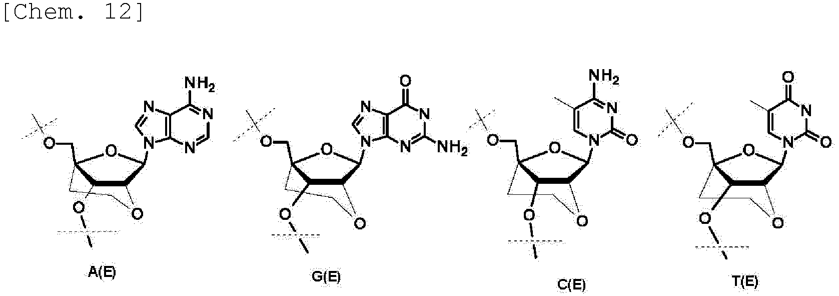 wherein the broken line indicates a point of attachment.

Another example of a 2'-O,4'-C-bridging modification is, for example, a 2'-O,4'-C-methylene-bridged nucleoside (sometimes referred to as "LNA" or a "LNA unit"; the symbol representing a sugar moiety is (L)) (Obika, S. et al. Tetrahedron Lett., 38, p.8735- (1997).; Obika, S. et al., Tetrahedron Lett., 39, p.5401- (1998).; A. A. Koshkin, A. A. et al. Tetrahedron, 54, p.3607 (1998).; Obika, S. Bioorg. Med. Chem., 9, p.1001 (2001).). The 2'-O,4'-C-methylene-bridged nucleoside corresponding to each base has a structure represented by one of the following formulae: 2'-O,4'-C-methylene-bridged adenosine may be represented as A(L), 2'-O,4'-C-methylene-bridged guanosine may be represented as G(L), 2'-O,4'-C-methylene-bridged-5-methylcytidine may be represented as C(L) (written as C(L) for convenience although the structure of the base is 5C; 2'-O,4'-C-methylene-bridged cytidine can also be used in replacement of 2'-O,4'-C-methylene-bridged-5-methylcytidine), 2'-O,4'-C-methylene-bridged uridine may be represented as U(L), and 2'-O,4'-C-methylene-bridged-5-methyluridine may be represented as T(L). A 2'-O,4'-C-methylene-bridged nucleoside whose base is not specified may be represented as N(L). 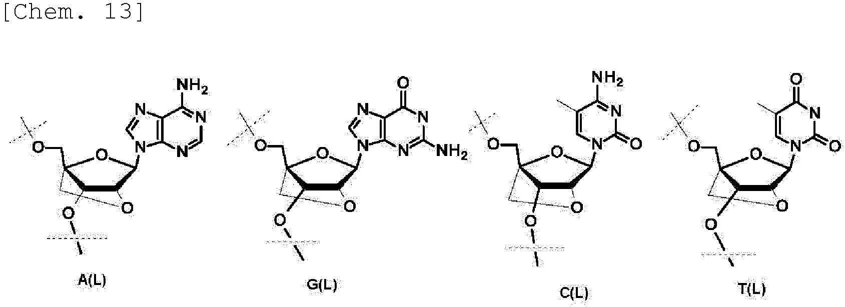 wherein the broken line indicates a point of attachment.

The oligonucleotide of the present invention or a salt thereof has an antisense strand region (sometimes referred to simply as an antisense strand) having a nucleotide sequence substantially complementary to the nucleotide sequence of a target sequence, and a sense strand region (sometimes referred to simply as a sense strand) having a nucleotide sequence substantially complementary to the nucleotide sequence of the complementary region of the antisense strand region. The sense strand region and the antisense strand region form a double-stranded structure in the nucleotide sequence of the complementary region, where the sense strand region and the antisense strand region are independent oligonucleotides and form a double-stranded oligonucleotide, or the 5'-position of the nucleotide at the 5'-terminus of one of the sense strand region and the antisense strand region and the 3'-position (2'-position in the case of a nucleotide modified at the 3'-position) of the nucleotide at the 3'-terminus of the other may be connected to form a single-stranded oligonucleotide. That is, the oligonucleotide of the present invention may be a bimolecular oligonucleotide, or a monomolecular oligonucleotide. When the oligonucleotide of the present invention is a monomolecular oligonucleotide, the form of connection between the sense strand region and the antisense strand region is not limited as long as the oligonucleotide of the present invention has an RNA interfering action and/or a gene expression suppressing action. Examples thereof include connection between the 5'-position of the nucleotide at the 5'-terminus of one of the sense strand region and the antisense strand region and the 3'-position (2'-position in the case of a nucleotide modified at the 3'-position) of the nucleotide at the 3'-terminus of the other via a desired chemical linker or oligonucleotide linker. Examples of such a linker include linkers described in WO2012/074038, and preferably a linker represented by the following formula (Z). The linker represented by the following formula (Z) can be appropriately adjusted according to WO 2012/074038. 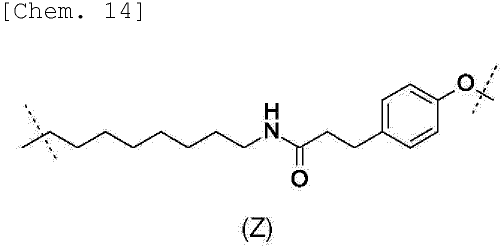 wherein the broken line represents a point of attachment, and means that the oxygen atom bonded to the phenyl group forms a phosphodiester bond with the 5'-phosphate group of the nucleotide at the 5'-terminus of an adjacent antisense region, and the methylene group at the other terminus forms a phosphodiester bond with a 3'-phosphate group (phosphate group at the 2'-position in the case of a nucleotide modified at the 3'-position) of the nucleotide at the 3'-terminus of an adjacent sense strand region, and each phosphodiester bond formed may be a chemically modified phosphodiester bond such as a phosphorothioate bond.

The residue at the 3'-position (2'-position in the case of a nucleotide modified at the 3'-position) of the nucleotide at the 3'-terminus and/or the residue at the 5'-position of the nucleotide at the 5'-terminus may be a hydroxyl group or a phosphate group, or may have a structure obtained by chemical modification of the hydroxyl group or the phosphate group. Examples of the structure obtained by chemical modification include a structure represented by the following formula (2). 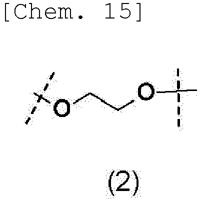 wherein the broken line represents a point of attachment, the point of attachment on one side means that an oxygen atom is bonded to an adjacent nucleotide, where the oxygen atom forms a phosphodiester bond with the 5'-phosphate group when bonded to the nucleotide at the 5'-terminus, and with the 3'-phosphate group (2'-phosphate group in the case of a nucleotide modified at the 3'-position) when bonded to the nucleotide at the 3'-terminus, and the point of attachment at the other side means that the oxygen atom is bonded to a hydrogen atom to form a hydroxyl group, and the phosphodiester bond formed may be a chemically modified phosphodiester bond such as a phosphorothioate bond.

The term "substantially identical nucleotide sequence" in the present specification includes not only a nucleotide sequence consisting of bases that are all identical to those of a nucleotide sequence of interest, but also a nucleotide sequence having a sequence identity of 70% or more with a nucleotide sequence of interest, and a nucleotide sequence which contains one or several non-identical bases, but enables oligonucleotides to exhibit a desired function. Here, the term "identical base" includes a base fully identical to an original base, and a base equivalent or superior to an original base in its ability to form a in base pair. As such a base, for example, a chemically modified base, which is equivalent or superior to an original base in its ability to form a base pair , is considered identical to the original base (for example, C and 5C, or U and T(5U)). The term "sequence identity" refers to a sequence having an identity of preferably 80% or more, more preferably 90% or more, even more preferably 91%, 92%, 93%, 94%, 95%, 96%, 97%, 98% or 99%, or a mismatch in 3 bases, 2 bases or 1 base, with a nucleotide sequence of interest. The identity of a nucleotide sequence can be calculated using known gene analysis software such as BLAST (registered trademark).

The term "substantially complementary nucleotide sequence" in the present specification includes not only a nucleotide sequence consisting of nucleotides that are all complementary to those of a nucleotide sequence of interest, but also a nucleotide sequence in which one or several (for example, five, four, three or two) nucleotides are not complementary nucleotides, but the nucleotide sequence allows oligonucleotides to form a base pair. In the present specification, the term "complementary base" refers to a base capable of forming a Watson-Crick base pair to a base of interest, and the base may be an unmodified base, or a chemically modified base. For example, 5-methyluracil and adenine are bases complementary to each other, and 5-methylcytosine and guanine are bases complementary to each other.

The term "double-stranded structure" of oligonucleotides in the present specification means that nucleotide sequences substantially complementary to each other form a Watson-Crick base pairs to give a structure with double strands. The nucleotide sequences forming a double-stranded structure may be substantially complementary nucleotide sequences between two different oligonucleotide molecules, or substantially complementary sequences in a single-stranded oligonucleotide.

In the present specification, the term "structural unit" refers to a chemical structural unit that imparts a desired function to an oligonucleotide. Examples of a desired function imparted to an oligonucleotide include a function of delivering an oligonucleotide to target tissues or target cells (also referred to as target tissues or the like), and a function of improving kinetics of an oligonucleotide in the body. The structural unit can be bound to an oligonucleotide by a known method, and for example, by providing an amidite structure in the structural unit, the structural unit can be bound to the 3'-position (2'-position in the case of a nucleotide modified at the 3'-position) of the nucleotide at the 3'-terminus or the 5'-position of the nucleotide at the 5'-terminus of the oligonucleotide. The ability of delivery to target tissues or the like can be imparted to an oligonucleotide by providing a structure for promoting delivery to a target tissue or the like, such as a ligand, in the structural unit, and examples of the structure include a GalNAc unit for delivery to liver cells, and a fatty acid unit for delivery to the liver and muscular tissues. In the present specification, such a structural unit for imparting the ability of delivery to target tissues or the like may be referred to, in particular, as a "unit for delivery".

### <2. RNAi-oligonucleotide>

In the present specification, the term "RNAi-oligonucleotide" refers to an oligonucleotide in which substantially complementary nucleotides contained in a sense strand region and an antisense strand region form Watson-Crick base pairs, and the oligonucleotide contains complementary regions forming a double-stranded structure, and has an RNA interfering action and/or a gene expression suppressing action on a target gene. As long as the oligonucleotide has an RNA interfering action and/or a gene expression suppressing action on a target gene, not all the bases of the complementary region contained in the RNAi-oligonucleotide are required to form a Watson-Crick base pair. In the present specification, the RNAi-oligonucleotide may be referred to as "siRNA".

In the present specification, the term "nucleotide sequence substantially identical to a target sequence" refers to a nucleotide sequence identical to a target sequence, and includes not only a sequence fully identical to a target sequence, but also a nucleotide sequence substantially identical to a target sequence as long as the RNAi-oligonucleotide has an RNA interfering action and/or a gene expression suppressing action on a target gene.

In the present specification, the term "nucleotide sequence substantially complementary to a target sequence" refers to a nucleotide sequence complementary to a target sequence, and includes not only a sequence fully complementary to a target sequence, but also a nucleotide sequence substantially complementary to a target sequence as long as the RNAi-oligonucleotide has an RNA interfering action and/or a gene expression suppressing action on a target gene.

The oligonucleotide containing a nucleotide sequence substantially complementary to a target sequence and having an RNA interfering action and/or a gene expression suppressing action on a target gene refers to an RNAi-oligonucleotide for a target gene.

The nucleotide sequence of the RNAi-oligonucleotide for a target gene is not limited as long as the oligonucleotide has an RNA interfering action and/or a gene expression suppressing action on the target gene. It can be determined on the basis of sequences predicted to have an RNA interfering action and/or a gene expression suppressing action on the target gene by using, for example, computer software (for example, GENETYX (registered trademark): manufactured by GENETYX CORPORATION). It is also possible to perform the determination by further confirming the RNA interfering action and/or gene expression suppressing action of the RNAi-oligonucleotide prepared on the basis of the selected sequences.

In the present specification, the gene expression suppressing action includes not only an action of fully suppressing the expression of a gene, but also an action of reducing the expression of a gene as compared to a control, and the gene expression suppressing action also includes gene silencing. In the present specification, the term "gene expression suppressing action" and the term "gene expression suppressing activity" are used interchangeably with each other.

The RNA interfering action and/or gene expression suppressing action can be confirmed by a method that is normally carried out by those skilled in the art. For example, a protein that is a translated product of a target gene is quantified after a certain period of time after administration of an RNAi-oligonucleotide for the target gene to cells in which the target gene is expressed by western blot analysis, and the level of expression of the protein is compared to that of a control to confirm the RNA interfering action and/or gene expression suppressing action. It is also possible to confirm the RNA interfering action and/or gene expression suppressing action by measuring the level of expression of the target gene in real time by a real-time PCR method after administration of the RNAi-oligonucleotide for the target gene to the target gene.

In the present specification, the term "target sequence" means a sequence of 18 or more nucleotides of any part of a mRNA nucleotide sequence for a gene encoding transferrin receptor 2, i.e., a target gene, which is targeted by the RNAi-oligonucleotide of the present invention. When the target sequence is known to have SNPs or the like, the target sequence also includes a sequence having such a variation. In the RNAi-oligonucleotide of the present invention, at least a part of the antisense strand region includes a sequence substantially complementary to a target sequence (hereinafter, a "target-matching sequence").

In the present specification, the term "complementary region" means regions in which the nucleotide sequences of the sense strand region and the antisense strand region contained in the RNAi-oligonucleotide are substantially complementary to each other. The sense strand region and the antisense strand region are not necessarily fully complementary in their complementary regions, but at least terminal bases in the complementary regions are complementary to each other. In the RNAi-oligonucleotide of the present invention, the complementary region of the antisense strand region includes a target-matching sequence, and may maintain complementarity to the sense strand region in regions obtained by further extending the 5'-terminus and/or the 3'-terminus of the complementary region.

The chain length of the target-matching sequence in the antisense strand region forming the RNAi-oligonucleotide of the present invention is not limited as long as the oligonucleotide has an RNA interfering action and/or a gene expression suppressing action. It may be any length that is equal to or longer than 18 nucleotides and equal to or smaller than the full length of the open reading frame (ORF) of a target gene. For example, the chain length of the target-matching sequence can be 19 to 29 nucleotides, and is preferably 18 to 24, 18 to 23, or 18 to 22, more preferably 18 to 21, and even more preferably 18 or 19.

The chain length of the complementary region in each of the sense strand region and the antisense strand region forming the RNAi-oligonucleotide of the present invention is not limited as long as the oligonucleotide has an RNA interfering action and/or a gene expression suppressing action. It may be any length. For example, the chain length of the target region can be 19 to 29 nucleotides, and is preferably 19 to 24, 19 to 23, or 19 to 22, more preferably 19 to 21, and even more preferably 19.

The chain length of each of the sense strand region and the antisense strand region forming the RNAi-oligonucleotide in the present invention is not limited as long as the oligonucleotide has an RNA interfering action and/or a gene expression suppressing action. It may be any length. The chain length of the sense strand region can be, for example, 19 to 39 nucleotides, and is preferably 19 to 29, more preferably 19 to 24, 19 to 23, or 19 to 22, even more preferably 19 to 21, and most preferably 19. The chain length of the antisense strand region can be, for example, 19 to 39 nucleotides, and is preferably 19 to 29, more preferably 19 to 24, 19 to 23, or 19 to 22, even more preferably 21 to 23, and most preferably 21.

In the RNAi-oligonucleotide of the present invention, when the sense strand region and the antisense strand region are present in different oligonucleotide molecules, the oligonucleotide is not required to have a double-stranded structure as a whole, and may have an overhang structure in which a part of the nucleosides at the 5'- and/or 3'-termini protrudes, or a double-stranded structure is not formed. Examples of the overhang structure include structures consisting of, for example, 1 to 5 nucleosides, preferably 1 to 3 nucleosides, and more preferably 2 nucleosides. The overhang structure may be present at one or both of the termini of the double-stranded oligonucleotide. In the present specification, the overhang structure may be expressed by representing the 5'-terminus of the sense strand region as S_{O5}, the 3'-terminus of the sense strand region as S_{O3}, the 5'-terminus of the antisense strand region as A₀₅, and the 3'-terminus of the antisense strand region as A₀₃. The nucleotide sequence of the overhang structure is not limited. Oligo T or oligo U can be used.

The RNAi-oligonucleotide has at least one property selected from the following (i) to (iv):
(i) the oligonucleotide has an RNA interfering action and/or a gene expression suppressing action on a target gene;
(ii) the oligonucleotide is stable against a ribonuclease, and has an RNA interfering action and/or a gene expression suppressing action on a target gene;
(iii) the oligonucleotide is stable against an exonuclease, and has an RNA interfering action and/or a gene expression suppressing action on a target gene; and
(iv) the oligonucleotide is stable against a ribonuclease and an exonuclease, and has an RNA interfering action and/or a gene expression suppressing action on a target gene.

An example of the RNAi-oligonucleotide is derived from a double-stranded oligonucleotide having an antisense strand region for a target gene, and a sense strand region having a nucleotide sequence substantially complementary to the antisense strand region, where the 5'-position of the nucleotide at the 5'-terminus of the antisense strand region and the 3'-position (2'-position in the case of a nucleotide modified at the 3'-position) of the nucleotide at the 3'-terminus of the sense strand region may be bound via a linker forming a phosphodiester bond which may be chemically modified at each end. Examples of the oligonucleotide can include oligonucleotides forming the structure of oligonucleotide 3'-P(=O)(OH)-[linker]-P(=O)(OH)-5'-oligonucleotide [herein, "oligonucleotide 3'" indicates a structure in which the hydrogen atom on the hydroxyl group is not present at the 3'-position (2'-position in the case of a nucleotide modified at the 3'-position) of the nucleotide at the 3'-terminus of the oligonucleotide, and "5'-oligonucleotide" indicates a structure in which the hydrogen atom on the hydroxyl group is not present at the 5'-position of the nucleotide at the 5'-terminus of the oligonucleotide]. Such a linker is described in, for example, WO 2012/074038 and the like, and can be synthesized by reference to the descriptions in that document.

A nucleoside forming the RNAi-oligonucleotide may be a natural nucleoside, or a sugar-modified nucleoside may be used. The inter-nucleoside bond is a phosphodiester bond that may be chemically modified. For example, a phosphodiester bond or a phosphorothioate bond can be appropriately selected.

The RNAi-oligonucleotide of the present invention can suppress expression of TfR2 mRNA. The RNAi-oligonucleotide of the present invention has low toxicity to cells, and can be used safely in a pharmaceutical product. The RNAi-oligonucleotide of the present invention, which suppresses expression of TfR2 mRNA, can thus suppress production of hepcidin which plays a key role in iron metabolism. The RNAi-oligonucleotide of the present invention, which suppresses expression of TfR2 mRNA, can thus increase the plasma iron concentration and/or the HGB concentration . Although the mechanism of action is not limited, the RNAi-oligonucleotide of the present invention can be used in the treatment or prevention of a disease that can be treated or prevented by suppressing expression of TfR2 mRNA and/or production of hepcidin. Examples of the disease include anemia. Although the mechanism of action is not limited, the RNAi-oligonucleotide of the present invention enables anemia to be treated or prevented by, for example, increasing the HGB concentration . The RNAi-oligonucleotide of the present invention can reduce the frequency and/or the amount of transfusion of blood cells to an anemia patient in need of transfusion of blood cells. For example, the frequency and/or the amount of transfusion of blood cells to an anemia patient for a given period of time (for example, 1 day, 1 week, 2 weeks, 4 weeks, 8 weeks, 12 weeks, 1 month, 2 months, 3 months, 6 months or 1 year) can be reduced by about 30%, about 40%, about 50%, about 60%, about 70%, about 80%, about 90%, or about 100%.

The present invention provides inventions for medical use, which include a pharmaceutical composition for use in the treatment or prevention of anemia, comprising the RNAi-oligonucleotide of the present invention as an active ingredient, a method for treating or preventing anemia, comprising administering an effective amount of the oligonucleotide, the oligonucleotide for use in the treatment or prevention of anemia, and use of the RNAi-oligonucleotide of the present invention in the production of a pharmaceutical composition for the treatment or prevention of anemia. Anemia that can be treated or prevented by the RNAi-oligonucleotide of the present invention includes, for example, anemia associated with chronic inflammation (anemia of chronic disease (ACD)), anemia associated with bone-marrow dysfunction, iron-deficiency anemia, iron refractory iron deficiency anemia (IRIDA), anemia resistant to an erythropoietin stimulating agent (ESA), anemia induced by chemotherapy, aplastic anemia, Diamond-Blackfan anemia, and inherited abnormality of hemoglobin such as β thalassemia and sickle cell disease. Anemia associated with chronic inflammation includes, for example, anemia associated with autoimmune disease such as articular rheumatism, systemic erythematosus or autoimmune hematolytic anemia, anemia associated with infectious disease, anemia associated with inflammatory enteric disease such as inflammatory colitis, anemia associated with heart failure, anemia associated with chronic kidney disease, cancerous anemia, anemia associated with macrophage activation syndrome, and anemia associated with Castleman disease. Anemia associated with chronic kidney disease includes, for example, kidney disease involving diabetes, kidney disease involving hypertension, nephrotic syndrome (for example, congenital nephrotic syndrome of the Finnish type, diffuse mesangial sclerosis, minimal lesion nephrotic syndrome, focal segmental glomerulosclerosis, membranous nephropathy, and Galloway-Mowat syndrome), kidney disease involving chronic glomerulonephritis (for example, IgA nephropathy, mesangial proliferative glomerulonephritis, membranoproliferative glomerulonephritis, purpura nephritis, antiglomerular basement membrane nephritis (Goodpasture syndrome), Alport's syndrome, Epstein syndrome, Lupus nephritis, microscopic polyangiitis, atypical hemolytic uremic syndrome, nail-patella syndrome, fibronectin nephropathy, and lipoprotein glomerulopathy), kidney disease involving chronic tubulointerstitial nephritis, kidney disease involving chronic pyelonephritis, kidney disease involving deposition of amyloid, autosomal dominant tubulointerstitial kidney disease, kidney disease involving nephronophthisis, and kidney disease involving kidney malformation (for example, polycystic kidney disease). Cancerous anemia includes, for example, anemia associated with cancer such as multiple myeloma, acute leukemia, chronic leukemia, lung cancer or breast cancer. Anemia associated with bone-marrow dysfunction includes, for example, anemia associated with myelofibrosis, anemia associated with myelodysplastic syndrome, anemia associated with chronic myelomonocytic leukemia (CMML), anemia associated with chemotherapeutic myelosuppression, aplastic anemia, Diamond-Blackfan anemia, and Fanconi anemia.

The RNAi-oligonucleotide of the present invention can be preferably used in the treatment or prevention of anemia associated with chronic inflammation and anemia associated with bone-marrow dysfunction, and can be more preferably used in the treatment or prevention of anemia associated with autoimmune disease such as articular rheumatism, systemic erythematosus or autoimmune hematolytic anemia, anemia associated with infectious disease, anemia associated with inflammatory enteric disease such as inflammatory colitis, anemia associated with heart failure, anemia associated with chronic kidney disease, cancerous anemia, anemia associated with Castleman disease, anemia associated with myelofibrosis, anemia associated with myelodysplastic syndrome, anemia associated with chronic myelomonocytic leukemia, and anemia associated with chemotherapeutic myelosuppression.

In the present specification, the term "combined use" means that two or more different drug agents are administered simultaneously, separately or sequentially. The present invention provides a method for treating or preventing anemia, comprising using the RNAi-oligonucleotide of the present invention in combination with one or more other drug agents. The RNAi-oligonucleotide of the present invention may be used in combination with one or more other drug agents as long as the effect of the present invention is exhibited. Preferably, use of the RNAi-oligonucleotide of the present invention in combination with other drug agents having a different mechanism of action on anemic symptoms can provide a greater therapeutic effect. For example, for myelodysplastic syndrome (MDS), a drug agent such as an erythropoiesis stimulating agent (ESA) having a mechanism of action of promoting proliferation of erythrocytes to treat anemia or luspatercept having a mechanism of action of promoting differentiation of erythrocytes to treat anemia, or a drug agent such as azacitidine having a mechanism of action of suppressing methylation of DNA and killing and wounding abnormal cells may be prescribed, and such a drug agent and TfR2 siRNA have different mechanisms of action. Therefore, combined use of such a drug agent and TfR2 siRNA can provide a therapeutic effect higher than that of a single agent on MDS. For anemia associated with chronic kidney disease, a drug agent such as ESA or a hypoxia inducible factor prolyl hydroxylase (HIFPHD) inhibitor having a mechanism of action of promoting proliferation of erythrocytes to treat anemia may be prescribed, and such a drug agent and TfR2 siRNA have different mechanisms of action. Therefore, combined use of such a drug agent and TfR2 siRNA can provide a therapeutic effect higher than that of a single agent on anemia associated with chronic kidney disease. For myelofibrosis, ruxolitinib is known to have a therapeutic effect mainly on symptoms such as splenomegaly (N Engl J Med 2012; 366: 799-807). However, the drug agent is known to have a limited effect or no effect on anemic symptoms, or worsen anemia (Journal of Hematology & Oncology 2013, 6: 79). For the mechanism in which ruxolitinib worsens anemia, it is considered that EPO signals required for proliferation of erythrocytic cells is suppressed by inhibiting JAK2. However, since ruxolitinib has a certain therapeutic effect on symptoms other than anemia in myelofibrosis, combined use of ruxolitinib and TfR2 siRNA can provide a beneficial therapeutic effect on anemia in myelofibrosis. Use in combination with a JAK2 inhibitor other than ruxolitinib can also provide a beneficial therapeutic effect on anemia in myelofibrosis. Examples of the other JAK2 inhibitor include fedratinib, pacritinib, and momelotinib.

When the RNAi-oligonucleotide of the present invention is used in combination with other drug agents, the timing of administration of the RNAi-oligonucleotide of the present invention is not limited as long as the effect of the present invention is exhibited. It may be administered simultaneously with the other drug agent, or may be administered before or after administration of the other drug agent. Examples of the drug agent that can be used in combination with the RNAi-oligonucleotide of the present invention include other drugs for treating anemia, or drugs for treating a disease that causes anemia. Examples of the other drug for treating anemia include ESA, a hypoxia inducible factor prolyl hydroxylase (HIFPHD) inhibitor, an oral iron supplement, an intravenous iron supplement, danazol, and luspatercept. Examples of ESA include epoetin alfa, epoetin beta, epoetin beta pegol, epoetin kappa, or darbepoetin alfa. Examples of a HIFPHD inhibitor include roxadustat, vadadustat, daprodustat, enarodustat, and molidustat. Examples of an oral iron supplement include ferrous citrate, ferric citrate, ferrous fumarate, soluble ferric pyrophosphate, and dry iron sulfide. Examples of an intravenous iron supplement include ferric carboxymaltose, and sugar-containing iron oxide. Examples of a drug for treating a disease that causes anemia include a drug for treating myelofibrosis, a drug for treating myelodysplastic syndrome, a drug for treating chronic kidney disease, or an anticancer agent. Examples of a drug for treating myelofibrosis include a JAK2 inhibitor. Examples of a JAK2 inhibitor include ruxolitinib, fedratinib, pacritinib, and momelotinib. Use in combination with ruxolitinib is preferred. Examples of a drug for treating myelodysplastic syndrome include azacytidine, and lenalidomide. Use in combination with azacytidine or lenalidomide is preferred. Examples of a drug for treating chronic kidney disease include a SGLT2 (sodium glucose cotransporter 2) inhibitor, a renin-angiotensin system inhibitor (for example, an angiotensin II receptor antagonist (ARB) and an angiotensin-converting enzyme (ACE) inhibitor), a calcium antagonist, a diuretic drug, a drug for treating diabetes, a drug for treating hyperlipidemia, a steroid, or an immunosuppressive drug. Examples of an anticancer agent include a chemotherapeutic drug. Examples of a chemotherapeutic drug include cisplatin, carboplatin, doxorubicin, paclitaxel, and amrubicin.

The RNAi-oligonucleotide of the present invention may be used in combination with a drug for treating iron overload disorder. When transfusion of blood cells is performed for treatment of anemia, iron overload disorder can develop due to frequent transfusion of blood cells. Iron overload disorder is a pathological condition caused by accumulation of iron derived from transfused blood cells in tissues of the liver or heart, and can produce liver failure, heart failure, diabetes, articular pain, hypothyroidism, loss of pituitary function, sexual dysfunction or the like. Therefore, a drug for treating iron overload disorder may be used for treating or preventing iron overload disorder in treatment of anemia, and can be preferably used in combination with the RNAi-oligonucleotide of the present invention. Examples of a drug for treating iron overload disorder include an iron chelating agent, and examples of an iron chelating agent include Deferasirox, Deferoxamine, and Deferiprone.

### <3. S3M-oligonucleotide>

The present invention provides an RNAi-oligonucleotide (hereinafter, also referred to as a "S3M-oligonucleotide") or a pharmaceutically acceptable salt thereof in which a sense strand region consists of an oligonucleotide represented by the following formula (I), an antisense strand region consists of an oligonucleotide represented by the following formula (II), and the RNAi-oligonucleotide or pharmaceutically acceptable salt thereof has the following characteristics (a) to (g):
sense strand region: 5' S_{O5}-Sₐ-S₁₉-S₁₈-S₁₇-S₁₆-S₁₅-S₁₄-S₁₃-S₁₂-S₁₁-S₁₀-S₉-S₈-S₇-S₆-S₅-S₄-S₃-S₂-S₁-S_{O3} 3' (I)
antisense strand region: 5' A₀₅-A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-Aₐ-A₀₃ 3' (II)
   (a) S₁ to S₁₉ each represent a nucleoside, S₁ is a 3'-modified nucleoside, Sₐ is 0 to 10, preferably 0 to 5 or 0 to 4, and more preferably 0 to 3, 0 to 2, or 0 to 1 nucleosides, S_{O3} and S_{O5} are each independently 0 to 5, preferably 0 to 4, and more preferably 0 to 3, 0 to 2, or 0 to 1 nucleosides, and each inter-nucleoside bond represents a phosphodiester bond that may be chemically modified;
   (b) A₁ to A₁₉ represent a nucleoside, at least one of A₁₁, A₁₂, A₁₃, A₁₄ and A₁₅ is DNA, RNA, a 2'-O,4'-C-bridging nucleoside, or 2'-MOE RNA, Aₐ is 0 to 10, preferably 0 to 5 or 0 to 4, and more preferably 0 to 3, 0 to 2, or 0 to 1 nucleosides, A_{O3} and A_{O5} are each independently 0 to 5, preferably 0 to 4, and more preferably 0 to 3, 0 to 2, or 0 to 1 nucleosides, and each inter-nucleoside bond represents a phosphodiester bond that may be chemically modified;
   (c) the nucleotide sequence between A₂ and Aₐ consists of a nucleotide sequence substantially complementary to a target sequence (target-matching sequence), A₁ is a nucleoside having a base complementary to a corresponding nucleoside of the target sequence, a nucleoside having adenine, a nucleoside having thymine, or a nucleoside having uracil, and when A₀₃ and A₀₅ are present, the nucleotide sequences thereof are each independently a nucleotide sequence selected independently of the corresponding nucleoside of the target sequence;
   (d) the nucleotide sequence between S₁ and Sₐ and the nucleotide sequence between A₁ and Aₐ are complementary regions consisting of nucleotide sequences substantially complementary to each other, and form a double-stranded structure;
   (e) when both S_{O5} and A_{O3} are present, S_{O5} and A_{O3} are nucleosides not complementary to each other;
   (f) when both S_{O3} and A_{O5} are present, S_{O3} and A_{O5} are nucleosides not complementary to each other; and
   (g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.

### <3-1. Sense strand region>

The sense strand region of the S3M-oligonucleotide refers to a region represented by the above formula (I), that is, S_{O5} to S_{O3}. The complementary region in the sense strand region of the S3M-oligonucleotide is represented as S₁ to S₁₉ numbered in the stated order from the nucleoside at the 3'-terminus, and Sₐ located on the 5' side of the complementary region. That is, the complementary region in the sense strand of the S3M-oligonucleotide is the region S₁ to Sₐ in the above formula (I). S₁ to S₁₉ each represent a nucleoside, and Sₐ represents 0 to 5, preferably 0 to 4, and more preferably 0 to 3, 0 to 2, or 0 to 1 nucleosides. Among the overhang structures of the sense strand region, an overhang structure added at the 5'-terminus is represented as S_{O5}, and an overhang structure added at the 3'-terminus is represented as S_{O3}, and S_{O5} and S_{O3} each represent 0 to 5, preferably 0 to 4, and more preferably 0 to 3, 0 to 2, or 0 to 1 nucleosides.

The S3M-oligonucleotide is characterized in that the nucleoside located at the 3'-terminus of the complementary region in the sense strand region (that is, S₁ in the above formula (I)) is a 3'-modified nucleoside. The 3'-modified nucleoside is not limited as long as the S3M-oligonicleotide has an RNA interfering action and/or a gene expression suppressing action. It is preferably 3'-deoxy-3'-fluoro RNA (3'-F RNA), 3'-OMe RNA, or 3'-MOE RNA, and more preferably 3'-OMe RNA. The residue at the 2'-position of the 3'-modified nucleoside may be a hydroxyl group, an optionally modified alkoxy group (preferably, a methoxy group, an ethoxy group or a methoxyethoxy group), or an optionally modified phosphate group (preferably, thiophosphate group, dithiophosphate group, methylphosphate group, or ethylphosphate group), and may be bound to the 5'-position of the nucleoside of the overhang structure (that is, S_{O3} in the above formula (I)), or may be bound to a linker structure. Preferably, the residue at the 2'-position is a hydroxyl group.

Nucleosides other than S₁ in the complementary region in the sense strand region of the S3M-oligonucleotide are not limited. Various natural nucleosides or sugar-modified nucleosides can be adopted. They are preferably, each independently, 2'-OMe RNA, 2'-F RNA or DNA.

An aspect of the complementary region in the sense strand region of the S3M-oligonucleotide is such that at least one of the 11th, 12th, 13th and 15th nucleosides from the 3'-terminus (that is, S₁₁, S₁₂, S₁₃ and S₁₅ in the above formula (I)) is 2'-F RNA, and other nucleosides are 2'-OMe RNA, 2'-F RNA, DNA or a combination thereof. Preferably, all of S₁₁, S₁₂, S₁₃ and S₁₅ are 2'-F RNA.

A preferred aspect of the complementary region in the sense strand region of the S3M-oligonucleotide is that the nucleosides of S₂ to S₁₀, S₁₄, S₁₆ to S₁₉ and Sₐ of the complementary region in the sense strand region are each independently 2'-OMe RNA, 2'-F RNA, DNA, or a modification pattern in which two selected therefrom are alternately arranged, and more preferably 2'-OMe RNA, DNA, or a modification pattern in which two selected therefrom are alternately arranged. More preferably, all of those nucleosides are 2'-OMe RNA. The number of nucleosides in Sₐ can be appropriately selected between 0 and 10, and from the 3' side thereof, nucleosides may be arranged in the order of S₂₀, S₂₁, S₂₂, S₂₃, S₂₄, S₂₅, S₂₆, S₂₇, S₂₈ and S₂₉ as necessary.

The nucleotide sequence of the complementary region in the sense strand region of the S3M-oligonucleotide (that is, S₁ to Sₐ in the above formula (I)) is substantially complementary to the complementary region in the antisense region (that is, A₁ to Aₐ in the above formula (II)).

The sense strand region of the S3M-oligonucleotide may have overhang structures at the 3'-terminus and/or the 5'-terminus of the complementary region thereof (that is, S_{O5} and S_{O5} in the above formula (I)). S_{O3} and S_{O5} each independently represent 0 to 5 (preferably, 4, 3, 2, 1 or 0) nucleosides, and the nucleosides are each independently 2'-OMe RNA, 2'-F RNA DNA or 2'-O,4'-C-bridging-modified nucleoside. Preferably, S_{O3} and S_{O5} are not present.

When S_{O3} and S_{O5} are present, the base thereof is not limited as long as a function as an overhang is exhibited. It is preferably oligo U or oligo T.

The following formulae (Ia) to (Ic) show examples of the modification pattern of the sense strand region of the S3M-oligonucleotide:

5'S_{O5}(N(M))-Sₐ(N(M)-N(M))-S₁₉(N(M))-S₁₈(N(M))-S₁₇(N(M))-S₁₆(N(M))-S₁₅(N(F))-S₁₄(N(M))-S₁₃(N(F))-S₁₂(N(F)) -S₁₁(N(F))-S₁₀(N(M))-S₉(N(M))-S₈(N(M))-S₇(N(M))-S₆(N(M))-S₅(N(M))-S₄(N(M))-S₃(N(M))-S₂(N(M))-S₁(N(3M))-2'H 3' (Ia)

5'Sₐ(N(M))-S₁₉(N(M))-S₁₈(N(M))-S₁₇(N(M))-S₁₆(N(M))-S₁₅(N(F))-S₁₄(N(M))-S₁₃(N(F))-S₁₂(N(F))-S₁₁(N(F))-S₁₀(N(M))-S₉(N(M))-S₈(N(M))-S₇(N(M))-S₆(N(M))-S₅(N(M))-S₄(N(M))-S₃(N(M))-S₂(N(M)-S₁(N(3M))-2'H 3' (Ib)

5'S₁₉(N(M))-S₁₈(N(M))-S₁₇(N(M))-S₁₆(N(M))-S₁₅(N(F))-S₁₄(N(M))-S₁₃(N(F))-S₁₂(N(F))-S₁₁(N(F))-S₁₀(N(M))-S₉(N(M)) -S₈(N(M))-S₇(N(M))-S₆(N(M))-S₅(N(M))-S₄(N(M))-S₃(N(M))-S₂(N(M)-S₁(N(3M))-2'H 3' (Ic)

wherein (N(M)) represents 2'-OMe RNA, (N(F)) represents 2'-F RNA, and (N(3M)) represents 3'-OMe RNA. S_{O5}(N(M)) and Sₐ(N(M)) represent a nucleoside consisting of 0 to 3 2'-OMe RNAs. Two inter-nucleoside bonds in three nucleosides from each of the 5'-terminal and the 3'-terminal of each oligonucleotide are phosphorothioate bonds, and other inter-nucleoside bonds are phosphodiester bonds. That is, each oligonucleotide has a total of four phosphorothioate bonds with two phosphorothioate bonds at each of the 3'-terminus and the 5'-terminus.

### <3-2. Antisense strand region>

The antisense strand region of the S3M-oligonucleotide refers to a region represented by the above formula (II), that is, A_{O5} to A_{O3}. The complementary region in the antisense strand region of the S3M-oligonucleotide is represented as A₁ to A₁₉ numbered in the stated order from the nucleoside at the 5'-terminus, and Aₐ located on the 3' side of the complementary region. That is, the complementary region of the antisense strand is the region of A₁ to Aₐ in the above formula (II). The nucleotide sequence of the complementary region in the antisense strand region of the S3M-oligonucleotide is only required to be substantially complementary to a target sequence, and may include, for example, a mismatch in 5 bases, 4 bases, 3 bases, 2 bases or 1 base with the target sequence, although it is preferred to be fully complementary to the target sequence. A₁ to A₁₉ each represent a nucleoside, and Aₐ represents 0 to 5 nucleosides. Among the overhang structures of the antisense strand region, an overhang structure added at the 5'-terminus is represented as A_{O5}, and an overhang structure added at the 3'-terminus is represented as A_{O3}.

A₁ may be substantially complementary to a corresponding base of the target sequence, and may be adenine, uracil or thymine independently of the target sequence. U.S. Patent No. 10093923 and the like indicate that good RNA interfering activity is exhibited when the base at the 3'-terminus in the complementary region of the sense strand of siRNA is adenine or uracil independently of the target sequence. In addition, the document Nature 465, 818-822 (2010) indicates that the nucleoside at the 5'-terminus of the antisense strand binds to Ago 2 protein involved in RNA interfering activity, and does not bind to target mRNA. It is also indicated that the nucleoside at the 5'-terminus of the antisense strand more strongly binds to Ago 2 protein when the nucleoside is an adenine or uracil nucleoside than when the nucleoside is guanine or cytosine, and therefore, the nucleoside at the 5'-terminus of the antisense strand is preferably an adenine or uracil nucleoside.

At least one (preferably two or more) of complementary regions A₁₁, A₁₂, A₁₃, A₁₄ and A₁₅ in the antisense strand region of the S3M-oligonucleotide is selected from DNA, RNA, a 2'-O,4'-C-bridging-modified nucleoside and 2'-MOE RNA. The 2'-O,4'-C-bridging-modified nucleoside used here is not limited. It is preferably LNA or ENA. In the antisense strand complementary region, nucleosides other that nucleosides identified as described above are not limited. Various natural nucleosides or sugar-modified nucleosides can be adopted. They are, each independently, 2'-OMe RNA, 2'-F RNA or DNA.

As an example of the present invention, an oligonucleotide is provided in which the complementary region A₁₄ in the antisense strand region of the S3M-oligonucleotide is RNA. Examples of the modification pattern of such an oligonucleotide include, but are not limited to, those shown as types 56 and 57 of Figure 2, types 61, 62, 66 and 67 of Figure 3, types 75 to 83 of Figure 5, and types 91 to 95 of Figure 6.

A preferred aspect of the antisense strand region of the oligonucleotide in which A₁₄ is RNA is that A₁₃ is a 2'-O,4'-C-bridging-modified nucleoside, 2'-OMe RNA or 2'-F RNA, more preferably a 2'-O,4'-C-bridging-modified nucleoside, and even more preferably ENA or LNA (types 76 to 83 of Figure 5 and types 91 to 93 of Figure 6). In such a S3M-oligonucleotide, the modification pattern of A₁₁-A₁₂-A₁₃-A₁₄-A₁₅ is preferably any of the following formulae (IIIa) to (IIIh).

A₁₁(2'-OMe RNA)-A₁₂(2'-OMe RNA)-A₁₃(ENA)-A₁₄(RNA)-A₁₅(2'-OMe RNA) (IIIa)

A₁₁(2'-F RNA)-A₁₂(2'-OMe RNA)-A₁₃(ENA)-A₁₄(RNA)-A₁₅(2'-OMe RNA) (IIIb)

A₁₁(2'-OMe RNA)-A₁₂(2'-F RNA)-A₁₃(ENA)-A₁₄(RNA)-A₁₅(2'-OMe RNA) (IIIc)

A₁₁(2'-OMe RNA)-A₁₂(2'-OMe RNA)-A₁₃(LNA)-A₁₄(RNA)-A₁₅(2'-OMe RNA) (IIId)

A₁₁(2'-F RNA)-A₁₂(2'-OMe RNA)-A₁₃(LNA)-A₁₄(RNA)-A₁₅(2'-OMe RNA) (IIIe)

A₁₁(2'-OMe RNA)-A₁₂(2'-F RNA)-A₁₃(LNA)-A₁₄(RNA)-A₁₅(2'-OMe RNA) (IIIf)

A₁₁(2'-F RNA)-A₁₂(2'-OMe RNA)-A₁₃(2'-OMe RNA)-A₁₄(RNA)-A₁₅(2'-OMe RNA) (IIIg)

A₁₁(2'-OMe RNA)-A₁₂(2'-F RNA)-A₁₃(2'-OMe RNA)-A₁₄(RNA)-A₁₅(2'-OMe RNA) (IIIh)

As an example of the present invention, an oligonucleotide is provided in which the complementary region A₁₄ in the antisense strand region of the S3M-oligonucleotide is a 2'-O,4'-C-bridging-modified nucleoside. Here, the 2'-O,4'-C-bridging-modified nucleoside is preferably ENA or LNA. Examples of the modification pattern of such an oligonucleotide include, but are not limited to, those shown as types 70 to 74 of Figure 4, and types 88 to 90 of Figure 6.

A preferred aspect of the oligonucleotide in which A₁₄ is a 2'-O,4'-C-bridging-modified nucleoside is that A₁₂ is DNA. In such a S3M-oligonucleotide, the modification pattern A₁₁-A₁₂-A₁₃-A₁₄-A₁₅ of the antisense strand complementary region is preferably any of the following formulae (IIIi) to (IIIl).

A₁₁(2'-F RNA)-A₁₂(DNA)-A₁₃(2'-OMe RNA)-A₁₄(ENA)-A₁₅(2'-OMe RNA) (IIIi)

A₁₁(2'-OMe RNA)-A₁₂(DNA)-A₁₃(2'-OMe RNA)-A₁₄(ENA)-A₁₅(2'-OMe RNA) (IIIj)

A₁₁(2'-F RNA)-A₁₂(DNA)-A₁₃(2'-OMe RNA)-A₁₄(LNA)-A₁₅(2'-OMe RNA) (IIIk)

A₁₁(2'-OMe RNA)-A₁₂(DNA)-A₁₃(2'-OMe RNA)-A₁₄(LNA)-A₁₅(2'-OMe RNA) (IIIl)

As an example of the present invention, an oligonucleotide is provided in which A₁₄ of the complementary region of the antisense strand region of the S3M-oligonucleotide is DNA. Examples of the modification pattern of such an oligonucleotide include, but are not limited to, those shown as types 58 and 59 of Figure 2, and types 63, 64, 68 and 69 of Figure 3.

As an example of the present invention, an oligonucleotide is provided in which A₁₅ of formula (II) in the antisense strand region of the S3M-oligonucleotide is 2'-MOE RNA. Examples of the modification pattern of such an oligonucleotide include, but are not limited to, those shown as types 79 and 83 of Figure 5.

As an example of the present invention, an oligonucleotide is provided in which any of the 11th to 13th nucleosides from the 5'-terminus of the complementary region of the antisense strand region of the S3M-oligonucleotide (A₁₁, A₁₂ and A₁₃ of formula (II)) is a 2'-O,4'-C-bridging-modified nucleoside. Examples of the modification pattern of such an oligonucleotide include, but are not limited to, those shown as types 43 and 45 of Figure 1, types 75 to 84 of Figure 4 and types 85 and 86 and 91 to 93 of figure 6 for ENA, and types 44 and 46 of Figure 1 and type 67 and 69 of Figure 3 for LNA.

As an example of the present invention, an oligonucleotide is provided in which any of the 11th to 13th nucleosides from the 5'-terminus of the complementary region of the antisense strand region of the S3M-oligonucleotide (A₁₁, A₁₂ and A₁₃ of formula (II)) is DNA. Examples of the modification pattern of such an oligonucleotide include, but are not limited to, those shown as types 41 and 42 of Figure 1, type 71 of Figure 4, and types 87 to 90 of Figure 6.

As an example of the present invention, an oligonucleotide is provided in which any of the 11th to 13th nucleosides from the 5'-terminus of the complementary region of the antisense strand region of the S3M-oligonucleotide (A₁₁, A₁₂ and A₁₃ of formula (II)) is 2'-MOE RNA. Examples of the modification pattern of such an oligonucleotide include, but are not limited to, those shown as types 47, 48 of Figure 1, and type 74 of figure 4.

For nucleosides in the complementary region in the antisense strand region of the S3M-oligonucleotide of the present invention other than the nucleosides specified as described above, various types of nucleosides can be appropriately selected, but preferably, at least one (preferably all) of the nucleosides of A₂, A₆ and A₁₆ is 2'-F RNA. A more preferred antisense strand region is such that, in addition to A₂, A₆ and A₁₆, one or two nucleosides selected from the group consisting of A₈, A₉ and A₁₀ are 2'-F RNA, and nucleosides other than those specified as described above (for example, A₁, A₃ to A₅, A₇, A₁₇ to A₁₉ and Aₐ) are 2'-OMe RNA or DNA.

Preferable modification patterns of regions other than A₁₁ to A₁₅ of the complementary region of the antisense strand region of the S3M-oligonucleotide of the present invention are exemplified in, for example, Tables A-1 and A-2 below.

**[Table A-1]**

| | A₁ | A₂ | A₃ | A₄ | A₅ | A₆ | A₇ | A₈ | A₉ | A₁₀ |
|---|---|---|---|---|---|---|---|---|---|---|
| Pattern 1 | (M) | (F) | (M) | (M) | (M) | (F) | (M) | (M) | (M) | (M) |
| Pattern 2 | (M) | (F) | (M) | (M) | (M) | (F) | (M) | (F) | (M) | (M) |
| Pattern 3 | (M) | (F) | (M) | (M) | (M) | (F) | (M) | (F) | (M) | (M) |
| Pattern 4 | (M) | (F) | (M) | (M) | (M) | (F) | (M) | (M) | (F) | (M) |
| Pattern 5 | (M) | (F) | (M) | (M) | (M) | (F) | (M) | (M) | (M) | (F) |
| Pattern 6 | (M) | (F) | (M) | (M) | (M) | (F) | (M) | (F) | (M) | (F) |
| Pattern 7 | (M) | (F) | (M) | (M) | (M) | (F) | (F) | (M) | (F) | (M) |
| Pattern 8 | (M) | (F) | (M) | (F) | (M) | (F) | (M) | (M) | (M) | (M) |
| Pattern 9 | (M) | (F) | (M) | (F) | (M) | (F) | (M) | (F) | (M) | (M) |
| Pattern 10 | (M) | (F) | (M) | (F) | (M) | (F) | (M) | (F) | (M) | (F) |
| Pattern 11 | (M) | (F) | (M) | (F) | (M) | (F) | (M) | (F) | (F) | (M) |
| Pattern 12 | (M) | (F) | (M) | (F) | (M) | (F) | (M) | (F) | (F) | (F) |
| Pattern 13 | (M) | (F) | (M) | (M) | (M) | (F) | (M) | (F) | (F) | (M) |
| Pattern 14 | (M) | (F) | (M) | (F) | (M) | (F) | (M) | (M) | (F) | (M) |
| Pattern 15 | (M) | (F) | (M) | (M) | (M) | (F) | (M) | (M) | (F) | (M) |
| Pattern 16 | (M) | (F) | (M) | (M) | (M) | (F) | (M) | (M) | (F) | (F) |

**[Table A-2]**

| | A₁₆ | A₁₇ | A₁₈ | A₁₉ | Aₐ | A_{O3} |
|---|---|---|---|---|---|---|
| Pattern 1 | (F) | (M) | (M) | (M) | (M) | (M) |
| Pattern 2 | (F) | (M) | (M) | (M) | (M) | (M) |
| Pattern 3 | (F) | (M) | (M) | (M) | (M) | (M) |
| Pattern 4 | (F) | (M) | (M) | (M) | (M) | (M) |
| Pattern 5 | (F) | (M) | (M) | (M) | (M) | (M) |
| Pattern 6 | (F) | (M) | (M) | (M) | (M) | (M) |
| Pattern 7 | (F) | (M) | (M) | (M) | (M) | (M) |
| Pattern 8 | (F) | (M) | (M) | (M) | (M) | (M) |
| Pattern 9 | (F) | (M) | (M) | (M) | (M) | (M) |
| Pattern 10 | (F) | (M) | (M) | (M) | (M) | (M) |
| Pattern 11 | (F) | (M) | (M) | (M) | (M) | (M) |
| Pattern 12 | (F) | (M) | (M) | (M) | (M) | (M) |
| Pattern 13 | (F) | (M) | (M) | (M) | (M) | (M) |
| Pattern 14 | (F) | (M) | (M) | (M) | (M) | (M) |
| Pattern 15 | (F) | (M) | (F) | (M) | (M) | (M) |
| Pattern 16 | (F) | (M) | (M) | (M) | (M) | (M) |

[In the tables above, (M) is 2'-OMe RNA, and (F) is 2'-F RNA. Aₐ is 0 to 5 nucleosides, where all the nucleosides are the same except when 0. A_{O3} is 0 to 5 nucleosides, where all the nucleosides are the same except when 0. A phosphodiester bond or a phosphorothioate bond is appropriately selected as the inter-nucleoside bond.]

The antisense strand region contained in the S3M-oligonucleotide may have overhang structures at the 3'-terminus and/or the 5'-terminus of the complementary region thereof (that is, A_{O3} and A_{O5} in the above formula (II)). A_{O3} and A_{O5} each independently represent 0 to 5 (preferably, 3, 2, 1 or 0) nucleosides, where the nucleosides are each independently 2'-OMe RNA, 2'-F RNA, DNA or a 2'-O,4'-C-bridging-modified nucleoside. Preferably, A_{O3} is an overhang consisting of two or three 2'-OMe RNAs, or an overhang consisting of one or two 2'-OMe RNAs and one or two 2'-O,4'-C-bridging-modified nucleosides, and A_{O5} is not present.

When A_{O3} and A_{O5} are present, the bases thereof are not limited as long as a function as an overhang is exhibited. They are preferably oligo U or oligo T.

### <3-3. Inter-nucleoside bond>

A phosphodiester bond that may be chemically modified can be appropriately selected for each inter-nucleoside bond in the S3M-oligonucleotide of the present invention, and a phosphodiester bond or a phosphorothioate bond is preferred. Those bonds can be used alone, or in combination. A combination of phosphodiester bonds and phosphorothioate bonds is preferred, and the phosphorothioate bond content in inter-nucleoside bonds contained in each of the sense strand region and the antisense strand region is 50% or less, 40% or less, 30% or less, 29% or less, 28% or less, 27% or less, 26% or less, or 25% or less.

When RNA and/or 2'-F RNA are used, the bond between the relevant nucleoside and a 3' adjacent nucleoside may be a phosphorothioate bond. When RNA is used, the bond between the relevant nucleoside and a 3' adjacent nucleoside is preferably a phosphorothioate bond.

In the S3M-oligonucleotide of the present invention, a phosphorothioate bond is preferably used for bonds between the 1st and 2nd nucleosides up to between the 4th and 5th nucleosides (the number of inter-nucleoside bonds is 1, 2, 3 or 4) from the 5'-terminus and/or the 3'-terminus of the oligonucleotide. In the oligonucleotide of the present invention, when the sense strand region and the antisense strand region are contained in separate oligonucleotides, bonds between the 1st and 2nd nucleosides up to between the 4th and 5th nucleosides from each of the 5'-terminus and the 3'-terminus of the sense strand region, and bonds between the 1st and 2nd nucleotides up to between the 4th and 5th nucleotides from each of the 5'-terminus and the 3'-terminus of the antisense strand region, may be phosphorothioate bonds. Preferably, bonds between the 1st and 2nd nucleosides up to between the 2nd and 3rd nucleosides (the number of inter-nucleoside bonds is 2) from each of the 5'-terminus and the 3'-terminus of the sense strand region, and bonds between the 1st and 2nd nucleosides up to between the 2nd and 3rd nucleosides (the number of inter-nucleoside bonds is 2) from the 5'-terminus and bonds between the 1st and 2nd nucleotides up to between the 3rd and 4th nucleosides (the number of inter-nucleoside bonds is 3) from the 3'-terminus of the antisense strand region, are phosphorothioate bonds.

### <4. Method for synthesizing oligonucleotide>

The method for preparing the oligonucleotide of the present invention is not limited as long as it enables synthesis of a desired oligonucleotide. A known chemical synthesis method (phosphotriester method, phosphoramidite method, a H-phosphonate method or the like) can be used. For example, the oligonucleotide can be synthesized with commercially available reagents for use in DNA/RNA using a commercially available nucleic acid synthesizer.

When a normal phosphoramidite method is used, an oligonucleotide having a desired nucleotide sequence can be synthesized by a method described in the art (Nucleic Acids Research, 12, 4539 (1984)) using a DNA synthesizer, for example, Model 392 made by PerkinElmer, Inc. based on the phosphoramidite method.

Amidite reagents corresponding to various nucleosides may be obtained by purchasing those that are commercially available, or performing synthesis by a known method.

The oligonucleotide of the present invention can be synthesized by a method described in the document Nucleic Acids Research, 12, 4539 (1984) using a commercially available DNA synthesizer (for example, Model 392 made by PerkinElmer, Inc. based on phosphoramidite method). Phosphoramidite reagents used here may be commercially available phosphoramidite reagents for natural nucleosides and 2'-O-methylnucleosides (that is, 2'-O-methylguanosine, 2'-O-methyladenosine, 2'-O-methylcytidine and 2'-O- methyluridine). The following applies to 2'-O-alkylguanosines, 2'-O-alkyladenosines, 2'-O-alkylsytidines and 2'-O-alkyluridines in which the number of carbon atoms in the 2'-O-alkyl group is 2 to 6.

For 2'-O-aminoethylguanosine, 2'-O-aminoethyladenosine, 2'-O-aminoethylcytidine and 2'-O-aminoethyluridine, corresponding phosphoramidite reagents can be synthesized according to the document Blommers et al. Biochemistry (1998), 37, 17714-17725.

For 2'-O-propylguanosine, 2'-O-propyladenosine, 2'-O-propylcytidine and 2'-O-propyluridine, corresponding phosphoramidite reagents can be synthesized according to the document Lesnik, E.A. et al. Biochemistry (1993), 32, 7832-7838.

For 2'-O-allylguanosine, 2'-O-allyladenosine, 2'-O-allylcytidine and 2'-O-allyluridine, commercially available phosphoramidite reagents can be used.

For 2'-O-methoxyethylguanosine, 2'-O-methoxyethyladenosine, 2'-O-methoxyethyl-5-methylcytidine and 2'-O-methoxyethyl-5-methyluridine for 2'-MOE RNA, corresponding phosphoramidite reagents can be synthesized according to US patent No.6261840, or the document Martin, P. Helv. Chim. Acta. (1995) 78, 486-504, or commercially available phosphoramidite reagents can be used.

For 2'-O-butylguanosine, 2'-O-butyladenosine, 2'-O-butylcytidine and 2'-O-butyluridine, corresponding phosphoramidite reagents can be synthesized according to the document Lesnik, E.A. et al. Biochemistry (1993), 32, 7832-7838.

For 2'-O-pentylguanosine, 2'-O-pentyladenosine, 2'-O-pentylcytidine and 2'-O-pentyluridine, corresponding phosphoramidite reagents can be synthesized according to the document Lesnik, E.A. et al. Biochemistry (1993), 32, 7832-7838.

For 2'-O-propargylguanosine, 2'-O-propargyladenosine, 2'-O-propargylcytidine and 2'-O-propargyluridine, commercially available phosphoramidite reagents can be used.

For 2'-deoxy-2'-fluoroguanisine, 2'-deoxy-2'-fluoroadenosine, 2'-deoxy-2'-fluorocytidine and 2'-deoxy-2'-fluorouridine for 2'-F RNA, corresponding phosphoramidite reagents can be synthesized according to the document J. Med. Chem. 36, 831 (1993), or commercially available phosphoramidite reagents can be used.

For 3'-F RNA that is a 3'-modified nucleoside, corresponding phosphoramidite reagents can be synthesized according to WO 2017027645 for 3'-deoxy-3'-fluoroguanosine, WO2018198076 for 3'-deoxy-3'-fluoroadenosine, US 2011/0196141 for 3'-deoxy-3'-fluorouridine. For 3'-deoxy-3'-fluorocytidine, the synthesis can be performed by reference to known methods in the above-mentioned documents and the like.

For 3'-OMe RNA that is a 3'-modified nucleoside, 3'-O-methylguanosine, 3'-O-methyladenosine, 3'-O-methylcytidine and 3'-O-methyluridine can be synthesized using commercially available phosphoramidite reagents.

For 3'-MOE RNA that is a 3'-modified nucleoside, 3'-O-methoxyethylguanosine, 3'-0-methoxyethyladenosine, 3'-O-methoxyethyl-5-methylcytidine and 3'-O-methoxyethyl-5-methyluridine, corresponding phosphoramidite reagents can be synthesized according to US 2003/0096979.

For 2'-O,4'-C-methyleneguanosine, 2'-O,4'-C-methyleneadenosine, 2'-O,4'-C-methylenecytidine, 2'-O, 4'-C-methylene-5-methylcytidine and 2'-O,4'-C-methylene-5-methyluridine for LNA, corresponding phosphoramidite reagents can be produced by a method described in WO 99/14226.

For 2'-O,4'-C-alkyleneguanosines, 2'-O,4'-C-alkyleneadenosines, 2'-O,4'-C-alkylenecytidines, 2'-O,4'-C-alkylene-5-methylcytidines and 2'-O,4'-C-alkylene-5-methyluridines in which the number of carbon atoms in the alkylene group of the 2'-O,4'-C-bridge moiety is 2 to 5, corresponding phosphoramidite reagents can be produced by a method described in WO 00/47599.

For nucleosides subjected to modification of D-ribofuranose with 2'-deoxy-2'-C,4'-C-methyleneoxymethylene, corresponding phosphoramidite reagents can be synthesized according to the document Wang, G. et al. Tetrahedron (1999), 55, 7707-7724.

For S-cEt (constrained ethyl), corresponding phosphoramidite reagents can be synthesized according to the document Seth, P.P. et al. J. Org. Chem (2010), 75, 1569-1581.

For AmNA, corresponding phosphoramidite reagents can be synthesized according to the document Yahara, A. et al. ChemBioChem (2012), 13, 2513-2516 or WO 2014/109384.

An antisense oligonucleotide having a phosphorothioate bond can be synthesized by coupling phosphoramidite reagents, followed by reaction with a reagent such as sulfur, tetraethylthiuram disulfide (TETD, Applied Biosystems), a Beaucage reagent (Glen Research) or xanthan hydride (Tetrahedron Letters, 32, 3005 (1991); J. Am. Chem. Soc. 112, 1253 (1990); PCT/WO 98/54198).

Commercially available controlled pore glass (CPG) and polystyrene solid-phase supports, to which a 2'-O-methylnucleoside and a 3'-O-methylnucleoside are bound, can be used in a synthesizer. For 2'-O,4'-C-methyleneguanosine, 2'-O,4'-C-methyleneadenosine, 2'-O,4'-C-methylene-5-methylcytidine and 2'-O,4'-C-methylene-5-methyluridine, nucleosides produced by a method described in WO 99/14226 can be bound to CPG, and for 2'-O,4'-C-alkyleneguanosines, 2'-O,4'-C-alkyleneadenosines, 2'-O,4'-C-alkylene-5-methylcytidines and 2'-O,4'-C-alkylene-5-methyluridines in which the number of carbon atoms in the alkylene is 2 to 5, nucleosides produced by a method described in WO 00/47599 can be bound to CPG according to the document Oligonucleotide Synthesis, Edited by M. J. Gait, Oxford University Press, 1984. The synthesis can be performed by bonding any of the above-described phosphoramidite reagents using universal resin. Use of modified CPG (described in Japanese Patent Laid-Open No. 7-87982, Example 12b) enables synthesis of an oligonucleotide in which a 2-hydroxyethylphosphate group is bound to the 3'-terminus. Use of 3'-amino-modifier C3 CPG, 3'-amino-Modifier C7 CPG, Glyceryl CPG, (Glen Research), 3'-specer C3 SynBase CPG 1000 or 3'-spacer C9 SynBase CPG 1000 (link technologies) enables synthesis of an oligonucleotide in which a hydroxyalkylphosphate group or an aminoalkylphosphate group is bound to the 3'-terminus.

When an oligonucleotide analog is thioate-modified, a thioate derivative can be obtained by a method described in the art (Tetrahedron Letters, 32, 3005 (1991), J. Am. Chem. Soc., 112, 1253 (1990)) using reagents such as tetraethylthiuram disulfide (TETD, Applied BioSystems), a Beaucage reagent, and a phenylacetyl disulfide/pyridine-acetonitrile (1 : 1 v/v) solution (Ravikumar, V.T. et al. Bioorg. Med. Chem. Lett. (2006) 16, p. 2513-2517) in addition to sulfur.

When a silyl-based protective group such as t-butyldimethylsilyl (TBS) is used as a protective group for the 2'-hydroxyl group of RNA, the protection can be eliminated using tetrabutylammonium fluoride (TBAF), HF-pyridine, HF-triethylamine or the like.

Protection of the acyl group of the base moiety and the cyanoethyl group of the phosphate moiety can be eliminated using concentrated aqueous ammonia, methanolic ammonia, ethanolic ammonia, a concentrated aqueous ammonia-ethanol (3 : 1 V/V) mixed liquid, a concentrated aqueous ammonia-40% methylamine aqueous solution (1 : 1 V/V) mixed liquid, methylamine, a 0.5 M LiOH aqueous solution, a 3.5 M triethylamine/methanol solution (1 : 10 V/V) mixed liquid, 0.05 M potassium carbonate-containing methanol or the like. Concentrated aqueous ammonia and a concentrated aqueous ammonia-ethanol mixed liquid (3 : 1 V/V) are preferred.

The oligonucleotide of the present invention can be obtained by performing purification through any purification process for use in normal nucleic acid purification, for example, various kinds of chromatography such as reversed-phase chromatography and ion-exchange chromatography (including high-performance liquid chromatography).

The oligonucleotide of the present invention includes an oligonucleotide to which a unit for delivery for delivering the oligonucleotide to target tissues or target cells is bound. Examples of the oligonucleotide to which a unit for delivery is bound can include oligonucleotides in which a unit of a desired chemical structure having an aminoalkylphosphate group (for example, the alkyl has 3 to 9 carbon atoms) is bound to the 5'-position of the nucleotide at the 5'-terminus and/or the 3'-position (2'-position in the case of a nucleotide modified at the 3'-position) of the nucleotide at the 3'-terminus of the oligonucleotide. As such a unit for delivery, for example, a GalNAc unit binding to asialoglyco-receptors (ASGRPs) of liver parenchymal cells, or fatty acid (for example, myristic acid, palmitic acid, stearic acid, arachidic acid or behenic acid) unit for delivery to liver and muscular tissues (see, for example, Nucleic Acids Res. (2020) 47, 6029-6044; WO 2017/19267) can be used as appropriate.

The GalNAc unit is a structural unit containing **N-**acetyl-D-galactosamine (GalNAc) capable of binding to ASGRP, and can be used for delivering the oligonucleotide of the present invention to the liver. The GalNAc unit may have a linear or branched linker structure, and can have, for example, a linear linker structure, or a branched linker structure that diverges into two or more, three or more, or four or more branches, or four or less, three or less, or two or less branches. The GalNAc unit may contain a phosphate group or a thiophosphate group for binding to an oligonucleotide. As long as the ability to bind to ASGRP is maintained, the number of GalNAcs contained in one GalNAc unit is not limited, and the GalNAc structure may be modified. As GalNAc units that can be used for the oligonucleotide of the present invention, for example, known GalNAc units described in WO 2021/049504, WO 2009/073809, WO 2014/076196, WO 2014/179620, WO 2015/006740, WO 2015/105083, WO 2016/055601, 2017/023817, WO 2017/084987, WO 2017/131236, Methods in Enzymology, 1999, Vol. 313, p. 297-321, and Bioorganic & Medicinal Chemistry Letters 26 (2016) 3690-3693, can be used as appropriate.

Examples of the GalNAc unit that can be used include those having the following formula, and can be appropriately adjusted by a method described in WO 2021/049504. 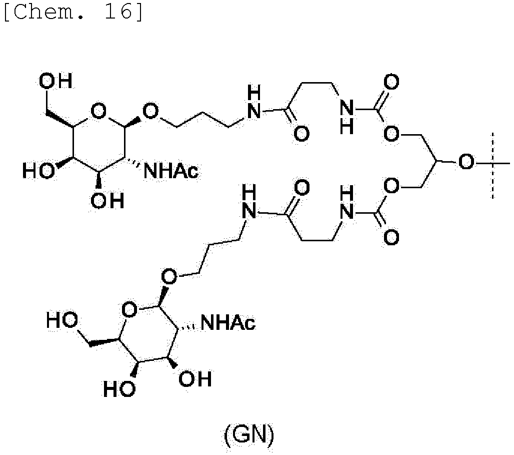 wherein the broken line represents a point of attachment, the oxygen atom at the point of attachment binds to an adjacent nucleotide, and forms a phosphodiester bond with the 5'-phosphate group when binding to the nucleotide at the 5'-terminus, and with the 3'-phosphate group (phosphate group at the 2'-position in the case of a nucleotide modified at the 3'-position) when binding to the nucleotide at the 3'-terminus, and the phosphodiester bond formed may be a chemically modified phosphodiester bond such as a phosphorothioate bond.

The oligonucleotide of the present invention also includes an oligonucleotide in which a cholesterol unit, a lipid unit or a vitamin E unit is introduced into the oligonucleotide (see, for example, Lorenz, C. et al. Bioorg. Med. Chem. Lett., 14, p. 4975-4977 (2004); Soutschek, J., et al. Nature, 432, p. 173-178, (2004); Wolfrum, C. et al. Nature Biotech. 25, p. 1149-1157, (2007))Kubo, T. et al. Oligonucleotides, 17, p. 1-20, (2007); Kubo, T., et al. Biochem. Biophys. Res. Comm. 365, p. 54-61, (2008); and Nishina, K., et al., Mol. Ther. 16, p. 734-740, (2008)), and an oligonucleotide in which an aptamer, which is a nucleic acid molecule capable of binding to a protein, is bound to a terminus of the oligonucleotide.

The oligonucleotide of the present invention also includes an oligonucleotide in which the oligonucleotide is bound to a monoclonal antibody (or an appropriate binding moiety thereof), or a protein (or an appropriate oligopeptide fragment thereof) (see, for example, Song, et al. Nature Biotech. 23, p. 709-717 (2005); and Xia et al. Pharm. Res. 24, p. 2309-2316 (2007), Kumar, et al. Nature, 448, p. 39-43 (2007)).

The oligonucleotide of the present invention also includes a positively charged complex obtained by adding a cationic polymer to the oligonucleotide (see, as examples of where distribution to organs and cells could be achieved, Leng et al. J. Gen. Med. 7, p. 977-986 (2005), Baigude et al. 2, p. 237-241, and ACS Chem. Biol. (2007), Yadava et al. Oligonucleotide 17, p. 213-222 (2007)).

The oligonucleotide of the present invention includes all pharmaceutically acceptable salts or esters of the oligonucleotides, or salts of such esters. Preferred examples of the pharmaceutically acceptable salt of the oligonucleotide of the present invention can include metal salts such as alkali metal salts such as sodium salts, potassium salts and lithium salts, alkaline earth metals such as calcium salts and magnesium salts, aluminum salts, iron salts, zinc salts, copper salts, nickel salts, and cobalt salts; amine salts such as inorganic amine salts such as ammonium salts, and organic amine salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts such as halogenated hydracid salts such as hydrofluoric acid salts, hydrochloric acid salts, hydrobromic acid salts and hydroiodic acid salts, nitric acid salts, perchloric acid salts, sulfuric acid salts, and phosphoric acid salts; organic acid salts such as lower alkanesulfonic acid salts such as methanesulfonic acid salts, trifluoromethanesulfonic acid salts and ethanesulfonic acid salts, arylsulfonic acid salts such as benzenesulfonic acid salts and p-toluenesulfonic acid salts, and acetic acid salts, malic acid salts, fumaric acid salts, succinic acid salts, citric acid salts, tartaric acid salts, oxalic acid salts, and maleic acid salts; and amino acid salts such as glycine salts, lysine salts, arginine salts, ornithine salts, glutamine acid salts, and asparagine acid salts.

The oligonucleotide or a pharmaceutically acceptable salt thereof according to the present invention can suppress expression of TfR2 mRNA. The oligonucleotide or a pharmaceutically acceptable salt thereof according to the present invention, which suppresses expression of TfR2 mRNA, can thus suppress production of hepcidin which plays a key role in iron metabolism. Although the mechanism of action is not limited, the oligonucleotide or a pharmaceutically acceptable salt thereof according to the present invention can be used in the treatment or prevention of a disease that can be treated or prevented by suppressing expression of TfR2 mRNA and/or production of hepcidin. A preferred aspect is administration to an anemia patient, where expression of TfR2 mRNA can be suppressed, and an effect of improving an anemic symptom can be expected. The oligonucleotide or a pharmaceutically acceptable salt thereof according to the present invention can also be used as a pharmaceutical composition containing the oligonucleotide or a pharmaceutically acceptable salt thereof as an active ingredient.

A pharmaceutical composition containing the oligonucleotide of the present invention is mixed, encapsulated or conjugated with another molecule, molecular structure, or mixture of compounds to form, for example, a liposome, a receptor-targeting molecule, an oral, rectal, or a local formulation or other formulation for assisting uptake, distribution and/or absorption. When the oligonucleotide of the present invention is used as a prophylactic drug or a therapeutic drug for a disease, the oligonucleotide or a pharmaceutically acceptable salt thereof can be administered itself, or mixed with an appropriate pharmaceutically acceptable excipient, diluent and the like, and orally administered as a tablet, a capsule, a granule, a powder, a syrup or the like, or parenterally administered as an injection, a suppository, a patch, an external preparation or the like.

These preparations are produced by well-known methods using additives such as excipients (examples thereof can include organic excipients such as sugar derivatives such as lactose, white sugar, glucose, mannitol and sorbitol; starch derivatives such as corn starch, potato starch, α-starch and dextrin; cellulose derivatives such as crystalline cellulose; gum arabic; dextran; and pullulan; and inorganic excipients such as silicic acid salt derivatives such as light anhydrous silicic acid, synthetic aluminum silicate, calcium silicate and magnesium aluminometasilicate; phosphoric acid salts such as calcium hydrogen phosphate; carbonic acid salts such as calcium carbonate; and sulfuric acid salts such as calcium sulfate), lubricants (examples thereof can include stearic acid, stearic acid metal salts such as calcium stearate and magnesium stearate; talc; colloid silica; waxes such as bead wax and spermaceti; boric acid; adipic acid; sulfuric acid salts such as sodium sulfate; glycol; fumaric acid; sodium benzoate; DL leucin; lauryl sulfuric acid salts such as sodium lauryl sulfate and magnesium lauryl sulfate; silicic acids such as anhydrous silicic acid and silicic acid hydrate; and the above-described starch derivatives), binders (examples thereof can include hydroxypropylcellulose, hydroxypropylmethylcellulose, polyvinylpyrrolidone, macrogol, and the same compounds as the above-described excipients), disintegrants (examples thereof can include cellulose derivatives such as low-substituted hydroxypropylcellulose, carboxymethylcellulose, carboxymethylcellulose calcium and internally cross-linked carboxymethylcellulose sodium; and chemically modified starch/celluloses such as carboxymethyl starch, carboxymethyl starch sodium and cross-linked polyvinylpyrrolidone), emulsifiers (examples thereof can include colloidal clays such as bentonite and veegum; metal hydroxides such as magnesium hydroxide and aluminum hydroxide; anionic surfactants such as sodium lauryl sulfate and calcium stearate; cationic surfactants such as benzalkonium chloride; and nonionic surfactants such polyoxyethylene alkyl ethers, polyoxyethylene sorbitan fatty acid esters and sucrose fatty acid esters), stabilizers (examples thereof can include paraoxybenzoic acid esters such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), taste and odor improvers (examples thereof can include sweeteners, sour agents and flavors as normally used), and diluents.

The body to be subjected to introduction of an oligonucleotide prepared as described above is not limited as long as it allows a target gene to be transcribed into RNA in its cell. The body to be subjected to introduction means a cell, a tissue or an individual.

The cell into which the oligonucleotide of the present invention is introduced may be a germline cell, a somatic cell, a differentiating totipotent cell, a multipotent cell, a cleaved cell, a non-cleaved cell, a parenchymal tissue cell, an epidermal cell, an immortalized cell, a transformed cell, a nerve cell, or an immune cell.

The tissue includes a single-cell embryo or a constitutive cell, or a multiple-cell embryo, a fetal tissue and the like. Examples of a differentiating cell include adipose cells, fibroblast cells, muscular cells, myocardial cells, endothelial cells, nerve cells, glial cells, blood cells, megakaryocytes, lymphocytes, macrophages, neutrophils, eosinophils, basophils, mast cells, leucocytes, granulocytes, keratinocytes, cartilage cells, osteoblast cells, osteoclastic cells, liver cells, and endocrine or exocrine cells. As examples of these cells, CHO-K1 cells (RIKEN Cell bank), Drosophila S2 cells (Schneider, I. et al., J. Embryol. Exp. Morph., 27, p. 353-365 (1972), human HeLa cells (ATCC: CCL-2), or human HEK 293 cells (ATCC: CRL-1573), and the like are preferably used.

Examples of an individual used as a body to be subjected to the introduction of the oligonucleotide of the present invention further include specifically those belonging to plants, animals, protozoa, viruses, bacteria or fungi. The plant may be a monocotyledonous plant, a dicotyledonous plant, or a gymnospermous plant, and the animal may be a vertebrate or an invertebrate. The vertebrate is preferably a mammal including a mouse, a rat, a monkey, a dog, or a human.

As a method for introducing the oligonucleotide of the present invention into a body to be subjected to the introduction, a calcium phosphate method, an electroporation method, a lipofection method, viral infection, immersion in a 3L5-oligonucleotide solution, a transformation method, or the like is used when the body to be subjected to the introduction is a cell or a tissue. Examples of the method for introduction into an embryo include microinjection, an electroporation method, and viral infection. When the body to be subjected to the introduction is a plant, injection or perfusion into the cavity or interstitial cells or the like of the plant, or a method of spraying, is used. In the case of an animal individual, a method of systemic introduction by oral, topical, subcutaneous, intramuscular, intravenous, parenteral, transvaginal, transrectal, transnasal, transocular or transmembrane administration or the like, an electroporation method, or viral infection, is used. As a method for oral introduction, a method can also be used in which the oligonucleotide of the present invention is directly mixed with food for an organism.

For a method for introducing the oligonucleotide of the present invention into a patient, a colloidal dispersion system can be used in addition to the above. The colloidal dispersion system is expected to have an effect of improving the *in vivo* stability of a compound, or an effect of efficiently delivering a compound to a specific organ, tissue or cell. The colloidal dispersion system is not limited as long as it is normally used. Examples thereof can include lipid-based dispersion systems including polymer complexes, nanocapsules, microspheres, beads, oil-in-water emulsifiers, micelles, mixed micelles and liposomes. A plurality of liposomes and artificial membrane vesicles which have an effect of efficiently delivering a compound to a specific organ, tissue or cell are preferred (Mannino et al., Biotechniques, 1988, 6, p.682; Blume and Cevc, Biochem. et Biophys. Acta, 1990, 1029, p.91; Lappalainen et al., Antiviral Res., 1994, 23, p.119; Chonn and Cullis, Current Op. Biotech., 1995, 6, p.698). Single-membrane liposomes having a size in the range of 0.2 to 0.4 µm can encapsulate a significant proportion of an aqueous buffer containing macromolecules, and a compound is encapsulated in the resulting aqueous inner membranes, and delivered in a biologically active form to brain cells (Fraley et al., Trends Biochem. Sci., 1981, 6, p. 77).

A liposome normally has a composition such that a lipid, in particular, a phospholipid, in particular, a phospholipid having a high phase transition temperature is normally combined with one or more steroids, in particular, cholesterols. Examples of a lipid useful for liposome production include phosphatidyl compounds such as phosphatidylglycerol, phosphatidylcholine, phosphatidylserine, sphingolipid, phosphatidylethanolamine, cerebroside and ganglioside.

Diacylphosphatidylglycerol is particularly useful. Here, the lipid moiety contains 14 to 18 carbon atoms, in particular, 16 to 18 carbon atoms, and is saturated (lacks a double bond in a chain of 14 to 18 carbon atoms) .

Typical phospholipids include phosphatidylcholine, dipalmitoyl phosphatidylcholine, and distearoyl phosphatidylcholine.

Targeting of a colloidal dispersion system of liposomes may be passive or active.

Passive targeting is achieved by using the intrinsic tendency of liposomes to be distributed to reticuloendothelial cells of an organ containing sinusoidal capillaries.

On the other hand, examples of active targeting can include bonding a specific ligand such as a viral protein coat (Morishita et al., Proc. Natl. Acad. Sci. (U.S.A), 1993. 90, p. 8474-), a monoclonal antibody (or an appropriate binding moiety thereof), a sugar, a glycolipid or a protein (or an oligopeptide fragment thereof) to liposomes, or a method in which liposomes are modified by changing the composition of the liposomes in order to achieve distribution to organs and cell types other than naturally occurring localization sites.

The surface of a targeted colloidal dispersion system can be modified in various ways. In a delivery system in which targeting is performed with liposomes, lipid groups can be incorporated into lipid bilayers of liposomes in order to maintain a target ligand in close association with the lipid bilayers. Various linking groups can be used for linking a lipid chain to the target ligand.

A target ligand binding to a specific cell surface molecule that is predominantly found on cells to which delivery of the oligonucleotide of the present invention is desired can be, for example, (1) a hormone, a growth factor or an appropriate oligopeptide fragment thereof which is bound to a specific cell receptor that is predominantly expressed by cells to which delivery is desired, or (2) a polyclonal or monoclonal antibody or an appropriate fragment thereof (for example, Fab; F(ab')2) which specifically binds to an antigenic epitope that is predominantly found on target cells. Two or more biologically active agents can also be combined in a single liposome, and administered.

A drug agent that improves the intracellular stability of contents and/or targeting can also be added to the colloidal dispersion system.

The amount of the oligonucleotide of the present invention or a pharmaceutically acceptable salt thereof used varies depending on the symptom, age and the like. It is desirable that it be administered to an adult one to three times a day depending on the symptom in an amount of not less than 1 mg (preferably 30 mg) and not more than 2000 mg (preferably 1500 mg) per dose in the case of oral administration, not less than 0.5 mg (preferably 5 mg) and not more than 1000 mg (preferably 250 mg) per dose in the case of intravenous administration or subcutaneous administration, not less than 0.5 mg (preferably 5 mg) and not more than 500 mg (preferably 250 mg) in the case of intratracheal administration, and not less than 0.05 mg (preferably 0.5 mg) and not more than 10 mg (preferably 5 mg) in the case of intraocular administration. It is desirable that the administration be performed, depending on the symptom, one to three times a week in the case of a drug agent having improved stability, and one to three times a month, or every 3, 6, 12, 18 or 24 months in the case of a drug agent having further improved stability.

A pharmaceutical composition and formulation for topical administration includes a transdermal patch, an ointment, a lotion, a cream, a gel, a lozenge, a suppository, a spray, a solution and a powder.

The oligonucleotide of the present invention can suppress expression of TfR2 mRNA. The oligonucleotide of the present invention has low toxicity to cells, and can be applied to a pharmaceutical product having high safety. The oligonucleotide of the present invention, which suppresses expression of TfR2 mRNA, can thus suppress production of hepcidin which plays a key role in iron metabolism. The oligonucleotide of the present invention, which suppresses expression of TfR2 mRNA, can thus increase the plasma iron concentration and/or the HGB concentration. Although the mechanism of action is not limited, the oligonucleotide of the present invention can be used in the treatment or prevention of a disease that can be treated or prevented by suppressing expression of TfR2 mRNA and/or production of hepcidin. Examples of the disease include anemia. Although the mechanism of action is not limited, the RNAi-oligonucleotide of the present invention enables anemia to be treated or prevented by, for example, increasing the HGB concentration. The RNAi-oligonucleotide of the present invention can reduce the frequency and/or the amount of transfusion of blood cells to an anemia patient in need of transfusion of blood cells. For example, the frequency and/or the amount of transfusion of blood cells to an anemia patient for a given period of time (for example, 1 day, 1 week, 2 weeks, 4 weeks, 8 weeks, 12 weeks, 1 month, 2 months, 3 months, 6 months or 1 year) can be reduced by about 30%, about 40%, about 50%, about 60%, about 70%, about 80%, about 90%, or about 100%.

The present invention provides inventions for medical use, which include a pharmaceutical composition for use in the treatment or prevention of anemia, comprising the oligonucleotide of the present invention as an active ingredient, a method of treating or preventing anemia, comprising administering an effective amount of the oligonucleotide, the oligonucleotide for use in the treatment or prevention of anemia, and use of the oligonucleotide of the present invention in the production of a pharmaceutical composition for the treatment or prevention of anemia. Anemia that can be treated or prevented by the oligonucleotide of the present invention includes, for example, anemia associated with chronic inflammation (anemia of chronic disease (ACD)), anemia associated with bone-marrow dysfunction, iron-deficiency anemia, iron refractory iron deficiency anemia (IRIDA), anemia resistant to an erythropoietin stimulating agent (ESA), anemia induced by chemotherapy, aplastic anemia, Diamond-Blackfan anemia, and inherited abnormality of hemoglobin such as β thalassemia or sickle cell disease. Anemia associated with chronic inflammation includes, for example, anemia associated with autoimmune disease such as articular rheumatism, systemic erythematosus or autoimmune hematolytic anemia, anemia associated with infectious disease, anemia associated with inflammatory enteric disease such as inflammatory colitis, anemia associated with heart failure, anemia associated with chronic kidney disease, cancerous anemia, anemia associated with macrophage activation syndrome, and anemia associated with Castleman disease. Anemia associated with chronic kidney disease includes, for example, kidney disease involving diabetes, kidney disease involving hypertension, nephrotic syndrome (for example, congenital nephrotic syndrome of the Finnish type, diffuse mesangial sclerosis, minimal lesion nephrotic syndrome, focal segmental glomerulosclerosis, chronic nephropathy, and Galloway-Mowat syndrome), kidney disease involving chronic glomerulonephritis (for example, IgA nephropathy, mesangial proliferative glomerulonephritis, membranoproliferative glomerulonephritis, purpura nephritis, antiglomerular basement membrane nephritis (Goodpasture syndrome), Alport's syndrome, Epstein syndrome, Lupus nephritis, microscopic polyangiitis, atypical hemolytic uremic syndrome, nail-patella syndrome, fibronectin nephropathy, and lipoprotein glomerulopathy), kidney disease involving chronic tubulointerstitial nephritis, kidney disease involving chronic pyelonephritis, kidney disease involving deposition of amyloid, autosomal dominant tubulointerstitial kidney disease, kidney disease involving nephronophthisis, and kidney disease involving kidney malformation (for example, polycystic kidney disease). Cancerous anemia includes, for example, anemia associated with cancer such as multiple myeloma, acute leukemia, chronic leukemia, lung cancer or breast cancer. Anemia associated with bone-marrow dysfunction includes, for example, anemia associated with myelofibrosis, anemia associated with myelodysplastic syndrome, anemia associated with chronic myelomonocytic leukemia (CMML), anemia associated with chemotherapeutic myelosuppression, aplastic anemia, Diamond-Blackfan anemia, and Fanconi anemia.

The oligonucleotide of the present invention can be preferably used in the treatment or prevention of anemia associated with chronic inflammation and anemia associated with bone-marrow dysfunction, and can be more preferably used in the treatment or prevention of anemia associated with autoimmune disease such as articular rheumatism, systemic erythematosus or autoimmune hematolytic anemia, anemia associated with infectious disease, anemia associated with inflammatory enteric disease such as inflammatory colitis, anemia associated with heart failure, anemia associated with chronic kidney disease, cancerous anemia, anemia associated with Castleman disease, anemia associated with myelofibrosis, anemia associated with myelodysplastic syndrome, anemia associated with chronic myelomonocytic leukemia, and anemia associated with chemotherapeutic myelosuppression.

The present invention provides a method of treating or preventing anemia, comprising using the oligonucleotide of the present invention in combination with one or more other drug agents. The oligonucleotide of the present invention may be used in combination with one or more other drug agents as long as the effect of the present invention is exhibited. Preferably, use of the oligonucleotide of the present invention in combination with other drug agents having a different mechanism of action on anemic symptoms can provide a greater therapeutic effect. For example, for myelodysplastic syndrome (MDS), a drug agent such as an erythropoiesis stimulating agent (ESA) having a mechanism of action of promoting proliferation of erythrocytes to treat anemia or luspatercept having a mechanism of action of promoting differentiation of erythrocytes to treat anemia, or a drug agent such as azacitidine having a mechanism of action of suppressing methylation of DNA and killing and wounding abnormal cells may be prescribed, and such a drug agent and TfR2 siRNA have different mechanisms of action. Therefore, combined use of such a drug agent and TfR2 siRNA can provide a therapeutic effect higher than that of a single agent on **MDS.** For anemia associated with chronic kidney disease, a drug agent such as ESA or a hypoxia inducible factor prolyl hydroxylase (HIFPHD) inhibitor having a mechanism of action of promoting proliferation of erythrocytes to treat anemia may be prescribed, and such a drug agent and TfR2 siRNA have different mechanisms of action. Therefore, combined use of such a drug agent and TfR2 siRNA can provide a therapeutic effect higher than that of a single agent on anemia associated with chronic kidney disease. For myelofibrosis, ruxolitinib is known to have a therapeutic effect mainly on symptoms such as splenomegaly (N Engl J Med 2012; 366: 799-807). However, the drug agent is known to have a limited effect or no effect on anemic symptoms, or to worsen anemia (Journal of Hematology & Oncology 2013, 6: 79). For the mechanism in which ruxolitinib worsens anemia, it is considered that EPO signals required for proliferation of erythrocytic cells are suppressed by inhibiting JAK2. However, since ruxolitinib has a certain therapeutic effect on symptoms other than anemia in myelofibrosis, combined use of ruxolitinib and TfR2 siRNA can provide a beneficial therapeutic effect on anemia in myelofibrosis. Use in combination with a JAK2 inhibitor other than ruxolitinib can also provide a beneficial therapeutic effect on anemia in myelofibrosis. Examples of the other JAK2 inhibitor include fedratinib, pacritinib, and momelotinib.

When the oligonucleotide of the present invention is used in combination with other drug agents, the timing of administration of the RNAi-oligonucleotide of the present invention is not limited as long as the effect of the present invention is exhibited. It may be administered simultaneously with the other drug agent used in combination, or may be administered before or after administration of the other drug agent used in combination. Examples of the drug agent that can be used in combination with the RNAi-oligonucleotide of the present invention include other drugs for treating anemia, or drugs for treating a disease that causes anemia. Examples of another drug for treating anemia include ESA, a hypoxia inducible factor prolyl hydroxylase (HIFPHD) inhibitor, an oral iron supplement, an intravenous iron supplement, danazol, and luspatercept. Examples of ESA include epoetin alfa, epoetin beta, epoetin beta pegol, epoetin kappa, or darbepoetin alfa. Examples of a HIFPHD inhibitor include roxadustat, vadadustat, daprodustat, enarodustat, and molidustat. Examples of an oral iron supplement include ferrous citrate, ferric citrate, ferrous fumarate, soluble ferric pyrophosphate, and dry iron sulfide. Examples of an intravenous iron supplement include ferric carboxymaltose, and sugar-containing iron oxide. Examples of a drug for treating a disease that causes anemia include a drug for treating myelofibrosis, a drug for treating myelodysplastic syndrome, a drug for treating chronic kidney disease, or an anticancer agent. Examples of a drug for treating myelofibrosis include a JAK2 inhibitor. Examples of a JAK2 inhibitor include ruxolitinib, fedratinib, pacritinib, and momelotinib. Use in combination with ruxolitinib is preferred. Examples of a drug for treating myelodysplastic syndrome include azacytidine, and lenalidomide. Use in combination with azacytidine or lenalidomide is preferred. Examples of a drug for treating chronic kidney disease include a SGLT2 (sodium glucose co-transporter 2) inhibitor, a renin-angiotensin system inhibitor (for example, an angiotensin II receptor antagonist (ARB) and an angiotensin-converting enzyme (ACE) inhibitor), a calcium antagonist, a diuretic drug, a drug for treating diabetes, a drug for treating hyperlipidemia, a steroid, or an immunosuppressive drug. Examples of the anticancer agent include a chemotherapeutic drug. Examples of a chemotherapeutic drug include cisplatin, carboplatin, doxorubicin, paclitaxel, and amrubicin.

The oligonucleotide of the present invention may be used in combination with a drug for treating iron overload disorder. When transfusion of blood cells is performed for treatment of anemia, iron overload disorder can develop due to frequent transfusion of blood cells. Iron overload disorder is a pathological condition caused by accumulation of iron derived from transfused blood cells in tissues of the liver or heart, and can produce liver failure, heart failure, diabetes, articular pain, hypothyroidism, loss of pituitary function, sexual dysfunction or the like. Therefore, a drug for treating iron overload disorder may be used for treating or preventing iron overload disorder in the treatment of anemia, and can be preferably used in combination with the oligonucleotide of the present invention. Examples of a drug for treating iron overload disorder include an iron chelating agent, and examples of the iron chelating agent include Deferasirox, Deferoxamine, and Deferiprone.

### Examples

### (Examples 1 to 82)

Each of the compounds of Examples 1 to 82 shown in Table 1 is a double-stranded oligonucleotide consisting of a sense strand region and an antisense strand region which are oligonucleotides that are independent of each other. The complementary regions of the sense strand region and the antisense strand region all consist of natural RNA (that is, A(R), G(R), C(R) and U(R)). Each inter-nucleoside bond is a phosphodiester bond that is not chemically modified. The antisense strand region in the compound of each Example is an oligonucleotide which has a complementary region consisting of a sequence fully complementary to each target sequence (nucleotide sequence sandwiched between the start position and the end position) shown in Table 1 and in which two consecutive T(D) are bound as an overhang structure to the 3'-terminus of the complementary region. The sense strand region in the compound of each Example is an oligonucleotide which has a complementary region consisting of a sequence fully complementary to the nucleotide sequence in the complementary region of the corresponding antisense strand region and in which two consecutive T(D) are bound as an overhang structure to the 3'-terminus of the complementary region. In the compound of Example 61, all 19 base pairs in AD-47826 which is siRNA for human TfR2 (hTfR2) shown in WO 2012/177921 are natural RNA, and two consecutive T(D) are bound as an overhang structure to the 3'-terminus of each of the sense strand region and the antisense strand region.

The compounds of Table 1 were synthesized using a phosphoramidite method (Nucleic Acids Research, 12, 4539 (1984), Nature Communications 6, Article number: 6317 (2015)). The compound of each Example was identified by negative ion ESI mass spectrometry. Table 1 shows the results. In the compound of each Example, a sense strand region and an antisense strand region, each of which had been separately synthesized, were put in equimolar amounts into a tube, and annealed, and then confirmed to form a double-stranded oligonucleotide by using an unmodified gel.

**[Table 1-1]**

| Example | Name of compound | Start position of target sequence | End position of target sequence | Molecular weight of sense strand region | Molecular weight of antisense strand region |
|---|---|---|---|---|---|
| 1 | TfR2-001 | 2657 | 2675 | 6646.37 | 6649.27 |
| 2 | TfR2-002 | 1114 | 1132 | 6624.72 | 6685.04 |
| 3 | TfR2-003 | 399 | 417 | 6734.91 | 6548.29 |
| 4 | TfR2-004 | 2157 | 2175 | 6652.11 | 6629.77 |
| 5 | TfR2-005 | 2497 | 2515 | 6583.01 | 6715.25 |
| 6 | TfR2-006 | 1669 | 1687 | 6667.45 | 6628.97 |
| 7 | TfR2-007 | 2543 | 2561 | 6551.67 | 6733.92 |
| 8 | TfR2-008 | 918 | 936 | 6653.14 | 6628.17 |
| 9 | TfR2-009 | 2658 | 2676 | 6644.50 | 6633.72 |
| 10 | TfR2-010 | 2180 | 2198 | 6559.74 | 6722.03 |
| 11 | TfR2-011 | 1446 | 1464 | 6563.57 | 6729.86 |
| 12 | TfR2-012 | 1843 | 1861 | 6752.25 | 6540.43 |
| 13 | TfR2-013 | 1236 | 1254 | 6692.84 | 6605.73 |
| 14 | TfR2-014 | 1824 | 1842 | 6552.53 | 6748.08 |
| 15 | TfR2-015 | 2667 | 2685 | 6734.68 | 6550.24 |
| 16 | TfR2-016 | 1042 | 1060 | 6480.05 | 6820.56 |
| 17 | TfR2-017 | 2659 | 2677 | 6607.34 | 6678.06 |
| 18 | TfR2-018 | 2680 | 2698 | 6611.58 | 6669.11 |
| 19 | TfR2-019 | 2847 | 2865 | 6655.76 | 6613.08 |
| 20 | TfR2-020 | 2544 | 2562 | 6529.26 | 6757.86 |
| 21 | TfR2-021 | 2181 | 2199 | 6520.08 | 6760.85 |
| 22 | TfR2-022 | 1671 | 1689 | 6649.91 | 6645.88 |
| 23 | TfR2-023 | 1788 | 1806 | 6518.94 | 6777.18 |
| 24 | TfR2-024 | 1233 | 1251 | 6706.11 | 6605.52 |
| 25 | TfR2-025 | 2499 | 2517 | 6564.16 | 6716.94 |
| 26 | TfR2-026 | 2422 | 2440 | 6654.04 | 6611.86 |
| 27 | TfR2-027 | 1141 | 1159 | 6695.40 | 6621.10 |
| 28 | TfR2-028 | 2175 | 2193 | 6682.89 | 6631.59 |
| 29 | TfR2-029 | 1837 | 1855 | 6696.91 | 6583.11 |
| 30 | TfR2-030 | 2534 | 2552 | 6523.62 | 6771.59 |
| 31 | TfR2-031 | 1787 | 1805 | 6559.13 | 6752.32 |
| 32 | TfR2-032 | 917 | 935 | 6666.78 | 6630.22 |
| 33 | TfR2-033 | 2636 | 2654 | 6643.05 | 6656.46 |
| 34 | TfR2-034 | 894 | 912 | 6626.34 | 6667.53 |
| 35 | TfR2-035 | 2145 | 2163 | 6671.56 | 6621.64 |

**[Table 1-2]**

| Example | Name of compound | Start position of target sequence | End position of target sequence | Molecular weight of sense strand region | Molecular weight of antisense strand region |
|---|---|---|---|---|---|
| 36 | TfR2-036 | 1308 | 1326 | 6625.92 | 6667.59 |
| 37 | TfR2-037 | 2645 | 2663 | 6628.83 | 6668.09 |
| 38 | TfR2-038 | 2824 | 2842 | 6764.86 | 6546.52 |
| 39 | TfR2-039 | 1536 | 1554 | 6652.30 | 6646.75 |
| 40 | TfR2-040 | 1841 | 1859 | 6778.08 | 6502.47 |
| 41 | TfR2-041 | 2545 | 2563 | 6523.60 | 6740.06 |
| 42 | TfR2-042 | 504 | 522 | 6695.27 | 6620.27 |
| 43 | TfR2-043 | 93 | 111 | 6565.85 | 6746.68 |
| 44 | TfR2-044 | 414 | 432 | 6643.58 | 6649.31 |
| 45 | TfR2-045 | 415 | 433 | 6620.18 | 6691.65 |
| 46 | TfR2-046 | 2143 | 2161 | 6670.24 | 6661.91 |
| 47 | TfR2-047 | 1743 | 1761 | 6645.61 | 6666.08 |
| 48 | TfR2-048 | 1998 | 2016 | 6689.93 | 6641.90 |
| 49 | TfR2-049 | 1845 | 1863 | 6677.87 | 6606.31 |
| 50 | TfR2-050 | 1842 | 1860 | 6780.09 | 6501.11 |
| 51 | TfR2-051 | 1069 | 1087 | 6543.54 | 6773.30 |
| 52 | TfR2-052 | 1742 | 1760 | 6672.60 | 6628.87 |
| 53 | TfR2-053 | 2646 | 2664 | 6627.88 | 6673.84 |
| 54 | TfR2-054 | 1623 | 1641 | 6623.58 | 6676.32 |
| 55 | TfR2-055 | 2425 | 2443 | 6660.18 | 6610.74 |
| 56 | TfR2-056 | 2423 | 2441 | 6675.54 | 6590.43 |
| 57 | TfR2-057 | 2634 | 2652 | 6630.83 | 6670.68 |
| 58 | TfR2-058 | 64 | 82 | 6701.99 | 6600.48 |
| 59 | TfR2-059 | 1585 | 1603 | 6652.70 | 6631.57 |
| 60 | TfR2-060 | 1668 | 1686 | 6687.20 | 6631.76 |
| 61 | TfR2-061 | 241 | 259 | 6610.06 | 6701.54 |
| 62 | TfR2-062 | 1746 | 1764 | 6567.92 | 6732.37 |
| 63 | TfR2-063 | 2004 | 2022 | 6671.13 | 6645.01 |
| 64 | TfR2-064 | 396 | 414 | 6696.31 | 6588.21 |
| 65 | TfR2-065 | 1748 | 1766 | 6565.91 | 6732.86 |
| 66 | TfR2-066 | 2800 | 2818 | 6605.37 | 6707.92 |
| 67 | TfR2-067 | 397 | 415 | 6696.17 | 6593.60 |
| 68 | TfR2-068 | 306 | 324 | 6557.51 | 6786.94 |
| 69 | TfR2-069 | 1234 | 1252 | 6708.31 | 6607.12 |
| 70 | TfR2-070 | 2427 | 2445 | 6660.36 | 6593.84 |

**[Table 1-3]**

| Example | Name of compound | Start position of target sequence | End position of target sequence | Molecular weight of sense strand region | Molecular weight of antisense strand region |
|---|---|---|---|---|---|
| 71 | TfR2-071 | 2681 | 2699 | 6589.66 | 6709.78 |
| 72 | TfR2-072 | 2799 | 2817 | 6605.45 | 6709.89 |
| 73 | TfR2-073 | 465 | 483 | 6624.81 | 6692.06 |
| 74 | TfR2-074 | 1312 | 1330 | 6591.52 | 6696.28 |
| 75 | TfR2-075 | 1687 | 1705 | 6671.36 | 6632.90 |
| 76 | TfR2-076 | 2553 | 2571 | 6551.07 | 6701.44 |
| 77 | TfR2-077 | 2684 | 2702 | 6535.41 | 6751.70 |
| 78 | TfR2-078 | 994 | 1012 | 6764.41 | 6547.17 |
| 79 | TfR2-079 | 1789 | 1807 | 6559.74 | 6737.30 |
| 80 | TfR2-080 | 66 | 84 | 6676.65 | 6641.69 |
| 81 | TfR2-081 | 1468 | 1486 | 6719.88 | 6610.66 |
| 82 | TfR2-082 | 2647 | 2665 | 6631.34 | 6671.27 |

The "start position of target sequence" and the "end position of target sequence" in the tables each indicate the position of a base in the nucleotide sequence of human TfR2 mRNA set forth as SEQ ID NO: 1. The compound of each Example is a double-stranded oligonucleotide whose target sequence is a nucleotide sequence sandwiched between the start position and the end position. That is, in each Example, the antisense strand region has a sequence complementary to the nucleotide sequence sandwiched between the start position and the end position of the target sequence, and the sense strand region has a sequence complementary to the antisense strand region. For example, in the compound of Example 19 (TfR2-019), the sequence of the complementary region of the sense strand region is 5' CGUGGAGUUUCAAUAUCAA 3'

(SEQ ID NO: 43), and the sequence of the complementary region of the antisense strand region is 5' UUGAUAUUGAAACUCCACG 3' (SEQ ID NO: 44). In the compound of Example 39 (TfR2-039), the sequence of the complementary region of the sense strand region is 5' ACCUCAAAGCCGUAGUGUA 3' (SEQ ID NO: 45), and the sequence of the complementary region of the antisense strand region is 5' UACACUACGGCUUUGAGGU 3' (SEQ ID NO: 46). Two consecutive T(D) are bound as an overhang structure to the 3'-terminus of the complementary region in each of the sense strand region and the antisense strand region of the compound of each Example in Table 1. The residue at each of the 5'-position of the nucleotide at the 5'-terminus and the 3'-position of the nucleotide at the 3'-terminus of each oligonucleotide is a hydroxyl group in which a hydrogen atom is bonded to an oxygen atom in the above structural diagram of each nucleoside. The "molecular weight" in the tables indicates a value measured by negative ion ESI mass spectrometry.

### (Examples 83 to 184)

Each of the compounds of Examples 83 to 133 shown in Table 2 is a single-stranded oligonucleotide in which the residue at the 3'-position (2'-position in the case of a nucleotide modified at the 3'-position) of the nucleotide at the 3'-terminus of the sense strand region and the residue at the 5'-position of the nucleotide at the 5'-terminus of the antisense strand region are connected by a linker represented by the above formula (Z). The complementary region of the sense strand region and the complementary region of the antisense strand region form a double-stranded structure. Each of the compounds of Examples 134 to 184 shown in Table 3 is a double-stranded oligonucleotide having a sense strand region and an antisense strand region which are oligonucleotides that are independent of each other.

The compounds of Example 180 (TfR2-019. 94DUG. s1) and Example 181 (TfR2-019. 94DUG. s2) are siRNA in which each of the overhang structures at the 3'-termini of the antisense strand regions in the compound of Example 169 (TfR2-019. 94DUG) is one nucleotide, or is not present. The compounds of Example 182 (TfR2-019. 94DUG. e1) and Example 183 (TfR2-019. 94DUG. e2) are siRNA that targets a sequence obtained by extending the target sequence in the compound of Example 19 (TfR2-019) (that is, the sequence from positions 2847 to 2865 in SEQ ID NO: 1) toward the 5'-terminus side by 1 or 2 nucleotides in chain length (that is, the 2846th-to-2865th sequence and the 2845th-to-2865th sequence, respectively, of SEQ ID NO: 1), and have complementary region chain lengths of 20 bp and 21 bp, respectively. The compound of Example 184 (TfR2-019. 94DUG. e3) is siRNA in which the overhang structure at the 3'-terminus of the antisense strand region in the compound of Example 169 (TfR2-019. 94DUG) is G(M)U(M).

The compounds of Examples 83 to 184 shown in Tables 2 and 3 were synthesized in the same manner as in Examples 1 to 82. DMT-ethane-Diol phosphoramidite (ChemGene, catalog No: CLP-2250) was used in the synthesis of the moiety "2" and a method described in WO 2012/074038, Example 12 was used in the synthesis of the moiety "Z" in the sequence. The moiety "2'-O-methylnucleoside" was synthesized using a phosphoramidite described in Nucleic Acids Research 17, 3373 (1989). The moiety "DNA" was synthesized using a phosphoramidite described in Nucleic Acids Research 11, 4539 (1984). The moiety "3'-O-methylnucleoside" was synthesized using 3'-O-Methyl Adenosine (n-bz) CED phosphoramidite (ChemGene, catalog No: ANP-2901), 3'-O-Methyl Cytidine (n-bz) CED phosphoramidite (ChemGene, catalog No: ANP-2902), 3'-O-Methyl Guanosine (n-ibu) CED phosphoramidite (ChemGene, catalog No: ANP-2903), or 3'-O-Methyl Uridine CED phosphoramidite (ChemGene, catalog No: ANP-2904). The moiety "2'-O,4'-C-methylenenucleoside" was synthesized using a phosphoramidite described in WO 99/14226. The moiety "2'-deoxy-2'-fluoronucleoside" was synthesized using a phosphoramidite described in J. Med. Chem. 36, 831 (1993). The moiety "2'-O-methoxyethylnucleoside" was synthesized using a phosphoramidite described in Helv. Chim. Acta 78, 486-504 (1995). The moiety "GN" was synthesized using a phosphoramidite described in WO 2019/172286, Reference Example 41. For the compounds of Examples 134 to 179, the nucleotide sequence of the sense strand region is shown in Tables 3-1 to 3-5, and the nucleotide sequence of the antisense strand region is shown in Tables 3-6 to 3-10. For the compounds of Examples 180 to 184, the nucleotide sequence of the sense strand region is shown in Table 3-11, and the nucleotide sequence of the antisense strand region is shown in Table 3-12.

In each of the compounds of Examples 134 to 184, a sense strand region and an antisense strand region were put in equimolar amounts into a tube, and annealed, and then confirmed to form a double-stranded oligonucleotide by using an unmodified gel or an HPLC.

**[Table 2-1]**

| Example | Name of compound | Nucleotide sequence (5'-3') | Molecular weight | Theoretical value | SEQ ID NO |
|---|---|---|---|---|---|
| 83 | TfR2-003. 5 | G(M)^U(M)^G(M)A(M)G(F)G(M)A(F)U(F)G(F)U(M)C(M)A(M)A(M)C(M)U(M)A(M)U(M)G(M)A(M)Z U(M)^C(F)^A(M)U(M)A(M)G(F)U(M)U(F)G(F)A(M)C(M)A(M)U(M)C(F)C(M)U(F)C(M)A(M)C(M)^U(M)^U(M) | 13637.49 | 13635.29 | 2 |
| 84 | TfR2-005. 5 | G(M)^C(M)^U(M)C(M)C(F)U(M)C(F)G(F)C(F)U(M)U(M)G(M)A(M)A(M)U(M)G(M)A(M)U(M)U(M)Z A(M)^A(F)^U(M)C(M)A(M)U(F)U(M)C(F)A(F)A(M)G(M)C(M)G(M)A(F)G(M)G(F)A(M)G(M)C(M)^U(M)^U(M) | 13651.48 | 13650.29 | 3 |
| 85 | TfR2-006, 5 | U(M)^G(M)^G(M)G(M)C(F)A(M)G(F)A(F)C(F)U(M)C(M)U(M)C(M)U(M)A(M)U(M)G(M)A(M)A(M)Z U(M)^U(F)^C(M)A(M)U(M)A(F)G(M)A(F)G(F)A(M)G(M)U(M)C(M)U(F)G(M)C(F)C(M)C(M)A(M)^U(M)^U(M) | 13647.17 | 13650.29 | 4 |
| 86 | TfR2-022. 5 | G(M)^G(M)^C(M)A(M)G(F)A(M)C(F)U(F)C(F)U(M)C(M)U(M)A(M)U(M)G(M)A(M)A(M)C(M)A(M)Z U(M)^G(F)^U(M)U(M)C(M)A(F)U(M)A(F)G(F)A(M)G(M)A(M)G(M)U(F)C(M)U(F)G(M)C(M)C(M)^U(M)^U(M) | 13648.50 | 13650.29 | 5 |
| 87 | TfR2-039. 5 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 13652.26 | 13650.29 | 6 |
| 88 | TfR2-046. 5 | G(M)^C(M)^G(M)A(M)C(F)U(M)G(F)A(F)C(F)A(M)C(M)G(M)C(M)A(M)U(M)G(M)U(M)A(M)C(M)Z G(M)^U(F)^A(M)C(M)A(M)U(F)G(M)C(F)G(F)U(M)G(M)U(M)C(M)A(F)G(M)U(F)C(M)G(M)C(M)^U(M)^U(M) | 13676.17 | 13680.29 | 7 |
| 89 | TfR2-065. 5 | A(M)^G(M)^C(M)A(M)G(F)U(M)G(F)C(F)C(F)U(M)A(M)U(M)U(M)C(M)C(M)U(M)U(M)C(M)A(M)Z U(M)^G(F)^A(M)A(M)G(M)G(F)A(M)A(F)U(F)A(M)G(M)G(M)C(M)A(F)C(M)U(F)G(M)C(M)U(M)^U(M)^U(M) | 13647.20 | 13650.29 | 8 |
| 90 | TfR2-007. 5 | A(M)^C(M)^C(M)U(M)C(F)U(M)C(F)A(F)U(F)U(M)G(M)C(M)U(M)G(M)A(M)U(M)C(M)A(M)A(M)Z U(M)^U(F)^G(M)A(M)U(M)C(F)A(M)G(F)C(F)A(M)A(M)U(M)G(M)A(F)G(M)A(F)G(M)G(M)U(M)^U(M)^U(M) | 13632.75 | 13635.29 | 9 |
| 91 | TfR2-013. 5 | G(M)^G(M)^C(M)U(M)A(F)G(M)U(F)G(F)G(F)U(M)C(M)A(M)A(M)C(M)A(M)A(M)U(M)C(M)A(M)Z U(M)^G(F)^A(M)U(M)U(M)G(F)U(M)U(F)G(F)A(M)C(M)C(M)A(M)C(F)U(M)A(F)G(M)C(M)C(M)^U(M)^U(M) | 13648.57 | 13650.29 | 10 |
| 92 | TfR2-019. 5 | C(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(F)G(F)A(M)A(M)A(M)C(M)U(F)C(M)C(F)A(M)C(M)G(M)^U(M)^U(M) | 13617.57 | 13620.29 | 11 |

**[Table 2-2]**

| Example | Name of compound | Nucleotide sequence (5'-3') | Molecular weight | Theoretical value | SEQ ID NO |
|---|---|---|---|---|---|
| 93 | TfR2-037. 5 | A(M)^C(M)^C(M)C(M)U(F)A(M)A(F)U(F)G(F)U(M)G(M)A(M)C(M)G(M)G(M)U(M)C(M)A(M)U(M)Z A(M)^U(F)^G(M)A(M)C(M)C(F)G(M)U(F)C(F)A(M)C(M)A(M)U(M)U(F)A(M)G(F)G(M)G(M)U(M)^U(M)^U(M) | 13652.76 | 13650.29 | 12 |
| 94 | TfR2-076. 5 | G(M)^C(M)^U(M)G(M)A(F)U(M)C(F)A(F)A(F)U(M)U(M)U(M)C(M)U(M)C(M)A(M)U(M)U(M)A(M)Z U(M)^A(F)^A(M)U(M)G(M)A(F)G(M)A(F)A(F)A(M)U(M)U(M)G(M)A(F)U(M)C(F)A(M)G(M)C(M)^U(M)^U(M) | 13602 76 | 13605.29 | 13 |
| 95 | TfR2-077. 5 | U(M)^A(M)^G(M)C(M)A(F)U(M)C(F)C(F)C(F)U(M)U(M)C(M)A(M)A(M)U(M)C(M)A(M)C(M)A(M)Z U(M)^G(F)^U(M)G(M)A(M)U(F)U(M)G(F)A(F)A(M)G(M)G(M)G(M)A(F)U(M)G(F)C(M)U(M)A(M)^U(M)^U(M) | 13632.45 | 13635.29 | 14 |
| 96 | TfR2-081. 5 | C(M)^U(M)^G(M)G(M)G(F)A(M)C(F)G(F)G(F)U(M)G(M)G(M)U(M)G(M)A(M)C(M)U(M)U(M)U(M)Z A(M)^A(F)^A(M)G(M)U(M)C(F)A(M)C(F)C(F)A(M)C(M)C(M)G(M)U(F)C(M)C(F)C(M)A(M)G(M)^U(M)^U(M) | 13682.43 | 13680.29 | 15 |
| 97 | TfR2-061. 5 | C(M)^A(M)^G(M)G(M)C(F)A(M)G(F)C(F)C(F)A(M)A(M)A(M)C(M)C(M)U(M)C(M)A(M)U(M)U(M)Z A(M)^A(F)^U(M)G(M)A(M)G(F)G(M)U(F)U(F)U(M)G(M)G(M)C(M)U(F)G(M)C(F)C(M)U(M)G(M)^U(M)^U(M) | 13666.44 | 13665.29 | 16 |
| 98 | TfR2-006. 3 | U(M)^G(M)^G(M)G(M)C(D)A(M)G(D)A(D)C(D)U(M)C(M)U(M)C(M)U(M)A(M)U(M)G(M)A(M)A(M)Z U(M)^T(D)^C(M)A(M)U(M)A(D)G(M)A(D)G(D)A(M)G(M)U(M)C(M)T(D)G(M)C(D)C(M)C(M)A(M)^U(M)^U(M) | 13496.61 | 13498.37 | 17 |
| 99 | TfR2-007. 3 | A(M)^C(M)^C(M)U(M)C(D)U(M)C(D)A(D)T(D)U(M)G(M)C(M)U(M)G(M)A(M)U(M)C(M)A(M)A(M)Z U(M)^T(D)^G(M)A(M)U(M)C(D)A(M)G(D)C(D)A(M)A(M)U(M)G(M)A(D)G(M)A(D)G(M)G(M)U(M)^U(M)^U(M) | 1348110 | 13483.36 | 18 |
| 100 | TfR2-013. 3 | G(M)^G(M)^C(M)U(M)A(D)G(M)T(D)G(D)G(D)U(M)C(M)A(M)A(M)C(M)A(M)A(M)U(M)C(M)A(M)Z U(M)^G(D)^A(M)U(M)U(M)G(D)U(M)T(D)G(D)A(M)C(M)C(M)A(M)C(D)U(M)A(D)G(M)C(M)C(M)^U(M)^U(M) | 13496.24 | 13498.40 | 19 |
| 101 | TfR2-019. 3 | C(M)^G(M)^U(M)G(M)G(D)A(M)G(D)T(D)T(D)U(M)C(M)A(M)A(M)U(M)A(M)U(M)C(M)A(M)A(M)Z U(M)^T(D)^G(M)A(M)U(M)A(D)U(M)T(D)G(D)A(M)A(M)A(M)C(M)T(D)C(M)C(D)A(M)C(M)G(M)^U(M)^U(M) | 13507.44 | 13510.47 | 20 |
| 102 | TfR2-039. 3 | A(M)^C(M)^C(M)U(M)C(D)A(M)A(D)A(D)G(D)C(M)C(M)G(M)U(M)A(M)G(M)U(M)G(M)U(M)A(M)Z U(M)^A(D)^C(M)A(M)C(M)T(D)A(M)C(D)G(D)G(M)C(M)U(M)U(M)T(D)G(M)A(D)G(M)G(M)U(M)^U(M)^U(M) | 13496.54 | 13498.37 | 21 |

**[Table 2-3]**

| Example | Name of compound | Nucleotide sequence (5'-3') | Molecular weight | Theoretical value | SEQ ID NO |
|---|---|---|---|---|---|
| 103 | TfR2-076. 3 | G(M)^C(M)^U(M)G(M)A(D)U(M)C(D)A(D)A(D)U(M)U(M)U(M)C(M)U(M)C(M)A(M)U(M)U(M)A(M)Z U(M)^A(D)^A(M)U(M)G(M)A(D)G(M)A(D)A(D)A(M)U(M)U(M)G(M)A(D)U(M)C(D)A(M)G(M)C(M)^U(M)^U(M) | 13422.42 | 13425.29 | 13 |
| 104 | TR2-006. 6 | U(M)^G(M)^G(M)G(M)C(F)A(M)G(F)A(F)C(F)U(M)C(M)U(M)C(M)U(M)A(M)U(M)G(M)A(M)A(M)Z U(M)^U(F)^C(M)A(M)U(M)A(F)G(M)A(M)G(F)A(M)G(M)U(M)C(M)U(F)G(M)C(F)C(M)C(M)A(M)^U(M)^U(M) | 13661.03 | 13662 32 | 4 |
| 105 | TfR2-007. 6 | A(M)^C(M)^C(M)U(M)C(F)U(M)C(F)A(F)U(F)U(M)G(M)C(M)U(M)G(M)A(M)U(M)C(M)A(M)A(M)Z U(M)^U(F)^G(M)A(M)U(M)C(F)A(M)G(M)C(F)A(M)A(M)U(M)G(M)A(F)G(M)A(F)G(M)G(M)U(M)^U(M)^U(M) | 13645-38 | 13647 32 | 9 |
| 106 | TfR2-013. 6 | G(M)^G(M)^C(M)U(M)A(F)G(M)U(F)G(F)G(F)U(M)C(M)A(M)A(M)C(M)A(M)A(M)U(M)C(M)A(M)Z U(M)^G(F)^A(M)U(M)U(M)G(F)U(M)U(M)G(F)A(M)C(M)C(M)A(M)C(F)U(M)A(F)G(M)C(M)C(M)^U(M)^U(M) | 13661.03 | 13662.32 | 10 |
| 107 | TfR2-019. 6 | C(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 13630.35 | 13632.32 | 11 |
| 108 | TfR2-038. 6 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 13660.75 | 13662.32 | 6 |
| 109 | TfR2-076. 6 | G(M)^C(M)^U(M)G(M)A(F)U(M)C(F)A(F)A(F)U(M)U(M)U(M)C(M)U(M)C(M)A(M)U(M)U(M)A(M)Z U(M)^A(F)^A(M)U(M)G(M)A(F)G(M)A(M)A(F)A(M)U(M)U(M)G(M)A(F)U(M)C(F)A(M)G(M)C(M)^U(M)^U(M) | 13615.42 | 13617.32 | 13 |
| 110 | TfR2-039. 8 | A(D)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 13635.56 | 13632.29 | 6 |
| 111 | TfR2-039. 9 | A(D)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(M)G(M)C(M)U(M)U(M)U(F)G(M)A(F)G(M)G(M)U(M)^U(M)^U(M) | 13633.74 | 13632.29 | 6 |
| 112 | TfR2-039. 10 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(F)G(F)G(M)C(M)U(M)U(M)U(F)G(M)A(F)G(M)G(M)U(M)^U(M)^U(M) | 13636.50 | 13634.29 | 22 |
| 113 | TfR2-039. 11 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(M)G(F)G(M)C(M)U(M)U(M)U(F)G(M)A(F)G(M)G(M)U(M)^U(M)^U(M) | 13649.50 | 13646.32 | 22 |

**[Table 2-4]**

| Example | Name of compound | Nucleotide sequence (5'-3') | Molecular weight | Theoretical value | SEQ ID NO |
|---|---|---|---|---|---|
| 114 | TfR2-039. 12 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(F)G(M)G(M)C(M)U(M)U(M)U(F)G(M)A(F)G(M)G(M)U(M)^U(M)^U(M) | 13650.20 | 13646.32 | 22 |
| 115 | TfR2-019, 8 | C(D)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 13605.92 | 13602.29 | 11 |
| 115 | TfR2-019. 9 | C(D)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(F)G(M)A(M)A(M)A(M)C(M)U(F)C(M)C(F)A(M)C(M)G(M)^U(M)^U(M) | 13599.79 | 13602.29 | 11 |
| 117 | TfR2-019. 15 | C(M)^G(M)^U(M)G(M)G(F)A(M)G(F)U(F)T(D)U(M)C(M)A(M)A(M)U(M)A(M)U(M)C(M)^A(M)^A(D)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 13633.59 | 13630.46 | 23 |
| 118 | TfR2-019. 17 | C(M)^G(M)^U(M)G(M)G(F)A(M)G(F)U(F)T(D)U(M)C(M)A(M)A(M)U(M)A(M)U(M)C(M)^A(M)^A(D)Z U(M)^U(F)^G(M)A(M)U(M)A(F)U(M)U(F)G(M)A(M)A(M)A(M)C(M)U(F)C(M)C(F)A(M)C(M)G(M)^U(M)^U(M) | 13634.22 | 13630.46 | 23 |
| 119 | TfR2-039. 19 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(M)G(M)C(M)U(M)U(M)U(F)G(M)A(F)G(M)G(M)U(M)^U(M)^U(M) | 13675.42 | 13674.35 | 6 |
| 120 | TfR2-039. 20 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(M)G(M)G(M)C(M)U(M)U(M)U(F)G(M)A(F)G(M)G(M)U(M)^U(M)^U(M) | 13662.17 | 13658.35 | 22 |
| 121 | TfR2-039. 21 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 13650.03 | 13650 29 | 6 |
| 122 | TfR2-039. 22 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 13663.25 | 13662.32 | 6 |
| 123 | TfR2-039. 23 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(F)G(M)G(M)C(M)U(M)U(M)U(F)G(M)A(F)G(M)G(M)U(M)^U(M)^U(M) | 13664.40 | 13662.32 | 6 |
| 124 | TfR2-039. 24 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(M)G(M)C(M)U(M)U(M)U(F)G(M)A(F)G(M)G(M)U(M)^U(M)^U(M) | 13669.00 | 13674.35 | 6 |

### [Table 2-5]

**[Table 2-5]**

| Example | Name of compound | Nucleotide sequence (5'-3') | Molecular weight | Theoretical value | SEQ ID NO |
|---|---|---|---|---|---|
| 125 | TfR2-039. 25 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(M)A(M)C(M)G(M)G(M)C(M)U(M)U(M)U(F)G(M)A(F)G(M)G(M)U(M)^U(M)^U(M) | 13690.27 | 13686.38 | 6 |
| 126 | TfR2-039. 26 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(M)G(M)C(M)U(M)U(M)U(F)G(M)A(M)G(M)G(M)U(M)^U(M)^U(M) | 13687.73 | 13686.38 | 6 |
| 127 | TfR2-039. 5G | GNA(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 14554.80 | 14547.45 | 6 |
| 128 | TfR2-019. 6G | GNC(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 14536.84 | 14529.45 | 11 |
| 129 | TfR2-019. 22 | C(M)^G(M)^U(M)G(M)G(F)A(M)G(F)U(F)U(F)U(M)C(M)A(M)A(M)U(M)A(M)U(M)C(M)A(M)A(3M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 13632.67 | 13632.32 | 11 |
| 130 | TfR2-019. 22s | C(M)^G(M)^U(M)G(M)G(F)A(M)G(F)U(F)U(F)U(M)C(M)A(M)A(M)U(M)A(M)U(M)C(M)^A(M)^A(3M)^Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 13679.53 | 13680.53 | 11 |
| 131 | AD47882. 5G | GNC(M)^C(M)^A(M)C(M)G(F)U(M)G(F)A(F)U(F)U(M)C(M)U(M)C(M)C(M)U(M)U(M)U(M)C(M)U(M)Z A(M)^G(F)^A(M)A(M)A(M)G(F)G(M)A(F)G(F)A(M)A(M)U(M)C(M)A(F)C(M)G(F)U(M)G(M)G(M)^U(M)^U(M) | 14555.13 | 14554.85 | 24 |
| 132 | AD47882. 23G | GNC(M)^C(M)^A(M)C(M)G(F)U(M)G(F)A(F)U(F)U(M)C(M)U(M)C(M)C(M)U(M)U(M)U(M)C(M)U(3M)Z A(M)^G(F)^A(M)A(M)A(M)G(F)G(M)A(F)G(F)A(M)A(M)U(M)C(M)A(F)C(M)G(F)U(M)G(M)G(M)^U(M)^U(M) | 14560.47 | 14559.47 | 24 |
| 133 | AD47882. 23sG | GNC(M)^C(M)^A(M)C(M)G(F)U(M)G(F)A(F)U(F)U(M)C(M)U(M)C(M)C(M)U(M)U(M)U(M)^C(M)^U(3M)^Z A(M)^G(F)^A(M)A(M)A(M)G(F)G(M)A(F)G(M)A(M)A(M)U(M)C(M)A(F)C(M)G(F)U(M)G(M)G(M)^U(M)^U(M) | 14615.25 | 14616.08 | 24 |

**[Table 2-6]**

| Name of compound | Nucleotide sequence (5'-3') | SEQ ID NO |
|---|---|---|
| TfR2-003.5 | G(M)^U(M)^G(M)A(M)G(F)G(M)A(F)U(F)G(F)U(M)C(M)A(M)A(M)C(M)U(M)A(M)U(M)G(M)A(M)Z U(M)^C(F)^A(M)U(M)A(M)G(F)U(M)U(F)G(F)A(M)C(M)A(M)U(M)C(F)C(M)U(F)C(M)A(M)C(M)^U(M)^U(M) | 2 |
| TIR2-005.5 | G(M)^C(M)^U(M)C(M)C(F)U(M)C(F)G(F)C(F)U(M)U(M)G(M)A(M)A(M)U(M)G(M)A(M)U(M)U(M)Z A(M)^A(F)^U(M)C(M)A(M)U(F)U(M)C(F)A(F)A(M)G(M)C(M)G(M)A(F)G(M)G(F)A(M)G(M)C(M)^U(M)^U(M) | 3 |
| TfR2-006.5 | U(M)^G(M)^G(M)G(M)C(F)A(M)G(F)A(F)C(F)U(M)C(M)U(M)C(M)U(M)A(M)U(M)G(M)A(M)A(M)Z U(M)^U(F)^C(M)A(M)U(M)A(F)G(M)A(F)G(F)A(M)G(M)U(M)C(M)U(F)G(M)C(F)C(M)C(M)A(M)^U(M)^U(M) | 4 |
| TfR2-022.5 | G(M)^G(M)^C(M)A(M)G(F)A(M)C(F)U(F)C(F)U(M)C(M)U(M)A(M)U(M)G(M)A(M)A(M)C(M)A(M)Z U(M)^G(F)^U(M)U(M)C(M)A(F)U(M)A(F)G(F)A(M)G(M)A(M)G(M)U(F)C(M)U(F)G(M)C(M)C(M)^U(M)^U(M) | 5 |
| TfR2-039.5 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 6 |
| TfR2-046.5 | G(M)^C(M)^G(M)A(M)C(F)U(M)G(F)A(F)C(F)A(M)C(M)G(M)C(M)A(M)U(M)G(M)U(M)A(M)C(M)Z G(M)^U(F)^A(M)C(M)A(M)U(F)G(M)C(F)G(F)U(M)G(M)U(M)C(M)A(F)G(M)U(F)C(M)G(M)C(M)^U(M)^U(M) | 7 |
| TfR2-065.5 | A(M)^G(M)^C(M)A(M)G(F)U(M)G(F)C(F)C(F)U(M)A(M)U(M)U(M)C(M)C(M)U(M)U(M)C(M)A(M)Z U(M)^G(F)^A(M)A(M)G(M)G(F)A(M)A(F)U(F)A(M)G(M)G(M)C(M)A(F)C(M)U(F)G(M)C(M)U(M)^U(M)^U(M) | 8 |
| TfR2-007.5 | A(M)^C(M)^C(M)U(M)C(F)U(M)C(F)A(F)U(F)U(M)G(M)C(M)U(M)G(M)A(M)U(M)C(M)A(M)A(M)Z U(M)^U(F)^G(M)A(M)U(M)C(F)A(M)G(F)C(F)A(M)A(M)U(M)G(M)A(F)G(M)A(F)G(M)G(M)U(M)^U(M)^U(M) | 9 |

**[Table 2-7]**

| Name of compound | Nucleotide sequence (5'-3') | SEQ ID NO |
|---|---|---|
| TfR2-013.5 | G(M)^G(M)^C(M)U(M)A(F)G(M)U(F)G(F)G(F)U(M)C(M)A(M)A(M)C(M)A(M)A(M)U(M)C(M)A(M)Z U(M)^G(F)^A(M)U(M)U(M)G(F)U(M)U(F)G(F)A(M)C(M)C(M)A(M)C(F)U(M)A(F)G(M)C(M)C(M)^U(M)^U(M) | 10 |
| TfR2-019.5 | C(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(F)G(F)A(M)A(M)A(M)C(M)U(F)C(M)C(F)A(M)C(M)G(M)^U(M)^U(M) | 11 |
| TfR2-037.5 | A(M)^C(M)^C(M)C(M)U(F)A(M)A(F)U(F)G(F)U(M)G(M)A(M)C(M)G(M)G(M)U(M)C(M)A(M)U(M)Z A(M)^U(F)^G(M)A(M)C(M)C(F)G(M)U(F)C(F)A(M)C(M)A(M)U(M)U(F)A(M)G(F)G(M)G(M)U(M)^U(M)^U(M) | 12 |
| TfR2-076.5 | G(M)^C(M)^U(M)G(M)A(F)U(M)C(F)A(F)A(F)U(M)U(M)U(M)C(M)U(M)C(M)A(M)U(M)U(M)A(M)Z U(M)^A(F)^A(M)U(M)G(M)A(F)G(M)A(F)A(F)A(M)U(M)U(M)G(M)A(F)U(M)C(F)A(M)G(M)C(M)^U(M)^U(M) | 13 |
| TfR2-077.5 | U(M)^A(M)^G(M)C(M)A(F)U(M)C(F)C(F)C(F)U(M)U(M)C(M)A(M)A(M)U(M)C(M)A(M)C(M)A(M)Z U(M)^G(F)^U(M)G(M)A(M)U(F)U(M)G(F)A(F)A(M)G(M)G(M)G(M)A(F)U(M)G(F)C(M)U(M)A(M)^U(M)^U(M) | 14 |
| TfR2-081.5 | C(M)^U(M)^G(M)G(M)G(F)A(M)C(F)G(F)G(F)U(M)G(M)G(M)U(M)G(M)A(M)C(M)U(M)U(M)U(M)Z A(M)^A(F)^A(M)G(M)U(M)C(F)A(M)C(F)C(F)A(M)C(M)C(M)G(M)U(F)C(M)C(F)C(M)A(M)G(M)^U(M)^U(M) | 15 |
| TfR2.061.5 | C(M)^A(M)^G(M)G(M)C(F)A(M)G(F)C(F)C(F)A(M)A(M)A(M)C(M)C(M)U(M)C(M)A(M)U(M)U(M)Z A(M)^A(F)^U(M)G(M)A(M)G(F)G(M)U(F)U(F)U(M)G(M)G(M)C(M)U(F)G(M)C(F)C(M)U(M)G(M)^U(M)^U(M) | 16 |
| TfR2-006.3 | U(M)^G(M)^G(M)G(M)C(D)A(M)G(D)A(D)C(D)U(M)C(M)U(M)C(M)U(M)A(M)U(M)G(M)A(M)A(M)Z U(M)^T(D)^C(M)A(M)U(M)A(D)G(M)A(D)G(D)A(M)G(M)U(M)C(M)T(D)G(M)C(D)C(M)C(M)A(M)^U(M)^U(M) | 17 |

**[Table 2-8]**

| Name of compound | Nucleotide sequence (5'-3') | SEQ ID NO |
|---|---|---|
| TfR2-007.3 | A(M)^C(M)^C(M)U(M)C(D)U(M)C(D)A(D)T(D)U(M)G(M)C(M)U(M)G(M)A(M)U(M)C(M)A(M)A(M)Z U(M)^T(D)^G(M)A(M)U(M)C(D)A(M)G(D)C(D)A(M)A(M)U(M)G(M)A(D)G(M)A(D)G(M)G(M)U(M)^U(M)^U(M) | 18 |
| TfR2-013.3 | G(M)^G(M)^C(M)U(M)A(D)G(M)T(D)G(D)G(D)U(M)C(M)A(M)A(M)C(M)A(M)A(M)U(M)C(M)A(M)Z U(M)^G(D)^A(M)U(M)U(M)G(D)U(M)T(D)G(D)A(M)C(M)C(M)A(M)C(D)U(M)A(D)G(M)C(M)C(M)^U(M)^U(M) | 19 |
| TfR2-019.3 | C(M)^G(M)^U(M)G(M)G(D)A(M)G(D)T(D)T(D)U(M)C(M)A(M)A(M)U(M)A(M)U(M)C(M)A(M)A(M)Z U(M)^T(D)^G(M)A(M)U(M)A(D)U(M)T(D)G(D)A(M)A(M)A(M)C(M)T(D)C(M)C(D)A(M)C(M)G(M)^U(M)^U(M) | 20 |
| TfR2-039.3 | A(M)^C(M)^C(M)U(M)C(D)A(M)A(D)A(D)G(D)C(M)C(M)G(M)U(M)A(M)G(M)U(M)G(M)U(M)A(M)Z U(M)^A(D)^C(M)A(M)C(M)T(D)A(M)C(D)G(D)G(M)C(M)U(M)U(M)T(D)G(M)A(D)G(M)G(M)U(M)^U(M)^U(M) | 21 |
| TfR2-076.3 | G(M)^C(M)^U(M)G(M)A(D)U(M)C(D)A(D)A(D)U(M)U(M)U(M)C(M)U(M)C(M)A(M)U(M)U(M)A(M)Z U(M)^A(D)^A(M)U(M)G(M)A(D)G(M)A(D)A(D)A(M)U(M)U(M)G(M)A(D)U(M)C(D)A(M)G(M)C(M)^U(M)^U(M) | 13 |
| TfR2-006.6 | U(M)^G(M)^G(M)G(M)C(F)A(M)G(F)A(F)C(F)U(M)C(M)U(M)C(M)U(M)A(M)U(M)G(M)A(M)A(M)Z U(M)^U(F)^C(M)A(M)U(M)A(F)G(M)A(M)G(F)A(M)G(M)U(M)C(M)U(F)G(M)C(F)C(M)C(M)A(M)^U(M)^U(M) | 4 |
| TfR2-007.6 | A(M)^C(M)^C(M)U(M)C(F)U(M)C(F)A(F)U(F)U(M)G(M)C(M)U(M)G(M)A(M)U(M)C(M)A(M)A(M)Z U(M)^U(F)^G(M)A(M)U(M)C(F)A(M)G(M)C(F)A(M)A(M)U(M)G(M)A(F)G(M)A(F)G(M)G(M)U(M)^U(M)^U(M) | 9 |
| TfR2-013.6 | G(M)^G(M)^C(M)U(M)A(F)G(M)U(F)G(F)G(F)U(M)C(M)A(M)A(M)C(M)A(M)A(M)U(M)C(M)A(M)Z U(M)^G(F)^A(M)U(M)U(M)G(F)U(M)U(M)G(F)A(M)C(M)C(M)A(M)C(F)U(M)A(F)G(M)C(M)C(M)^U(M)^U(M) | 10 |

**[Table 2-9]**

| Name of compound | Nucleotide sequence (5'-3') | SEQ ID NO |
|---|---|---|
| TfR2-019.6 | C(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 11 |
| TfR2-039.6 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 6 |
| TfR2-076.6 | G(M)^C(M)^U(M)G(M)A(F)U(M)C(F)A(F)A(F)U(M)U(M)U(M)C(M)U(M)C(M)A(M)U(M)U(M)A(M)Z U(M)^A(F)^A(M)U(M)G(M)A(F)G(M)A(M)A(F)A(M)U(M)U(M)G(M)A(F)U(M)C(F)A(M)G(M)C(M)^U(M)^U(M) | 13 |
| TfR2-039.8 | A(D)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 6 |
| TfR2-039.9 | A(D)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(M)G(M)C(M)U(M)U(M)U(F)G(M)A(F)G(M)G(M)U(M)^U(M)^U(M) | 6 |
| TfR2-039.10 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(F)G(F)G(M)C(M)U(M)U(M)U(F)G(M)A(F)G(M)G(M)U(M)^U(M)^U(M) | 22 |
| TfR2-039.11 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(M)G(F)G(M)C(M)U(M)U(M)U(F)G(M)A(F)G(M)G(M)U(M)^U(M)^U(M) | 22 |
| TfR2-039.12 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(F)G(M)G(M)C(M)U(M)U(M)U(F)G(M)A(F)G(M)G(M)U(M)^U(M)^U(M) | 22 |

**[Table 2-10]**

| Name of compound | Nucleotide sequence (5'-3') | SEQ ID NO |
|---|---|---|
| TfR2-019.8 | C(D)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 11 |
| TfR2-019.9 | C(D)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(F)G(M)A(M)A(M)A(M)C(M)U(F)C(M)C(F)A(M)C(M)G(M)^U(M)^U(M) | 11 |
| TfR2-019.15 | C(M)^G(M)^U(M)G(M)G(F)A(M)G(F)U(F)T(D)U(M)C(M)A(M)A(M)U(M)A(M)U(M)C(M)^A(M)^A(D)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 23 |
| TfR2-019.17 | C(M)^G(M)^U(M)G(M)G(F)A(M)G(F)U(F)T(D)U(M)C(M)A(M)A(M)U(M)A(M)U(M)C(M)^A(M)^A(D)Z U(M)^U(F)^G(M)A(M)U(M)A(F)U(M)U(F)G(M)A(M)A(M)A(M)C(M)U(F)C(M)C(F)A(M)C(M)G(M)^U(M)^U(M) | 23 |
| TfR2-039.19 | A(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(M)G(M)G(M)C(M)U(M)U(M)U(F)G(M)A(F)G(M)G(M)U(M)^U(M)^U(M) | 6 |
| TfR2-039.20 | A(M)^C(M)^C(M)U(M)C(F)A(M)A(F)A(F)G(F)C(M)C(M)G(M)U(M)A(M)G(M)U(M)G(M)U(M)A(M)Z T(D)^A(F)^C(M)A(M)C(M)U(F)A(M)C(M)G(M)G(M)C(M)U(M)U(M)U(F)G(M)A(F)G(M)G(M)U(M)^U(M)^U(M) | 22 |
| TfR2-039.21 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 6 |
| TfR2-039.22 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(F)G(M)C(M)U(M)U(M)U(F)G(M)A(F)G(M)G(M)U(M)^U(M)^U(M) | 6 |

**[Table 2-11]**

| Name of compound | Nucleotide sequence (5'-3') | SEQ ID NO |
|---|---|---|
| TfR2-039.23 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(F)G(M)G(M)C(M)U(M)U(M)U(F)G(M)A(F)G(M)G(M)U(M)^U(M)^U(M) | 6 |
| TfR2-039.24 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(M)G(M)C(M)U(M)U(M)U(F)G(M)A(F)G(M)G(M)U(M)^U(M)^U(M) | 6 |
| TfR2-039.25 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(M)A(M)C(M)G(M)G(M)C(M)U(M)U(M)U(F)G(M)A(F)G(M)G(M)U(M)^U(M)^U(M) | 6 |
| TfR2-039.26 | A(M)^C(M)^C(M)U(M)C(F)A(M)A(F)A(F)G(F)C(M)C(M)G(M)U(M)A(M)G(M)U(M)G(M)U(M)A(3M)Z U(M)^A(F)^C(M)A(M)C(M)U(F)A(M)C(M)G(M)G(M)C(M)U(M)U(M)U(F)G(M)A(M)G(M)G(M)U(M)^U(M)^U(M) | 6 |
| TfR2-039.5G | GNA(M)^C(M)^C(M)U(M)C(F)A(M)A(F)A(F)G(F)C(M)C(M)G(M)U(M)A(M)G(M)U(M)G(M)U(M)A(M)Z U(M)^A(F)^C(M)A(M)C(M)U(F)A(M)C(F)G(F)G(M)C(M)U(M)U(M)U(F)G(M)A(F)G(M)G(M)U(M)^U(M)^U(M) | 6 |
| TfR2-019.6G | GNC(M)^G(M)^U(M)G(M)G(F)A(M)G(F)U(F)U(F)U(M)C(M)A(M)A(M)U(M)A(M)U(M)C(M)A(M)A(M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 11 |
| TfR2-019.22 | C(M)^G(M)^U(M)G(M)G(F)A(M)G(F)U(F)U(F)U(M)C(M)A(M)A(M)U(M)A(M)U(M)C(M)A(M)A(3M)Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 11 |
| TfR2-019.22s | C(M)^G(M)^U(M)G(M)G(F)A(M)G(F)U(F)U(F)U(M)C(M)A(M)A(M)U(M)A(M)U(M)C(M)^A(M)^A(3M)^Z U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 11 |

**[Table 2-12]**

| Name of compound | Nucleotide sequence (5'-3') | SEQ ID NO |
|---|---|---|
| AD47882.5G | GNC(M)^C(M)^A(M)C(M)G(F)U(M)G(F)A(F)U(F)U(M)C(M)U(M)C(M)C(M)U(M)U(M)U(M)C(M)U(M)Z A(M)^G(F)^A(M)A(M)A(M)G(F)G(M)A(F)G(F)A(M)A(M)U(M)C(M)A(F)C(M)G(F)U(M)G(M)G(M)^U(M)^U(M) | 24 |
| AD47882.23G | GNC(M)^C(M)^A(M)C(M)G(F)U(M)G(F)A(F)U(F)U(M)C(M)U(M)C(M)C(M)U(M)U(M)U(M)C(M)U(3M)Z A(M)^G(F)^A(M)A(M)A(M)G(F)G(M)A(F)G(F)A(M)A(M)U(M)C(M)A(F)C(M)G(F)U(M)G(M)G(M)^U(M)^U(M) | 24 |
| AD47882.23sG | GNC(M)^C(M)^A(M)C(M)G(F)U(M)G(F)A(F)U(F)U(M)C(M)U(M)C(M)C(M)U(M)U(M)U(M)^C(M)^U(3M)^Z A(M)^G(F)^A(M)A(M)A(M)G(F)G(M)A(F)G(M)A(M)A(M)U(M)C(M)A(F)C(M)G(F)U(M)G(M)G(M)^U(M)^U(M) | 24 |

**[Table 3-1]**

| Exam ple | Name of compo und | Na me of sen se stra nd | Nucleotide sequence of sense strand region (5'-3') | Molec ular weight | Theoret ical value | SE Q ID NO |
|---|---|---|---|---|---|---|
| 134 | TfR2-039. 28DU | TfR 2-039. 28S | A(D)^C(E)^C(M)U(M)C(F)A(M)A(F)A(F)G(F)C(M)C(M)G(M)U(M)A(M)G( M)U(M)G(M)^U(M)^A(M) | 6325.1 7 | 6323.4 2 | 25 |
| 135 | TfR2-039. 29DU | TfR 2-039. 29S | A(D)^C(M)^C(E)U(M)C(F)A(M)A(F)A(F)G(F)C(M)C(M)G(M)U(M)A(M)G( M)U(M)G(M)^U(M)^A(M) | 6323.2 2 | 6323.4 2 | 25 |
| 136 | TfR2-039. 30DU | TfR 2-039. 30S | A(D)^C(M)^C(M)T(E)C(F)A(M)A(F)A(F)G(F)C(M)C(M)G(M)U(M)A(M)G( M)U(M)G(M)^U(M)^A(M) | 6321.9 6 | 6323.4 1 | 47 |
| 137 | TfR2-039. 31DU | TfR 2-039. 31S | A(D)^C(M)^C(M)U(M)C(E)A(M)A(F)A(F)G(F)C(M)C(M)G(M)U(M)A(M)G( M)U(M)G(M)^U(M)^A(M) | 6336.6 4 | 6335.4 5 | 25 |
| 138 | TfR2-039. 32DU | TfR 2-039. 32S | A(D)^C(M)^C(M)U(M)C(F)A(E)A(F)A(F)G(F)C(M)C(M)G(M)U(M)A(M)G( M)U(M)G(M)^U(M)^A(M) | 6309.9 1 | 6309.3 9 | 25 |
| 139 | TfR2-039. 9DU | TfR 2-039. 9S | A(D)^C(M)^C(M)U(M)C(F)A(M)A(F)A(F)G(F)C(M)C(M)G(M)U(M)A(M)G( M)U(M)G(M)^U(M)^A(M) | 6296.1 3 | 6297.3 8 | 25 |
| 140 | TfR2-019. 29DU | TfR 2-019. 29S | C(D)^G(M)^T(E)G(M)G(F)A(M)G(F)U(F)U(F)U(M)C(M)A(M)A(M)U(M)A( M)U(M)C(M)^A(M)^A(M) | 6322.2 0 | 6325.3 9 | 26 |
| 141 | TfR2-019. 34DU | TfR 2-019. 34S | C(D)^G(M)^T(E)G(M)G(F)A(M)G(F)U(F)U(F)U(M)C(M)A(M)A(M)U(M)A( M)U(M)C(M)^A(M)^A(M) | 6321.4 9 | 6325.3 9 | 26 |
| 142 | TfR2-019. 22DU | TfR 2-019. 22S | C(M)^G(M)^U(M)G(M)G(F)A(M)G(F)U(F)U(F)U(M)C(M)A(M)A(M)U(M)A (M)U(M)C(M)^A(M)^A(3M) | 6330.0 0 | 6329.3 9 | 27 |
| 143 | TfR2-019. 22sDU | TfR 2-019. 22s S | C(M)^G(M)^U(M)G(M)G(F)A(M)G(F)U(F)U(F)U(M)C(M)A(M)A(M)U(M)A (M)U(M)C(M)^A(M)^A(3M)^2 | 6470.0 0 | 6469.4 9 | 27 |

**[Table 3-2]**

| Exam ple | Name of compo und | Na me of sen se stra nd | Nucleotide sequence of sense strand region (5'-3') | Molec ular weight | Theoret ical value | SE Q ID NO |
|---|---|---|---|---|---|---|
| 144 | TfR2-039. 23DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 145 | TfR2-039. 35DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 146 | TfR2-039. 36DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 147 | TfR2-039. 37DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 148 | TfR2-039. 38DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 149 | TfR2-039. 39DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 150 | TfR2-039. 40DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 151 | TfR2-039. 41DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 152 | TfR2-039. 42DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 153 | TfR2-039. 43DUG | TfR 2-039.23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |
| 154 | TfR2-039. 45DUG | TfR 2-039. 23S G | GNA(M)^C(M)^C(M)U(M)C(F)A(M)A(F)A(F)G(F)C(M)C(M)G(M)U(M)A(M )G(M)U(M)G(M)^U(M)^A(3M) | 7228.2 8 | 7228.3 2 | 25 |

**[Table 3-3]**

| Exam ple | Name of compo und | Na me of sen se stra nd | Nucleotide sequence of sense strand region (5'-3') | Molec ular weight | Theoret ical value | SE Q ID NO |
|---|---|---|---|---|---|---|
| 155 | TfR2-019. 22DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 156 | TfR2-019. 36DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 157 | TfR2-019. 38DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 158 | TfR2-019. 85DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 159 | TfR2-019. 86DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 160 | TfR2-019. 87DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 161 | TfR2-019. 71DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 162 | TfR2-019. 88DUG | TfR 2-019.22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 163 | TfR2-019. 89DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 164 | TfR2-019. 90DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |
| 165 | TfR2-019. 80DUG | TfR 2-019. 22S G | GNC(M)^G(M)^U(M)G(M)G(F)A(M)G(F)U(F)U(F)U(M)C(M)A(M)A(M)U( M)A(M)U(M)C(M)^A(M)^A(3M) | 7230.3 0 | 7230.2 8 | 27 |

**[Table 3-4]**

| Exam ple | Name of compo und | Name of sense strand | Nucleotide sequence of sense strand region (5'-3') | Molec ular weight | Theore tical value | SE Q ID N O |
|---|---|---|---|---|---|---|
| 166 | TfR2-019. 91DU G | TfR2-019. 22SG | GNC(M)^G(M)^U(M)G(M)G(F)A(M)G(F)U(F)U(F)U(M)C(M)A(M)A(M)U( M)A(M)U(M)C(M)^A(M)^A(3M) | 7230. 30 | 7230.2 8 | 27 |
| 167 | TfR2-019. 92DU G | TfR2-019. 22SG | GNC(M)^G(M)^U(M)G(M)G(F)A(M)G(F)U(F)U(F)U(M)C(M)A(M)A(M)U( M)A(M)U(M)C(M)^A(M)^A(3M) | 7230. 30 | 7230.2 8 | 27 |
| 168 | TfR2-019. 93DU G | TfR2-019. 22SG | GNC(M)^G(M)^U(M)G(M)G(F)A(M)G(F)U(F)U(F)U(M)C(M)A(M)A(M)U( M)A(M)U(M)C(M)^A(M)^A(3M) | 7230. 30 | 7230.2 8 | 27 |
| 169 | TfR2-019. 94DU G | TfR2-019. 22SG | GNC(M)^G(M)^U(M)G(M)G(F)A(M)G(F)U(F)U(F)U(M)C(M)A(M)A(M)U( M)A(M)U(M)C(M)^A(M)^A(3M) | 7230. 30 | 7230.2 8 | 27 |
| 170 | TfR2-019. 95DU G | TfR2-019. 22SG | GNC(M)^G(M)^U(M)G(M)G(F)A(M)G(F)U(F)U(F)U(M)C(M)A(M)A(M)U( M)A(M)U(M)C(M)^A(M)^A(3M) | 7230. 30 | 7230.2 8 | 27 |
| 171 | AD478 82. 23DU G | AD47 882. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 172 | AD478 82. 23sDU G | AD47 882. 23sS G | GNC(M)^C(M)^A(M)C(M)G(F)U(M)G(F)A(F)U(F)U(M)C(M)U(M)C(M)C( M)U(M)U(M)U(M)^C(M)^U(3M)^2 | 7200. 00 | 7199.9 9 | 28 |
| 173 | AD478 82.41DU G | AD47 882. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 174 | AD478 82. 88DU G | AD47 882. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 175 | AD478 82. 89DU G | AD47 882. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 176 | AD478 82. 91DU G | AD47 882. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |

**[Table 3-5]**

| Exam ple | Name of compo und | Name of sense strand | Nucleotide sequence of sense strand region (5'-3') | Molec ular weight | Theoret ical value | SE Q ID N O |
|---|---|---|---|---|---|---|
| 177 | AD478 82. 92DU G | AD478 82. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 178 | AD478 82. 93DU G | AD478 82. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |
| 179 | AD478 82. 94DU G | AD478 82. 23SG | GNC(M)^C(M)^A(M)C(M)G(F)U(M)G(F)A(F)U(F)U(M)C(M)U(M)C(M)C( M)U(M)U(M)U(M)^C(M)^U(3M) | 7056. 15 | 7056.2 0 | 28 |

**[Table 3-6]**

| Exam ple | Name of compo und | Name of antise nse strand | Nucleotide sequence of antisense strand region (5'-3') | Molec ular weight | Theore tical value | SE Q ID N O |
|---|---|---|---|---|---|---|
| 134 | TfR2-039. 28DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 135 | TfR2-039. 29DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 136 | TfR2-039. 30DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 137 | TfR2-039. 31DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 138 | TfR2-039. 32DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 139 | TfR2-039. 9DU | TfR2-039. 9AS | U(M)^A(F)^C(M)A(M)C(M)U(F)A(M)C(F)G(M)G(M)C(M)U(M)U(M)U(F)G (M)A(F)G(M)G(M)U(M)^U(M)^U(M) | 6949. 20 | 6949.7 2 | 29 |
| 140 | TfR2-019. 29DU | TfR2-019. 29AS | U(M)^U(F)^G(M)A(M)U(M)A(F)U(M)U(F)G(M)A(M)A(M)A(M)C(M)U(F)C( M)C(F)A(M)C(M)G(M)^U(M)^U(M) | 6917. 88 | 6917.7 4 | 30 |
| 141 | TfR2-019. 34DU | TfR2-019. 34AS | U(M)^U(F)^G(M)A(M)U(M)A(F)U(M)U(M)G(F)A(M)A(M)A(M)C(M)U(F)C( M)C(F)A(M)C(M)G(M)^U(M)^U(M) | 6914. 60 | 6917.7 4 | 30 |
| 142 | TfR2-019. 22DU | TfR2-019. 22AS | U(M)^U(F)^G(M)A(M)U(M)A(F)U(M)U(M)G(F)A(M)A(M)A(M)C(M)U(F)C( M)C(F)A(M)C(M)G(M)^U(M)^U(M) | 6918. 0 | 6917.7 4 | 30 |
| 143 | TfR2-019. 22sDU | TfR2-019. 22AS | U(M)^U(F)^G(M)A(M)U(M)A(F)U(M)U(M)G(F)A(M)A(M)A(M)C(M)U(F)C( M)C(F)A(M)C(M)G(M)^U(M)^U(M) | 6918. 0 | 6917.7 4 | 30 |

**[Table 3-7]**

| Exam ple | Name of compo und | Name of antise nse strand | Nucleotide sequence of antisense strand region (5'-3') | Molec ular weigh t | Theore tical value | SE Q ID N O |
|---|---|---|---|---|---|---|
| 144 | TfR2-039. 23DU G | TfR2-039. 23AS | U(M)^A(F)^C(M)A(M)C(M)U(F)A(M)C(F)G(M)G(M)C(M)U(M)U(M)U(F)G( M)A(F)G(M)G(M)U(M)^U(M)^U(M) | 6950. 0 | 6949.7 2 | 29 |
| 145 | TfR2-039. 35DU G | TfR2-039. 35AS | U(M)^A(F)^C(M)A(M)C(M)U(F)A(M)C(F)G(M)G(M)C(M)U(M)U(M)U(F)G( M)A(F)G(M)G(M)^U(M)^U(M)^U(M) | 6963. 03 | 6965.7 9 | 29 |
| 146 | TfR2-039.36DU G | TfR2-039. 36AS | U(M)^A(F)^C(M)A(M)C(M)U(F)A(M)C(M)G(M)G(M)C(M)U(F)U(M)U(F)G( M)A(F)G(M)G(M)^U(M)^U(M)^U(M) | 6964. 35 | 6965.7 9 | 29 |
| 147 | TfR2-039. 37DU G | TfR2-039. 37AS | U(M)^A(F)^C(M)A(M)C(M)U(F)A(M)C(M)G(M)G(M)C(M)U(F)^U(M)U(F)G (M)A(F)G(M)G(M)^U(M)^U(M)^U(M) | 6977. 99 | 6981.8 6 | 29 |
| 148 | TfR2-039. 38DU G | TfR2-039. 38AS | U(M)^A(F)^C(M)A(M)C(M)U(F)A(M)C(F)G(F)G(M)C(M)U(F)U(M)U(F)G( M)A(F)G(M)G(M)^U(M)^U(M)^U(M) | 6942. 14 | 6941.7 3 | 29 |
| 149 | TfR2-039. 39DU G | TfR2-039. 39AS | U(M)^A(F)^C(M)A(M)C(M)U(F)A(M)C(F)G(F)G(M)C(M)U(F)^U(M)U(F)G( M)A(F)G(M)G(M)^U(M)^U(M)^U(M) | 6957. 52 | 6957.8 0 | 29 |
| 150 | TfR2-039. 40DU G | TfR2-039. 40AS | U(M)^A(F)^C(M)A(M)C(M)U(F)A(M)C(F)^G(F)^G(M)C(M)U(F)^U(M)U(F) G(M)A(F)G(M)G(M)^U(M)^U(M)^U(M) | 6987. 83 | 6989.9 4 | 29 |
| 151 | TfR2-039. 41DU G | TfR2-039. 41AS | U(M)^A(F)^C(M)A(M)C(M)U(F)A(M)C(F)G(M)G(M)C(M)T(D)U(M)U(F)G( M)A(F)G(M)G(M)^U(M)^U(M)^U(M) | 6949. 06 | 6949.7 9 | 31 |
| 152 | TfR2-039. 42DU G | TfR2-039. 42AS | U(M)^A(F)^C(M)A(M)C(M)U(F)A(M)C(F)G(M)G(M)C(M)T(D)^U(M)U(F)G (M)A(F)G(M)G(M)^U(M)^U(M)^U(M) | 6964. 35 | 6965.8 6 | 31 |
| 153 | TfR2-039. 43DU G | TfR2-039. 43AS | U(M)^A(F)^C(M)A(M)C(M)U(F)A(M)C(F)G(M)G(M)C(E)U(M)U(M)U(F)G( M)A(F)G(M)G(M)^U(M)^U(M)^U(M) | 6994. 48 | 6991.8 3 | 29 |
| 154 | TfR2-039. 45DU G | TfR2-039. 45AS | U(M)^A(F)^C(M)A(M)C(M)U(F)A(M)C(F)G(M)G(M)C(M)U(M)T(E)U(F)G( M)A(F)G(M)G(M)^U(M)^U(M)^U(M) | 6994. 74 | 6991.8 2 | 32 |

**[Table 3-8]**

| Exam ple | Name of compo und | Name of antise nse strand | Nucleotide sequence of antisense strand region (5'-3') | Molec ular weight | Theore tical value | SE Q ID N O |
|---|---|---|---|---|---|---|
| 155 | TfR2-019. 22DU G | TfR2-019. 22AS | U(M)^U(F)^G(M)A(M)U(M)A(F)U(M)U(M)G(F)A(M)A(M)A(M)C(M)U(F)C( M)C(F)A(M)C(M)G(M)^U(M)^U(M) | 6918. 0 | 6917.7 4 | 30 |
| 156 | TfR2-019. 36DU G | TfR2-019. 36AS | U(M)^U(F)^G(M)A(M)U(M)A(F)U(M)U(M)G(M)A(M)A(M)A(F)C(M)U(F)C( M)C(F)A(M)C(M)^G(M)^U(M)^U(M) | 6929. 97 | 6929.9 9 | 30 |
| 157 | TfR2-019. 38DU G | TfR2-019. 38AS | U(M)^U(F)^G(M)A(M)U(M)A(F)U(M)U(F)G(F)A(M)A(M)A(F)C(M)U(F)C( M)C(F)A(M)C(M)^G(M)^U(M)^U(M) | 6905. 91 | 6905.9 5 | 30 |
| 158 | TfR2-019.85DU G | TfR2-019.85AS | U(M)^U(F)^G(M)A(M)U(M)A(F)U(M)U(M)G(F)A(M)A(M)A(M)C(E)U(F)C( M)C(F)A(M)C(M)^G(M)^U(M)^U(M) | 6955. 94 | 6956.0 0 | 30 |
| 159 | TfR2-019. 86DU G | TfR2-019. 86AS | U(M)^U(F)^G(M)A(M)U(M)A(F)U(M)U(M)G(F)A(M)A(E)A(M)C(M)U(F)C( M)C(F)A(M)C(M)^G(M)^U(M)^U(M) | 6941. 94 | 6941.9 9 | 30 |
| 160 | TfR2-019. 87DU G | TfR2-019. 87AS | U(M)^U(F)^G(M)A(M)U(M)A(F)U(M)U(M)G(F)A(M)A(M)A(D)C(M)U(F)C( M)C(F)A(M)C(M)^G(M)^U(M)^U(M) | 6899. 95 | 6899.9 8 | 30 |
| 161 | TfR2-019. 71DU G | TfR2-019. 71AS | U(M)^U(F)^G(M)A(M)U(M)A(F)U(M)U(F)G(F)A(M)A(M)A(D)C(M)T(E)C( M)C(F)A(M)C(M)^G(M)^U(M)^U(M) | 6926. 06 | 6925.9 9 | 33 |
| 162 | TfR2-019. 88DU G | TfR2-019. 88AS | U(M)^U(F)^G(M)A(F)U(M)A(F)U(M)U(M)G(F)A(M)A(M)A(D)C(M)T(E)C( M)C(F)A(M)C(M)^G(M)^U(M)^U(M) | 6926. 05 | 6925.9 9 | 33 |
| 163 | TfR2-019. 89DU G | TfR2-019. 89AS | U(M)^U(F)^G(M)A(M)U(M)A(F)U(M)U(M)G(F)A(F)A(M)A(D)C(M)T(E)C( M)C(F)A(M)C(M)^G(M)^U(M)^U(M) | 6926. 11 | 6925.9 9 | 33 |
| 164 | TfR2-019. 90DU G | TfR2-019. 90AS | U(M)^U(F)^G(M)A(M)U(M)A(F)U(M)U(M)G(F)A(M)A(M)A(D)C(M)T(E)C( M)C(F)A(M)C(F)^G(M)^U(M)^U(M) | 6926. 00 | 6925.9 9 | 33 |
| 165 | TfR2-019. 80DU G | TfR2-019. 80AS | U(M)^U(F)^G(M)A(M)U(M)A(F)U(M)U(F)G(F)A(M)A(M)A(M)C(E)U(R)^C (M)C(F)A(M)C(M)^G(M)^U(M)^U(M) | 6958. 09 | 6957.9 6 | 30 |

**[Table 3-9]**

| Exam ple | Name of compo und | Name of antise nse strand | Nucleotide sequence of antisense strand region (5'-3') | Molec ular weight | Theore tical value | SE Q ID N O |
|---|---|---|---|---|---|---|
| 166 | TfR2-019. 91DU G | TfR2-019. 91AS | U(M)^U(F)^G(M)A(M)U(M)A(F)U(M)U(M)G(F)A(F)A(M)A(M)C(E)U(R)^C (M)C(F)A(M)C(M)^G(M)^U(M)^U(M) | 6957. 96 | 6957.9 6 | 30 |
| 167 | TfR2-019. 92DU G | TfR2-019. 92AS | U(M)^U(F)^G(M)A(M)U(M)A(F)U(M)U(M)G(F)A(M)A(F)A(M)C(E)U(R)^C (M)C(F)A(M)C(M)^G(M)^U(M)^U(M) | 6958. 07 | 6957.9 6 | 30 |
| 168 | TfR2-019. 93DU G | TfR2-019. 93AS | U(M)^U(F)^G(M)A(M)U(M)A(F)U(M)U(M)G(F)A(M)A(M)A(F)C(E)U(R)^C (M)C(F)A(M)C(M)^G(M)^U(M)^U(M) | 6958. 06 | 6957.9 6 | 30 |
| 169 | TfR2-019. 94DU G | TfR2-019. 94AS | U(M)^U(F)^G(M)A(M)U(M)A(F)U(M)U(M)G(F)A(M)A(F)A(M)C(M)U(R)^C (M)C(F)A(M)C(M)^G(M)^U(M)^U(M) | 6935. 66 | 6935.6 9 | 30 |
| 170 | TfR2-019.95DU G | TfR2-019.95AS | U(M)^U(F)^G(M)A(M)U(M)A(F)U(M)U(M)G(F)A(M)A(M)A(F)C(M)U(R)^C (M)C(F)A(M)C(M)^G(M)^U(M)^U(M) | 6935. 68 | 6935.6 9 | 30 |
| 171 | AD478 82. 23DU G | AD47 882. 9AS | A(M)^G(F)^A(M)A(M)A(M)G(F)G(M)A(F)G(M)A(M)A(M)U(M)C(M)A(F)C( M)G(F)U(M)G(M)G(M)^U(M)^U(M) | 7118. 03 | 7118.1 2 | 34 |
| 172 | AD478 82. 23sDU G | AD47 882. 9AS | A(M)^G(F)^A(M)A(M)A(M)G(F)G(M)A(F)G(M)A(M)A(M)U(M)C(M)A(F)C( M)G(F)U(M)G(M)G(M)^U(M)^U(M) | 7118. 03 | 7118.1 2 | 34 |
| 173 | AD478 82. 41DU G | AD47 882. 41AS | A(M)^G(F)^A(M)A(M)A(M)G(F)G(M)A(F)G(M)A(M)A(M)T(D)C(M)A(F)C( M)G(F)U(M)G(M)^G(M)^U(M)^U(M) | 7119. 06 | 7118.1 | 35 |
| 174 | AD478 82. 88DU G | AD47 882. 88AS | A(M)^G(F)^A(M)A(F)A(M)G(F)G(M)A(M)G(F)A(M)A(M)T(D)C(M)A(E)C( M)G(F)U(M)G(M)^G(M)^U(M)^U(M) | 7130. 10 | 7130.1 0 | 35 |
| 175 | AD478 82. 89DU G | AD47 882. 89AS | A(M)^G(F)^A(M)A(M)A(M)G(F)G(M)A(M)G(F)A(F)A(M)T(D)C(M)A(E)C( M)G(F)U(M)G(M)^G(M)^U(M)^U(M) | 7130. 11 | 7130.1 0 | 35 |
| 176 | AD478 82. 91DU G | AD47 882. 91AS | A(M)^G(F)^A(M)A(M)A(M)G(F)G(M)A(M)G(F)A(F)A(M)U(M)C(E)A(R)^C( M)G(F)U(M)G(M)^G(M)^U(M)^U(M) | 7162. 09 | 7162.0 7 | 34 |

**[Table 3-10]**

| Exam ple | Name of compo und | Name of antise nse strand | Nucleotide sequence of antisense strand region (5'-3') | Molec ular weight | Theore tical value | SE Q ID N O |
|---|---|---|---|---|---|---|
| 177 | AD478 82. 92DU G | AD47 882. 92AS | A(M)^G(F)^A(M)A(M)A(M)G(F)G(M)A(M)G(F)A(M)A(F)U(M)C(E)A(R)^C( M)G(F)U(M)G(M)^G(M)^U(M)^U(M) | 7163. 08 | 7162.0 7 | 34 |
| 178 | AD478 82. 93DU G | AD47 882. 93AS | A(M)^G(F)^A(M)A(M)A(M)G(F)G(M)A(M)G(F)A(M)A(M)U(F)C(E)A(R)^C( M)G(F)U(M)G(M)^G(M)^U(M)^U(M) | 7162. 05 | 7162.0 7 | 34 |
| 179 | AD478 82. 94DU G | AD47 882. 94AS | A(M)^G(F)^A(M)A(M)A(M)G(F)G(M)A(M)G(F)A(M)A(F)U(M)C(M)A(R)^C (M)G(F)U(M)G(M)^G(M)^U(M)^U(M) | 7136. 04 | 7136.0 6 | 34 |

**[Table 3-11]**

| Exam ple | Name of compo und | Nam e of sens e stran d | Nucleotide sequence of sense strand region (5'-3') | Molec ular weigh t | Theore tical value | SE Q ID N O |
|---|---|---|---|---|---|---|
| 180 | TfR2-019. 94DU G.s1 | TfR2-019. 22SG | GNC(M)^G(M)^U(M)G(M)G(F)A(M)G(F)U(F)U(F)U(M)C(M)A(M)A(M)U(M) A(M)U(M)C(M)^A(M)^A(3M) | 7230. 30 | 7230.2 8 | 27 |
| 181 | TfR2-019. 94DU G.s2 | TfR2-019. 22SG | GNC(M)^G(M)^U(M)G(M)G(F)A(M)G(F)U(F)U(F)U(M)C(M)A(M)A(M)U(M) A(M)U(M)C(M)^A(M)^A(3M) | 7230. 30 | 7230.2 8 | 27 |
| 182 | TfR2-019. 94DU G.e1 | TfR2-019. 22SG .e1 | GNG(M)^C(M)^G(M)U(M)G(M)G(F)A(M)G(F)U(F)U(F)U(M)C(M)A(M)A(M )U(M)A(M)U(M)C(M)^A(M)^A(3M) | 7589. 35 | 7589.3 5 | 36 |
| 183 | TfR2-019. 94DU G.e2 | TfR2-019. 22SG .e2 | GNG(M)^G(M)^C(M)G(M)U(M)G(M)G(F)A(M)G(F)U(F)U(F)U(M)C(M)A(M )A(M)U(M)A(M)U(M)C(M)^A(M)^A(3M) | 7948. 42 | 7948.4 1 | 37 |
| 184 | TfR2-019. 94DU G.e3 | TfR2-019. 22SG | GNC(M)^G(M)^U(M)G(M)G(F)A(M)G(F)U(F)U(F)U(M)C(M)A(M)A(M)U(M) A(M)U(M)C(M)^A(M)^A(3M) | 7230. 30 | 7230.2 8 | 27 |

**[Table 3-12]**

| Exam ple | Name of compo und | Name of antise nse strand | Nucleotide sequence of antisense strand region (5'-3') | Molec ular weight | Theore tical value | SE Q ID N O |
|---|---|---|---|---|---|---|
| 180 | TfR2-019. 94DU G.s1 | TfR2-019. 94AS. s1 | U(M)^U(F)^G(M)A(M)U(M)A(F)U(M)U(M)G(F)A(M)A(F)A(M)C(M)U(R)^C(M)C(F) A(M)C(M)^G(M)^U(M) | 6599 | 6599.5 8 | 38 |
| 181 | TfR2-019. 94DU G.s2 | TfR2-019. 94AS. s2 | U(M)^U(F)^G(M)A(M)U(M)A(F)U(M)U(M)G(F)A(M)A(F)A(M)C(M)U(R)^C(M)C(F) A(M)C(M)^G(M) | 6263 | 6263.3 1 | 39 |
| 182 | TfR2-019. 94DU G.e1 | TfR2-019. 94AS. e1 | U(M)^U(F)^G(M)A(M)U(M)A(F)U(M)U(M)G(F)A(M)A(F)A(M)C(M)U(R)^C(M)C(F) A(M)C(M)G(M)^C(M)^U(M)^U(M) | 7254 | 7255.0 6 | 40 |
| 183 | TfR2-019. 94DU G.e2 | TfR2-019. 94AS. e2 | U(M)^U(F)^G(M)A(M)U(M)A(F)U(M)U(M)G(F)A(M)A(F)A(M)C(M)U(R)^C(M)C(F) A(M)C(M)G(M)C(M)^C(M)^U(M)^U(M) | 7574 | 7574.2 7 | 41 |
| 184 | TfR2-019. 94DU G.e3 | TfR2-019. 94AS. e3 | U(M)^U(F)^G(M)A(M)U(M)A(F)U(M)U(M)G(F)A(M)A(F)A(M)C(M)U(R)^C(M)C(F) A(M)C(M)^G(M)^G(M)^U(M) | 6974 | 6974.8 8 | 42 |

**[Table 3-13]**

| Name of sense strand | Nucleotide sequence of sense strand region (5'-3') | SEQ ID NO |
|---|---|---|
| TfR2-039.28S | A(D)^C(E)^C(M)U(M)C(F)A(M)A(F)A(F)G(F)C(M)C(M)G(M)U(M)A(M)G(M)U(M)G(M)^U( M)^A(M) | 25 |
| TfR2-039.29S | A(D)^C(M)^C(E)U(M)C(F)A(M)A(F)A(F)G(F)C(M)C(M)G(M)U(M)A(M)G(M)U(M)G(M)^U( M)^A(M) | 25 |
| TfR2-039.30S | A(D)^C(M)^C(M)T(E)C(F)A(M)A(F)A(F)G(F)C(M)C(M)G(M)U(M)A(M)G(M)U(M)G(M)^U( M)^A(M) | 47 |
| TfR2-039.31S | A(D)^C(M)^C(M)U(M)C(E)A(M)A(F)A(F)G(F)C(M)C(M)G(M)U(M)A(M)G(M)U(M)G(M)^U( M)^A(M) | 25 |
| TfR2-039.32S | A(D)^C(M)^C(M)U(M)C(F)A(E)A(F)A(F)G(F)C(M)C(M)G(M)U(M)A(M)G(M)U(M)G(M)^U( M)^A(M) | 25 |
| TfR2-039.9S | A(D)^C(M)^C(M)U(M)C(F)A(M)A(F)A(F)G(F)C(M)C(M)G(M)U(M)A(M)G(M)U(M)G(M)^U( M)^A(M) | 25 |
| TfR2-019.29S | C(D)^G(M)^T(E)G(M)G(F)A(M)G(F)U(F)U(F)U(M)C(M)A(M)A(M)U(M)A(M)U(M)C(M)^A( M)^A(M) | 26 |
| TfR2-019.34S | C(D)^G(M)^T(E)G(M)G(F)A(M)G(F)U(F)U(F)U(M)C(M)A(M)A(M)U(M)A(M)U(M)C(M)^A( M)^A(M) | 26 |

**[Table 3-14]**

| Name of sense strand | Nucleotide sequence of sense strand region (5'-3') | SEQ ID NO |
|---|---|---|
| TfR2-019.22S | C(M)^G(M)^U(M)G(M)G(F)A(M)G(F)U(F)U(F)U(M)C(M)A(M)A(M)U(M)A(M)U(M)C(M)^A(M)^ A(3M) | 27 |
| TfR2-019.22sS | C(M)^G(M)^U(M)G(M)G(F)A(M)G(F)U(F)U(F)U(M)C(M)A(M)A(M)U(M)A(M)U(M)C(M)^A(M)^ A(3M)^2 | 27 |
| TfR2-039.23SG | GNA(M)^C(M)^C(M)U(M)C(F)A(M)A(F)A(F)G(F)C(M)C(M)G(M)U(M)A(M)G(M)U(M)G(M)^U( M)^A(3M) | 25 |
| TfR2-019.22SG | GNC(M)^G(M)^U(M)G(M)G(F)A(M)G(F)U(F)U(F)U(M)C(M)A(M)A(M)U(M)A(M)U(M)C(M)^A( M)^A(3M) | 27 |
| AD47882.23SG | GNC(M)^C(M)^A(M)C(M)G(F)U(M)G(F)A(F)U(F)U(M)C(M)U(M)C(M)C(M)U(M)U(M)U(M)^C( M)^U(3M) | 28 |
| AD47882.23sSG | GNC(M)^C(M)^A(M)C(M)G(F)U(M)G(F)A(F)U(F)U(M)C(M)U(M)C(M)C(M)U(M)U(M)U(M)^C( M)^U(3M)^2 | 28 |
| TfR2-019. 22SG.e1 | GNG(M)^C(M)^G(M)U(M)G(M)G(F)A(M)G(F)U(F)U(F)U(M)C(M)A(M)A(M)U(M)A(M)U(M)C( M)^A(M)^A(3M) | 36 |
| TfR2-019. 22SG.e2 | GNG(M)^G(M)^C(M)G(M)U(M)G(M)G(F)A(M)G(F)U(F)U(F)U(M)C(M)A(M)A(M)U(M)A(M)U( M)C(M)^A(M)^A(3M) | 37 |

**[Table 3-15]**

| Name of antisens e strand | Nucleotide sequence of antisense strand region (5'-3') | SEQ ID NO |
|---|---|---|
| TfR2-039. 9AS | U(M)^A(F)^C(M)A(M)C(M)U(F)A(M)C(F)G(M)G(M)C(M)U(M)U(M)U(F)G(M)A(F)G(M)G(M)U(M)^U(M )^U(M) | 29 |
| TfR2-019. 29AS | U(M)^U(F)^G(M)A(M)U(M)A(F)U(M)U(F)G(M)A(M)A(M)A(M)C(M)U(F)C(M)C(F)A(M)C(M)G(M)^U(M )^U(M) | 30 |
| TfR2-019. 34AS | U(M)^U(F)^G(M)A(M)U(M)A(F)U(M)U(M)G(F)A(M)A(M)A(M)C(M)U(F)C(M)C(F)A(M)C(M)G(M)^U(M )^U(M) | 30 |
| TfR2-019. 22AS | U(M)^U(F)^G(M)A(M)U(M)A(F)U(M)U(M)G(F)A(M)A(M)A(M)C(M)U(F)C(M)C(F)A(M)C(M)G(M)^U(M )^U(M) | 30 |
| TfR2-039. 23AS | U(M)^A(F)^C(M)A(M)C(M)U(F)A(M)C(F)G(M)G(M)C(M)U(M)U(M)U(F)G(M)A(F)G(M)G(M)U(M)^U(M )^U(M) | 29 |
| TfR2-039. 35AS | U(M)^A(F)^C(M)A(M)C(M)U(F)A(M)C(F)G(M)G(M)C(M)U(M)U(M)U(F)G(M)A(F)G(M)G(M)^U(M)^U( M)^U(M) | 29 |
| TfR2-039. 36AS | U(M)^A(F)^C(M)A(M)C(M)U(F)A(M)C(M)G(M)G(M)C(M)U(F)U(M)U(F)G(M)A(F)G(M)G(M)^U(M)^U( M)^U(M) | 29 |
| TfR2-039. 37AS | U(M)^A(F)^C(M)A(M)C(M)U(F)A(M)C(M)G(M)G(M)C(M)U(F)^U(M)U(F)G(M)A(F)G(M)G(M)^U(M)^U (M)^U(M) | 29 |

**[Table 3-16]**

| Name of antisens e strand | Nucleotide sequence of antisense strand region (5'-3') | SEQ ID NO |
|---|---|---|
| TfR2-039. 38AS | U(M)^A(F)^C(M)A(M)C(M)U(F)A(M)C(F)G(F)G(M)C(M)U(F)U(M)U(F)G(M)A(F)G(M)G(M)^U(M)^U(M )^U(M) | 29 |
| TfR2-039. 39AS | U(M)^A(F)^C(M)A(M)C(M)U(F)A(M)C(F)G(F)G(M)C(M)U(F)^U(M)U(F)G(M)A(F)G(M)G(M)^U(M)^U( M)^U(M) | 29 |
| TfR2-039. 40AS | U(M)^A(F)^C(M)A(M)C(M)U(F)A(M)C(F)^G(F)^G(M)C(M)U(F)^U(M)U(F)G(M)A(F)G(M)G(M)^U(M)^ U(M)^U(M) | 29 |
| TfR2-039. 41AS | U(M)^A(F)^C(M)A(M)C(M)U(F)A(M)C(F)G(M)G(M)C(M)T(D)U(M)U(F)G(M)A(F)G(M)G(M)^U(M)^U( M)^U(M) | 31 |
| TfR2-039. 42AS | U(M)^A(F)^C(M)A(M)C(M)U(F)A(M)C(F)G(M)G(M)C(M)T(D)^U(M)U(F)G(M)A(F)G(M)G(M)^U(M)^U( M)^U(M) | 31 |
| TfR2-039. 43AS | U(M)^A(F)^C(M)A(M)C(M)U(F)A(M)C(F)G(M)G(M)C(E)U(M)U(M)U(F)G(M)A(F)G(M)G(M)^U(M)^U( M)^U(M) | 29 |
| TfR2-039. 45AS | U(M)^A(F)^C(M)A(M)C(M)U(F)A(M)C(F)G(M)G(M)C(M)U(M)T(E)U(F)G(M)A(F)G(M)G(M)^U(M)^U( M)^U(M) | 32 |
| TfR2-019. 36AS | U(M)^U(F)^G(M)A(M)U(M)A(F)U(M)U(M)G(M)A(M)A(M)A(F)C(M)U(F)C(M)C(F)A(M)C(M)^G(M)^U( M)^U(M) | 30 |

**[Table 3-17]**

| Name of antisens e strand | Nucleotide sequence of antisense strand region (5'-3') | SEQ ID NO |
|---|---|---|
| TfR2-019. 38AS | U(M)^U(F)^G(M)A(M)U(M)A(F)U(M)U(F)G(F)A(M)A(M)A(F)C(M)U(F)C(M)C(F)A(M)C(M)^G(M)^U(M )^U(M) | 30 |
| TfR2-019. 85AS | U(M)^U(F)^G(M)A(M)U(M)A(F)U(M)U(M)G(F)A(M)A(M)A(M)C(E)U(F)C(M)C(F)A(M)C(M)^G(M)^U( M)^U(M) | 30 |
| TfR2-019. 86AS | U(M)^U(F)^G(M)A(M)U(M)A(F)U(M)U(M)G(F)A(M)A(E)A(M)C(M)U(F)C(M)C(F)A(M)C(M)^G(M)^U( M)^U(M) | 30 |
| TfR2-019. 87AS | U(M)^U(F)^G(M)A(M)U(M)A(F)U(M)U(M)G(F)A(M)A(M)A(D)C(M)U(F)C(M)C(F)A(M)C(M)^G(M)^U( M)^U(M) | 30 |
| TfR2-019. 71AS | U(M)^U(F)^G(M)A(M)U(M)A(F)U(M)U(F)G(F)A(M)A(M)A(D)C(M)T(E)C(M)C(F)A(M)C(M)^G(M)^U(M )^U(M) | 33 |
| TfR2-019. 88AS | U(M)^U(F)^G(M)A(F)U(M)A(F)U(M)U(M)G(F)A(M)A(M)A(D)C(M)T(E)C(M)C(F)A(M)C(M)^G(M)^U(M )^U(M) | 33 |
| TfR2-019. 89AS | U(M)^U(F)^G(M)A(M)U(M)A(F)U(M)U(M)G(F)A(F)A(M)A(D)C(M)T(E)C(M)C(F)A(M)C(M)^G(M)^U(M )^U(M) | 33 |
| TfR2-019. 90AS | U(M)^U(F)^G(M)A(M)U(M)A(F)U(M)U(M)G(F)A(M)A(M)A(D)C(M)T(E)C(M)C(F)A(M)C(F)^G(M)^U(M )^U(M) | 33 |

**[Table 3-18]**

| Name of antisens e strand | Nucleotide sequence of antisense strand region (5'-3') | SEQ ID NO |
|---|---|---|
| TfR2-019. 80AS | U(M)^U(F)^G(M)A(M)U(M)A(F)U(M)U(F)G(F)A(M)A(M)A(M)C(E)U(R)^C(M)C(F)A(M)C(M)^G(M)^U( M)^U(M) | 30 |
| TfR2-019. 91AS | U(M)^U(F)^G(M)A(M)U(M)A(F)U(M)U(M)G(F)A(F)A(M)A(M)C(E)U(R)^C(M)C(F)A(M)C(M)^G(M)^U( M)^U(M) | 30 |
| TfR2-019. 92AS | U(M)^U(F)^G(M)A(M)U(M)A(F)U(M)U(M)G(F)A(M)A(F)A(M)C(E)U(R)^C(M)C(F)A(M)C(M)^G(M)^U( M)^U(M) | 30 |
| TfR2-019. 93AS | U(M)^U(F)^G(M)A(M)U(M)A(F)U(M)U(M)G(F)A(M)A(M)A(F)C(E)U(R)^C(M)C(F)A(M)C(M)^G(M)^U( M)^U(M) | 30 |
| TfR2-019. 94AS | U(M)^U(F)^G(M)A(M)U(M)A(F)U(M)U(M)G(F)A(M)A(F)A(M)C(M)U(R)^C(M)C(F)A(M)C(M)^G(M)^U( M)^U(M) | 30 |
| TfR2-019. 95AS | U(M)^U(F)^G(M)A(M)U(M)A(F)U(M)U(M)G(F)A(M)A(M)A(F)C(M)U(R)^C(M)C(F)A(M)C(M)^G(M)^U( M)^U(M) | 30 |
| AD4788 2. 9AS | A(M)^G(F)^A(M)A(M)A(M)G(F)G(M)A(F)G(M)A(M)A(M)U(M)C(M)A(F)C(M)G(F)U(M)G(M)G(M)^U(M )^U(M) | 34 |
| AD4788 2. 41AS | A(M)^G(F)^A(M)A(M)A(M)G(F)G(M)A(F)G(M)A(M)A(M)T(D)C(M)A(F)C(M)G(F)U(M)G(M)^G(M)^U( M)^U(M) | 35 |

**[Table 3-19]**

| Name of antisens e strand | Nucleotide sequence of antisense strand region (5'-3') | SEQ ID NO |
|---|---|---|
| AD4788 2. 88AS | A(M)^G(F)^A(M)A(F)A(M)G(F)G(M)A(M)G(F)A(M)A(M)T(D)C(M)A(E)C(M)G(F)U(M)G(M)^G(M)^U(M )^U(M) | 35 |
| AD4788 2. 89AS | A(M)^G(F)^A(M)A(M)A(M)G(F)G(M)A(M)G(F)A(F)A(M)T(D)C(M)A(E)C(M)G(F)U(M)G(M)^G(M)^U(M )^U(M) | 35 |
| AD4788 2. 91AS | A(M)^G(F)^A(M)A(M)A(M)G(F)G(M)A(M)G(F)A(F)A(M)U(M)C(E)A(R)^C(M)G(F)U(M)G(M)^G(M)^U( M)^U(M) | 34 |
| AD4788 2. 92AS | A(M)^G(F)^A(M)A(M)A(M)G(F)G(M)A(M)G(F)A(M)A(F)U(M)C(E)A(R)^C(M)G(F)U(M)G(M)^G(M)^U( M)^U(M) | 34 |
| AD4788 2. 93AS | A(M)^G(F)^A(M)A(M)A(M)G(F)G(M)A(M)G(F)A(M)A(M)U(F)C(E)A(R)^C(M)G(F)U(M)G(M)^G(M)^U( M)^U(M) | 34 |
| AD4788 2. 94AS | A(M)^G(F)^A(M)A(M)A(M)G(F)G(M)A(M)G(F)A(M)A(F)U(M)C(M)A(R)^C(M)G(F)U(M)G(M)^G(M)^U( M)^U(M) | 34 |
| TfR2-019. 94AS.s1 | U(M)^U(F)^G(M)A(M)U(M)A(F)U(M)U(M)G(F)A(M)A(F)A(M)C(M)U(R)^C(M)C(F)A(M)C(M)^G(M)^U( M) | 38 |
| TfR2-019. 94AS.s2 | U(M)^U(F)^G(M)A(M)U(M)A(F)U(M)U(M)G(F)A(M)A(F)A(M)C(M)U(R)^C(M)C(F)A(M)C(M)^G(M) | 39 |

**[Table 3-20]**

| Name of antisens e strand | Nucleotide sequence of antisense strand region (5'-3') | SEQ ID NO |
|---|---|---|
| TfR2-019. 94AS.e 1 | U(M)^U(F)^G(M)A(M)U(M)A(F)U(M)U(M)G(F)A(M)A(F)A(M)C(M)U(R)^C(M)C(F)A(M)C(M)G(M)^C(M) ^U(M)^U(M) | 40 |
| TfR2-019. 94AS.e 2 | U(M)^U(F)^G(M)A(M)U(M)A(F)U(M)U(M)G(F)A(M)A(F)A(M)C(M)U(R)^C(M)C(F)A(M)C(M)G(M)C(M)^ C(M)^U(M)^U(M) | 41 |
| TfR2-019. 94AS.e 3 | U(M)^U(F)^G(M)A(M)U(M)A(F)U(M)U(M)G(F)A(M)A(F)A(M)C(M)U(R)^C(M)C(F)A(M)C(M)^G(M)^G( M)^U(M) | 42 |

The "molecular weight" in the tables indicates a value measured by negative ion ESI mass spectrometry. In the "nucleotide sequence" in the tables, N(R) represents RNA, N(D) represents DNA, N(M) represents 2'-OMe RNA, N(m) represents 2'-MOE RNA, N(3M) represents 3'-OMe RNA, N(F) represents 2'-F RNA, N(L) represents LNA, and N(E) represents ENA. The structures of the nucleosides are represented by the above structural formulae, respectively.

In the tables, "GN" indicates a GalNAc unit represented by the above formula (GN). In the tables, "2" indicates -O(CH₂)₂O- (represented by the above formula (2)), and "Z" indicates -O(CH₂)₈NHC(=O) (CH₂)₂PhO-(represented by the above formula (Z)). For the bond between nucleosides, or the bond between the nucleoside and the GalNAc unit, "2" or "Z", "^" in the tables indicates a phosphorothioate bond (-P(=S) (OH)-, represented by the above structural diagram (PS) of the inter-nucleoside bond). The bond is a phosphodiester bond (-P(=O)(OH)-, represented by (P) in the above structural diagram of inter-nucleoside bonds) if no specific indication is given.

The residue at each of the 5'-position of the nucleotide at the 5'-terminus and the 3'-position (2'-position in the case of a nucleotide modified at the 3'-position) of the nucleotide at the 3'-terminus of an oligonucleotide is a hydroxyl group in which a hydrogen atom is bonded to an oxygen atom in the above structural diagram of each nucleoside. When "2" forms a terminus, a hydroxyl group is formed with a hydrogen atom being bonded to an oxygen atom on the side where there is no binding to an oligonucleotide in the above structural diagram of "2". When "^" forms a terminus, a hydroxyl group is bonded to a phosphorus atom in the above structural diagram of "PS". However, when "GN" forms a terminus, bonding of a hydrogen atom is not necessary.

### Test Example 1: Screening of HepG2 cells for TfR2 siRNA by forward transfection method

The following experiment was conducted using the compounds of Examples 1 to 82 as TfR2 siRNA. On the day before transfection, HepG2 cells in 100 µL of DMEM medium (manufactured by Gibco) containing 10% FBS (manufactured by Gibco) were seeded at 2.5 × 10⁴ cells per well of a 96-well plate (manufactured by Corning Incorporated). On the day of transfection, each siRNA sample, a negative control (NT) using distilled water instead of siRNA, and TfR2-061 (Example 61) as a positive control were prepared for two wells respectively. Preparation of a reagent for performing transfection of one siRNA sample is as follows. 50 µL of Opti-MEM and 1 µL of siRNA (concentration: 10 µM) were added to a mixture of 50 µL of Opti-MEM (manufactured by Life Technologies Corporation) and 3 µL of RNAi MAX (manufactured by Thermo Fisher Scientific Inc.), , and the mixture was incubated at room temperature for 15 minutes. Thereafter, 1 mL of pre-prepared DMEM containing 10% FBS, warmed to 37°C, was added. To the 96-well plate on which the HepG2 cells were seeded and the medium had been removed, 110 µL of the mixture was added.

The final concentration of siRNA was 10 nM for all the samples. Preparation of a reagent for the negative control is as follows. 50 µL of Opti-MEM and 1 µL of distilled water were added to a mixture of 50 µL of Opti-MEM (manufactured by Life Technologies Corporation) and 3 µL of RNAi MAX (manufactured by Thermo Fisher Scientific Inc.), and the mixture was incubated at room temperature for 15 minutes.

Two days after the transfection, the cells were washed once with PBS (manufactured by Thermo Fisher Scientific Inc.), and RNA was extracted. The RNA was extracted by using RNeasy Mini Kit (manufactured by QUAGEN) and following the protocol of the kit. Using High Capacity RNA to cDNA Kit (manufactured by Applied Biosystems), 300 ng of obtained RNA was subjected to a reverse transcription reaction to synthesize cDNA. Using the obtained cDNA, the mRNA level of endogenous hTfR2 was measured by quantitative PCR. The mRNA level of human 18S ribosomal RNA (h18S) was measured as the housekeeping gene.

Using IDT PrimeTime Gene Expression Master Mix (manufactured by Integrated DNA Technologies, Inc.), quantitative PCR was performed in the following manner. The obtained cDNA was diluted 10-fold with distilled water (15 µL of cDNA and 135 µL of distilled water were mixed). In a 384-well PCR plate (manufactured by Applied Biosystems), 5 µL of IDT PrimeTime Gene Expression Master Mix, 0.5 µL of a hTfR2 primer (Hs. PT. 58. 20599434, manufactured by Integrated DNA Technologies, Inc.), 0.5 µL of a h18S primer (Hs. PT. 39a. 22214856.g, manufactured by Integrated DNA Technologies, Inc.), 2 µL of distilled water and 2 µL of cDNA (total 10 µL) were mixed per well, the mixture was prepared in a 2-well-equivalent amount for each cDNA, and quantitative PCR was performed. The mRNA expression level of each of hTfR2 and h18S was given as an average value of two wells. The mRNA expression level of hTfR2 was standardized with the mRNA expression level of h18S which is the housekeeping gene. As the hTfR2 mRNA expression level in the negative control is assumed to be 1.0, relative hTfR2 mRNA expression levels of the siRNA-treated samples were calculated and recorded. Table 4 shows the results.

**[Table 4-1]**

| Name of compound | Relative Value |
|---|---|
| TfR2-001 | 0.35 |
| TfR2-002 | 0.41 |
| TfR2-003 | 0.41 |
| TfR2-004 | 0.43 |
| TfR2-005 | 0.42 |
| TfR2-006 | 0.43 |
| TfR2-007 | 0.35 |
| TfR2-008 | 0.53 |
| TfR2-009 | 0.40 |
| TfR2-010 | 0.42 |
| TfR2-011 | 0.53 |
| TfR2-012 | 0.49 |
| TfR2-013 | 0.31 |
| TfR2-014 | 0.49 |
| TfR2-015 | 0.33 |
| TfR2-016 | 0.50 |
| TfR2-017 | 0.48 |
| TfR2-018 | 0.39 |
| TfR2-019 | 0.30 |
| TfR2-020 | 0.49 |
| TfR2-021 | 1.01 |
| TfR2-022 | 0.43 |
| TfR2-023 | 1.18 |
| TfR2-024 | 0.41 |
| TfR2-025 | 0.81 |
| TfR2-026 | 1.16 |
| TfR2-027 | 0.61 |
| TfR2-028 | 0.45 |
| NT | 1.00 |
| TfR2-061 | 0.19 |

**[Table 4-2]**

| Name of compound | Relative Value |
|---|---|
| TfR2-029 | 0.55 |
| TfR2-030 | 0.98 |
| TfR2-031 | 1.22 |
| TfR2-032 | 0.55 |
| TfR2-033 | 0.53 |
| TfR2-034 | 0.48 |
| TfR2-035 | 0.40 |
| TfR2-036 | 0.48 |
| TfR2-037 | 0.35 |
| TfR2-038 | 0.48 |
| TfR2-039 | 0.36 |
| TfR2-040 | 0.65 |
| TfR2-041 | 0.39 |
| TfR2-042 | 1.02 |
| TfR2-043 | 0.44 |
| TfR2-044 | 0.58 |
| TfR2-045 | 0.90 |
| TfR2-046 | 0.38 |
| TfR2-047 | 0.76 |
| TfR2-048 | 0.77 |
| TfR2-049 | 0.87 |
| TfR2-050 | 0.50 |
| TfR2-051 | 0.45 |
| TfR2-052 | 0.40 |
| TfR2-053 | 0.32 |
| TfR2-054 | 0.45 |
| TfR2-055 | 0.54 |
| TfR2-056 | 0.43 |
| NT | 1.00 |
| TfR2-061 | 0.22 |

**[Table 4-3]**

| Name of compound | Relative Value |
|---|---|
| TfR2-057 | 0.46 |
| TfR2-058 | 0.50 |
| TfR2-059 | 0.63 |
| TfR2-060 | 0.63 |
| TfR2-062 | 0.61 |
| TfR2-063 | 0.73 |
| TfR2-064 | 0.47 |
| TfR2-065 | 0.43 |
| TfR2-066 | 0.63 |
| TfR2-067 | 0.49 |
| TfR2-068 | 0.69 |
| TfR2-069 | 0.50 |
| TfR2-070 | 0.54 |
| TfR2-071 | 0.52 |
| TfR2-072 | 0.53 |
| TfR2-073 | 0.55 |
| TfR2-074 | 0.58 |
| TfR2-075 | 0.57 |
| TfR2-076 | 0.39 |
| TfR2-077 | 0.40 |
| TfR2-078 | 0.52 |
| TfR2-079 | 0.90 |
| TfR2-080 | 0.50 |
| TfR2-081 | 0.38 |
| TfR2-082 | 0.35 |
| NT | 1.00 |
| TfR2-061 | 0.35 |

### Test Example 1-1: Evaluation of cytotoxicity using HepG2 cells

Evaluation of cytotoxicity was performed 4 days after siRNA was introduced into HepG2 cells by a reverse transfection method in the same manner as in Test Example 2. As siRNA, TfR2-061 (Example 61), TfR2-019 (Example 19) and TfR2-039 (Example 39) were used.

For the cytotoxicity of siRNA to liver cells, a test was conducted using Cell Counting Kit-8 (CCK8, DOJINDO LABORATORIES). The culture supernatant was removed, and a solution obtained by preparing CCK8 reagent in an amount of 10 µL per 100 µL of DMEM containing 10% FBS was then added at 110 µL/well. Thereafter, the cells were incubated in a CO₂ incubator at 37°C for 30 minutes. Thereafter, the OD 450 nm absorbance was measured using a microplate reader. As the OD 450 nm value in the negative control (NT) is assumed to be 1.0, relative OD 450 nm values for wells in which siRNA had been treated were calculated, and recorded. The results are shown in Figure 11.

These results show that TfR2-019 and TfR2-039 have particularly low toxicity to liver cells.

### Test Example 2: Screening of HepG2 cells for TfR2 siRNA by reverse transfection method

The following experiment was conducted using the compounds of Examples as TfR2 siRNA. Screening for siRNA was performed in the following manner by a reverse transfection method. On the day of transfection, each siRNA sample , a negative control (NT) using distilled water instead of siRNA, and TfR2-061 (Example 61) or siRNA for hTfR2 described in WO 2012/177921 (AD-52590), as a positive control were prepared for two wells respectively.

5 µL of siRNA was added per well to a liquid mixture of 14.8 µL of Opti-MEM and 0.2 µL of RNAiMAX. The mixture was incubated at room temperature. For preparation of a reagent for the negative control, 5 µL of distilled water was added, instead of siRNA, to the liquid mixture of Opti-MEM and RNAiMAX. For preparation of a reagent for the positive control, 5 µL of TfR2-061 (Example 61) or AD-52590 was added to the liquid mixture of Opti-MEM and RNAiMAX. Next, the liquid mixture containing siRNA, the negative control or the positive control was added to a 96-well cell culture plate (manufactured by SUMIRON Co., Ltd.) at 20 µL per well. In the 96-well plate to which the liquid mixture had been added, 2 × 10⁴ cells/mL HepG2 cells, prepared using DMEM containing 10% FBS and warmed in advance to 37°C, were seeded at 80 µL per well. The final concentration of siRNA was adjusted by a 10-fold or 50-fold dilution series from 10 nM.

One or two days after transfection, the cells were washed once with PBS. Using Superprep Cell Lysis RT Kit for qPCR (Takara Bio Inc.), RNA extraction and a reverse transcription reaction were carried out in accordance with the protocol of the cDNA synthesis kit. Using the obtained cDNA, the mRNA expression level of endogenous hTfR2 was measured by quantitative PCR. For the housekeeping gene, the mRNA expression level of h18S was measured.

The quantitative PCR was performed in the following manner using IDT PrimeTime Gene Expression Master Mix (manufactured by Integrated DNA Technologies, Inc.). The obtained cDNA was used as a stock solution. In a 384-well PCR plate (manufactured by Applied Biosystems), 5 µL of IDT PrimeTime Gene Expression Master Mix, 0.25 µL of a hTfR2 primer (Hs. PT. 58. 20599434, manufactured by Integrated DNA Technologies, Inc.) or 0.25 µL of a h18S primer (Hs. PT. 39a. 22214856.g, manufactured by Integrated DNA Technologies, Inc.), 2.75 µL of distilled water and 2 µL of cDNA (total 10 µL) were mixed per well, the mixture was prepared in a 2-well-equivalent amount for each cDNA, and quantitative PCR was performed. The mRNA expression level of each of hTfR2 and h18S was given as an average value of two wells. The mRNA expression level of hTfR2 was standardized with the mRNA expression level of h18S, which is the housekeeping gene. As the hTfR2 mRNA expression level in the negative control is assumed to be 1.0, relative hTfR2 mRNA expression levels of the siRNA-treated samples were calculated and recorded. Tables 5 to 20 show the results.

**[Table 5-1]**

| Name of compound | Concentration (nM) | Relative Value |
|---|---|---|
| NT | - | 1.00 |
| TfR2-061 | 10 | 0.07 |
| | 1 | 0.07 |
| | 0.1 | 0.11 |
| TfR2-007 | 10 | 0.19 |
| | 1 | 0.20 |
| | 0.1 | 0.21 |
| TfR2-013 | 10 | 0.11 |
| | 1 | 0.17 |
| | 0.1 | 0.14 |
| TfR2-015 | 10 | 0.38 |
| | 1 | 0.47 |
| | 0.1 | 0.37 |
| TfR2-019 | 10 | 0.19 |
| | 1 | 0.19 |
| | 0.1 | 0.66 |
| TfR2-037 | 10 | 0.37 |
| | 1 | 0.32 |
| | 0.1 | 0.28 |
| TfR2-076 | 10 | 0.39 |
| | 1 | 0.74 |
| | 0.1 | 0.28 |
| TfR2-077 | 10 | 0.26 |
| | 1 | 0.19 |
| | 0.1 | 0.30 |
| TfR2-081 | 10 | 0.13 |
| | 1 | 0.15 |
| | 0.1 | 0.27 |

**[Table 5-2]**

| Name of compound | Concentration (nM) | Relative Value |
|---|---|---|
| NT | - | 1.00 |
| TfR2-061 | 10 | 0.10 |
| | 1 | 0.10 |
| | 0.1 | 0.13 |
| TfR2-003 | 10 | 0.56 |
| | 1 | 0.43 |
| | 0.1 | 0.83 |
| TfR2-005 | 10 | 0.36 |
| | 1 | 0.26 |
| | 0.1 | 0.27 |
| TfR2-006 | 10 | 0.31 |
| | 1 | 0.37 |
| | 0.1 | 0.75 |
| TfR2-022 | 10 | 0.25 |
| | 1 | 0.22 |
| | 0.1 | 0.20 |
| TfR2-039 | 10 | 0.21 |
| | 1 | 0.25 |
| | 0.1 | 0.23 |
| TfR2-046 | 10 | 0.30 |
| | 1 | 0.27 |
| | 0.1 | 0.24 |
| TfR2-065 | 10 | 0.14 |
| | 1 | 0.15 |
| | 0.1 | 0.15 |

**[Table 6-1]**

| Name of compound | Concentration (nM) | Relative Value |
|---|---|---|
| NT | - | 1.00 |
| AD-52590 | 1 | 0.05 |
| | 0.02 | 0.44 |
| | 0.0004 | 0.81 |
| TfR2-003.5 | 1 | 0.10 |
| | 0.02 | 0.46 |
| | 0.0004 | 0.87 |
| TfR2-005.5 | 1 | 0.07 |
| | 0.02 | 0.20 |
| | 0.0004 | 0.56 |
| TfR2-006.5 | 1 | 0.08 |
| | 0.02 | 0.34 |
| | 0.0004 | 0.76 |
| TfR2-007.5 | 1 | 0.12 |
| | 0.02 | 0.24 |
| | 0.0004 | 1.03 |
| TfR2-013.5 | 1 | 0.12 |
| | 0.02 | 0.44 |
| | 0.0004 | 0.75 |
| TfR2-019.5 | 1 | 0.05 |
| | 0.02 | 0.31 |
| | 0.0004 | 0.82 |
| TfR2-022.5 | 1 | 0.36 |
| | 0.02 | 0.37 |
| | 0.0004 | 0.78 |
| TfR2-037.5 | 1 | 0.16 |
| | 0.02 | 0.31 |
| | 0.0004 | 0.58 |

**[Table 6-2]**

| Name of compound | Concentration (nM) | Relative Value |
|---|---|---|
| NT | - | 1.00 |
| AD-52590 | 1 | 0.03 |
| | 0.02 | 0.24 |
| | 0.0004 | 0.60 |
| TfR2-039.5 | 1 | 0.09 |
| | 0.02 | 0.38 |
| | 0.0004 | 0.54 |
| TfR2-061.5 | 1 | 0.02 |
| | 0.02 | 0.11 |
| | 0.0004 | 0.38 |
| TfR2-065.5 | 1 | 0.22 |
| | 0.02 | 0.16 |
| | 0.0004 | 0.36 |
| TfR2-076.5 | 1 | 0.04 |
| | 0.02 | 0.10 |
| | 0.0004 | 0.50 |
| TfR2-077.5 | 1 | 0.06 |
| | 0.02 | 0.12 |
| | 0.0004 | 0.43 |
| TfR2-081.5 | 1 | 0.02 |
| | 0.02 | 0.26 |
| | 0.0004 | 0.43 |

**[Table 7-1]**

| Name of compound | Concentration (nM) | Relative Value |
|---|---|---|
| NT | - | 1.00 |
| AD-52590 | 0.001 | 0.92 |
| | 0.01 | 0.72 |
| | 0.1 | 0.44 |
| | 1 | 0.14 |
| | 10 | 0.09 |
| TfR2-006.5 | 0.001 | 0.86 |
| | 0.01 | 0.69 |
| | 0.1 | 0.36 |
| | 1 | 0.20 |
| | 10 | 0.15 |
| TfR2-013.5 | 0.001 | 0.84 |
| | 0.01 | 0.67 |
| | 0.1 | 0.46 |
| | 1 | 0.23 |
| | 10 | 0.16 |
| TfR2-039.5 | 0.001 | 0.70 |
| | 0.01 | 0.72 |
| | 0.1 | 0.51 |
| | 1 | 0.27 |
| | 10 | 0.23 |

**[Table 7-2]**

| Name of compound | Concentration (nM) | Relative Value |
|---|---|---|
| NT | - | 1.00 |
| AD-52590 | 0.001 | 0.80 |
| | 0.01 | 0.57 |
| | 0.1 | 0.26 |
| | 1 | 0.08 |
| | 10 | 0.08 |
| TfR2-007.5 | 0.001 | 0.68 |
| | 0.01 | 0.53 |
| | 0.1 | 0.25 |
| | 1 | 0.19 |
| | 10 | 0.16 |
| TfR2-019.5 | 0.001 | 0.58 |
| | 0.01 | 0.31 |
| | 0.1 | 0.14 |
| | 1 | 0.08 |
| | 10 | 0.07 |
| TfR2-076.5 | 0.001 | 0.64 |
| | 0.01 | 0.27 |
| | 0.1 | 0.28 |
| | 1 | 0.17 |
| | 10 | 0.15 |

**[Table 8-1]**

| Name of compound | Concentration (nM) | Relative Value |
|---|---|---|
| NT | - | 1.00 |
| AD-52590 | 0.001 | 1.02 |
| | 0.01 | 0.77 |
| | 0.1 | 0.45 |
| | 1 | 0.14 |
| | 10 | 0.15 |
| TfR2-006.3 | 0.001 | 1.06 |
| | 0.01 | 1.02 |
| | 0.1 | 0.67 |
| | 1 | 0.34 |
| | 10 | 0.39 |
| TfR2-013.3 | 0.001 | 1.04 |
| | 0.01 | 1.16 |
| | 0.1 | 0.81 |
| | 1 | 0.45 |
| | 10 | 0.37 |
| TfR2-039.3 | 0.001 | 0.95 |
| | 0.01 | 1.05 |
| | 0.1 | 0.92 |
| | 1 | 0.79 |
| | 10 | 0.81 |

**[Table 8-2]**

| Name of compound | Concentration (nM) | Relative Value |
|---|---|---|
| NT | - | 1.00 |
| AD-52590 | 0.001 | 0.90 |
| | 0.01 | 0.62 |
| | 0.1 | 0.31 |
| | 1 | 0.13 |
| | 10 | 0.12 |
| TfR2-007.3 | 0.001 | 0.95 |
| | 0.01 | 0.94 |
| | 0.1 | 0.86 |
| | 1 | 0.76 |
| | 10 | 0.67 |
| TfR2-019.3 | 0.001 | 0.77 |
| | 0.01 | 0.55 |
| | 0.1 | 0.36 |
| | 1 | 0.25 |
| | 10 | 0.25 |
| TfR2-076.3 | 0.001 | 0.80 |
| | 0.01 | 0.57 |
| | 0.1 | 0.39 |
| | 1 | 0.27 |
| | 10 | 0.28 |

**[Table 8-3]**

| Name of compound | Concentration (nM) | Relative Value |
|---|---|---|
| NT | - | 1.00 |
| AD-52590 | 0.001 | 0.72 |
| | 0.01 | 0.71 |
| | 0.1 | 0.45 |
| | 1 | 0.24 |
| | 10 | 0.04 |
| TfR2-006.6 | 0.001 | 0.93 |
| | 0.01 | 0.91 |
| | 0.1 | 0.39 |
| | 1 | 0.17 |
| | 10 | 0.10 |
| TfR2-013.6 | 0.001 | 0.77 |
| | 0.01 | 1.16 |
| | 0.1 | 0.44 |
| | 1 | 0.15 |
| | 10 | 0.14 |
| TfR2-039.6 | 0.001 | 0.91 |
| | 0.01 | 0.88 |
| | 0.1 | 0.50 |
| | 1 | 0.17 |
| | 10 | 0.22 |

**[Table 8-4]**

| Name of compound | Concentration (nM) | Relative Value |
|---|---|---|
| NT | - | 1.00 |
| AD-52590 | 0.001 | 0.78 |
| | 0.01 | 0.44 |
| | 0.1 | 0.24 |
| | 1 | 0.04 |
| | 10 | 0.04 |
| TfR2-007.6 | 0.001 | 0.91 |
| | 0.01 | 0.58 |
| | 0.1 | 0.34 |
| | 1 | 0.16 |
| | 10 | 0.07 |
| TfR2-019.6 | 0.001 | 0.54 |
| | 0.01 | 0.31 |
| | 0.1 | 0.18 |
| | 1 | 0.09 |
| | 10 | 0.11 |
| TfR2-076.6 | 0.001 | 0.79 |
| | 0.01 | 0.45 |
| | 0.1 | 0.27 |
| | 1 | 0.16 |
| | 10 | 0.18 |

**[Table 9]**

| Name of compound | Concentration (nM) | Relative Value |
|---|---|---|
| NT | - | 1.00 |
| AD-52590 | 0.001 | 0.73 |
| | 0.01 | 0.47 |
| | 0.1 | 0.22 |
| | 1 | 0.06 |
| | 10 | 0.06 |
| TfR2-039.5 | 0.001 | 0.82 |
| | 0.01 | 0.69 |
| | 0.1 | 0.36 |
| | 1 | 0.20 |
| | 10 | 0.21 |
| TfR2-019.6 | 0.001 | 0.56 |
| | 0.01 | 0.32 |
| | 0.1 | 0.17 |
| | 1 | 0.09 |
| | 10 | 0.06 |
| TfR2-039.5G | 0.001 | 0.73 |
| | 0.01 | 0.64 |
| | 0.1 | 0.39 |
| | 1 | 0.19 |
| | 10 | 0.19 |
| TfR2-019.6G | 0.001 | 0.53 |
| | 0.01 | 0.33 |
| | 0.1 | 0.17 |
| | 1 | 0.08 |
| | 10 | 0.06 |

**[Table 10]**

| Name of compound | Concentration (nM) | Relative Value |
|---|---|---|
| NT | - | 1.00 |
| AD-52590 | 0.001 | 0.97 |
| | 0.01 | 0.81 |
| | 0.1 | 0.34 |
| | 1 | 0.14 |
| | 10 | 0.12 |
| TfR2-039.5 | 0.001 | 0.98 |
| | 0.01 | 0.83 |
| | 0.1 | 0.50 |
| | 1 | 0.28 |
| | 10 | 0.29 |
| TfR2-039.10 | 0.001 | 0.87 |
| | 0.01 | 0.81 |
| | 0.1 | 0.55 |
| | 1 | 0.29 |
| | 10 | 0.26 |
| TfR2-039.11 | 0.001 | 0.95 |
| | 0.01 | 0.65 |
| | 0.1 | 0.47 |
| | 1 | 0.32 |
| | 10 | 0.28 |
| TfR2-039.12 | 0.001 | 0.81 |
| | 0.01 | 0.73 |
| | 0.1 | 0.53 |
| | 1 | 0.32 |
| | 10 | 0.35 |

**[Table 11-1]**

| Name of compound | Concentration (nM) | Relative Value |
|---|---|---|
| NT | - | 1.00 |
| AD-52590 | 0.001 | 0.92 |
| | 0.01 | 0.74 |
| | 0.1 | 0.39 |
| | 1 | 0.14 |
| | 10 | 0.10 |
| TfR2-039.5 | 0.001 | 1.09 |
| | 0.01 | 0.99 |
| | 0.1 | 0.63 |
| | 1 | 0.31 |
| | 10 | 0.40 |
| TfR2-039.19 | 0.001 | 1.12 |
| | 0.01 | 0.91 |
| | 0.1 | 0.53 |
| | 1 | 0.36 |
| | 10 | 0.29 |
| TfR2-039.20 | 0.001 | 1.08 |
| | 0.01 | 0.96 |
| | 0.1 | 0.63 |
| | 1 | 0.32 |
| | 10 | 0.29 |
| TfR2-039.8 | 0.001 | 0.81 |
| | 0.01 | 0.75 |
| | 0.1 | 0.46 |
| | 1 | 0.31 |
| | 10 | 0.22 |

**[Table 11-2]**

| Name of compound | Concentration (nM) | Relative Value |
|---|---|---|
| NT | - | 1.00 |
| TfR2-019.6 | 0.001 | 0.72 |
| | 0.01 | 0.51 |
| | 0.1 | 0.28 |
| | 1 | 0.22 |
| | 10 | 0.12 |
| TfR2-019.8 | 0.001 | 0.84 |
| | 0.01 | 0.41 |
| | 0.1 | 0.25 |
| | 1 | 0.19 |
| | 10 | 0.11 |
| TfR2-019.9 | 0.001 | 0.73 |
| | 0.01 | 0.38 |
| | 0.1 | 0.29 |
| | 1 | 0.14 |
| | 10 | 0.09 |
| TfR2-019.15 | 0.001 | 0.78 |
| | 0.01 | 0.55 |
| | 0.1 | 0.28 |
| | 1 | 0.17 |
| | 10 | 0.12 |
| TfR2-019.17 | 0.001 | 0.75 |
| | 0.01 | 0.42 |
| | 0.1 | 0.27 |
| | 1 | 0.15 |
| | 10 | 0.11 |

**[Table 12-1]**

| Name of compound | Concentration (nM) | Relative Value |
|---|---|---|
| NT | - | 1.00 |
| AD-52590 | 0.001 | 0.91 |
| | 0.01 | 0.72 |
| | 0.1 | 0.35 |
| | 1 | 0.14 |
| | 10 | 0.10 |
| TfR2-039.5 | 0.001 | 1.09 |
| | 0.01 | 0.79 |
| | 0.1 | 0.51 |
| | 1 | 0.30 |
| | 10 | 0.31 |
| TfR2-039.21 | 0.001 | 1.03 |
| | 0.01 | 0.83 |
| | 0.1 | 0.60 |
| | 1 | 0.30 |
| | 10 | 0.30 |
| TfR2-039.22 | 0.001 | 0.93 |
| | 0.01 | 0.96 |
| | 0.1 | 0.60 |
| | 1 | 0.33 |
| | 10 | 0.26 |
| TfR2-039.23 | 0.001 | 1.05 |
| | 0.01 | 0.83 |
| | 0.1 | 0.54 |
| | 1 | 0.32 |
| | 10 | 0.36 |

**[Table 12-2]**

| Name of compound | Concentration (nM) | Relative Value |
|---|---|---|
| NT | - | 1.00 |
| TfR2-039.5 | 0.001 | 0.81 |
| | 0.01 | 0.77 |
| | 0.1 | 0.57 |
| | 1 | 0.29 |
| | 10 | 0.36 |
| TfR2-039.24 | 0.001 | 1.11 |
| | 0.01 | 1.03 |
| | 0.1 | 0.60 |
| | 1 | 0.34 |
| | 10 | 0.34 |
| TfR2-039.25 | 0.001 | 1.40 |
| | 0.01 | 0.91 |
| | 0.1 | 0.73 |
| | 1 | 0.44 |
| | 10 | 0.39 |
| TfR2-039.26 | 0.001 | 1.22 |
| | 0.01 | 1.10 |
| | 0.1 | 0.87 |
| | 1 | 0.42 |
| | 10 | 0.53 |

**[Table 13-1]**

| Name of compound | Concentration (nM) | Relative Value |
|---|---|---|
| NT | - | 1.00 |
| AD-52590 | 0.01 | 0.46 |
| | 0.1 | 0.19 |
| | 1 | 0.03 |
| | 10 | 0.05 |
| TfR2-039.5 | 0.01 | 0.60 |
| | 0.1 | 0.34 |
| | 1 | 0.22 |
| | 10 | 0.21 |
| TfR2-039.9 | 0.01 | 0.64 |
| | 0.1 | 0.33 |
| | 1 | 0.17 |
| | 10 | 0.19 |
| TfR2-039.9DU | 0.01 | 0.92 |
| | 0.1 | 0.57 |
| | 1 | 0.20 |
| | 10 | 0.13 |
| TfR2-039.28DU | 0.01 | 1.44 |
| | 0.1 | 0.94 |
| | 1 | 0.29 |
| | 10 | 0.19 |

**[Table 13-2]**

| Name of compound | Concentration (nM) | Relative Value |
|---|---|---|
| NT | - | 1.00 |
| AD-52590 | 0.01 | 0.52 |
| | 0.1 | 0.21 |
| | 1 | 0.07 |
| | 10 | 0.06 |
| TfR2-039.9 | 0.01 | 0.81 |
| | 0.1 | 0.45 |
| | 1 | 0.31 |
| | 10 | 0.32 |
| TfR2-039.29DU | 0.01 | 1.14 |
| | 0.1 | 0.80 |
| | 1 | 0.35 |
| | 10 | 0.27 |
| TfR2-039.30DU | 0.01 | 1.08 |
| | 0.1 | 0.95 |
| | 1 | 0.32 |
| | 10 | 0.30 |
| TfR2-039.31DU | 0.01 | 1.19 |
| | 0.1 | 0.74 |
| | 1 | 0.32 |
| | 10 | 0.30 |

**[Table 13-3]**

| Name of compound | Concentration (nM) | Relative Value |
|---|---|---|
| NT | - | 1.00 |
| AD-52590 | 0.01 | 0.48 |
| | 0.1 | 0.18 |
| | 1 | 0.07 |
| | 10 | 0.07 |
| TfR2-039.5 | 0.01 | 0.63 |
| | 0.1 | 0.28 |
| | 1 | 0.22 |
| | 10 | 0.23 |
| TfR2-039.9 | 0.01 | 0.85 |
| | 0.1 | 0.35 |
| | 1 | 0.25 |
| | 10 | 0.28 |
| TfR2-039.32DU | 0.01 | 1.05 |
| | 0.1 | 0.93 |
| | 1 | 0.43 |
| | 10 | 0.38 |

**[Table 14]**

| Name of compound | Concentration (nM) | Relative Value |
|---|---|---|
| NT | - | 1.00 |
| AD-52590 | 0.001 | 0.84 |
| | 0.01 | 0.66 |
| | 0.1 | 0.31 |
| | 1 | 0.07 |
| | 10 | 0.07 |
| TfR2-019.6 | 0.001 | 0.57 |
| | 0.01 | 0.37 |
| | 0.1 | 0.22 |
| | 1 | 0.12 |
| | 10 | 0.08 |
| TfR2-019.29DU | 0.001 | 0.69 |
| | 0.01 | 0.43 |
| | 0.1 | 0.22 |
| | 1 | 0.12 |
| | 10 | 0.06 |
| TfR2-019.34DU | 0.001 | 0.71 |
| | 0.01 | 0.42 |
| | 0.1 | 0.21 |
| | 1 | 0.11 |
| | 10 | 0.06 |

**[Table 15]**

| Name of compound | Concentration (nM) | Relative Value |
|---|---|---|
| NT | - | 1.00 |
| TfR2-019.6 | 0.001 | 0.65 |
| | 0.01 | 0.36 |
| | 0.1 | 0.23 |
| | 1 | 0.14 |
| | 10 | 0.11 |
| TfR2-019.22 | 0.001 | 0.66 |
| | 0.01 | 0.34 |
| | 0.1 | 0.22 |
| | 1 | 0.14 |
| | 10 | 0.08 |
| TfR2-019.22s | 0.001 | 0.68 |
| | 0.01 | 0.36 |
| | 0.1 | 0.21 |
| | 1 | 0.13 |
| | 10 | 0.07 |
| TfR2-019.22DU | 0.001 | 0.79 |
| | 0.01 | 0.48 |
| | 0.1 | 0.28 |
| | 1 | 0.15 |
| | 10 | 0.11 |
| TfR2-019.22sDU | 0.001 | 0.80 |
| | 0.01 | 0.47 |
| | 0.1 | 0.28 |
| | 1 | 0.15 |
| | 10 | 0.08 |

**[Table 16-1]**

| Name of compound | Concentration (nM) | Relative Value |
|---|---|---|
| NT | - | 1.00 |
| TfR2-019.6G | 0.001 | 0.66 |
| | 0.01 | 0.33 |
| | 0.1 | 0.18 |
| | 1 | 0.13 |
| | 10 | 0.13 |
| TfR2-039.5G | 0.001 | 0.93 |
| | 0.01 | 0.71 |
| | 0.1 | 0.40 |
| | 1 | 0.32 |
| | 10 | 0.29 |
| TfR2-039.23DUG | 0.001 | 1.02 |
| | 0.01 | 0.94 |
| | 0.1 | 0.75 |
| | 1 | 0.43 |
| | 10 | 0.39 |
| TfR2-039.38DUG | 0.001 | 0.93 |
| | 0.01 | 0.95 |
| | 0.1 | 0.59 |
| | 1 | 0.30 |
| | 10 | 0.24 |
| TfR2-039.41DUG | 0.001 | 0.96 |
| | 0.01 | 0.84 |
| | 0.1 | 0.50 |
| | 1 | 0.28 |
| | 10 | 0.26 |

**[Table 16-2]**

| Name of compound | Concentration (nM) | Relative Value |
|---|---|---|
| NT | - | 1.00 |
| TfR2-039.23DUG | 0.001 | 1.00 |
| | 0.01 | 0.95 |
| | 0.1 | 0.89 |
| | 1 | 0.49 |
| | 10 | 0.42 |
| TfR2-039.35DUG | 0.001 | 1.03 |
| | 0.01 | 0.93 |
| | 0.1 | 0.76 |
| | 1 | 0.42 |
| | 10 | 0.29 |
| TfR2-039.36DUG | 0.001 | 0.93 |
| | 0.01 | 0.93 |
| | 0.1 | 0.65 |
| | 1 | 0.32 |
| | 10 | 0.30 |
| TfR2-039.37DUG | 0.001 | 0.96 |
| | 0.01 | 0.89 |
| | 0.1 | 0.62 |
| | 1 | 0.31 |
| | 10 | 0.25 |
| TfR2-039.38DUG | 0.001 | 1.03 |
| | 0.01 | 0.92 |
| | 0.1 | 0.62 |
| | 1 | 0.30 |
| | 10 | 0.24 |

**[Table 17-1]**

| Name of compound | Concentration (nM) | Relative Value |
|---|---|---|
| NT | - | 1.00 |
| TfR2-039.23DUG | 0.001 | 0.91 |
| | 0.01 | 0.72 |
| | 0.1 | 0.60 |
| | 1 | 0.35 |
| | 10 | 0.27 |
| TfR2-039.39DUG | 0.001 | 0.79 |
| | 0.01 | 0.81 |
| | 0.1 | 0.42 |
| | 1 | 0.18 |
| | 10 | 0.13 |
| TfR2-039.40DUG | 0.001 | 0.83 |
| | 0.01 | 0.81 |
| | 0.1 | 0.47 |
| | 1 | 0.15 |
| | 10 | 0.14 |
| TfR2-039.41DUG | 0.001 | 0.81 |
| | 0.01 | 0.63 |
| | 0.1 | 0.31 |
| | 1 | 0.13 |
| | 10 | 0.14 |
| TfR2-039.42DUG | 0.001 | 0.66 |
| | 0.01 | 0.73 |
| | 0.1 | 0.37 |
| | 1 | 0.16 |
| | 10 | 0.12 |

**[Table 17-2]**

| Name of compound | Concentration (nM) | Relative Value |
|---|---|---|
| NT | - | 1.00 |
| TfR2-039.23DUG | 0.001 | 0.75 |
| | 0.01 | 0.76 |
| | 0.1 | 0.59 |
| | 1 | 0.25 |
| | 10 | 0.21 |
| TfR2-039.36DUG | 0.001 | 0.58 |
| | 0.01 | 0.59 |
| | 0.1 | 0.39 |
| | 1 | 0.12 |
| | 10 | 0.09 |
| TfR2-039.41DUG | 0.001 | 0.66 |
| | 0.01 | 0.63 |
| | 0.1 | 0.26 |
| | 1 | 0.08 |
| | 10 | 0.07 |
| TfR2-039.43DUG | 0.001 | 0.72 |
| | 0.01 | 0.52 |
| | 0.1 | 0.59 |
| | 1 | 0.42 |
| | 10 | 0.35 |
| TfR2-039.45DUG | 0.001 | 0.48 |
| | 0.01 | 0.47 |
| | 0.1 | 0.44 |
| | 1 | 0.23 |
| | 10 | 0.21 |

**[Table 18-1]**

| Name of compound | Concentration (nM) | Relative Value |
|---|---|---|
| NT | - | 1.00 |
| TfR2-019.6G | 0.001 | 0.88 |
| | 0.01 | 0.37 |
| | 0.1 | 0.20 |
| | 1 | 0.13 |
| | 10 | 0.14 |
| TfR2-019.22DUG | 0.001 | 0.87 |
| | 0.01 | 0.46 |
| | 0.1 | 0.23 |
| | 1 | 0.12 |
| | 10 | 0.15 |
| TfR2-019.38DUG | 0.001 | 0.89 |
| | 0.01 | 0.54 |
| | 0.1 | 0.30 |
| | 1 | 0.16 |
| | 10 | 0.12 |
| TfR2-019.36DUG | 0.001 | 0.88 |
| | 0.01 | 0.49 |
| | 0.1 | 0.25 |
| | 1 | 0.14 |
| | 10 | 0.13 |
| TfR2-019.87DUG | 0.001 | 0.95 |
| | 0.01 | 0.43 |
| | 0.1 | 0.25 |
| | 1 | 0.15 |
| | 10 | 0.13 |

**[Table 18-2]**

| Name of compound | Concentration (nM) | Relative Value |
|---|---|---|
| NT | - | 1.00 |
| TfR2-019.6G | 0.001 | 0.84 |
| | 0.01 | 0.44 |
| | 0.1 | 0.25 |
| | 1 | 0.12 |
| | 10 | 0.11 |
| TfR2-019.22DUG | 0.001 | 0.89 |
| | 0.01 | 0.51 |
| | 0.1 | 0.22 |
| | 1 | 0.13 |
| | 10 | 0.10 |
| TfR2-019.85DUG | 0.001 | 0.64 |
| | 0.01 | 0.36 |
| | 0.1 | 0.17 |
| | 1 | 0.15 |
| | 10 | 0.09 |
| TfR2-019.86DUG | 0.001 | 0.72 |
| | 0.01 | 0.34 |
| | 0.1 | 0.19 |
| | 1 | 0.15 |
| | 10 | 0.10 |
| TfR2-039.41DUG | 0.001 | 0.93 |
| | 0.01 | 0.89 |
| | 0.1 | 0.57 |
| | 1 | 0.33 |
| | 10 | 0.28 |

**[Table 19-1]**

| Name of compound | Concentration (nM) | Relative Value |
|---|---|---|
| NT | - | 1.00 |
| TfR2-061.5 | 0.001 | 0.75 |
| | 0.01 | 0.50 |
| | 0.1 | 0.33 |
| | 1 | 0.15 |
| | 10 | 0.15 |
| TfR2-019.22DUG | 0.001 | 1.07 |
| | 0.01 | 0.61 |
| | 0.1 | 0.33 |
| | 1 | 0.25 |
| | 10 | 0.22 |
| TfR2-019.71DUG | 0.001 | 0.97 |
| | 0.01 | 0.88 |
| | 0.1 | 0.57 |
| | 1 | 0.32 |
| | 10 | 0.31 |
| TfR2-019.88DUG | 0.001 | 0.86 |
| | 0.01 | 0.76 |
| | 0.1 | 0.46 |
| | 1 | 0.28 |
| | 10 | 0.24 |
| TfR2-019.89DUG | 0.001 | 0.81 |
| | 0.01 | 0.71 |
| | 0.1 | 0.40 |
| | 1 | 0.30 |
| | 10 | 0.19 |

**[Table 19-2]**

| Name of compound | Concentration (nM) | Relative Value |
|---|---|---|
| NT | - | 1.00 |
| TfR2-019.6G | 0.001 | 0.84 |
| | 0.01 | 0.62 |
| | 0.1 | 0.30 |
| | 1 | 0.21 |
| | 10 | 0.16 |
| TfR2-019.22DUG | 0.001 | 1.09 |
| | 0.01 | 0.73 |
| | 0.1 | 0.30 |
| | 1 | 0.20 |
| | 10 | 0.15 |
| TfR2-019.90DUG | 0.001 | 1.02 |
| | 0.01 | 0.93 |
| | 0.1 | 0.45 |
| | 1 | 0.29 |
| | 10 | 0.22 |
| TfR2-019.80DUG | 0.001 | 1.06 |
| | 0.01 | 0.76 |
| | 0.1 | 0.35 |
| | 1 | 0.26 |
| | 10 | 0.21 |
| TfR2-019.91DUG | 0.001 | 1.02 |
| | 0.01 | 0.66 |
| | 0.1 | 0.33 |
| | 1 | 0.25 |
| | 10 | 0.17 |

**[Table 19-3]**

| Name of compound | Concentration (nM) | Relative Value |
|---|---|---|
| NT | - | 1.00 |
| TfR2-019.6G | 0.001 | 0.79 |
| | 0.01 | 0.53 |
| | 0.1 | 0.35 |
| | 1 | 0.29 |
| | 10 | 0.19 |
| TfR2-019.92DUG | 0.001 | 0.88 |
| | 0.01 | 0.67 |
| | 0.1 | 0.33 |
| | 1 | 0.24 |
| | 10 | 0.24 |
| TfR2-019.22DUG | 0.001 | 0.83 |
| | 0.01 | 0.55 |
| | 0.1 | 0.34 |
| | 1 | 0.23 |
| | 10 | 0.24 |
| TfR2-019.93DUG | 0.001 | 0.85 |
| | 0.01 | 0.67 |
| | 0.1 | 0.40 |
| | 1 | 0.26 |
| | 10 | 0.24 |
| TfR2-046.5 | 0.001 | 0.84 |
| | 0.01 | 0.78 |
| | 0.1 | 0.55 |
| | 1 | 0.35 |
| | 10 | 0.37 |

**[Table 20]**

| Name of compound | Concentration (nM) | Relative value |
|---|---|---|
| NT | - | 1.00 |
| TfR2-019.92DUG | 0.001 | 0.85 |
| | 0.01 | 0.52 |
| | 0.1 | 0.35 |
| | 1 | 0.16 |
| | 10 | 0.13 |
| TfR2-019.93DUG | 0.001 | 0.68 |
| | 0.01 | 0.42 |
| | 0.1 | 0.22 |
| | 1 | 0.11 |
| | 10 | 0.08 |
| TfR2-019.94DUG | 0.001 | 0.65 |
| | 0.01 | 0.48 |
| | 0.1 | 0.28 |
| | 1 | 0.15 |
| | 10 | 0.16 |
| TfR2-019.95DUG | 0.001 | 0.91 |
| | 0.01 | 0.53 |
| | 0.1 | 0.25 |
| | 1 | 0.18 |
| | 10 | 0.09 |

### Test Example 2-1: hTfR2 knockdown activity using HepG2 cells

In the same manner as in Test Example 2, the mRNA expression level of hTfR2 was evaluated 2 days after siRNA was introduced into HepG2 cells by a reverse transfection method. TfR2-019 (Example 19) and TfR2-019.94DUG (Example 169) were used as siRNA.

For evaluation of the hTfR2 mRNA expression level, relative hTfR2 mRNA expression levels of the siRNA-treated samples were calculated and recorded as relative values when the hTfR2 mRNA expression level in the negative control (NT) is assumed to be 1.0. The results are shown in Figure 12.

These results demonstrate that TfR2-019.94DUG has particularly high knockdown activity against hTfR2 mRNA.

### Test Example 3: Test of single administration of TfR2 siRNA to male cynomolgus monkeys

The plasma iron concentration and the plasma hepcidin concentration after subcutaneous administration of TfR2-039.5G (Example 127) or TfR2-039.23DUG (Example 144) at a dose of 30 mg/kg were measured. The plasma iron concentration was measured using a Metallo-assay iron measurement kit (manufactured by Metallogenics Co., Ltd.). The plasma hepcidin concentration was measured using LC/MS. Table 21 shows the results for administration of TfR2-039.5G (Example 127), and Table 22 shows the results for TfR2-039.23DUG (Example 144).

**[Table 21]**

| | Monkey 1 | |
|---|---|---|
| days after administration | plasma Fe ug/dL | Hepcidin (nM) |
| 0 | 146.5 | 15.5 |
| 3 | 209.0 | 11.3 |
| 7 | 214.0 | 11.6 |
| 14 | 271.0 | 11.2 |
| 21 | 300.0 | 13.1 |
| 29 | 285.5 | 13.9 |
| 35 | 204.0 | 20.0 |
| 42 | 219.5 | 17.6 |
| 49 | 203.5 | 15.6 |
| 56 | 187.5 | 18.8 |

**[Table 22]**

| | Monkey 2 | | Monkey 3 | |
|---|---|---|---|---|
| days after administration | plasma Fe ug/dL | Hepcidin (nM) | plasma Fe ug/dL | Hepcidin (nM) |
| -14 | 108.5 | 16.4 | 121.5 | 13.0 |
| 0 | 142.5 | 16.4 | 140.5 | 11.7 |
| 7 | 183.0 | 11.9 | 121.0 | 12.4 |
| 14 | 215.0 | 9.45 | 140.5 | 9.83 |
| 21 | 198.5 | 9.77 | 180.0 | 8.88 |
| 28 | 197.5 | 10.4 | 168.0 | 9.60 |
| 37 | 169.5 | 10.8 | 196.0 | 10.4 |
| 42 | 183.5 | 11.5 | 131.0 | 4.95 |
| 49 | 156.5 | not determined | 124.0 | not determined |

### Test Example 4: Preparation of siRNA-encapsulating nucleic acid lipid particles using TfR2 siRNA

### (1) Preparation of siRNA-encapsulating nucleic acid lipid particles

Distearoylphosphatidylcholine (1,2-distearoyl-sn-glycero-3-phosphocholine: hereinafter, referred to as DSPC, NOF CORPORATION), cholesterol(hereinafter, referred to as Chol, Sigma-Aldrich, Inc.), (7R,9Z)-18-({[3-(dimethylamino)propyloxy]carbonyl}oxy)octacosa-9-en-7yl acetate (compound described in WO 2015/005253, Example 28) (hereinafter, referred to as LP), and 1,2-dimyristoyl-sn-glycerol methoxypolyethylene glycol having a molecular weight of about 2,000 in terms of polyethylene glycol (1,2-dimyristoyl-sn-glycero-3-methoxypolyethylene glycol; hereinafter, referred to as PEG-DMG, NOF CORPORATION) were dissolved at a molar ratio of 10 : 48 : 40 : 2 in ethanol at a total lipid concentration of 10 mM.

On the other hand, as mouse TfR2 siRNA, AD-47882 (siRNA for mouse TfR2 which is described in WO 2012/177921) was prepared by dilution with a citrate buffer solution (20 mM citrate buffer, pH 4.0).

A crude dispersion of nucleic acid lipid particles was obtained by mixing the lipid solution and a siRNA solution in microchannels with NanoAssemblr BenchTop (Precision Nanosystems Inc.) such that the weight ratio of total lipid to siRNA was 11, and the volume ratio of the lipid solution and the siRNA solution was 1 : 3. The dispersion of nucleic acid lipid particles was dialyzed against about 25 to 50 times its amount of a phosphate buffer solution for 12 to 18 hours (Float-A-Lyzer G2, MWCO: 1,000 kD, Spectra/Por) to remove ethanol, thereby obtaining a purified dispersion of siRNA-encapsulating nucleic acid lipid particles. LP was synthesized by a method described in WO 2015/005253, Example 28.

### (2) Evaluation of characteristics of siRNA-encapsulating nucleic acid lipid particles

The following characteristics were evaluated for the dispersion containing nucleic acid lipid particles as prepared in (1).

### (2-1) Ratio of encapsulation of siRNA

Using Quant-iT RiboGreen RNA Assay Kit (Invitrogen), the ratio of encapsulation of siRNA was measured according to the product document. Specifically, siRNA in the dispersion of nucleic acid lipid particles was quantified with and without a 0.015% Triton X-100 surfactant, followed by calculation of the ratio of encapsulation from the following equation. Ratio of encapsulation (%) = {([siRNA level with surfactant] - [siRNA level without surfactant])/[siRNA level with surfactant]} × 100(%)

### (2-2) Ratio of siRNA and lipid

A dispersion of nucleic acid lipid particles was prepared by dilution with 1.0% Triton X-100, and the amount of siRNA in the dispersion of nucleic acid lipid particles was measured by ion-exchange chromatography (system: Agilent 1260 series, column: Dionex BioLC DNAPac PA200 (8 µm, 4.0 mm × 250 mm) (Thermo Fisher Scientific Inc.), Buffer A: 10 mM Tris-HCl buffer solution, 25 mM sodium perchlorate, 20% ethanol (pH 7.0), Buffer B: 10 mM Tris HCl buffer solution, 250 mM sodium perchlorate, 20% ethanol (pH 7.0), (B%): 20-70% (15 min), flow rate: 0.5 mL/min, temperature: 40°C, detection: 260 nm).

A dispersion of nucleic acid lipid particles was prepared by dilution with ethanol, and the amount of each lipid in the dispersion of nucleic acid lipid particles was measured by reversed-phase chromatography (system: DIONEX UltiMate 3000, column: XSelect CSH C18 (130 Å, 3.5 µm, 3.0 mm × 150 mm) (Waters catalog # 186005263), Buffer A: 0.2% formic acid, Buffer B: 0.2% formic acid, methanol, (B%): 75-95% (15 min), 95% (2 min), flow rate: 0.45 mL/min, temperature: 50°C, detection: Corona CAD (charged aerosol detector)). The ratio of total lipid to siRNA was calculated from the following equation. Amount of total lipid to siRNA (wt/wt) = [total lipid concentration]/[siRNA concentration] (wt/wt)

### (2-3) Average particle diameter

The particle diameter of the nucleic acid lipid particles was measured by Zeta Potential/Particle Sizer NICOMPTM 380ZLS (Particle Sizing Systems, LLC.). The average particle diameter in the tables indicates a volume average particle diameter, where the number following ± indicates an error. Table 23 shows the results of evaluation of the characteristics.

**[Table 23]**

| | Ratio of encapsulation (%) | Particle diameter (nm) | Total lipid/siRNA (wt/wt) |
|---|---|---|---|
| AD-47882 | 95 | 91±26 | 11 |

From the above results, it became apparent that in these nucleic acid lipid particles, about 95% of siRNA was encapsulated in the lipid particles, and the average particle diameter was about 90 nm.

### Test Example 5: Drug efficacy evaluation test for nucleic acid lipid particles encapsulating TfR2 siRNA in a mouse model of anemia associated with loss of bone-marrow function

Using nucleic acid lipid particles encapsulating AD-47882 (siRNA for mouse TfR2 which is described in WO 2012/177921) as nucleic acid lipid particles encapsulating mouse TfR2 siRNA, the following experiment was conducted. The nucleic acid lipid particles were prepared in the same manner as in Test Example 4. To induce loss of bone-marrow function, 100 mg/kg of carboplatin (manufactured by Tokyo Chemical Industry Co., Ltd.) was administered intraperitoneally (i.p.) to male C57BL/6NJcl mice (from CLEA Japan, Inc.). To a control group in which loss of bone-marrow function was not induced (control), PBS was i.p. administered instead of carboplatin (n = 5). Four days after the administration of carboplatin, grouping was performed on the basis of the plasma iron concentration and the HGB concentration, followed by drug agent treatment (n = 5/group). The drug agent was intravenously (i.v.) administered once in the form of nucleic acid lipid particles for each of 0.5 mg/kg (0.5 mpk) of AD-47882 and 0.25 mg/kg (0.25 mpk) of AD-47882. PBS was i.v. administered instead of the drug agent to a vehicle group. Before administration of AD-47882 (Day 0), 3 days after administration of AD-47882 (Day 3), 7 days after the administration (Day 7), 10 days after the administration (Day 10) and 11 days after the administration (Day 11), blood was collected from the tail vein, and the HGB concentration was measured using QuantiChrom Whole Blood HB Kit (manufactured by Bioassay Systems, LLC.). The results for the HGB concentration are shown in Figure 7C. Before administration of AD-47882 (Day 0), 3 days after the administration (Day 3) and 7 days after the administration (Day 7), the plasma iron concentration and the plasma hepcidin concentration were measured. The plasma iron concentration was measured using a Metallo-assay iron measurement kit (manufactured by Metallogenics Co., Ltd.). The plasma hepcidin concentration was measured using LC/MS. The results for the plasma iron concentration and the plasma hepcidin concentration are shown in Figures 7A and 7B, respectively. In addition, 11 days after treatment with AD-47882 (Day 11), blood was collected from the abdominal large vein under anesthesia with isoflurane, and the mean corpuscular hemoglobin (MCH) amount was measured using a multi-item automatic corpuscle analyzer XT 2000 (manufactured by Sysmex Corporation). The results for the MCH amount are shown in Figure 7D.

The mice were painlessly killed by bloodletting, the liver was then isolated, and the mRNA expression levels of mouse TfR2 (mTfR2) and mouse hepcidin (mHepcidin) were measured. Using RNeasy Mini Kit (manufactured by QUAGEN), RNA was extracted in accordance with the protocol of the kit. 5000 ng of obtained RNA was subjected to a reverse transcription reaction using High Capacity RNA to cDNA Kit (manufactured by Applied Biosystems). Using the thus-obtained cDNA, the mRNA level of endogenous mouse TfR2 and Hepcidin was measured by quantitative PCR.

The quantitative PCR was performed in the following manner using IDT PrimeTime Gene Expression Master Mix (manufactured by Integrated DNA Technologies, Inc.). The obtained cDNA was diluted 10-fold with distilled water (15 µL of cDNA and 135 µL of distilled water were mixed). In a 384-well PCR plate (manufactured by Applied Biosystems), 5 µL of IDT PrimeTime Gene Expression Master Mix, 0.25 µL of a mouse TfR2 (mTfR2) primer (Mm.PT.58.7860185, manufactured by Integrated DNA Technologies, Inc.) or 0.25 µL of a mouse hepcidin (mHepcidin) primer (Mm.PT.58.43563393.g, manufactured by Integrated DNA Technologies, Inc.), 2.75 µL of distilled water, and 2 µL of cDNA (total 10 µL) were mixed per well, the mixture was prepared in a 2-well-equivalent amount for each cDNA, and quantitative PCR was performed. For the housekeeping gene, a mouse β-actin (mActb) primer (Mm. PT. 39a, 22214843. g, manufactured by Integrated DNA Technologies, Inc.) was used, and mixed so as to meet a ratio as described above, the mixture was prepared in a 2-well-equivalent amount for each cDNA, and quantitative PCR was performed. The mRNA level of each of mTfR2, mHepcidin and mActb was given as an average value of two wells. The mRNA expression level of mTfR2 and mHepcidin was standardized with the mRNA expression level of mActb that is a housekeeping gene. As the mRNA level in the control group in which loss of bone-marrow function was not induced is assumed to be 1.0, relative mRNA levels in the vehicle group and the AD-47882 treatment group were calculated and recorded (average value ± standard error). The results for the mRNA expression levels of mTfR2 and mHepcidin in the liver are shown in Figure 7E and 7F, respectively.

These results show that by treatment with TfR2 siRNA, the mRNA expression level of TfR2 may be suppressed to increase the HGB concentration, thus providing a therapeutic effect on anemic diseases such as anemia associated with bone-marrow dysfunction, for example, anemia associated with myelofibrosis.

### Test example 6: Drug efficacy evaluation test for GalNAc-conjugated TfR2 siRNA in mouse model of anemia associated with loss of bone-marrow function

Using AD47882.23DUG (Example 171) as GalNAc-conjugated TfR2 siRNA, the following experiment was conducted. To induce loss of bone-marrow function, 100 mg/kg of carboplatin was administered intraperitoneally (i.p.). PBS was i.p. administered instead of carboplatin (n = 5) to a control group in which loss of bone-marrow function was not induced (control). Four days after the administration of carboplatin, grouping was performed on the basis of the HGB concentration, followed by drug agent treatment (n = 9 or 10/group). The drug agent was administered subcutaneously once at a dose of 3 mg/kg (3 mpk) to a GalNAc-conjugated TfR2 siRNA treatment group. Fc-conjugated ActRIIB (ActRIIB-Fc) as a drug agent for trapping a ligand for activin receptor type IIB (ActRIIB) was administered subcutaneously at a dose of 10 mg/kg every 3 or 4 days to a positive control group, with the administration performed three times in total. ActRIIB-Fc is a protein preparation obtained by bonding the extracellular domain of luspatercept (ACE-536) to the Fc domain of mouse IgG2a (WO 2016090188, Blood. 2014; 123(25): 3864-3872), and is known to promote differentiation of erythrocytes by suppressing Smad 2/3 signals, and to exhibit a therapeutic effect on a mouse model of β thalassemia and a mouse model of myelodysplastic syndrome (Blood. 2014; 123(25): 3864-3872, Nature Medicine volume 20, pages 408-414 (2014)). PBS was administered subcutaneously every 3 or 4 days to a vehicle group, with the administration performed three times in total. siRNA was administered subcutaneously once and ActRIIB-Fc was administered subcutaneously three times in total as described above to a group for treatment with GalNAc-conjugated TfR2 siRNA in combination with ActRIIB-Fc.

Before drug agent treatment (Day 0), 3 days after the treatment (Day 3) and 8 days after the treatment (Day 8), blood was collected from the tail vein, and the HGB concentration was measured using QuantiChrom Whole Blood HB Kit (manufactured by Bioassay Systems, LLC.). The results for the HGB concentration are shown in Figure 8A (average value ± standard error). Figure 8B shows the results for the HGB concentration 3 days after the drug agent treatment, which indicate an effect of the combined use of GalNAc-conjugated TfR2 siRNA and ActRIIB-Fc. Nine days after the drug agent treatment (Day 9), blood was collected from the abdominal large vein under anesthesia with isoflurane, and the HGB concentration, the MCH amount, the hematocrit (HCT) value and the number of red blood cells (RBCs) were measured using a multi-item automatic corpuscle analyzer XT 2000 (manufactured by Sysmex Corporation). The results are shown in Figures 8D to 8G (average value ± standard error), respectively. The mice were painlessly killed by bloodletting, the liver was then isolated, and the mRNA expression levels of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 8C. As the mRNA level in the control group in which loss of bone-marrow function was not induced is assumed to be 1.0, relative mRNA levels in other groups were calculated and recorded (average value ± standard error).

These results show that by treatment with GalNAc-conjugated TfR2 siRNA, the mRNA expression level of TfR2 may be suppressed to increase the HGB concentration, thus providing a therapeutic effect on anemic diseases such as anemia associated with bone-marrow dysfunction, for example, anemia associated with myelofibrosis. In addition, the results show that combined administration of GalNAc-conjugated TfR2 siRNA and luspatercept may enable provision of a therapeutic effect higher than that of a single agent on anemic disease such as anemia associated with myelofibrosis.

### Test Example 7: Drug efficacy evaluation test for GalNAc-conjugated TfR2 siRNA in a mouse model of anemia associated with inflammation

Using the compounds of AD47882.5G (Example 131), AD47882.23G (Example 132), AD47882.23sG (Example 133), AD47882.23DUG (Example 171) and AD47882.23sDUG (Example 172) as GalNAc-conjugated TfR2 siRNA, the following experiment was conducted. Each GalNAc-conjugated TfR2 siRNA was administered subcutaneously to male C57BL/6NJcl mice (from CLEA Japan, Inc.) once at a dose of 1 mg/kg (1 mpk) (Day 0). PBS was administered subcutaneously to a vehicle group. Five days after the treatment with siRNA or PBS (Day 5), turpentine was administered subcutaneously at 150 µL/mouse to induce inflammation. PBS was s.c. administered instead of turpentine to a control group in which inflammation was not induced (control). In the present test, n = 5/group. Two days after the first administration of turpentine (Day 7), the plasma iron concentration and the plasma hepcidin concentration were measured. The plasma iron concentration was measured using a Metallo-assay iron measurement kit (manufactured by Metallogenics Co., Ltd.). The plasma hepcidin concentration was measured using LC/MS. The results are shown in Figures 9A and 9B, respectively.

Six days after the first administration of turpentine (Day 11), turpentine was administered subcutaneously again at 150 µL/mouse. Two days after the second administration of turpentine (Day 13), blood was collected from the abdominal large vein under anesthesia with isoflurane, and the HGB concentration, the MCH amount, and the number of RBCs were measured using a multi-item automatic corpuscle analyzer XT 2000 (manufactured by Sysmex Corporation). The results are shown in Figures 9C to 9E, respectively. The mice were painlessly killed by bloodletting, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 9F. As the mRNA level in the control group in which inflammation was not induced is assumed to be 1.0, relative mRNA levels in other groups were calculated and recorded (average value ± standard error).

These results show that by treatment with GalNAc-conjugated TfR2 siRNA, the mRNA expression level of TfR2 may be suppressed to increase the HGB concentration, thus providing of a therapeutic effect on anemic diseases such as anemia associated with inflammation, for example, anemia associated with chronic kidney disease and cancerous anemia.

### Test Example 8: Drug efficacy evaluation test for GalNAc-conjugated TfR2 siRNA in a mouse model of anemia associated with inflammation

Using AD47882.41DUG (Example 173), AD47882.88DUG (Example 174), AD47882.89DUG (Example 175), AD47882.91DUG (Example 176), AD47882.92DUG (Example 177) and AD47882.93DUG (Example 178) as GalNAc-conjugated TfR2 siRNA, the following experiment was conducted. Each GalNAc-conjugated TfR2 siRNA was administered to male C57BL/6NJcl mice (from CLEA Japan, Inc.) once at a dose of 1 mg/kg (1 mpk) (Day 0). PBS was administered subcutaneously to the vehicle group. Four days after the treatment with siRNA or PBS (Day 4), turpentine was administered subcutaneously at 150 µL/mouse to induce inflammation. PBS was administered s.c. instead of turpentine to the control group that would not undergo induction of inflammation. In the present test, n = 5/group. Two days after the first administration of turpentine (Day 6), the plasma iron concentration was measured. The plasma iron concentration was measured using a Metallo-assay iron measurement kit (manufactured by Metallogenics Co., Ltd.). The results are shown in Figure 10A, respectively.

Seven days after the first administration of turpentine (Day 11), turpentine was administered subcutaneously again at 150 µL/mouse. Three days after the second administration of turpentine (Day 14), blood was collected from the abdominal large vein under anesthesia with isoflurane, and the HGB concentration, the MCH amount and the RBC number were measured using a multi-item automatic corpuscle analyzer XT 2000 (manufactured by Sysmex Corporation). The results are shown in Figures 10B to 10D, respectively. The mice were painlessly killed by bloodletting, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 10E. As the mRNA level in the control group in which inflammation was not induced is assumed to be 1.0, relative mRNA levels in other groups were calculated and recorded (average value ± standard error).

These results show that by treatment with GalNAc-conjugated TfR2 siRNA, the mRNA expression level of TfR2 may be suppressed to increase the HGB concentration, thus providing a therapeutic effect on anemic diseases such as anemia associated with inflammation, for example, anemia associated with chronic kidney disease and cancerous anemia.

### Test Example 9: Screening of HepG2 cells for TfR2 siRNA by a reverse transfection method

Using TfR2-019.94DUG (Example 169), TfR2-019.94DUG.sl (Example 180), TfR2-019.94DUG.s2 (Example 181), TfR2-019.94DUG.el (Example 182), TfR2-019.94DUG.e2 (Example 183) and TfR2-019.94DUG.e3 (Example 184) as siRNA, a test for the mRNA expression level of hTfR2 was conducted in the same manner as in Test Example 2. As the hTfR2 mRNA expression level in the negative control (NT) is assumed to be 1.0, relative hTfR2 mRNA expression levels of the siRNA-treated samples were calculated and recorded. Table 24 below shows the results.

**[Table 24-1]**

| Name of compound | Concentration (nM) | Relative Value |
|---|---|---|
| NT | - | 1.00 |
| 19.94DUG | 0.001 | 0.97 |
| | 0.01 | 0.58 |
| | 0.1 | 0.26 |
| | 1 | 0.05 |
| | 10 | 0.03 |
| 19.94DUG.s1 | 0.001 | 1.13 |
| | 0.01 | 0.48 |
| | 0.1 | 0.17 |
| | 1 | 0.07 |
| | 10 | 0.02 |
| 19.94DUG.s2 | 0.001 | 1.02 |
| | 0.01 | 0.53 |
| | 0.1 | 0.26 |
| | 1 | 0.08 |
| | 10 | 0.04 |
| 19.94DUG.e1 | 0.001 | 0.92 |
| | 0.01 | 0.40 |
| | 0.1 | 0.20 |
| | 1 | 0.06 |
| | 10 | 0.02 |
| 19.94DUG.e2 | 0.001 | 0.89 |
| | 0.01 | 0.51 |
| | 0.1 | 0.21 |
| | 1 | 0.08 |
| | 10 | 0.04 |

**[Table 24-2]**

| Name of compound | Concentration (nM) | Relative Value |
|---|---|---|
| NT | - | 1.00 |
| 19.94DUG | 0.001 | 0.86 |
| | 0.01 | 0.63 |
| | 0.1 | 0.30 |
| | 1 | 0.10 |
| | 10 | 0.06 |
| 19.94DUG e3 | 0.001 | 0.99 |
| | 0.01 | 0.60 |
| | 0.1 | 0.27 |
| | 1 | 0.09 |
| | 10 | 0.03 |

### Test Example 10: Drug efficacy evaluation test for GalNAc-conjugated TfR2 siRNA in normal mice

Using AD47882.5G (Example 131) and AD47882.94DUG (Example 179) as GalNAc-conjugated siRNA, the following experiment was conducted. As GalNAc-conjugated siRNA, AD47882.5G was administered subcutaneously (s.c.) to male C57BL/6NJcl mice (from CLEA Japan, Inc.) at a dose of 10 mg/kg (10 mpk), 3 mg/kg (3 mpk) or 1 mg/kg (1 mpk) (n = 5/group). PBS was administered subcutaneously to a vehicle group (n = 5/group). Before treatment with siRNA or PBS (Day 0), 7 days after the treatment (Day 7), 21 days after the treatment (Day 21) and 28 days after the treatment (Day 28), blood was collected from the tail vein, and the plasma iron concentration was measured using a Metallo-assay iron measurement kit (manufactured by Metallogenics Co., Ltd.). The results for the plasma iron concentration are shown in Figure 13A (average value ± standard error).

As GalNAc-conjugated siRNA, AD47882.94DUG was administered subcutaneously (s.c.) to male C57BL/6NJcl mice (from CLEA Japan, Inc.) at a dose of 3 mg/kg (3 mpk), 1 mg/kg (1 mpk) or 0.3 mg/kg (0.3 mpk) (n = 5/group). PBS was administered subcutaneously to a vehicle group (n = 5/group). Before treatment with siRNA or PBS (Day 0), 7 days after the treatment (Day 7), 21 days after the treatment (Day 21) and 28 days after the treatment (Day 28), blood was collected from the tail vein, and the plasma iron concentration was measured using a Metallo-assay iron measurement kit (manufactured by Metallogenics Co., Ltd.). The results for the plasma iron concentration are shown in Figure 13B (average value ± standard error).

These results show that in particular, AD47882.94DUG stably suppresses the mRNA expression level of TfR2 *in vivo* to increase the plasma iron concentration. As another test, normal mice were similarly treated with AD47882.88DUG (compound of Example 174), AD47882.89DUG (compound of Example 175), AD47882.91DUG (compound of Example 176), AD47882.92DUG (compound of Example 177), AD47882.93DUG (compound of Example 178), AD47882.71DUG (compound obtained by applying the type 71 modification pattern to the AD47882.88DUG compound), AD47882.80DUG (compound obtained by applying the type 80 modification pattern to the AD47882.88DUG compound) or AD47882.90DUG (compound obtained by applying the type 90 modification pattern to the AD47882.88DUG compound), and it was confirmed that the plasma iron concentration increased 28 days after the treatment. These results suggest that by applying the same modification pattern as those of the above-mentioned compounds to TfR2 siRNA with different target sequences, siRNA capable of stably suppressing the mRNA expression level of TfR2 to increase the plasma iron concentration can be obtained.

### Test Example 11: Drug efficacy evaluation test for GalNAc-conjugated TfR2 siRNA in a mouse model of anemia associated with loss of bone-marrow function

Using AD47882.23DUG (Example 171) and AD47882.94DUG (Example 179) as GalNAc-conjugated TfR2 siRNA, the following experiment was conducted. To induce loss of bone-marrow function, 100 mg/kg of carboplatin was administered intraperitoneally (i.p.) to male C57BL/6NJcl mice (from CLEA Japan, Inc.). Grouping was performed on the basis of the HGB concentration and the plasma iron concentration 4 days after the administration of carboplatin, followed by drug agent treatment (n = 6/group). The drug agent was administered subcutaneously (s.c.) once at a dose of 3 mg/kg (3 mpk) to a GalNAc-conjugated TfR2 siRNA treatment group. PBS was administered subcutaneously once to a vehicle group.

Before the drug agent treatment (Day 0), blood was collected from the tail vein, and the HGB concentration was measured using QuantiChrom Whole Blood HB Kit (manufactured by Bioassay Systems, LLC.). The results for the HGB concentration before the drug agent treatment are shown in Figure 14A (average value ± standard error). The plasma iron concentration was measured using a Metallo-assay iron measurement kit (manufactured by Metallogenics Co., Ltd.). The results for the plasma iron concentration before the drug agent treatment are shown in Figure 14B (average value ± standard error). Figure 14C shows results for the plasma iron concentration 8 days after the drug agent treatment (Day 8). Nine days after the drug agent treatment (Day 9), blood was collected from the abdominal large vein under anesthesia with isoflurane, and the HGB concentration, the MCH amount and the number of RBCs were measured using a multi-item automatic corpuscle analyzer XT 2000 (manufactured by Sysmex Corporation). The results are shown in Figures 14D to 14F (average value ± standard error), respectively.

These results show that by treatment with GalNAc-conjugated TfR2 siRNA, the plasma iron concentration may be increased in order to increase the HGB concentration, thus providing a therapeutic effect on anemic diseases such as anemia associated with bone-marrow dysfunction, for example, anemia associated with myelofibrosis.

### Test Example 12: Drug efficacy evaluation test for GalNAc-conjugated TfR2 siRNA in a mouse model of anemia associated with inflammation

Using AD47882.92DUG (Example 177) and AD47882.94DUG (Example 179) as GalNAc-conjugated TfR2 siRNA, the following experiment was conducted. To induce inflammation, turpentine was administered subcutaneously (s.c.) to male C57BL/6NJcl mice (from CLEA Japan, Inc.) at 150 µL/mouse three times a week. PBS was administered subcutaneously instead of turpentine to a control group in which inflammation was not induced (control). Seven days after the first administration of turpentine (Day 0), blood was collected from the tail vein, and the plasma iron concentration and the HGB concentration were measured. The plasma iron concentration was measured using a Metallo-assay iron measurement kit (manufactured by Metallogenics Co., Ltd.). The HGB concentration was measured using QuantiChrom Whole Blood HB Kit (manufactured by Bioassay Systems, LLC.). The results are shown in Figures 15A and 15B (average value ± standard error), respectively. The drug agent was administered to male C57BL/6NJcl mice (from CLEA Japan, Inc.) (n = 5/group). The drug agent was administered subcutaneously once at a dose of 3 mg/kg (3 mpk) to a GalNAc-conjugated TfR2 siRNA treatment group. PBS was administered subcutaneously once to a vehicle group.

Four days after the drug agent treatment (Day 4) and 8 days after the treatment (Day 8), blood was collected from the tail vein, and the plasma iron concentration was measured using a Metallo-assay iron measurement kit (manufactured by Metallogenics Co., Ltd.). The results are shown in Figure 15A (average value ± standard error). In addition, 8 days after the drug agent treatment, blood was collected from the abdominal large vein under anesthesia with isoflurane, and the HGB concentration, the MCH amount and the number of RBCs were measured using a multi-item automatic corpuscle analyzer XT 2000 (manufactured by Sysmex Corporation). The results are shown in Figures 15C to 15E (average value ± standard error), respectively. The mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 15F. As the mRNA level in the control group which did not undergo induction of inflammation is assumed to be 1.0, relative mRNA levels in other groups were calculated and recorded (average value ± standard error).

These results show that by treatment with GalNAc-conjugated TfR2 siRNA, the mRNA expression level of TfR2 may be suppressed in order to increase the HGB concentration, thus providing a therapeutic effect on anemic diseases such as anemia associated with inflammation, for example, anemia associated with chronic kidney disease and cancerous anemia.

### Test Example 13: Drug efficacy evaluation test for GalNAc-conjugated TfR2 siRNA in NUP98-HOXD13 mice

NUP98-HOXD13 mice (C57BL/6-Tg (Vav1-NUP98/HOXD13)G2Apla/J) are known as a mouse model of myelodysplastic syndrome (MDS) characterized by ineffective hematopoiesis and insufficient differentiation and maturation of erythrocytes (Nature Medicine volume 20, pages 408-414 (2014)). In order to verify the therapeutic effect of TfR2 siRNA on MDS, a GalNAc-conjugated TfR2 siRNA administration test was conducted on NUP98-HOXD13 mice. The compound used was AD47882.23DUG (Example 171).

NUP98-HOXD13 mice were purchased from The Jackson Laboratory Japan, Inc., and used in the experiment. The mice were grouped on the basis of plasma iron concentration, the HGB concentration and body weight in four-month-old NUP98-HOXD13 mice, followed by drug agent treatment (n = 8/group). The drug agent was administered subcutaneously (s.c.) once a week at a dose of 5 mg/kg (5 mpk) to a GalNAc-conjugated TfR2 siRNA treatment group. PBS was subcutaneously administered once a week to a vehicle group.

Two weeks, four weeks and eight weeks after the start of drug agent treatment, blood was collected from the tail vein, and the plasma iron concentration was measured using a Metallo-assay iron measurement kit (manufactured by Metallogenics Co., Ltd.). The results are shown in Figure 16A (average value ± standard error). Two weeks, four weeks, six weeks and eight weeks after the start of drug agent treatment, blood was collected from the tail vein, and the HGB concentration was measured using QuantiChrom Whole Blood HB Kit (manufactured by Bioassay Systems, LLC.). The results are shown in Figure 16B (average value ± standard error). Eight weeks after the start of drug agent treatment, blood was collected from the abdominal large vein under anesthesia with isoflurane, and the HGB concentration, the MCH amount and the number of RBCs were measured using a multi-item automatic corpuscle analyzer XT 2000 (manufactured by Sysmex Corporation). The results are shown in Figure 16C to 16E (average value ± standard error), respectively. The mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 16F. As the mRNA level in the vehicle group is assumed to be 1.0, relative mRNA levels in other groups were calculated and recorded (average value ± standard error).

These results show that by treatment with GalNAc-conjugated TfR2 siRNA, the mRNA expression level of TfR2 may be suppressed in order to increase the plasma iron concentration and resulting in an increase in the HGB concentration, thus providing a therapeutic effect on MDS that is an anemic disease associated with loss of bone-marrow function.

### Test Example 14: Drug efficacy evaluation test for GalNAc-conjugated TfR2 siRNA in a mouse model of anemia associated with chronic kidney disease

Using the compound of AD47882.94DUG (Example 179) as GalNAc-conjugated TfR2 siRNA, the following experiment was conducted. To induce chronic kidney disease and anemia associated therewith, a food prepared so as to have an adenine (010-11513 manufactured by Wako Pure Chemical Industries, Ltd.) content of 0.2% was given to male C57BL/6NJcl mice (from CLEA Japan, Inc.). Two weeks after the 0.2% adenine food was given, blood was collected from the tail vein, and the plasma iron concentration and the HGB concentration were measured. The plasma iron concentration was measured using a Metallo-assay iron measurement kit (manufactured by Metallogenics Co., Ltd.). The HGB concentration was measured using QuantiChrom Whole Blood HB Kit (manufactured by Bioassay Systems, LLC.). The results are shown in Figures 17A to 17B (average value ± standard error), respectively. Grouping was performed on the basis of the plasma iron concentration, the HGB concentration and body weight, followed by drug agent treatment (n = 6/group). The drug agent was administered subcutaneously once a week at a dose of 3 mg/kg (3 mpk), 1 mg/kg (1 mpk) or 0.3 mg/kg (0.3 mpk) to a GalNAc-conjugated TfR2 siRNA treatment group. PBS was administered subcutaneously once a week to a vehicle group. A food free of 0.2% adenine was given to a control group in which anemia associated with chronic kidney disease was not induced (control).

Six weeks after the start of the drug agent treatment, blood was collected from the tail vein, and the plasma iron concentration was measured using a Metallo-assay iron measurement kit (manufactured by Metallogenics Co., Ltd.). The results are shown in Figure 17C (average value ± standard error). Six weeks after the start of the drug agent treatment, the HGB concentration, the MCH amount and the number of RBCs were measured by applying a multi-item automatic corpuscle analyzer XT 2000 (manufactured by Sysmex Corporation) on blood collected from the abdominal large vein under anesthesia with isoflurane. The results are shown in Figures 17D to 17F (average value ± standard error), respectively. The mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 17G. As the mRNA level in the vehicle group is assumed to be 1.0, the relative mRNA levels in other groups were calculated and recorded (average value ± standard error).

These results show that by treatment with GalNAc-conjugated TfR2 siRNA, the mRNA expression level of TfR2 may be suppressed in order to increase the plasma iron concentration and the HGB concentration, thus providing a therapeutic effect on anemia associated with chronic kidney disease.

### Test Example 15: Drug efficacy evaluation test for GalNAc-conjugated TfR2 siRNA in a mouse model of anemia associated with administration of ruxolitinib (JAK 1/JAK 2 inhibitor)

To verify the effect of TfR2 siRNA on anemia caused by administration of ruxolitinib, the following experiment was conducted using the compound of AD47882.23DUG (Example 171) as GalNAc-conjugated TfR2 siRNA.

For inducing anemia caused by administration of ruxolitinib, a food prepared so as to have a ruxolitinib (R-6688 manufactured by LC Laboratories) content of 0.1% was given to male C57BL/6NJcl mice (from CLEA Japan, Inc.). Six weeks after the 0.1% ruxolitinib-containing food was given, blood was collected from the tail vein, and the HGB concentration was measured using a multi-item automatic corpuscle analyzer XT 2000 (manufactured by Sysmex Corporation). The results are shown in Figure 18A (average value ± standard error). Grouping was performed on the basis of the HGB concentration and body weight, followed by drug agent treatment (n = 5/group). The drug agent was administered subcutaneously (s.c.) once a week at a dose of 3 mg/kg (3 mpk) to a GalNAc-conjugated TfR2 siRNA treatment group. PBS was administered subcutaneously once a week to a vehicle group. A food free of 0.1% ruxolitinib was given to a control group in which anemia caused by ruxolitinib was not induced (control).

Three weeks after the start of the drug agent treatment, blood was collected from the tail vein, and the plasma iron concentration was measured using a Metallo-assay iron measurement kit (manufactured by Metallogenics Co., Ltd.). The results are shown in Figure 18B (average value ± standard error). Six weeks after the start of the drug agent treatment, the HGB concentration, the MCH amount and the number of RBCs were measured by applying a multi-item automatic corpuscle analyzer XT 2000 (manufactured by Sysmex Corporation) on blood collected from the abdominal large vein under anesthesia with isoflurane. The results are shown in Figures 18C to 18E (average value ± standard error), respectively. The mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 18F. As the mRNA level in the control group which was not given the 0.1% ruxolitinib-containing food is assumed to be 1.0, relative mRNA levels in other groups were calculated and recorded (average value ± standard error).

These results show that it may be possible to provide a combined effect of a JAK 2 inhibitor, such as ruxolitinib, and TfR2 siRNA in the treatment of myelofibrosis.

### Test Example 16: Drug efficacy evaluation test for GalNAc-conjugated TfR2 siRNA in human TfR2-expressing mice

To verify the effect of GalNAc-conjugated TfR2 siRNA on human TfR2 mRNA, the following experiment was conducted using male or female TfR2 humanized mice having a human TfR2 gene locus and generated in Institute of Immunology Co., Ltd. TfR2 humanized mice were produced using bacterial artificial chromosome (BAC) clones including the human TFR2 gene locus (RP11-264N5). As GalNAc-conjugated TfR2 siRNA, TfR2-019.88DUG (compound of Example 162), TfR2-019.92DUG (compound of Example 167), TfR2-019.93DUG (compound of Example 168) and TfR2-019.94DUG (compound of Example 169) were used.

The siRNA was administered subcutaneously (s.c.) at a dose of 3 mg/kg (3 mpk) or 0.3 mg/kg (0.3 mpk) once (n = 3/group) to the GalNAc-conjugated TfR2 siRNA treatment group. PBS was administered subcutaneously once (n = 4/group) to the vehicle group.

Seven days after the start of the drug agent treatment, the mice were painlessly killed by bloodletting under anesthesia with isoflurane, the liver was then isolated, and the mRNA expression level of hTfR2 was measured. Extraction of RNA and quantitative PCR were performed in the manner described in Test Example 5. The mRNA expression level of hTfR2 was standardized with the mRNA expression level of mActb, which is the housekeeping gene. As the primer for hTfR2, those described in Test Examples 1 and 2 were used. The results for the mRNA expression level of hTfR2 are shown in Figure 19. As the mRNA level in the vehicle group is assumed to be 1.0, relative mRNA levels in other groups were calculated and recorded (average value ± standard error).

These results demonstrate that treatment with GalNAc-conjugated TfR2 siRNA enables suppression of the expression level of human TfR2 mRNA. In addition, they indicate that suppression of the expression level of human TfR2 mRNA may increase the HGB concentration, and provide a therapeutic effect for anemic diseases such as anemia associated with bone-marrow dysfunction, for example, anemia associated with bone-marrow fibrosis, and anemic diseases such as anemia associated with inflammation, for example, anemia associated with chronic kidney disease. As another test, mice obtained by further knocking out endogenous mouse TfR2 from human TfR2-expressing mice were treated with TfR2-019.94DUG (compound of Example 169), and it was confirmed that even after 42 days, expression of the human TfR2 gene was suppressed, and thus TfR2-019.94DUG had a stable human TfR2 gene expression-suppressing effect.

### Test Example 17: Drug efficacy evaluation test for GalNAc-conjugated TfR2 siRNA using human primary liver cells

GalNAc-conjugated siRNA is known to be incorporated into the liver via asialogycoprotein receptor 1 (ASGPR) expressed in liver hepatocytes. To verify the effect of GalNAc-conjugated TfR2 siRNA under conditions that do not involve the use of a transfection reagent, a drug efficacy evaluation test for GalNAc-conjugated TfR2 siRNA was conducted using human primary liver cells. As GalNAc-conjugated TfR2 siRNA, TfR2-019.88DUG (Example 162), TfR2-019.89DUG (Example 163), TfR2-019.90DUG (Example 164), TfR2-019.91DUG (Example 166), TfR2-019.92DUG (Example 167),TfR2-019.93DUG (Example 168), TfR2-019.94DUG (Example 169) and TfR2-019.95DUG (Example 170) were used.

### (Culture of human primary liver cells)

Human primary liver cells (HMCPIS manufactured by Life Technologies Corporation) stored in a liquid nitrogen tank were thawed using a warm bath at 37°C, and poured into 10 mL of a cryopreserved hepatocytes recovery medium (CHRM) (CM7000 manufactured by Life Technologies Corporation) warmed in advance to 37°C, and inversion mixing was performed. Thereafter, the mixture was centrifuged (100 g, 10 minutes), and the supernatant was removed. A cell suspension was then prepared using 5 mL of a cell seeding medium warmed in advance to 37°C. The cell seeding medium was prepared by adding a hepatocyte plating supplement pack (CM3000 manufactured by Life Technologies Corporation) to William's medium E (A1217601 manufactured by Life Technologies Corporation). Thereafter, the number of living cells was counted, and adjusted to 5 × 10⁵ cells/mL using the cell seeding medium. The cells were seeded at 5 × 10⁴ cells per well of a collagen-coated 96-well plate, and cultured in a CO₂ incubator for 4 to 6 hours. Using a microscope, it was confirmed that the cells adhered to the plate. Thereafter, an experiment of adding GalNAc-conjugated TfR2 siRNA was conducted.

### (Addition of GalNAc-conjugated TfR2 siRNA)

A medium containing GalNAc-conjugated TfR2 siRNA was prepared using a cell culturing medium warmed in advance to 37°C. A 2-well-equivalent amount of a siRNA sample and a 2-well-equivalent amount of a negative control (NT) with distilled water used instead of siRNA were prepared. The samples were prepared such that the concentration of siRNA was changed from 10,000 nM to a final concentration of 4.8 nM using a common dilution ratio of 5. The cell culturing medium was prepared by adding a hepatocyte maintenance supplement pack (CM4000 manufactured by Life Technologies Corporation) to William's medium E (A1217601 manufactured by Life Technologies Corporation). The cell seeding medium in each well for human primary liver cells seeded on the 96-well plate was removed by suction, and 100 µL of the prepared medium containing GalNAc-conjugated TfR2 siRNA was then added to each well. Three days after the compound was added, the cells were washed once with PBS, and RNA was extracted.

### (Extraction of RNA and qPCR)

Extraction of RNA and quantitative PCR were performed in the manner described in Test Example 1. The mRNA expression level of each of hTfR2 and h18S was given as an average value of two wells. The mRNA expression level of hTfR2 was standardized with the mRNA expression level of h18S, which is the housekeeping gene. As the hTfR2 mRNA expression level in the negative control (NT) is assumed to be 1.0, relative hTfR2 mRNA expression levels of the siRNA-treated samples were calculated and recorded (average value ± standard error). The results are shown in Figures 20A to 20C, respectively.

These results show that by treatment with GalNAc-conjugated TfR2 siRNA, the GalNAc-conjugated TfR2 siRNA is incorporated into human liver hepatocytes, thus suppressing the human TfR2 mRNA expression level. The suppression of the human TfR2 mRNA expression level may increase the HGB concentration, thus providing a therapeutic effect on anemic diseases such as anemia associated with bone-marrow dysfunction, for example, anemia associated with myelofibrosis, and anemic diseases such as anemia associated with inflammation, for example anemia associated with chronic kidney disease.

### Test Example 18: Effect of TfR2 siRNA with HepG2 cells on hepcidin gene expression

The mRNA expression level of hepcidin was evaluated 2 days after introduction of siRNA into HepG2 cells by a reverse transfection method in the same manner as in Test Example 2. As a primer for hepcidin, Hs00221783_m1 (Thermo Fisher Scientific Inc.) was used. As siRNA, TfR2-019 (Example 19), TfR2-039 (Example 39), TfR2-019.94DUG (Example 169) and TfR2-039.23DUG (Example 144) were used.

To evaluate the hepcidin mRNA expression level, the relative hepcidin mRNA expression levels of the siRNA-treated samples were calculated and recorded as relative values as the hepcidin mRNA expression level in the negative control (NT) is assumed to be 1.0. The results are shown in Figure 21.

### Industrial Applicability

The siRNA of the present invention is capable of suppressing expression of TfR2 mRNA, and can suppress production of hepcidin which plays a key role in iron metabolism. The siRNA of the present invention is beneficial in the treatment or prevention of a disease that can be treated or prevented by suppressing expression of TfR2 mRNA and/or production of hepcidin.

### Free Text of Sequence Listing

SEQ ID NO: 1 - Nucleotide sequence of hTfR2 mRNA
SEQ ID NOS: 2 to 24 - Nucleotide sequences of single-stranded oligonucleotides
SEQ ID NOS: 25 to 28 - Nucleotide sequences of sense strand regions of double-stranded oligonucleotides
SEQ ID NOS: 29 to 35 - Nucleotide sequences of antisense strand regions of double-stranded oligonucleotides
SEQ ID NOS: 36 and 37 - Nucleotide sequences of sense strand regions of double-stranded oligonucleotides
SEQ ID NOS: 38 to 42 - Nucleotide sequences of antisense strand regions of double-stranded oligonucleotides
SEQ ID NO: 43 - Nucleotide sequence of a complementary region of a sense strand region in TfR2-019
SEQ ID NO: 44 - Nucleotide sequence of a complementary region of an antisense strand region in TfR2-019
SEQ ID NO: 45 - Nucleotide sequence of a complementary region of a sense strand region in TfR2-039
SEQ ID NO: 46 - Nucleotide sequence of a complementary region of an antisense strand region in TfR2-039
SEQ ID NO: 47 - Nucleotide sequence of a sense strand region of a double-stranded oligonucleotide

## Claims

1. An oligonucleotide or a pharmaceutically acceptable salt thereof comprising the following antisense strand region (A) and sense strand region (B), and having a knockdown action on mRNA of transferrin receptor 2:
(A) an antisense strand region which is 18 to 31 bases in length and consists of a target-matching sequence substantially complementary to a target sequence consisting of 18 to 21 consecutive bases in a region between nucleotide positions 2845 and 2867 or a region between nucleotide positions 1534 and 1556 in SEQ ID NO: 1, in which a nucleoside at the 5'-terminus of the target-matching sequence is a nucleoside substantially complementary to a nucleoside at the 3'-terminus of the target sequence, a nucleoside having adenine, or a nucleoside having uracil, and an overhang structure of 5 or less bases may be present at the 5'-terminus and/or the 3'-terminus of the target-matching sequence; and
(B) a sense strand region which is 18 to 31 bases in length and contains a nucleotide sequence substantially complementary to the target-matching sequence of the antisense strand region, in which an overhang structure of 5 or less bases is optionally present at the 5'-terminus and/or the 3'-terminus of the nucleotide sequence.

2. The oligonucleotide or a pharmaceutically acceptable salt thereof according to claim 1, wherein the target sequence is a nucleotide sequence consisting of 19 bases from nucleotide positions 2847 to 2865 or from nucleotide positions 1536 to 1554 in SEQ ID NO: 1.

3. The oligonucleotide or a pharmaceutically acceptable salt thereof according to claim 1 or 2, wherein the nucleoside at the 5'-terminus of the target-matching sequence of the antisense strand region is a nucleoside having adenine or uracil.

4. The oligonucleotide or a pharmaceutically acceptable salt thereof according to any one of claims 1 to 3, wherein the target-matching sequence of the antisense strand region is a nucleotide sequence fully complementary to the target sequence consisting of 19 bases from nucleotide positions 2847 to 2865 or from nucleotide positions 1536 to 1554 in SEQ ID NO: 1.

5. The oligonucleotide or a pharmaceutically acceptable salt thereof according to any one of claims 1 to 4, wherein at least one of sugars and/or phosphodiester bonds forming the oligonucleotide is modified.

6. The oligonucleotide or a pharmaceutically acceptable salt thereof according to claim 5, wherein the sugar forming the oligonucleotide is D-ribofuranose, and the modification of the sugar is modification of a hydroxyl group at the 2'-position of D-ribofuranose.

7. The oligonucleotide or a pharmaceutically acceptable salt thereof according to claim 6, wherein the modification of the sugar is 2'-deoxidation, 2'-O-alkylation, 2'-0-alkoxyalkylation, 2'-halogenation and/or 2'-O,4'-C-alkylenation of D-ribofuranose.

8. The oligonucleotide or a pharmaceutically acceptable salt thereof according to claim 7, wherein the 2'-O-alkylation is 2'-O-methylation, the 2'-0-alkoxyalkylation is 2'-O-methoxyethylation, the 2'-halogenation is 2'-fluorination, and the 2'-O,4'-C-alkylenation is 2'-O,4'-C-methylenation and/or 2'-O,4'-C-ethylenation.

9. The oligonucleotide or a pharmaceutically acceptable salt thereof according to any one of claims 5 to 8, wherein the phosphodiester bond is modified to be phosphorothioate.

10. The oligonucleotide or a pharmaceutically acceptable salt thereof according to any one of claims 5 to 9, wherein the D-ribofuranose in the nucleoside at the 3'-terminus of the sense strand region and/or the antisense strand region is 3'-O-alkylated.

11. The oligonucleotide or a pharmaceutically acceptable salt thereof according to claim 10, wherein the 3'-O-alkylation is 3'-O-methylation.

12. The oligonucleotide or a pharmaceutically acceptable salt thereof according to any one of claims 1 to 11, wherein an overhang structure of 3 or less bases is present at the 5'-terminus and/or the 3'-terminus of the sense strand region and/or the antisense strand region.

13. The oligonucleotide or a pharmaceutically acceptable salt thereof according to claim 12, wherein the overhang structure has 2 bases.

14. The oligonucleotide or a pharmaceutically acceptable salt thereof according to claim 13, wherein the overhang structure is nucleosides containing two consecutive thymines, or nucleosides containing two consecutive uracils.

15. The oligonucleotide or a pharmaceutically acceptable salt thereof according to claim 14, wherein the overhang structure is two consecutive U(M) present at the 3'-terminus of the antisense strand region.

16. The oligonucleotide or a pharmaceutically acceptable salt thereof according to claim 1, wherein the sense strand region consists of an oligonucleotide represented by the following formula (I), and the antisense strand region consists of an oligonucleotide represented by the following formula (II), and having the following characteristics (a) to (g),:
sense strand region: 5' S_{O5}-Sₐ-S₁₉-S₁₈-S₁₇-S₁₆-S₁₅-S₁₄-S₁₃-S₁₂-S₁₁-S₁₀-S₉-S₈-S₇-S₆-S₅-S₄-S₃-S₂-S₁-S_{O3} 3' (I)
antisense strand region: 5' A₀₅-A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-Aₐ-A₀₃ 3' (I I)
(a) S₁ is a 3'-modified nucleoside, S₂ to S₁₉ each represent a nucleoside, and are each independently a 2'-OMe RNA, a 2'-F RNA or a DNA, Sₐ represents 0 to 3 nucleosides, where the nucleosides each independently represent 2'-OMe RNA, 2'-F RNA or DNA, S_{O3} and S_{O5} each independently represent 0 to 3 nucleosides, where the nucleosides each independently represent 2'-OMe RNA, 2'-F RNA, DNA or a 2 '-O,4'-C-bridging-modified nucleoside, and each inter-nucleoside bond represents a phosphodiester bond that may be chemically modified;
(b) A₁₁, A₁₂, A₁₃, A₁₄ and A₁₅ each represent a nucleoside, at least one of A₁₁, A₁₂, A₁₃, A₁₄ and A₁₅ is DNA, RNA, a 2 '-O,4'-C-bridging-modified nucleoside or 2'-MOE RNA, the others are 2'-OMe RNA or 2'-F RNA, A₁ to A₁₀ and A₁₆ to A₁₉ each represent a nucleoside, and each independently represent 2'-OMe RNA, 2'-F RNA or DNA, Aₐ represents 0 to 3 nucleosides, where the nucleosides each independently represent 2'-OMe RNA, 2'-F RNA or DNA, A_{O3} and A_{O5} each independently represent 0 to 3 nucleosides, where the nucleosides each independently represent 2'-OMe RNA, 2'-F RNA, DNA or a 2 '-O,4'-C-bridging-modified nucleoside, and each inter-nucleoside bond represents a phosphodiester bond that may be chemically modified;
(c) the nucleotide sequence between A₂ and Aₐ consists of a nucleotide sequence substantially complementary to a target sequence, A₁ is a nucleoside having a base complementary to a corresponding nucleoside of the target sequence, a nucleoside having adenine, a nucleoside having thymine, or a nucleoside having uracil, and when A₀₃ and A₀₅ are present, the nucleotide sequences thereof are each independently a nucleotide sequence selected independently of a corresponding nucleoside of the target sequence;
(d) the nucleotide sequence between S₁ and Sₐ and the nucleotide sequence between A₁ and Aₐ are nucleotide sequences substantially complementary to each other, and form a double-stranded structure;
(e) when both S_{O5} and A_{O3} are present, S_{O5} and A_{O3} are nucleotide sequences not complementary to each other;
(f) when both S_{O3} and A_{O5} are present, S_{O3} and A_{O5} are nucleotide sequences not complementary to each other; and
(g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.

17. The oligonucleotide or a pharmaceutically acceptable salt thereof according to claim 16, wherein in formula (II), S₁ is 3'-OMe RNA.

18. The oligonucleotide or a pharmaceutically acceptable salt thereof according to claim 16 or 17, wherein the 2'-O,4'-C-bridging-modified nucleoside is LNA or ENA.

19. The oligonucleotide or a pharmaceutically acceptable salt thereof according to any one of claims 16 to 18, wherein in formula (II), two or more of A₁₁, A₁₂, A₁₃, A₁₄ and A₁₅ are the same or different, and are DNA, RNA, a 2 '-O,4'-C-bridging-modified nucleoside or 2'-MOE RNA.

20. The oligonucleotide or a pharmaceutically acceptable salt thereof according to claim 19, wherein in formula (II), A₁₄ is RNA or a 2 '-O,4'-C-bridging-modified nucleoside.

21. The oligonucleotide or a pharmaceutically acceptable salt thereof according to claim 20, wherein in formula (II), A₁₃ is a 2 '-O,4'-C-bridging-modified nucleoside or 2' -OMe RNA, and A₁₄ is RNA.

22. The oligonucleotide or a pharmaceutically acceptable salt thereof according to claim 21, wherein in formula (II), A₁₁-A₁₂-A₁₃-A₁₄-A₁₅ is one of:
A₁₁(2'-OMe RNA)-A12(2'-OMe RNA)-A₁₃(ENA)-A₁₄(RNA)-A₁₅(2'-OMe RNA),
A₁₁(2'-F RNA)-A12(2'-OMe RNA)-A₁₃(ENA)-A₁₄(RNA)-A₁₅(2'-OMe RNA),
A₁₁(2'-OMe RNA)-A₁₂(2'-F RNA)-A₁₃(ENA)-A₁₄(RNA)-A₁₅(2'-OMe RNA),
A₁₁(2'-OMe RNA)-A12(2'-OMe RNA)-A₁₃(LNA)-A₁₄(RNA)-A₁₅(2'-OMe RNA),
A₁₁(2'-F RNA)-A₁₂(2'-OMe RNA)-A₁₃(LNA)-A₁₄(RNA)-A₁₅(2'-OMe RNA),
A₁₁(2'-OMe RNA)-A₁₂(2'-F RNA)-A₁₃(LNA)-A₁₄(RNA)-A₁₅(2'-OMe RNA),
A₁₁(2'-F RNA)-A₁₂(2'-OMe RNA)-A₁₃(2'-OMe RNA)-A₁₄(RNA) - A₁₅(2'-OMe RNA), and,
A₁₁(2'-OMe RNA)-A₁₂ (2'-F RNA)-A₁₃(2'-OMe RNA)-A₁₄(RNA) - A₁₅(2'-OMe RNA) .

23. The oligonucleotide or a pharmaceutically acceptable salt thereof according to claim 20, wherein in formula (II), A₁₂ is DNA, and A₁₄ is a 2 '-O,4'-C-bridging-modified nucleoside.

24. The oligonucleotide or a pharmaceutically acceptable salt thereof according to claim 23, wherein in formula (II), A₁₁-A₁₂-A₁₃-A₁₄-A₁₅ is one of:
A₁₁(2'-F RNA)-A₁₂(DNA)-A₁₃(2'-OMe RNA)-A₁₄(ENA)-A₁₅(2'-OMe RNA),
A₁₁(2'-OMe RNA)-A₁₂(DNA)-A₁₃(2'-OMe RNA)-A₁₄(ENA)-A₁₅(2'-OMe RNA), A₁₁(2'-F RNA)-A₁₂(DNA)-A₁₃(2'-OMe RNA)-A₁₄(LNA)-A₁₅ (2'-OMe RNA), and,
A₁₁(2'-OMe RNA)-A₁₂(DNA)-A₁₃(2'-OMe RNA)-A₁₄(LNA)-A₁₅(2'-OMe RNA) .

25. The oligonucleotide or a pharmaceutically acceptable salt thereof according to any one of claims 16 to 24, wherein in formula (II), A₂, A₆ and A₁₆ are 2'-F RNA.

26. The oligonucleotide or a pharmaceutically acceptable salt thereof according to claim 25, wherein in formula (II), one or two nucleosides selected from A₈, A₉ and A₁₀ are 2'-F RNA, and the nucleosides A₁, A₃ to A₅, A₇, A₁₇ to A₁₉ and Aₐ are 2' -OMe RNA.

27. The oligonucleotide or a pharmaceutically acceptable salt thereof according to claim 26, wherein in formula (II), A₂, A₆, A₈, A₁₀ and A₁₆ are 2'-F RNA, and the nucleosides A₁, A₃ to A₅, A₇, A₉, A₁₇ to A₁₉ and Aₐ are 2'-OMe RNA.

28. The oligonucleotide or a pharmaceutically acceptable salt thereof according to any one of claims 16 to 27, wherein in formula (I), S₁₁, S₁₂, S₁₃ and S₁₅ are 2'-F RNA.

29. The oligonucleotide or a pharmaceutically acceptable salt thereof according to claim 28, wherein in formula (I), S₂ to S₁₀, S₁₄, S₁₆ to S₁₉ and Sₐ are 2'-OMe RNA.

30. The oligonucleotide or a pharmaceutically acceptable salt thereof according to any one of claims 16 to 29, wherein when RNA is used in formula (II), a bond between the RNA and a 3' adjacent nucleoside is a phosphorothioate bond.

31. The oligonucleotide or a pharmaceutically acceptable salt thereof according to any one of claims 16 to 30, wherein in 2 to 5 nucleotides from each of the 5'-terminus and the 3'-terminus of the oligonucleotide, each inter-nucleoside bond is a phosphorothioate bond.

32. The oligonucleotide or a pharmaceutically acceptable salt thereof according to any one of claims 16 to 31, wherein the number of nucleosides in each of S_{O3}, S_{O5,} A_{O5} and A_{O3} is 0 to 2.

33. The oligonucleotide or a pharmaceutically acceptable salt thereof according to claim 32, wherein the number of nucleosides in S_{O3} is 0.

34. The oligonucleotide or a pharmaceutically acceptable salt thereof according to claim 33, wherein the number of nucleosides in each of S_{O3}, S_{O5} and A_{O5} is 0, and the number of nucleosides in A_{O3} is 2.

35. The oligonucleotide or a pharmaceutically acceptable salt thereof according to any one of claims 1 to 34, wherein the antisense strand region and the sense strand region form a double-stranded oligonucleotide as independent oligonucleotides.

36. The oligonucleotide or a pharmaceutically acceptable salt thereof according to any one of claims 1 to 34, wherein the nucleoside at the 5'-terminus of the antisense strand region and the nucleoside at the 3'-terminus of the sense strand region are connected by a linker structure.

37. The oligonucleotide or a pharmaceutically acceptable salt thereof according to claim 36, wherein the linker structure is represented by the following formula: 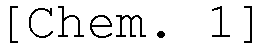 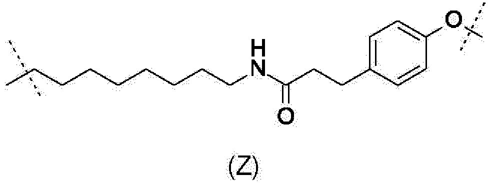 wherein the broken line represents a point of attachment, and means that the oxygen atom bonded to the phenyl group forms a phosphodiester bond or a phosphorothioate bond with a 5'-phosphate group of a nucleotide at the 5'-terminus of an adjacent antisense strand region, and the methylene group at the other end forms a phosphodiester bond or a phosphorothioate bond with a 3'-phosphate group (phosphate group at the 2'-position in the case of a nucleotide modified at the 3'-position) of a nucleotide at the 3'-terminus of an adjacent sense strand region.

38. The oligonucleotide or a pharmaceutically acceptable salt thereof according to any one of claims 1 to 37, wherein there is a chemical modification with a GalNAc unit at the 5'-position of a nucleoside at the 5'-terminus of the oligonucleotide and/or the 3'-position of a nucleoside at the 3'-terminus of the oligonucleotide, or the 2'-position of the nucleoside at the 3'-terminus of the oligonucleotide in the case where the nucleoside at the 3'-terminus is a 3'-modified nucleoside.

39. The oligonucleotide or a pharmaceutically acceptable salt thereof according to claim 38, wherein the GalNAc unit is represented by: 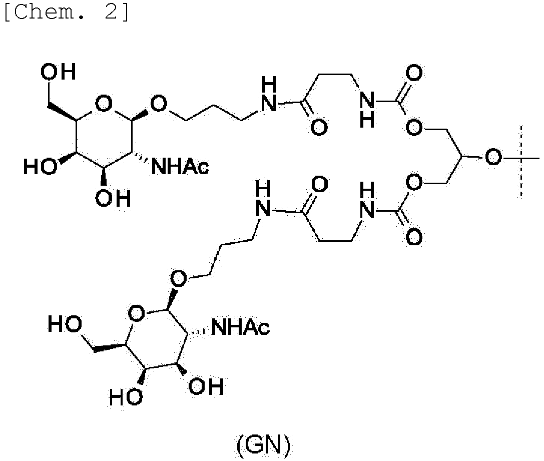 wherein the broken line represents a phosphodiester bond formed with a phosphate group at the 5'-terminus and/or a phosphate group (phosphate group at the 2'-position in the case of a nucleotide modified at the 3'-position) at the 3'-terminus of an adjacent nucleotide.

40. The oligonucleotide or a pharmaceutically acceptable salt thereof according to claim 1, wherein the sense strand region excluding the overhang structure has the following formula (I-1), the antisense strand region excluding the overhang structure has any of the following formulae (II-1) to (II-10), the sense strand region and the antisense strand region form a double-stranded oligonucleotide as independent oligonucleotides, and the sense strand region and the antisense strand region may each independently contain an overhang structure:
(formulae)
5' C(M)G(M)U(M)G(M)G(F)A(M)G(F)U(F)U(F)U(M)C(M)A(M)A(M)U(M)A (M)U(M)C(M)A(M)A(3M) 3' (I-1)
5' U(M)U(F)G(M)A(M)U(M)A(F)U(M)U(F)G(F)A(M)A(M)A(D)C(M)T(E)C (M)C(F)A(M)C(M)G(M) 3' (II-1)
5' U(M)U(F)G(M)A(F)U(M)A(F)U(M)U(M)G(F)A(M)A(M)A(D)C(M)T(E)C (M)C(F)A(M)C(M)G(M) 3' (11-2)
5' U(M)U(F)G(M)A(M)U(M)A(F)U(M)U(M)G(F)A(F)A(M)A(D)C(M)T(E)C (M)C(F)A(M)C(M)G(M) 3' (II-3)
5' U(M)U(F)G(M)A(M)U(M)A(F)U(M)U(M)G(F)A(M)A(M)A(D)C(M)T(E)C (M)C(F)A(M)C(F)G(M) 3' (11-4)
5' U(M)U(F)G(M)A(M)U(M)A(F)U(M)U(F)G(F)A(M)A(M)A(M)C(E)U(R)^ C(M)C(F)A(M)C(M)G(M) 3' (II-5)
5' U(M)U(F)G(M)A(M)U(M)A(F)U(M)U(M)G(F)A(F)A(M)A(M)C(E)U(R)^ C(M)C(F)A(M)C(M)G(M) 3' (11-6)
5' U(M)U(F)G(M)A(M)U(M)A(F)U(M)U(M)G(F)A(M)A(F)A(M)C(E)U(R)^ C(M)C(F)A(M)C(M)G(M) 3' (11-7)
5' U(M)U(F)G(M)A(M)U(M)A(F)U(M)U(M)G(F)A(M)A(M)A(F)C(E)U(R)^ C(M)C(F)A(M)C(M)G(M) 3' (II-8)
5' U(M)U(F)G(M)A(M)U(M)A(F)U(M)U(M)G(F)A(M)A(F)A(M)C(M)U(R)^ C(M)C(F)A(M)C(M)G(M) 3' (II-9)
5' U(M)U(F)G(M)A(M)U(M)A(F)U(M)U(M)G(F)A(M)A(M)A(F)C(M)U(R)^ C(M)C(F)A(M)C(M)G(M) 3' (II-10)
wherein for nucleic acid bases, A represents adenine, U represents uracil, T represents thymine, G represents guanine, and C represents cytosine (provided that C represents 2'-O,4'-C-ethylene-bridged-5-methylcytidine when C is ENA), and for nucleic acids, (R) represents RNA, (D) represents DNA, (M) represents 2'-OMe RNA, (3M) represents 3'-OMe RNA, (F) represents 2'-F RNA, and (E) represents ENA; and "^" means that the bond between nucleosides is a phosphorothioate bond (-P(=S) (OH)-), and the bond between nucleosides is a phosphodiester bond (-P(=O)(OH)-) if no specific indication is given, but two inter-nucleoside bonds in three nucleosides from each of the 5'-terminus and the 3'-terminus of the sense strand region, and two inter-nucleoside bonds in three nucleosides from the 5'-terminus and three inter-nucleoside bonds in four nucleosides from the 3'-terminus of the antisense strand region are phosphorothioate bonds, even if no specific indication is given.

41. The oligonucleotide or a pharmaceutically acceptable salt thereof according to claim 40, wherein an overhang structure of 3 or less bases is present at the 5'-terminus and/or the 3'-terminus of the sense strand region and/or the antisense strand region.

42. The oligonucleotide or a pharmaceutically acceptable salt thereof according to claim 41, wherein the overhang structure has 2 bases.

43. The oligonucleotide or a pharmaceutically acceptable salt thereof according to claim 42, wherein the overhang structure is nucleosides containing two consecutive thymines, or nucleosides containing two consecutive uracils.

44. The oligonucleotide or a pharmaceutically acceptable salt thereof according to claim 43, wherein the overhang structure is two consecutive U(M) added at the 3'-terminus of the antisense strand region.

45. The oligonucleotide or a pharmaceutically acceptable salt thereof according to any one of claims 40 to 44, wherein the 5'-terminus of the sense strand region is chemically modified with a GalNAc unit represented by the following formula: 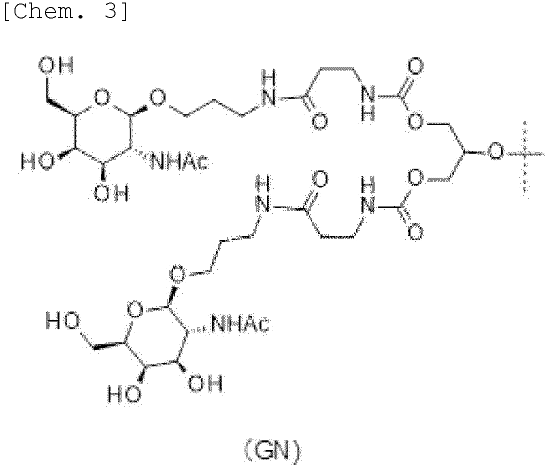 wherein the broken line represents a phosphodiester bond formed with a 5'-phosphate group of a nucleotide at the 5'-terminus of the adjacent sense strand region.

46. The oligonucleotide or a pharmaceutically acceptable salt thereof according to claim 1, wherein the sense strand region has the following formula (I-2), the antisense strand region has any of the following formulae (II-11) to (II-20), and the sense strand region and the antisense strand region form a double-stranded oligonucleotide as independent oligonucleotides:
(formulae)
5' GNC(M)^G(M)^U(M)G(M)G(F)A(M)G(F)U(F)U(F)U(M)C(M)A(M)A(M)U (M)A(M)U(M)C(M)^A(M)^A(3M) 3' (I-22)
5' U(M)^U(F)^G(M)A(M)U(M)A(F)U(M)U(F)G(F)A(M)A(M)A(D)C(M)T(E )C(M)C(F)A(M)C(M)^G(M)^U(M)^U(M) 3' (II-11)
5' U(M)^U(F)^G(M)A(F)U(M)A(F)U(M)U(M)G(F)A(M)A(M)A(D)C(M)T(E )C(M)C(F)A(M)C(M)^G(M)^U(M)^U(M) 3' (II-12)
5' U(M)^U(F)^G(M)A(M)U(M)A(F)U(M)U(M)G(F)A(F)A(M)A(D)C(M)T(E )C(M)C(F)A(M)C(M)^G(M)^U(M)^U(M) 3' (II-13)
5' U(M)^U(F)^G(M)A(M)U(M)A(F)U(M)U(M)G(F)A(M)A(M)A(D)C(M)T(E )C(M)C(F)A(M)C(F)^G(M)^U(M)^U(M) 3' (II-14)
5' U(M)^U(F)^G(M)A(M)U(M)A(F)U(M)U(F)G(F)A(M)A(M)A(M)C(E)U(R )^C(M)C(F)A(M)C(M)^G(M)^U(M)^U(M) 3' (II-15)
5' U(M)^U(F)^G(M)A(M)U(M)A(F)U(M)U(M)G(F)A(F)A(M)A(M)C(E)U(R )^C(M)C(F)A(M)C(M)^G(M)^U(M)^U(M) 3' (II-16)
5' U(M)^U(F)^G(M)A(M)U(M)A(F)U(M)U(M)G(F)A(M)A(F)A(M)C(E)U(R )^C(M)C(F)A(M)C(M)^G(M)^U(M)^U(M) 3' (II-17)
5' U(M)^U(F)^G(M)A(M)U(M)A(F)U(M)U(M)G(F)A(M)A(M)A(F)C(E)U(R )^C(M)C(F)A(M)C(M)^G(M)^U(M)^U(M) 3' (II-18)
5' U(M)^U(F)^G(M)A(M)U(M)A(F)U(M)U(M)G(F)A(M)A(F)A(M)C(M)U(R )^C(M)C(F)A(M)C(M)^G(M)^U(M)^U(M) 3' (II-19)
5' U(M)^U(F)^G(M)A(M)U(M)A(F)U(M)U(M)G(F)A(M)A(M)A(F)C(M)U(R )^C(M)C(F)A(M)C(M)^G(M)^U(M)^U(M) 3' (II-20)
wherein "GN" represents a GalNAc unit represented by the following formula: 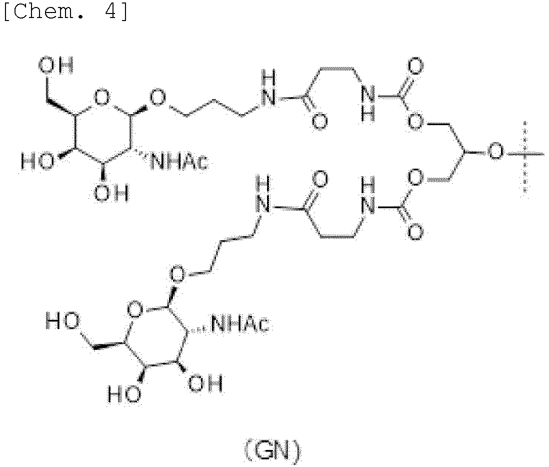 wherein the broken line represents a phosphodiester bond formed with a 5'-phosphate group of a nucleotide at the 5'-terminus of the adjacent sense strand region; for nucleobases, A represents adenine, U represents uracil, T represents thymine, G represents guanine, and C represents cytosine (provided that C represents 2'-O,4'-C-ethylene-bridged-5-methylcytidine when C is ENA), and for nucleic acids, (R) represents RNA, (D) represents DNA, (M) represents 2'-OMe RNA, (3M) represents 3'-OMe RNA, (F) represents 2'-F RNA, and (E) represents ENA; "^" means that the bond between nucleosides is a phosphorothioate bond (-P(=S) (OH)-), and the bond between nucleosides is a phosphodiester bond (-P(=O) (OH)-) if no specific indication is given; and each of residues at the 3'-position of the nucleotide at the 3'-terminus of the sense strand region, at the 5'-position of the nucleotide at the 5'-terminus of the antisense strand region, and the 3'-position of the nucleotide at the 3'-terminus of the antisense strand region is a hydroxyl group.

47. A pharmaceutical composition comprising the oligonucleotide or a pharmaceutically acceptable salt thereof according to any one of claims 1 to 46 as an active ingredient.

48. The pharmaceutical composition according to claim 47, for treating or preventing a disease that can be treated or prevented by suppressing expression of transferrin receptor 2.

49. The pharmaceutical composition according to claim 48, for preventing or treating anemia.

50. The pharmaceutical composition according to claim 49, wherein the anemia is anemia associated with chronic inflammation or anemia associated with bone-marrow dysfunction.

51. The pharmaceutical composition according to claim 50, wherein the anemia associated with chronic inflammation is anemia associated with an autoimmune disease, anemia associated with an infectious disease, anemia associated with an inflammatory enteric disease, anemia associated with heart failure, anemia associated with chronic kidney disease, cancerous anemia, or anemia associated with Castleman disease.

52. The pharmaceutical composition according to claim 50, wherein the anemia associated with bone-marrow dysfunction is anemia associated with myelofibrosis, anemia associated with myelodysplastic syndrome, anemia associated with chronic myelomonocytic leukemia, or anemia associated with chemotherapeutic myelosuppression.

53. The pharmaceutical composition according to any one of claims 47 to 52, which is intended for a patient undergoing treatment with a drug for treating anemia, a drug for treating a disease that causes anemia, or a drug for treating iron overload disorder.

54. The pharmaceutical composition according to claim 53, wherein the drug for treating anemia, the drug for treating a disease that causes anemia, or the drug for treating iron overload disorder is luspatercept or ruxolitinib.

55. A method for treating or preventing anemia, comprising administering an effective amount of the oligonucleotide or a pharmaceutically acceptable salt thereof according to any one of claims 1 to 46 to a subject.

56. The oligonucleotide or a pharmaceutically acceptable salt thereof according to any one of claims 1 to 46, for use in the treatment or prevention of anemia.

57. Use of the oligonucleotide or a pharmaceutically acceptable salt thereof according to any one of claims 1 to 46 in production of the pharmaceutical composition according to any one of claims 47 to 54.
